# EUROPEAN PATENT SPECIFICATION

(11) **EP 2 699 575 B1**
(45) Date of publication and mention of the grant of the patent: **25.03.2015**
(21) Application number: 12721779.2
(22) Date of filing: 16.04.2012
(51) Int. Cl.: A61K 45/06, C07D 471/04, A61K 31/437

(54) **TRIAZOLOPYRIDINES**
TRIAZOLOPYRIDINE
TRIAZOLOPYRIDINES

(30) Priority: 21.04.2011 EP 11163342; 27.05.2011 EP 11167872
(43) Date of publication of application: 26.02.2014
(73) Proprietor: Bayer Intellectual Property GmbH, 40789 Monheim (DE)
(72) Inventor: SCHULZE, Volker, 16562 Hohen Neuendorf, Bergfelde (DE); KOSEMUND, Dirk, 12587 Berlin (DE); WENGNER, Antje, Margret, 13189 Berlin (DE); SIEMEISTER, Gerhard, 13503 Berlin (DE); STÖCKIGT, Detlef, 14471 Potsdam (DE); LIENAU, Philip, 10967 Berlin (DE); SCHIROK, Hartmut, 40764 Langenfeld (DE); BRIEM, Hans, 10719 Berlin (DE)
(74) Representative: BIP Patents
(86) International application number: PCT/EP2012/056914
(87) International publication number: WO 2012/143329

(56) References cited:
- WO-A1-2008/025821
- WO-A1-2011/063908

## Description

The present invention relates to triazolopyridine compounds of general formula (I) as described and defined herein, to methods of preparing said compounds, to pharmaceutical compositions and combinations comprising said compounds, to the use of said compounds for manufacturing a pharmaceutical composition for the treatment or prophylaxis of a disease, as well as to intermediate compounds useful in the preparation of said compounds.

### BACKGROUND OF THE INVENTION

The present invention relates to chemical compounds that inhibit Mps-1 (Monopolar Spindle 1) kinase (also known as Tyrosine Threonine Kinase, TTK). Mps-1 is a dual specificity Ser/Thr kinase which plays a key role in the activation of the mitotic checkpoint (also known as spindle checkpoint, spindle assembly checkpoint) thereby ensuring proper chromosome segregation during mitosis [Abrieu A et al., Cell, 2001, 106, 83-93]. Every dividing cell has to ensure equal separation of the replicated chromosomes into the two daughter cells. Upon entry into mitosis, chromosomes are attached at their kinetochores to the microtubules of the spindle apparatus. The mitotic checkpoint is a surveillance mechanism that is active as long as unattached kinetochores are present and prevents mitotic cells from entering anaphase and thereby completing cell division with unattached chromosomes [Suijkerbuijk SJ and Kops GJ, Biochemica et Biophysica Acta, 2008, 1786, 24-31; Musacchio A and Salmon ED, Nat Rev Mol Cell Biol., 2007, 8, 379-93]. Once all kinetochores are attached in a correct amphitelic, *i.e.* bipolar, fashion with the mitotic spindle, the checkpoint is satisfied and the cell enters anaphase and proceeds through mitosis. The mitotic checkpoint consists of complex network of a number of essential proteins, including members of the MAD (mitotic arrest deficient, MAD 1-3) and Bub (Budding uninhibited by benzimidazole, Bub 1-3) families, the motor protein CENP-E, Mps-1 kinase as well as other components, many of these being over-expressed in proliferating cells (e.g. cancer cells) and tissues [Yuan B et al., Clinical Cancer Research, 2006, 12, 405-10]. The essential role of Mps-1 kinase activity in mitotic checkpoint signalling has been shown by shRNA-silencing, chemical genetics as well as chemical inhibitors of Mps-1 kinase [Jelluma N et al., PLos ONE, 2008, 3, e2415; Jones MH et al., Current Biology, 2005, 15, 160-65; Dorer RK et al., Current Biology, 2005, 15, 1070-76; Schmidt M et al., EMBO Reports, 2005, 6, 866-72].

There is ample evidence linking reduced but incomplete mitotic checkpoint function with aneuploidy and tumorigenesis [Weaver BA and Cleveland DW, Cancer Research, 2007, 67, 10103-5; King RW, Biochimica et Biophysica Acta, 2008, 1786, 4-14]. In contrast, complete inhibition of the mitotic checkpoint has been recognised to result in severe chromosome missegregation and induction of apoptosis in tumour cells [Kops GJ et al., Nature Reviews Cancer, 2005, 5, 773-85; Schmidt M and Medema RH, Cell Cycle, 2006, 5, 159-63; Schmidt M and Bastians H, Drug Resistance Updates, 2007, 10, 162-81]. Therefore, mitotic checkpoint abrogation through pharmacological inhibition of Mps-1 kinase or other components of the mitotic checkpoint represents a new approach for the treatment of proliferative disorders including solid tumours such as carcinomas and sarcomas and leukaemias and lymphoid malignancies or other disorders associated with uncontrolled cellular proliferation.

Different compounds have been disclosed in prior art which show an inhibitory effect on Mps-1 kinase:
WO 2009/024824 A1 discloses 2-Anilinopurin-8-ones as inhibitors of Mps-1 for the treatment of proliferate disorders. WO 2010/124826 A1 discloses substituted imidazoquinoxaline compounds as inhibitors of Mps-1 kinase or
TTK. WO 2011/026579 A1 discloses substituted aminoquinoxalines as Mps-1 inhibitors.

Substituted triazopyridine compounds have been disclosed for the treatment or prophylaxis of different diseases:
WO 2008/025821 A1 (Cellzome (UK) Ltd) relates to triazole derivatives as kinase inhibitors, especially inhibitors of ITK or PI3K, for the treatment or prophylaxis of immunological, inflammatory or allergic disorders. Said triazole derivatives are exemplified as possessing an amide, urea, carbamate or aliphatic amine substituent in position 2.

WO 2009/010530 A1 discloses bicyclic heterorayl compounds and their use as phosphatidylinositol (PI) 3-kinase. Among other compounds also substituted triazolopyridines are mentioned.

WO 2009/027283 A1 discloses triazolopyridine compounds and their use as ASK (apoptosis signal-regulating kinase) inhibitors for the treatment of autoimmune diseases and neurodegenerative diseases.

WO 2009/047514 A1 (Cancer Research Technology Limited) relates to [1,2,4]-triazolo-[1,5-a]-pyridine and [1,2,4]-triazolo-[1,5-c]-pyrimidine compounds which inhibit AXL receptor tyrosine kinase function, and to the treatment of diseases and conditions that are mediated by AXL receptor tyrosine kinase, that are ameliorated by the inhibition of AXL receptor tyrosine kinase function etc., including proliferative conditions such as cancer, etc. Said compounds are exemplified as possessing a substituent in the 5-position of said compounds and a substituent in the 2-position.

WO 2010/092041 A1 (Fovea Pharmaceuticals SA) relates to [1,2,4]-triazolo- [1,5-a]-pyridines, which are useful as selective kinase inhibitors, to methods for producing such compounds and methods for treating or ameliorating kinase- mediated disorder.

However, the state of the art described above does not describe the triazolopyridine compounds of general formula (I) of the present invention, or a stereoisomer, a tautomer, an N-oxide, a hydrate, a solvate, or a salt thereof, or a mixture of same, as described herein and defined in the claims, and as referred to in this text as "compounds of the present invention", or their pharmacological activity.

In particular, said compounds of the present invention have surprisingly been found to effectively inhibit Mps-1 kinase and may therefore be used for the treatment or prophylaxis of diseases of uncontrolled cell growth, proliferation and/or survival, inappropriate cellular immune responses, or inappropriate cellular inflammatory responses or diseases which are accompanied with uncontrolled cell growth, proliferation and/or survival, inappropriate cellular immune responses, or inappropriate cellular inflammatory responses, particularly in which the uncontrolled cell growth, proliferation a nd/or survival, inappropriate cellular immune responses, or inappropriate cellular inflammatory responses is mediated by Mps-1 kinase, such as, for example, haemotological tumours, solid tumours, and/or metastases thereof, e.g. leukaemias and myelodysplastic syndrome, malignant lymphomas, head and neck tumours including brain tumours and brain metastases, tumours of the thorax including non-small cell and small cell lung tumours, gastrointestinal tumours, endocrine tumours, mammary and other gynaecological tumours, urological tumours including renal, bladder and prostate tumours, skin tumours, and sarcomas, and/or metastases thereof.

WO2011/063908 is also related to triazolopyridine compounds as Mps-1 inhibitors. The effectiveness in inhibiting Mps-1 kinase was measured in an Mps-1 kinase assay with a concentration of 10 µM adenosine triphosphate (ATP).

The cellular concentration of ATP in mammals is in the millimolar range. Therefore it is important that a drug substance is also effective in inhibiting Mps-1 kinase in a kinase assay with a concentration of ATP in the millimolar range, e.g. 2 mM ATP, in order to potentially achieve an antiproliferative effect in a cellular assay.

For oral dosing it is essential, that a drug substance is hydrolytically stable in acidic medium, e.g. at pH 2, to avoid hydrolysis of the drug compound before absorption.

The half maximal inhibitory concentration (IC₅₀) of the most potent compounds specified in WO2011/063908, determined in an Mps-1 kinase assay with a concentration of 10 µM ATP, was lower than 2 nM (more potent than 2 nM). However, all these compounds show either an IC₅₀ higher than 30 nM (less potent than 30 nM) in an Mps-1 kinase assay with a concentration of 2 mM ATP, or they show a low hydrolytic stability at pH 2 with more than 15 % decay after 24 h.

Surprisingly it was found, that the compounds of the present invention are characterized by
- an IC₅₀ lower than 2 nM (more potent than 2 nM) in an Mps-1 kinase assay with a concentration of 10 µM ATP, and
- an IC₅₀ lower than 30 nM (more potent than 30 nM) in an Mps-1 kinase assay with a concentration of 2 mM ATP, and
- a high hydrolytic stability, with less than 10 % decay after 24 h at pH 2.

Hence, the compounds of the present invention have surprising and advantageous properties. These unexpected findings give rise to the present selection invention. The compounds of the present invention are purposively selected from the general formula of WO2011/063908 due to their superior inhibitory and stability properties.

### SUMMARY of the INVENTION

The present invention provides novel compounds of general formula (I): 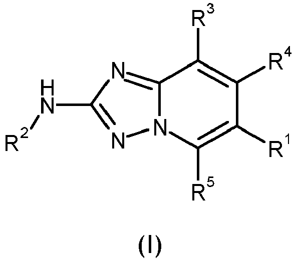 in which:
- R¹: represents a phenyl or pyridyl group
- which is substituted, one or more times, identically or differently, with a substituent selected from:
   R⁶-(C₁-C₆-alkoxy)-, R⁶-O-, -C(=O)R⁶, -C(=O)O-R⁶, -N(H)C(=O)R⁶, -N(H)C(=O)NR⁶R⁷, -NR⁶R⁷, -C(=O)N(H)R⁶, -C(=O)NR⁶R⁷, R⁶-S-, R⁶-S(=O)₂-, -N(H)S(=O)₂R⁶, -S(=O)₂N(H)R⁶; and
- which is optionally substituted, one or more times, identically or differently, with a substituent selected from: halo-, hydroxy-, nitro-, C₁-C₆-alkyl-, C₁-C₆-alkoxy-, hydroxy-C₁-C₆-alkyl-, - N(H)C(=O)R⁸, -N(H)C(=O)NR⁸R⁷, -C(=O)N(H)R⁸, -N(H)S(=O)₂R⁸;
- R²: represents a 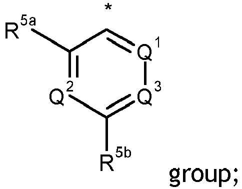 wherein * indicates the point of attachment of said group with the rest of the molecule;
Q¹ represents a group selected from: N, CH, C-(C₁-C₆-alkyl), C-(C₁-C₆-alkoxy), C-halo;
Q² represents a group selected from: N, CH, CR^{5b};
Q³ represents a group selected from: N, CH, CR^{5b};
R^{5a} represents a group selected from:
halo-, nitro-, C₁-C₆-alkyl-, halo-C₁-C₆-alkyl-, C₁-C₆-alkoxy-, halo-C₁-C₆-alkoxy-, hydroxy-C₁-C₆-alkyl-, C₁-C₆-alkoxy-C₁-C₆-alkyl-, halo-C₁-C₆-alkoxy-C₁-C₆-alkyl-, R⁸-(C₁-C₆-alkoxy)-, R⁸-O-, -NR⁸R⁷, R⁸-S-, R⁸-S(=O)-, R⁸-S(=O)₂-, (C₃-C₆-cycloalkyl)-(CH₂)ₙ-O-;
R^{5b} represents a group selected from:
halo-, hydroxy-, cyano-, nitro-, C₁-C₆-alkyl-, halo-C₁-C₆-alkyl-, C₁-C₆-alkoxy-, halo-C₁-C₆-alkoxy-, hydroxy-C₁-C₆-alkyl-, C₁-C₆-alkoxy-C₁-C₆-alkyl-, halo-C₁-C₆-alkoxy-C₁-C₆-alkyl-, R⁸-(C₁-C₆-alkyl)-, R⁸-(CH₂)ₙ(CHOH)(CH₂)ₘ-, R⁸-(C₁-C₆-alkoxy)-, R⁸-(CH₂)ₙ(CHOH)(CH₂)ₚ-O-, R⁸-(C₁-C₆-alkoxy-C₁-C₆-alkyl)-, R⁸-(C₁-C₆-alkoxy-C₁-C₆-alkyl)-O-, -O-(CH₂)ₙ-C(=O)NR⁸R⁷, R⁸-O-, -C(=O)R⁸, -C(=O)O-R⁸, -OC(=O)-R⁸, -N(H)C(=O)R⁸, -N(R⁷)C(=O)R⁸, -N(H)C(=O)NR⁸R⁷, -N(R⁷)C(=O)NR⁸R⁷, -NR⁸R⁷, -NR⁷, -C(=O)N(H)R⁸, -C(=O)NR⁸R⁷, R⁸-S-, R⁸-S(=O)-, R⁸-S(=O)₂-, -N(H)S(=O)R⁸, -N(R⁷)S(=O)R⁸, -S(=O)N(H)R⁸, -S(=O)NR⁸R⁷, -N(H)S(=O)₂R⁸, -N(R⁷)S(=O)₂R⁸, -S(=O)₂N(H)R⁸, -S(=O)₂NR⁸R⁷, -S(=O)(=NR⁸)R⁷, -S(=O)(=NR⁷)R⁸, -N=S(=O)(R⁸)R⁷;
- R³: represents a hydrogen atom, a halogen atom, a hydroxy-, amino-, cyano-, nitro-, C₁-C₄-alkyl-, halo-C₁-C₄-alkyl-, C₁-C₄-alkoxy-, halo-C₁-C₄-alkoxy-, hydroxy-C₁-C₄-alkyl-, C₁-C₄-alkoxy-C₁-C₄-alkyl-, halo-C₁-C₄-alkoxy-C₁-C₄-alkyl-, C₂-C₆-alkenyl-, C₂-C₆-alkynyl-, halo-C₂-C₆-alkenyl-, halo-C₂-C₆-alkynyl-, C₃-C₆-cycloalkyl-, or halo-C₃-C₆-cycloalkyl- group;
- R⁴: represents a hydrogen atom, a halogen atom, a hydroxy-, amino-, cyano-, nitro-, C₁-C₄-alkyl-, halo-C₁-C₄-alkyl-, C₁-C₄-alkoxy-, halo-C₁-C₄-alkoxy-, hydroxy-C₁-C₄-alkyl-, C₁-C₄-alkoxy-C₁-C₄-alkyl-, halo-C₁-C₄-alkoxy-C₁-C₄-alkyl-, C₂-C₆-alkenyl-, C₂-C₆-alkynyl-, halo-C₂-C₆-alkenyl-, halo-C₂-C₆-alkynyl-, C₃-C₆-cycloalkyl-, or halo-C₃-C₆-cycloalkyl- group;
- R⁵: represents a hydrogen atom;
- R⁶: represents a group selected from C₃-C₆-cycloalkyl-, 3- to 10-membered heterocyclyl-, aryl-, heteroaryl-, -(CH₂)_{q}-(C₃-C₆-cycloalkyl), -(CH₂)_{q}-(3- to 10-membered heterocyclyl), -(CH₂)_{q}-aryl, or -(CH₂)_{q}-heteroaryl,
wherein said group being optionally substituted, one or more times, identically or differently, with a substituent selected from:
halo-, hydroxy-, cyano-, nitro-, C₁-C₆-alkyl-, halo-C₁-C₆-alkyl-, C₁-C₆-alkoxy-, halo-C₁-C₆-alkoxy-, hydroxy-C₁-C₆-alkyl-, C₁-C₆-alkoxy-C₁-C₆-alkyl-, halo-C₁-C₆-alkoxy-C₁-C₆-alkyl-, R⁸-(C₁-C₆-alkyl)-, R⁸-(CH₂)ₙ(CHOH)(CH₂)m-, R⁸-(C₁-C₆-alkoxy)-, R⁸-(CH₂)ₙ(CHOH)(CH₂)ₚ-O-, R⁸-(C₁-C₆-alkoxy-C₁-C₆-alkyl)-, R⁸-(C₁-C₆-alkoxy-C₁-C₆-alkyl)-O-, aryl-, R⁸-O-, -C(=O)R⁸, -C(=O)O-R⁸, -OC(=O)-R⁸, -N(H)C(=O)R⁸, -N(R⁷)C(=O)R⁸, -N(H)C(=O)NR⁸R⁷, -N(R⁷)C(=O)NR⁸R⁷, -NR⁸R⁷, -C(=O)N(H)R⁸, -C(=O)NR⁸R⁷, R⁸-S-, R⁸-S(=O)-, R⁸-S(=O)₂-, -N(H)S(=O)R⁸, -N(R⁷)S(=O)R⁸, -S(=O)N(H)R⁸, -S(=O)NR⁸R⁷, -N(H)S(=O)₂R⁸, -N(R⁷)S(=O)₂R⁸, -S(=O)₂N(H)R⁸, -S(=O)₂NR⁸R⁷, -S(=O)(=NR⁸)R⁷,- S(=O)(=NR⁷)R⁸, -N=S(=O)(R⁸)R⁷;
- R⁷: represents a hydrogen atom, a C₁-C₆-alkyl- or a C₃-C₆-cycloalkyl- group;
- R⁸: represents a hydrogen atom or a C₁-C₆-alkyl- or C₃-C₆-cycloalkyl- group, wherein said C₁-C₆-alkyl- or C₃-C₆-cycloalkyl- group is optionally substituted, one or more times, identically or differently, with a substituent selected from:
halo-, hydroxy-, -NHR⁷, -NR⁷R⁷, -N(C₁-C₃-alkyl)-C(=O)R⁷, -N(C₁-C₃-alkyl)-C(=O)OR⁷, C₁-C₃-alkyl-, R⁷-S(=O)₂-, C₁-C₃-alkoxy-, halo-C₁-C₃-alkoxy-;
- n, m, p,: represent, independently from each other, an integer of 0, 1, 2 or 3;
and
- q: represents an integer of 0, 1, 2 or 3;
or a stereoisomer, a tautomer, an N-oxide, a hydrate, a solvate, or a salt thereof, or a mixture of same.

The triazolopyridine compounds of general formula (I) effectively inhibit Mps-1 kinase - even at high ATP concentrations - and show a high hydrolytic stability. The present invention relates to the triazolopyridine compounds of general formula (I) as described and defined herein, to methods of preparing said compounds, to pharmaceutical compositions and combinations comprising said compounds, to the use of said compounds for manufacturing a pharmaceutical composition for the treatment or prophylaxis of a disease, as well as to intermediate compounds useful in the preparation of said compounds. Preferred embodiments of the present invention are specified hereinafter as well as in the dependent claims.

### DETAILED DESCRIPTION of the INVENTION

The terms as mentioned in the present text have preferably the following meanings :

The term "halogen atom" or "halo-" is to be understood as meaning a fluorine, chlorine, bromine or iodine atom.

The term "C₁-C₆-alkyl" is to be understood as preferably meaning a linear or branched, saturated, monovalent hydrocarbon group having 1, 2, 3, 4, 5, or 6 carbon atoms, e.g. a methyl, ethyl, propyl, butyl, pentyl, hexyl, iso-propyl, iso-butyl, sec-butyl, tert-butyl, iso-pentyl, 2-methylbutyl, 1-methylbutyl, 1-ethylpropyl, 1,2-dimethylpropyl, neo-pentyl, 1,1-dimethylpropyl, 4-methylpentyl, 3-methylpentyl, 2-methylpentyl, 1-methylpentyl, 2-ethylbutyl, 1-ethylbutyl, 3,3-dimethylbutyl, 2,2-dimethylbutyl, 1,1-dimethylbutyl, 2,3-dimethylbutyl, 1,3-dimethylbutyl, or 1,2-dimethylbutyl group, or an isomer thereof. Particularly, said group has 1, 2, 3 or 4 carbon atoms ("C₁-C₄-alkyl"), *e.g.* a methyl, ethyl, propyl, butyl, iso-propyl, iso-butyl, sec-butyl, tert-butyl group, more particularly 1, 2 or 3 carbon atoms ("C₁-C₃-alkyl"), e.g. a methyl, ethyl, n-propyl- or iso-propyl group.

The term "halo-C₁-C₆-alkyl" is to be understood as preferably meaning a linear or branched, saturated, monovalent hydrocarbon group in which the term "C₁-C₆-alkyl" is defined *supra*, and in which one or more hydrogen atoms is replaced by a halogen atom, in identically or differently, *i.e.* one halogen atom being independent from another. Particularly, said halogen atom is F. Said halo-C₁-C₆-alkyl group is, for example, -CF₃, -CHF₂, -CH₂F, -CF₂CF₃, or - CH₂CF₃.

The term "C₁-C₆-alkoxy" is to be understood as preferably meaning a linear or branched, saturated, monovalent, hydrocarbon group of formula -O-(C₁-C₆-alkyl), in which the term "C₁-C₆-alkyl" is defined *supra*, *e.g.* a methoxy, ethoxy, n-propoxy, iso-propoxy, n-butoxy, iso-butoxy, tert-butoxy, sec-butoxy, pentoxy, iso-pentoxy, or n-hexoxy group, or an isomer thereof.

The term "halo-C₁-C₆-alkoxy" is to be understood as preferably meaning a linear or branched, saturated, monovalent C₁-C₆-alkoxy group, as defined *supra,* in which one or more of the hydrogen atoms is replaced, in identically or differently, by a halogen atom. Particularly, said halogen atom is F. Said halo-C₁-C₆-alkoxy group is, for example, -OCF₃, -OCHF₂, -OCH₂F, -OCF₂CF₃, or - OCH₂CF₃.

The term "C₁-C₆-alkoxy-C₁-C₆-alkyl" is to be understood as preferably meaning a C₁-C₆-alkyl group, as defined *supra,* in which one or more of the hydrogen atoms is replaced, in identically or differently, by a C₁-C₆-alkoxy group, as defined *supra*, *e.g.* methoxyalkyl, ethoxyalkyl, propyloxyalkyl, iso-propoxyalkyl, butoxyalkyl, iso-butoxyalkyl, tert-butoxyalkyl, sec-butoxyalkyl, pentyloxyalkyl, iso-pentyloxyalkyl, hexyloxyalkyl group, or an isomer thereof.

The term "halo-C₁-C₆-alkoxy-C₁-C₆-alkyl" is to be understood as preferably meaning a linear or branched, saturated, monovalent C₁-C₆-alkoxy-C₁-C₆-alkyl group, as defined *supra,* in which one or more of the hydrogen atoms is replaced, in identically or differently, by a halogen atom. Particularly, said halogen atom is F. Said halo-C₁-C₆-alkoxy-C₁-C₆-alkyl group is, for example, - CH₂CH₂OCF₃, -CH₂CH₂OCHF₂, -CH₂CH₂OCH₂F, -CH₂CH₂OCF₂CF₃, or - CH₂CH₂OCH₂CF_{3.}

The term "C₂-C₆-alkenyl" is to be understood as preferably meaning a linear or branched, monovalent hydrocarbon group, which contains one or more double bonds, and which has 2, 3, 4, 5 or 6 carbon atoms, particularly 2 or 3 carbon atoms ("C₂-C₃-alkenyl"), it being understood that in the case in which said alkenyl group contains more than one double bond, then said double bonds may be isolated from, or conjugated with, each other. Said alkenyl group is, for example, a vinyl, allyl, (E)-2-methylvinyl, (Z)-2-methylvinyl, homoallyl, (E)-but-2-enyl, (Z)-but-2-enyl, (E)-but-1-enyl, (Z)-but-1-enyl, pent-4-enyl, (E)-pent-3-enyl, (Z)-pent-3-enyl, (E)-pent-2-enyl, (Z)-pent-2-enyl, (E)-pent-1-enyl, (Z)-pent-1-enyl, hex-5-enyl, (E)-hex-4-enyl, (Z)-hex-4-enyl, (E)-hex-3-enyl, (Z)-hex-3-enyl, (E)-hex-2-enyl, (Z)-hex-2-enyl, (E)-hex-1-enyl, (Z)-hex-1-enyl, isopropenyl, 2-methylprop-2-enyl, 1-methylprop-2-enyl, 2-methylprop-1-enyl, (E)-1-methylprop-1-enyl, (Z)-1-methylprop-1-enyl, 3-methylbut-3-enyl, 2-methylbut-3-enyl, 1-methylbut-3-enyl, 3-methylbut-2-enyl, (E)-2-methylbut-2-enyl, (Z)-2-methylbut-2-enyl, (E)-1-methylbut-2-enyl, (Z)-1-methylbut-2-enyl, (E)-3-methylbut-1-enyl, (Z)-3-methylbut-1-enyl, (E)-2-methylbut-1-enyl, (Z)-2-methylbut-1-enyl, (E)-1-methylbut-1-enyl, (Z)-1-methylbut-1-enyl, 1,1-dimethylprop-2-enyl, 1-ethylprop-1-enyl, 1-propylvinyl, 1-isopropylvinyl, 4-methylpent-4-enyl, 3-methylpent-4-enyl, 2-methylpent-4-enyl, 1-methylpent-4-enyl, 4-methylpent-3-enyl, (E)-3-methylpent-3-enyl, (Z)-3-methylpent-3-enyl, (E)-2-methylpent-3-enyl, (Z)-2-methylpent-3-enyl, (E)-1-methylpent-3-enyl, (Z)-1-methylpent-3-enyl, (E)-4-methylpent-2-enyl, (Z)-4-methylpent-2-enyl, (E)-3-methylpent-2-enyl, (Z)-3-methylpent-2-enyl, (E)-2-methylpent-2-enyl, (Z)-2-methylpent-2-enyl, (E)-1-methylpent-2-enyl, (Z)-1-methylpent-2-enyl, (E)-4-methylpent-1-enyl, (Z)-4-methylpent-1-enyl, (E)-3-methylpent-1-enyl, (Z)-3-methylpent-1-enyl, (E)-2-methylpent-1-enyl, (Z)-2-methylpent-1-enyl, (E)-1-methylpent-1-enyl, (Z)-1-methylpent-1-enyl, 3-ethylbut-3-enyl, 2-ethylbut-3-enyl, 1-ethylbut-3-enyl, (E)-3-ethylbut-2-enyl, (Z)-3-ethylbut-2-enyl, (E)-2-ethylbut-2-enyl, (Z)-2-ethylbut-2-enyl, (E)-1-ethylbut-2-enyl, (Z)-1-ethylbut-2-enyl, (E)-3-ethylbut-1-enyl, (Z)-3-ethylbut-1-enyl, 2-ethylbut-1-enyl, (E)-1-ethylbut-1-enyl, (Z)-1-ethylbut-1-enyl, 2-propylprop-2-enyl, 1-propylprop-2-enyl, 2-isopropylprop-2-enyl, 1-isopropylprop-2-enyl, (E)-2-propylprop-1-enyl, (Z)-2-propylprop-1-enyl, (E)-1-propylprop-1-enyl, (Z)-1-propylprop-1-enyl, (E)-2-isopropylprop-1-enyl, (Z)-2-isopropylprop-1-enyl, (E)-1-isopropylprop-1-enyl, (Z)-1-isopropylprop-1-enyl, (E)-3,3-dimethylprop-1-enyl, (Z)-3,3-dimethylprop-1-enyl, 1-(1,1-dimethylethyl)ethenyl, buta-1,3-dienyl, penta-1,4-dienyl, hexa-1,5-dienyl, or methylhexadienyl group. Particularly, said group is vinyl or allyl.

The term "C₂-C₆-alkynyl" is to be understood as preferably meaning a linear or branched, monovalent hydrocarbon group which contains one or more triple bonds, and which contains 2, 3, 4, 5 or 6 carbon atoms, particularly 2 or 3 carbon atoms ("C₂-C₃-alkynyl"). Said C₂-C₆-alkynyl group is, for example, ethynyl, prop-1-ynyl, prop-2-ynyl, but-1-ynyl, but-2-ynyl, but-3-ynyl, pent-1-ynyl, pent-2-ynyl, pent-3-ynyl, pent-4-ynyl, hex-1-ynyl, hex-2-inyl, hex-3-inyl, hex-4-ynyl, hex-5-ynyl, 1-methylprop-2-ynyl, 2-methylbut-3-ynyl, 1-methylbut-3-ynyl, 1-methylbut-2-ynyl, 3-methylbut-1-ynyl, 1-ethylprop-2-ynyl, 3-methylpent-4-ynyl, 2-methylpent-4-ynyl, 1-methylpent-4-ynyl, 2-methylpent-3-ynyl, 1-methylpent-3-ynyl, 4-methylpent-2-ynyl, 1-methylpent-2-ynyl, 4-methylpent-1-ynyl, 3-methylpent-1-ynyl, 2-ethylbut-3-ynyl, 1-ethylbut-3-ynyl, 1-ethylbut-2-ynyl, 1-propylprop-2-ynyl, 1-isopropylprop-2-ynyl, 2,2-dimethylbut-3-inyl, 1,1-dimethylbut-3-ynyl, 1,1-dimethylbut-2-ynyl, or 3,3-dimethylbut-1-ynyl group. Particularly, said alkynyl group is ethynyl, prop-1-ynyl, or prop-2-inyl.

The term "C₃-C₆-cycloalkyl" is to be understood as preferably meaning a saturated, monovalent, mono-, or bicyclic hydrocarbon ring which contains 3, 4, 5 or 6 carbon atoms ("C₃-C₆-cycloalkyl"). Said C₃-C₆-cycloalkyl group is for example, a monocyclic hydrocarbon ring, e.g. a cyclopropyl, cyclobutyl, cyclopentyl, or cyclohexyl or a bicyclic hydrocarbon ring. Said cycloalkyl ring can optionally contain one or more double bonds e.g. cycloalkenyl, such as a cyclopropenyl, cyclobutenyl, cyclopentenyl or cyclohexenyl group, wherein the bond between said ring with the rest of the molecule may be to any carbon atom of said ring, be it saturated or unsaturated.

The term "heterocyclic ring", as used in the term "4-, 5-, 6-, 7-, 8-, 9- or 10-membered heterocyclic ring", or "4- to 6-membered heterocyclic ring" or "5-to 6-membered heterocyclic ring", for example, as used in the definition of compounds of general formula (I) as defined herein, is to be understood as meaning a saturated or partially unsaturated, mono-, bi- or poly-cyclic nitrogen atom-containing ring, said nitrogen atom being the point of attachment of said heterocyclic ring with the rest of the molecule. Said nitrogen atom-containing ring optionally further contains 1 or 2 heteroatom-containing groups selected from O, C(=O), S, S(=O), S(=O)₂, NR' in which R' represents C₁-C₆-alkyl-, C₃-C₆-cycloalkyl-, -C(=O)-(C₁-C₆-alkyl) or -C(=O)-(C₁-C₆-cycloalkyl). Particularly, without being limited thereto, said nitrogen atom-containing ring can be a 4-membered ring, such as an azetidinyl ring, for example, or a 5-membered ring, such as a pyrrolidinyl ring, for example, or a 6-membered ring, such as a piperidinyl, piperazinyl, morpholinyl, or thiomorpholinyl ring, for example, or a 7-membered ring, such as a diazepanyl ring ring, for example, or an 8-, 9-, or 10-membered ring, such as a cycloheptylaminyl, cyclooctylaminyl, or cyclononylaminyl ring, respectively, for example ; it being reiterated that any of the above-mentioned nitrogen atom-containing rings can further contain 1 or 2 heteroatom-containing groups selected from O, C(=O), S, S(=O), S(=O)₂, NR' in which R' is as defined *supra.*

As mentioned *supra,* said nitrogen atom-containing ring can be bicyclic, such as, without being limited thereto, a 5,5-membered ring, e.g. a hexahydrocyclopenta[c]pyrrol-2(1H)-yl) ring, or a 5,6-membered bicyclic ring, *e.g.* a hexahydropyrrolo[1,2-a]pyrazin-2(1H)-yl ring, or for example. As mentioned *supra,* said nitrogen atom-containing ring can be partially unsaturated, *i.e.* it can contain one or more double bonds, such as, without being limited thereto, a 2,5-dihydro-1H-pyrrolyl, 4H-[1,3,4]thiadiazinyl, 4,5-dihydrooxazolyl, or 4H-[1,4]thiazinyl ring, for example, or, it may be benzo-fused, such as, without being limited thereto, a dihydroisoquinolinyl ring, for example.

The term "3- to 10-membered heterocycloalkyl" is to be understood as preferably meaning a saturated or partially unsaturated, monovalent, mono- or bicyclic hydrocarbon ring which contains 2, 3, 4, 5, 6, 7, 8, or 9 carbon atoms, and one or more heteroatom-containing groups selected from C(=O), O, S, S(=O), S(=O)₂, NH, NR', wherein R' represents a C₁-C₆-alkyl-, C₃-C₆-cycloalkyl-, - C(=O)-(C₁-C₆-alkyl) or -C(=O)-(C₁-C₆-cycloalkyl). Particularly, said ring can contain 2, 3, 4, or 5 carbon atoms, and one or more of the above-mentioned heteroatom-containing groups (a "3- to 6-membered heterocycloalkyl"), more particularly said ring can contain 4 or 5 carbon atoms, and one or more of the above-mentioned heteroatom-containing groups (a "5- to 6-membered heterocycloalkyl"). Said heterocycloalkyl ring is for example, a monocyclic heterocycloalkyl ring such as an oxyranyl, oxetanyl, aziridinyl, azetidinyl, tetrahydrofuranyl, pyrrolidinyl, imidazolidinyl, pyrazolidinyl, pyrrolinyl, tetrahydropyranyl, piperidinyl, morpholinyl, dithianyl, thiomorpholinyl, piperazinyl, trithianyl, or chinuclidinyl group. Optionally, said heterocycloalkyl ring can contain one or more double bonds, e.g. 4H-pyranyl, 2H-pyranyl, 3H-diazirinyl, 2,5-dihydro-1H-pyrrolyl, [1,3]dioxolyl, 4H-[1,3,4]thiadiazinyl, 2,5-dihydrofuranyl, 2,3-dihydrofuranyl, 2,5-dihydrothiophenyl, 2,3-dihydrothiophenyl, 4,5-dihydrooxazolyl, or 4H-[1,4]thiazinyl group, or, it may be benzo fused.

The term "aryl" is to be understood as preferably meaning a monovalent, aromatic or partially aromatic, mono-, or bi- or tricyclic hydrocarbon ring having 6, 7, 8, 9, 10, 11, 12, 13 or 14 carbon atoms (a "C₆-C₁₄-aryl" group), particularly a ring having 6 carbon atoms (a "C₆-aryl" group), e.g. a phenyl group, or a biphenyl group, or a ring having 9 carbon atoms (a "C₉-aryl" group), *e.g.* an indanyl or indenyl group, or a ring having 10 carbon atoms (a "C₁₀-aryl" group), e.g. a tetralinyl, dihydronaphthyl, or naphthyl group, or a ring having 13 carbon atoms, (a "C₁₃-aryl" group), e.g. a fluorenyl group, or a ring having 14 carbon atoms, (a "C₁₄-aryl" group), e.g. an anthranyl group.

The term "heteroaryl" is understood as preferably meaning a monovalent, aromatic, mono- or bicyclic aromatic ring system having 5, 6, 7, 8, 9, 10, 11, 12, 13 or 14 ring atoms (a "5- to 14-membered heteroaryl" group), particularly 5 or 6 or 9 or 10 atoms, and which contains at least one heteroatom which may be identical or different, said heteroatom being such as oxygen, nitrogen or sulfur, and can be monocyclic, bicyclic, or tricyclic, and in addition in each case can be benzocondensed. Particularly, heteroaryl is selected from thienyl, furanyl, pyrrolyl, oxazolyl, thiazolyl, imidazolyl, pyrazolyl, isoxazolyl, isothiazolyl, oxadiazolyl, triazolyl, thiadiazolyl, thia-4H-pyrazolyl *etc.*, and benzo derivatives thereof, such as, for example, benzofuranyl, benzothienyl, benzoxazolyl, benzisoxazolyl, benzimidazolyl, benzotriazolyl, indazolyl, indolyl, isoindolyl, *etc.*; or pyridyl, pyridazinyl, pyrimidinyl, pyrazinyl, triazinyl, *etc.*, and benzo derivatives thereof, such as, for example, quinolinyl, quinazolinyl, isoquinolinyl, *etc.*; or azocinyl, indolizinyl, purinyl, *etc.*, and benzo derivatives thereof; or cinnolinyl, phthalazinyl, quinazolinyl, quinoxalinyl, naphthpyridinyl, pteridinyl, carbazolyl, acridinyl, phenazinyl, phenothiazinyl, phenoxazinyl, xanthenyl, or oxepinyl, *etc.* More particularly, heteroaryl is selected from pyridyl, benzofuranyl, benzisoxazolyl, indazolyl, quinazolinyl, thienyl, quinolinyl, benzothienyl, pyrazolyl, or furanyl.

The term "alkylene" is understood as preferably meaning an optionally substituted hydrocarbon chain (or "tether") having 1, 2, 3, 4, 5, or 6 carbon atoms, *i.e.* an optionally substituted -CH₂- ("methylene" or "single membered tether" or, for example -C(Me)₂-), -CH₂-CH₂- ("ethylene", "dimethylene", or "two-membered tether"), -CH₂-CH₂-CH₂- ("propylene", "trimethylene", or "three-membered tether"), -CH₂-CH₂-CH₂-CH₂- ("butylene", "tetramethylene", or "four-membered tether"), -CH₂-CH₂-CH₂-CH₂-CH₂- ("pentylene", "pentamethylene" or "five-membered ether"), or -CH₂-CH₂-CH₂-CH₂-CH₂-CH₂-("hexylene", "hexamethylene", or six-membered tether") group. Particularly, said alkylene tether has 1, 2, 3, 4, or 5 carbon atoms, more particularly 1 or 2 carbon atoms.

The term "C₁-C₆", as used throughout this text, e.g. in the context of the definition of "C₁-C₆-alkyl", "C₁-C₆-haloalkyl", "C₁-C₆-alkoxy", or "C₁-C₆-haloalkoxy" is to be understood as meaning an alkyl group having a finite number of carbon atoms of 1 to 6, *i.e.* 1, 2, 3, 4, 5, or 6 carbon atoms. It is to be understood further that said term "C₁-C₆" is to be interpreted as any sub-range comprised therein, e.g. C₁-C₆, C₂-C₅, C₃-C₄, C₁-C₂, C₁-C₃, C₁-C₄, C₁-C₅, C₁-C_{6;} particularly C₁-C₂, C₁-C₃, C₁-C₄, C₁-C₅, C₁-C_{6;} more particularly C₁-C₄; in the case of "C₁-C₆-haloalkyl" or "C₁-C₆-haloalkoxy" even more particularly C₁-C₂.

Similarly, as used herein, the term "C₂-C₆", as used throughout this text, e.g. in the context of the definitions of "C₂-C₆-alkenyl" and "C₂-C₆-alkynyl", is to be understood as meaning an alkenyl group or an alkynyl group having a finite number of carbon atoms of 2 to 6, *i.e.* 2, 3, 4, 5, or 6 carbon atoms. It is to be understood further that said term "C₂-C₆" is to be interpreted as any sub-range comprised therein, e.g. C₂-C₆, C₃-C₅, C₃-C₄, C₂-C₃, C₂-C₄, C₂-C₅; particularly C₂-C₃.

Further, as used herein, the term "C₃-C₆", as used throughout this text, e.g. in the context of the definition of "C₃-C₆-cycloalkyl", is to be understood as meaning a cycloalkyl group having a finite number of carbon atoms of 3 to 6, *i.e.* 3, 4, 5 or 6 carbon atoms. It is to be understood further that said term "C₃-C₆" is to be interpreted as any sub-range comprised therein, e.g. C₃-C₆ , C₄-C₅, C₃-C₅, C₃-C₄, C₄-C₆, C₅-C₆; particularly C₃-C₆.

As used herein, the term "leaving group" refers to an atom or a group of atoms that is displaced in a chemical reaction as stable species taking with it the bonding electrons. Preferably, a leaving group is selected from the group comprising: halo, in particular chloro, bromo or iodo, methanesulfonyloxy, p-toluenesulfonyloxy, trifluoromethanesulfonyloxy, nonafluorobutanesulfonyloxy, (4-bromo-benzene)sulfonyloxy, (4-nitro-benzene)sulfonyloxy, (2-nitro-benzene)-sulfonyloxy, (4-isopropyl-benzene)sulfonyloxy, (2,4,6-tri-isopropyl-benzene)-sulfonyloxy, (2,4,6-trimethyl-benzene)sulfonyloxy, (4-tertbutyl-benzene)sulfonyloxy, benzenesulfonyloxy, and (4-methoxy-benzene)sulfonyloxy.

As used herein, the term "one or more times", e.g. in the definition of the substituents of the compounds of the general formulae of the present invention, is understood as meaning "one, two, three, four or five times, particularly one, two, three or four times, more particularly one, two or three times, even more particularly one or two times".

Where the plural form of the word compounds, salts, polymorphs, hydrates, solvates and the like, is used herein, this is taken to mean also a single compound, salt, polymorph, isomer, hydrate, solvate or the like.

The compounds of this invention may contain one or more asymmetric centre, depending upon the location and nature of the various substituents desired. Asymmetric carbon atoms may be present in the (R) or (S) configuration, resulting in racemic mixtures in the case of a single asymmetric centre, and diastereomeric mixtures in the case of multiple asymmetric centres. In certain instances, asymmetry may also be present due to restricted rotation about a given bond, for example, the central bond adjoining two substituted aromatic rings of the specified compounds.

Substituents on a ring may also be present in either cis or trans form. It is intended that all such configurations (including enantiomers and diastereomers), are included within the scope of the present invention.

Preferred compounds are those which produce the more desirable biological activity. Separated, pure or partially purified isomers and stereoisomers or racemic or diastereomeric mixtures of the compounds of this invention are also included within the scope of the present invention. The purification and the separation of such materials can be accomplished by standard techniques known in the art.

The optical isomers can be obtained by resolution of the racemic mixtures according to conventional processes, for example, by the formation of diastereoisomeric salts using an optically active acid or base or formation of covalent diastereomers. Examples of appropriate acids are tartaric, diacetyltartaric, ditoluoyltartaric and camphorsulfonic acid. Mixtures of diastereoisomers can be separated into their individual diastereomers on the basis of their physical and/or chemical differences by methods known in the art, for example, by chromatography or fractional crystallisation. The optically active bases or acids are then liberated from the separated diastereomeric salts. A different process for separation of optical isomers involves the use of chiral chromatography (e.g., chiral HPLC columns), with or without conventional derivatisation, optimally chosen to maximise the separation of the enantiomers. Suitable chiral HPLC columns are manufactured by Diacel, e.g., Chiracel OD and Chiracel OJ among many others, all routinely selectable. Enzymatic separations, with or without derivatisation, are also useful. The optically active compounds of this invention can likewise be obtained by chiral syntheses utilizing optically active starting materials.

In order to limit different types of isomers from each other reference is made to IUPAC Rules Section E (Pure Appl Chem 45, 11-30, 1976).

The invention also includes all suitable isotopic variations of a compound of the invention. An isotopic variation of a compound of the invention is defined as one in which at least one atom is replaced by an atom having the same atomic number but an atomic mass different from the atomic mass usually or predominantly found in nature. Examples of isotopes that can be incorporated into a compound of the invention include isotopes of hydrogen, carbon, nitrogen, oxygen, phosphorus, sulphur, fluorine, chlorine, bromine and iodine, such as ²H (deuterium), ³H (tritium), ¹³C, ¹⁴C, ¹⁵N, ¹⁷O, ¹⁸O, ³²P, ³³P, ³³S, ³⁴S, ³⁵S, ³⁶S_{,} ¹⁸F, ³⁶Cl, ⁸²Br, ¹²³I, ¹²⁴I, ¹²⁹I and ¹³¹I, respectively. Certain isotopic variations of a compound of the invention, for example, those in which one or more radioactive isotopes such as ³H or ¹⁴C are incorporated, are useful in drug and/or substrate tissue distribution studies. Tritiated and carbon-14, i.e., ¹⁴C, isotopes are particularly preferred for their ease of preparation and detectability. Further, substitution with isotopes such as deuterium may afford certain therapeutic advantages resulting from greater metabolic stability, for example, increased in vivo half-life or reduced dosage requirements and hence may be preferred in some circumstances. Isotopic variations of a compound of the invention can generally be prepared by conventional procedures known by a person skilled in the art such as by the illustrative methods or by the preparations described in the examples hereafter using appropriate isotopic variations of suitable reagents.

The present invention includes all possible stereoisomers of the compounds of the present invention as single stereoisomers, or as any mixture of said stereoisomers, in any ratio. Isolation of a single stereoisomer, e.g. a single enantiomer or a single diastereomer, of a compound of the present invention may be achieved by any suitable state of the art method, such as chromatography, especially chiral chromatography, for example.

Further, the compounds of the present invention may exist as tautomers. For example, any compound of the present invention which contains a pyrazole moiety as a heteroaryl group for example can exist as a 1 H tautomer, or a 2H tautomer, or even a mixture in any amount of the two tautomers, or a triazole moiety for example can exist as a 1 H tautomer, a 2H tautomer, or a 4H tautomer, or even a mixture in any amount of said 1 H, 2H and 4H tautomers, *viz.* :

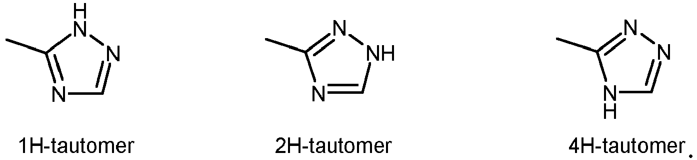

The present invention includes all possible tautomers of the compounds of the present invention as single tautomers, or as any mixture of said tautomers, in any ratio.

Further, the compounds of the present invention can exist as N-oxides, which are defined in that at least one nitrogen of the compounds of the present invention is oxidised. The present invention includes all such possible N-oxides.

The present invention also relates to useful forms of the compounds as disclosed herein, such as metabolites, hydrates, solvates, prodrugs, salts, in particular pharmaceutically acceptable salts, and co-precipitates.

The compounds of the present invention can exist as a hydrate, or as a solvate, wherein the compounds of the present invention contain polar solvents, in particular water, methanol or ethanol for example as structural element of the crystal lattice of the compounds. The amount of polar solvents, in particular water, may exist in a stoichiometric or non-stoichiometric ratio. In the case of stoichiometric solvates, *e.g.* a hydrate, hemi-, (semi-), mono-, sesqui-, di-, tri-, tetra-, penta- *etc.* solvates or hydrates, respectively, are possible. The present invention includes all such hydrates or solvates.

Further, the compounds of the present invention can exist in free form, e.g. as a free base, or as a free acid, or as a zwitterion, or can exist in the form of a salt. Said salt may be any salt, either an organic or inorganic addition salt, particularly any pharmaceutically acceptable organic or inorganic addition salt, customarily used in pharmacy.

The term "pharmaceutically acceptable salt" refers to a relatively non-toxic, inorganic or organic acid addition salt of a compound of the present invention. For example, see S. M. Berge, et al. "Pharmaceutical Salts," J. Pharm. Sci. 1977, 66, 1-19.

A suitable pharmaceutically acceptable salt of the compounds of the present invention may be, for example, an acid-addition salt of a compound of the present invention bearing a nitrogen atom, in a chain or in a ring, for example, which is sufficiently basic, such as an acid-addition salt with an inorganic acid, such as hydrochloric, hydrobromic, hydroiodic, sulfuric, bisulfuric, phosphoric, or nitric acid, for example, or with an organic acid, such as formic, acetic, acetoacetic, pyruvic, trifluoroacetic, propionic, butyric, hexanoic, heptanoic, undecanoic, lauric, benzoic, salicylic, 2-(4-hydroxybenzoyl)-benzoic, camphoric, cinnamic, cyclopentanepropionic, digluconic, 3-hydroxy-2-naphthoic, nicotinic, pamoic, pectinic, persulfuric, 3-phenylpropionic, picric, pivalic, 2-hydroxyethanesulfonate, itaconic, sulfamic, trifluoromethanesulfonic, dodecylsulfuric, ethansulfonic, benzenesulfonic, para-toluenesulfonic, methansulfonic, 2-naphthalenesulfonic, naphthalinedisulfonic, camphorsulfonic acid, citric, tartaric, stearic, lactic, oxalic, malonic, succinic, malic, adipic, alginic, maleic, fumaric, D-gluconic, mandelic, ascorbic, glucoheptanoic, glycerophosphoric, aspartic, sulfosalicylic, hemisulfuric, or thiocyanic acid, for example.

Further, another suitably pharmaceutically acceptable salt of a compound of the present invention which is sufficiently acidic, is an alkali metal salt, for example a sodium or potassium salt, an alkaline earth metal salt, for example a calcium or magnesium salt, an ammonium salt or a salt with an organic base which affords a physiologically acceptable cation, for example a salt with N-methyl-glucamine, dimethyl-glucamine, ethyl-glucamine, lysine, dicyclohexylamine, 1,6-hexadiamine, ethanolamine, glucosamine, sarcosine, serinol, tris-hydroxy-methyl-aminomethane, aminopropandiol, sovak-base, 1-amino-2,3,4-butantriol. Additionally, basic nitrogen containing groups may be quaternised with such agents as lower alkyl halides such as methyl, ethyl, propyl, and butyl chlorides, bromides and iodides; dialkyl sulfates like dimethyl, diethyl, and dibutyl sulfate ; and diamyl sulfates, long chain halides such as decyl, lauryl, myristyl and strearyl chlorides, bromides and iodides, aralkyl halides like benzyl and phenethyl bromides and others.

Those skilled in the art will further recognise that acid addition salts of the claimed compounds may be prepared by reaction of the compounds with the appropriate inorganic or organic acid via any of a number of known methods. Alternatively, alkali and alkaline earth metal salts of acidic compounds of the invention are prepared by reacting the compounds of the invention with the appropriate base via a variety of known methods.

The present invention includes all possible salts of the compounds of the present invention as single salts, or as any mixture of said salts, in any ratio.

As used herein, the term "*in vivo* hydrolysable ester" is understood as meaning an *in vivo* hydrolysable ester of a compound of the present invention containing a carboxy or hydroxy group, for example, a pharmaceutically acceptable ester which is hydrolysed in the human or animal body to produce the parent acid or alcohol. Suitable pharmaceutically acceptable esters for carboxy include for example alkyl, cycloalkyl and optionally substituted phenylalkyl, in particular benzyl esters, C₁-C₆ alkoxymethyl esters, e.g. methoxymethyl, C₁-C₆ alkanoyloxymethyl esters, e.g. pivaloyloxymethyl, phthalidyl esters, C₃-C₈ cycloalkoxy-carbonyloxy-C₁-C₆ alkyl esters, e.g. 1-cyclohexylcarbonyloxyethyl ; 1,3-dioxolen-2-onylmethyl esters, e.g. 5-methyl-1,3-dioxolen-2-onylmethyl ; and C₁-C₆-alkoxycarbonyloxyethyl esters, e.g. 1-methoxycarbonyloxyethyl, and may be formed at any carboxy group in the compounds of this invention.

An *in vivo* hydrolysable ester of a compound of the present invention containing a hydroxy group includes inorganic esters such as phosphate esters and [alpha]-acyloxyalkyl ethers and related compounds which as a result of the *in vivo* hydrolysis of the ester breakdown to give the parent hydroxy group. Examples of [alpha]-acyloxyalkyl ethers include acetoxymethoxy and 2,2-dimethylpropionyloxymethoxy. A selection of *in vivo* hydrolysable ester forming groups for hydroxy include alkanoyl, benzoyl, phenylacetyl and substituted benzoyl and phenylacetyl, alkoxycarbonyl (to give alkyl carbonate esters), dialkylcarbamoyl and N-(dialkylaminoethyl)-N-alkylcarbamoyl (to give carbamates), dialkylaminoacetyl and carboxyacetyl. The present invention covers all such esters.

Furthermore, the present invention includes all possible crystalline forms, or polymorphs, of the compounds of the present invention, either as single polymorphs, or as a mixture of more than one polymorphs, in any ratio.

In accordance with a first aspect, the present invention covers compounds of general formula (I): 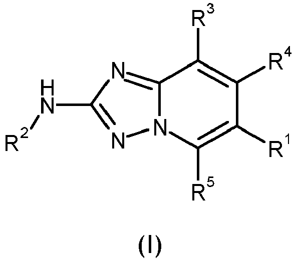 in which:
- R¹: represents a phenyl or a pyridyl group
- which is substituted, one or more times, identically or differently, with a substituent selected from:
   R⁶-(C₁-C₆-alkoxy)-, R⁶-O-, -C(=O)R⁶, -C(=O)O-R⁶, -N(H)C(=O)R⁶, -N(H)C(=O)NR⁶R⁷, -NR⁶R⁷, -C(=O)N(H)R⁶, -C(=O)NR⁶R⁷, R⁶-S-, R⁶-S(=O)₂-, -N(H)S(=O)₂R⁶, -S(=O)₂N(H)R⁶; and
- which is optionally substituted, one or more times, identically or differently, with a substituent selected from:
   halo-, hydroxy-, nitro-, C₁-C₆-alkyl-, C₁-C₆-alkoxy-, hydroxy-C₁-C₆-alkyl-, - N(H)C(=O)R⁸, -N(H)C(=O)NR⁸R⁷, -C(=O)N(H)R⁸, -N(H)S(=O)₂R⁸;
- R²: represents a 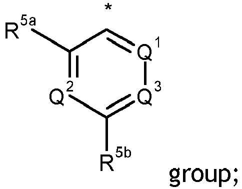 wherein * indicates the point of attachment of said group with the rest of the molecule;
Q¹ represents a group selected from: N, CH, C-(C₁-C₆-alkyl), C-(C₁-C₆-alkoxy), C-halo;
Q² represents a group selected from: N, CH, CR^{5b};
Q³ represents a group selected from: N, CH, CR^{5b};
R^{5a} represents a group selected from:
halo-, nitro-, C₁-C₆-alkyl-, halo-C₁-C₆-alkyl-, C₁-C₆-alkoxy-, halo-C₁-C₆-alkoxy-, hydroxy-C₁-C₆-alkyl-, C₁-C₆-alkoxy-C₁-C₆-alkyl-, halo-C₁-C₆-alkoxy-C₁-C₆-alkyl-, R⁸-(C₁-C₆-alkoxy)-, R⁸-O-, -NR⁸R⁷,
R⁸-S-, R⁸-S(=O)-, R⁸-S(=O)₂-, (C₃-C₆-cycloalkyl)-(CH₂)ₙ-O-;
R^{5b} represents a group selected from:
halo-, hydroxy-, cyano-, nitro-, C₁-C₆-alkyl-, halo-C₁-C₆-alkyl-, C₁-C₆-alkoxy-, halo-C₁-C₆-alkoxy-, hydroxy-C₁-C₆-alkyl-, C₁-C₆-alkoxy-C₁-C₆-alkyl-, halo-C₁-C₆-alkoxy-C₁-C₆-alkyl-, R⁸-(C₁-C₆-alkyl)-, R⁸-(CH₂)ₙ(CHOH)(CH₂)ₘ-, R⁸-(C₁-C₆-alkoxy)-, R⁸-(CH₂)ₙ(CHOH)(CH₂)ₚ-O-, R⁸-(C₁-C₆-alkoxy-C₁-C₆-alkyl)-, R⁸-(C₁-C₆-alkoxy-C₁-C₆-alkyl)-O-, -O-(CH₂)ₙ-C(=O)NR⁸R⁷, R⁸-O-, -C(=O)R⁸, -C(=O)O-R⁸, -OC(=O)-R⁸, -N(H)C(=O)R⁸, -N(R⁷)C(=O)R⁸, -N(H)C(=O)NR⁸R⁷, -N(R⁷)C(=O)NR⁸R⁷, -NR⁸R⁷, -NR⁷R⁷, -C(=O)N(H)R⁸, -C(=O)NR⁸R⁷, R⁸-S-, R⁸-S(=O)-, R⁸-S(=O)₂-, -N(H)S(=O)R⁸, -N(R⁷)S(=O)R⁸, -S(=O)N(H)R⁸, -S(=O)NR⁸R⁷, -N(H)S(=O)₂R⁸, -N(R⁷)S(=O)₂R⁸, -S(=O)₂N(H)R⁸, -S(=O)₂NR⁸R⁷, -S(=O)(=NR⁸)R⁷, -S(=O)(=NR⁷)R⁸, -N=S(=O)(R⁸)R⁷;
- R³: represents a hydrogen atom, a halogen atom, a hydroxy-, amino-, cyano-, nitro-, C₁-C₄-alkyl-, halo-C₁-C₄-alkyl-, C₁-C₄-alkoxy-, halo-C₁-C₄-alkoxy-, hydroxy-C₁-C₄-alkyl-, C₁-C₄-alkoxy-C₁-C₄-alkyl-, halo-C₁-C₄-alkoxy-C₁-C₄-alkyl-, C₂-C₆-alkenyl-, C₂-C₆-alkynyl-, halo-C₂-C₆-alkenyl-, halo-C₂-C₆-alkynyl-, C₃-C₆-cycloalkyl-, or halo-C₃-C₆-cycloalkyl- group;
- R⁴: represents a hydrogen atom, a halogen atom, a hydroxy-, amino-, cyano-, nitro-, C₁-C₄-alkyl-, halo-C₁-C₄-alkyl-, C₁-C₄-alkoxy-, halo-C₁-C₄-alkoxy-, hydroxy-C₁-C₄-alkyl-, C₁-C₄-alkoxy-C₁-C₄-alkyl-, halo-C₁-C₄-alkoxy-C₁-C₄-alkyl-, C₂-C₆-alkenyl-, C₂-C₆-alkynyl-, halo-C₂-C₆-alkenyl-, halo-C₂-C₆-alkynyl-, C₃-C₆-cycloalkyl-, or halo-C₃-C₆-cycloalkyl- group;
- R⁵: represents a hydrogen atom;
- R⁶: represents a group selected from C₃-C₆-cycloalkyl-, 3- to 10-membered heterocyclyl-, aryl-, heteroaryl-, -(CH₂)_{q}-(C₃-C₆-cycloalkyl), -(CH₂)_{q}-(3- to 10-membered heterocyclyl), -(CH₂)_{q}-aryl, or -(CH₂)_{q}-heteroaryl,
wherein said group being optionally substituted, one or more times, identically or differently, with a substituent selected from:
halo-, hydroxy-, cyano-, nitro-, C₁-C₆-alkyl-, halo-C₁-C₆-alkyl-, C₁-C₆-alkoxy-, halo-C₁-C₆-alkoxy-, hydroxy-C₁-C₆-alkyl-, C₁-C₆-alkoxy-C₁-C₆-alkyl-, halo-C₁-C₆-alkoxy-C₁-C₆-alkyl-, R⁸-(C₁-C₆-alkyl)-, R⁸-(CH₂)ₙ(CHOH)(CH₂)ₘ-, R⁸-(C₁-C₆-alkoxy)-, R⁸-(CH₂)ₙ(CHOH)(CH₂)ₚ-O-, R⁸-(C₁-C₆-alkoxy-C₁-C₆-alkyl)-, R⁸-(C₁-C₆-alkoxy-C₁-C₆-alkyl)-O-, aryl-, R⁸-O-, -C(=O)R⁸, -C(=O)O-R⁸, -OC(=O)-R⁸, -N(H)C(=O)R⁸, -N(R⁷)C(=O)R⁸, -N(H)C(=O)NR⁸R⁷, -N(R⁷)C(=O)NR⁸R⁷, -NR⁸R⁷, -C(=O)N(H)R⁸, -C(=O)NR⁸R⁷, R⁸-S-, R⁸-S(=O)-, R⁸-S(=O)₂-, -N(H)S(=O)R⁸, -N(R⁷)S(=O)R⁸, -S(=O)N(H)R⁸, -S(=O)NR⁸R⁷, -N(H)S(=O)₂R⁸, -N(R⁷)S(=O)₂R⁸, -S(=O)₂N(H)R⁸, -S(=O)₂NR⁸R⁷, -S(=O)(=NR⁸)R⁷,- S(=O)(=NR⁷)R⁸, -N=S(=O)(R⁸)R⁷;
- R⁷: represents a hydrogen atom, a C₁-C₆-alkyl- or a C₃-C₆-cycloalkyl- group;
- R⁸: represents a hydrogen atom or a C₁-C₆-alkyl- or C₃-C₆-cycloalkyl- group, wherein said C₁-C₆-alkyl- or C₃-C₆-cycloalkyl- group is optionally substituted, one or more times, identically or differently, with a substituent selected from:
halo-, hydroxy-, -NHR⁷, -NR⁷R⁷, -N(C₁-C₃-alkyl)-C(=O)R⁷, -N(C₁-C₃-alkyl)-C(=O)OR⁷, C₁-C₃-alkyl-, R⁷-S(=O)₂-, C₁-C₃-alkoxy-, halo-C₁-C₃-alkoxy-;
- n, m, p,: represent, independently from each other, an integer of 0, 1, 2 or 3;
and
- q: represents an integer of 0, 1, 2 or 3.

As defined *supra*, R¹ is a substituted phenyl or pyridyl group. Preferably, R¹ is a substituted phenyl group.

In a preferred embodiment, the invention relates to compounds of formula (I), wherein :
- R¹: represents a phenyl group
- which is substituted, one or more times, identically or differently, with a substituent selected from:
   R⁶-(C₁-C₆-alkoxy)-, R⁶-O-, -C(=O)R⁶, -C(=O)O-R⁶, -N(H)C(=O)R⁶, -N(H)C(=O)NR⁶R⁷, -NR⁶R⁷, -C(=O)N(H)R⁶, -C(=O)NR⁶R⁷, R⁶-S-, R⁶-S(=O)₂-, -N(H)S(=O)₂R^{b}, -S(=O)₂N(H)R⁶; and
- which is optionally substituted, one or more times, identically or differently, with a substituent selected from:
   halo-, C₁-C₆-alkyl-, C₁-C₆-alkoxy-, -N(H)C(=O)R⁸, -C(=O)N(H)R⁸.

In another preferred embodiment, the invention relates to compounds of formula (I), wherein :
- R¹: represents a phenyl group
- which is substituted, one or more times, identically or differently, with a substituent selected from:
   R⁶-(C₁-C₆-alkoxy)-, R⁶-O-, -N(H)C(=O)R⁶, -N(H)C(=O)NR⁶R⁷, -C(=O)N(H)R⁶, -C(=O)NR⁶R⁷; and
- which is optionally substituted, one or more times, identically or differently, with a substituent selected from:
   halo-, hydroxy-, nitro-, C₁-C₆-alkyl-, C₁-C₆-alkoxy-, hydroxy-C₁-C₆-alkyl-, - N(H)C(=O)R⁸, -N(H)C(=O)NR⁸R⁷, -C(=O)N(H)R⁸, -N(H)S(=O)₂R⁸.

In another preferred embodiment, the invention relates to compounds of formula (I), wherein :
- R¹: represents a phenyl group
- which is substituted, one or more times, identically or differently, with a substituent selected from:
   R⁶-(C₁-C₆-alkoxy)-, R⁶-O-, -N(H)C(=O)R⁶,
   -N(H)C(=O)NR⁶R⁷, -C(=O)N(H)R⁶, -C(=O)NR⁶R⁷; and
- which is optionally substituted, one or more times, identically or differently, with a substituent selected from:
   halo-, C₁-C₆-alkyl-, C₁-C₆-alkoxy-, -N(H)C(=O)R⁸, -C(=O)N(H)R⁸.

In another preferred embodiment, the invention relates to compounds of formula (I), wherein :
- R¹: represents a phenyl group
- which is substituted, one or more times, identically or differently, with a substituent selected from:
   -N(H)C(=O)R⁶, -C(=O)N(H)R⁶; and
- which is optionally substituted, one or more times, identically or differently, with a substituent selected from:
   halo-, C₁-C₆-alkyl-, C₁-C₆-alkoxy-, -N(H)C(=O)R⁸, -C(=O)N(H)R⁸.

In another preferred embodiment, the invention relates to compounds of formula (I), wherein :
- R¹: represents a phenyl group
- which is substituted, one or more times, identically or differently, with a substituent selected from:
   R⁶-(C₁-C₆-alkoxy)-, R⁶-O-, -N(H)C(=O)R⁶, -N(H)C(=O)NR⁶R⁷, -C(=O)N(H)R⁶, - C(=O)NR⁶R⁷; and
- which is optionally substituted, one or more times, identically or differently, with a substituent selected from:
   halo-, C₁-C₆-alkyl-, C₁-C₆-alkoxy-.

In another preferred embodiment, the invention relates to compounds of formula (I), wherein :
- R¹: represents a phenyl group
- which is substituted, one or more times, identically or differently, with a substituent selected from:
   -N(H)C(=O)R⁶, -C(=O)N(H)R⁶; and
- which is optionally substituted, one or more times, identically or differently, with a substituent selected from:
   halo-, C₁-C₆-alkyl-, C₁-C₆-alkoxy-.

In a preferred embodiment, the invention relates to compounds of formula (I), wherein :
- R¹: represents a phenyl group
- which is substituted, one or more times, identically or differently, with a -N(H)C(=O)R⁶ substituent, and
- which is optionally substituted, one or more times, identically or differently, with a substituent selected from:
   halo-, hydroxy-, nitro-, C₁-C₆-alkyl-, C₁-C₆-alkoxy-, hydroxy-C₁-C₆-alkyl-, - N(H)C(=O)R⁸, -N(H)C(=O)NR⁸R⁷, -C(=O)N(H)R⁸, -N(H)S(=O)₂R⁸.

In a preferred embodiment, the invention relates to compounds of formula (I), wherein :
- R¹: represents a phenyl group
- which is substituted, one or more times, identically or differently, with a -C(=O)N(H)R⁶ substituent, and
- which is optionally substituted, one or more times, identically or differently, with a substituent selected from:
   halo-, hydroxy-, nitro-, C₁-C₆-alkyl-, C₁-C₆-alkoxy-, hydroxy-C₁-C₆-alkyl-, - N(H)C(=O)R⁸, -N(H)C(=O)NR⁸R⁷, -C(=O)N(H)R⁸, -N(H)S(=O)₂R⁸.

In a preferred embodiment, the invention relates to compounds of formula (I), wherein :
- R¹: represents a phenyl group
- which is substituted, one or more times, identically or differently, with a -N(H)C(=O)R⁶ substituent, and
- which is optionally substituted, one or more times, identically or differently, with a substituent selected from:
   halo-, C₁-C₆-alkyl-, C₁-C₆-alkoxy-.

In a preferred embodiment, the invention relates to compounds of formula (I), wherein :
- R¹: represents a phenyl group
- which is substituted, one or more times, identically or differently, with a -C(=O)N(H)R⁶ substituent, and
- which is optionally substituted, one or more times, identically or differently, with a substituent selected from:
   halo-, C₁-C₆-alkyl-, C₁-C₆-alkoxy-.

In another preferred embodiment, the invention relates to compounds of formula (I), wherein :
- R¹: represents a phenyl group
- which is substituted, in para-position to the point of attachment of the phenyl group with the rest of the molecule, with
   -N(H)C(=O)R⁶; and
- which is optionally substituted, one or more times, identically or differently, with a substituent selected from:
   halo-, hydroxy-, nitro-, C₁-C₆-alkyl-, C₁-C₆-alkoxy-, hydroxy-C₁-C₆-alkyl-, - N(H)C(=O)R⁸, -N(H)C(=O)NR⁸R⁷, -C(=O)N(H)R⁸, -N(H)S(=O)₂R⁸.

In another preferred embodiment, the invention relates to compounds of formula (I), wherein :
- R¹: represents a phenyl group
- which is substituted, in para-position to the point of attachment of the phenyl group with the rest of the molecule, with
   -C(=O)N(H)R⁶; and
- which is optionally substituted, one or more times, identically or differently, with a substituent selected from:
   halo-, hydroxy-, nitro-, C₁-C₆-alkyl-, C₁-C₆-alkoxy-, hydroxy-C₁-C₆-alkyl-, - N(H)C(=O)R⁸, -N(H)C(=O)NR⁸R⁷, -C(=O)N(H)R⁸, -N(H)S(=O)₂R⁸.

In another preferred embodiment, the invention relates to compounds of formula (I), wherein :
- R¹: represents a phenyl group which is para-substituted with respect to the point of attachement of the phenyl group with the rest of the molecule, as depicted in formula (I), with a substituent selected from:
R⁶-(C₁-C₆-alkoxy)-, R⁶-O-, -C(=O)R⁶, -C(=O)O-R⁶, -N(H)C(=O)R⁶, -N(H)C(=O)NR⁶R⁷, -NR⁶R⁷, -C(=O)N(H)R⁶, -C(=O)NR⁶R⁷, R⁶-S-, R⁶-S(=O)₂-, -N(H)S(=O)₂R^{b}, -S(=O)₂N(H)R⁶; and
- which is optionally substituted, one or more times, identically or differently, with a substituent selected from:
   halo-, C₁-C₆-alkyl-, C₁-C₆-alkoxy-, -N(H)C(=O)R⁸, -C(=O)N(H)R⁸.

In another preferred embodiment, the invention relates to compounds of formula (I), wherein :
- R¹: represents a phenyl group which is para-substituted with respect to the point of attachement of the phenyl group with the rest of the molecule, as depicted in formula (I), with a substituent selected from:
R⁶-(C₁-C₆-alkoxy)-, R⁶-O-, -N(H)C(=O)R⁶, -N(H)C(=O)NR⁶R⁷, -C(=O)N(H)R⁶, - C(=O)NR⁶R⁷; and
- which is optionally substituted, one or more times, identically or differently, with a substituent selected from:
   halo-, C₁-C₆-alkyl-, C₁-C₆-alkoxy-, -N(H)C(=O)R⁸, -C(=O)N(H)R⁸.

In another preferred embodiment, the invention relates to compounds of formula (I), wherein :
- R¹: represents a phenyl group which is para-substituted with respect to the point of attachement of the phenyl group with the rest of the molecule, as depicted in formula (I), with a substituent selected from:
-N(H)C(=O)R⁶, -C(=O)N(H)R⁶; and
- which is optionally substituted, one or more times, identically or differently, with a substituent selected from:
   halo-, C₁-C₆-alkyl-, C₁-C₆-alkoxy-, -N(H)C(=O)R⁸, -C(=O)N(H)R⁸.

In another preferred embodiment, the invention relates to compounds of formula (I), wherein :
- R¹: represents a phenyl group which is para-substituted with respect to the point of attachement of the phenyl group with the rest of the molecule, as depicted in formula (I), with a substituent selected from:
-N(H)C(=O)R⁶, -C(=O)N(H)R⁶; and
- which is optionally substituted, one or more times, identically or differently, with a substituent selected from:
   halo-, C₁-C₆-alkyl-, C₁-C₆-alkoxy-.

In another preferred embodiment, the invention relates to compounds of formula (I), wherein :
- R¹: represents a group selected from: 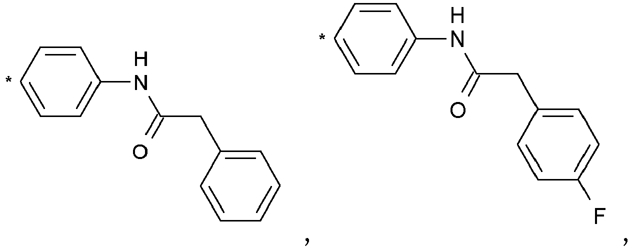 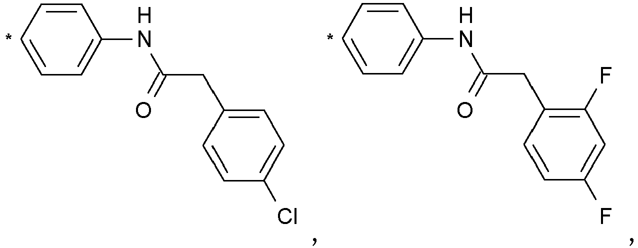 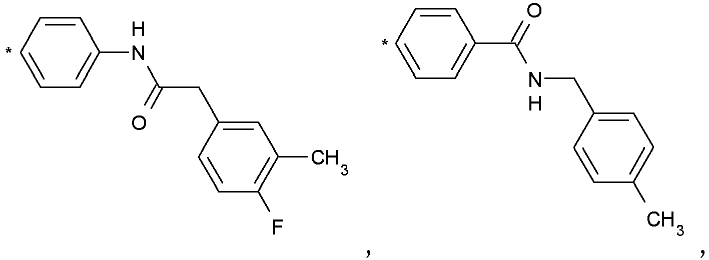 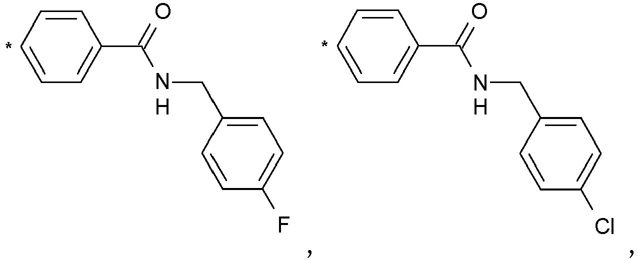 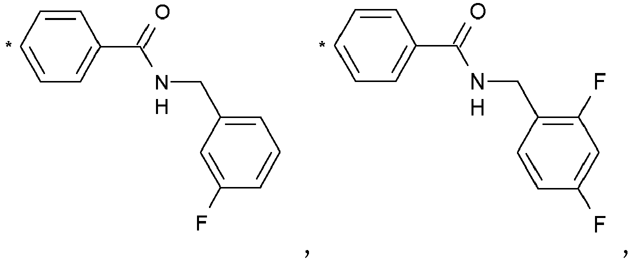 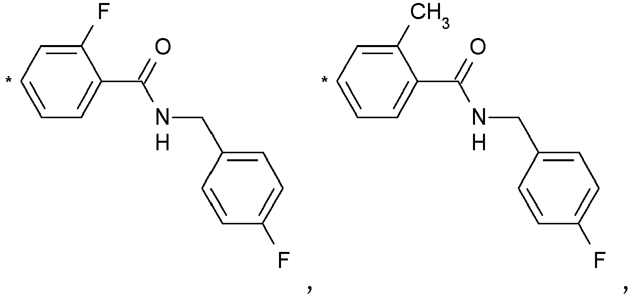 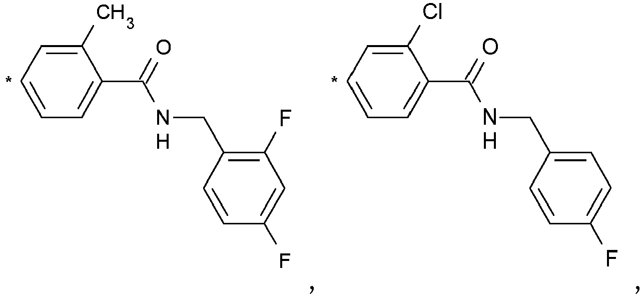 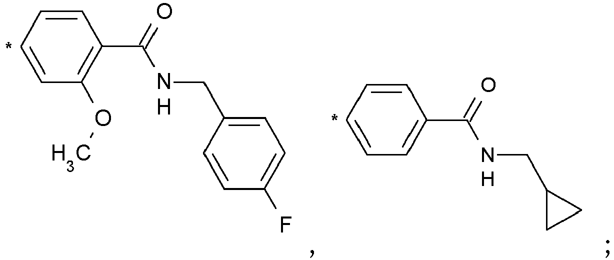
wherein * indicates the point of attachment of said group with the rest of the molecule.

In another preferred embodiment, the invention relates to compounds of formula (I), wherein :
- Q¹: represents a group selected from: CH, C-(C₁-C₆-alkyl), C-(C₁-C₆-alkoxy), C-halo.

Preferably, Q¹ represents CH.

In another preferred embodiment, the invention relates to compounds of formula (I), wherein :
- Q²: represents a group selected from: N, CH, C-R^{5c};
wherein R^{5c} is selected from the groups consisting of:
halo-, cyano-, C₁-C₆-alkyl-, C₁-C₆-alkoxy-, -N(H)C(=O)R⁷, -N(R⁷)C(=O)R⁷, -C(=O)N(H)R⁸, -C(=O)NR⁸R⁷, R⁸-S(=O)-, R⁸-S(=O)₂-, -S(=O)(=NR⁷)R⁸.

Preferably, Q² represents CH.

In another preferred embodiment, the invention relates to compounds of formula (I), wherein :
- Q³: represents a group selected from: N, CH, C-R^{5c},
wherein R^{5c} is selected from the groups consisting of:
halo-, cyano-, C₁-C₆-alkyl-, C₁-C₆-alkoxy-, -N(H)C(=O)R⁷, -N(R⁷)C(=O)R⁷, -C(=O)N(H)R⁸, -C(=O)NR⁸R⁷, R⁸-S(=O)-, R⁸-S(=O)₂-, -S(=O)(=NR⁷)R⁸.

Preferably, Q³ represents CH or N.

In another preferred embodiment, the invention relates to compounds of formula (I), wherein :
Q¹ and Q² represent CH, and Q³ represents CH or N.

In another preferred embodiment, the invention relates to compounds of formula (I), wherein :
- R³: represents a hydrogen atom, halo-, hydroxy-, C₁-C₄-alkyl-, halo-C₁-C₄-alkyl-, or C₁-C₄-alkoxy- group.

Preferably, R³ represents a hydrogen atom.

In another preferred embodiment, the invention relates to compounds of formula (I), wherein :
- R⁴: represents a hydrogen atom, halo-, a C₁-C₆-alkyl-, halo-C₁-C₆-alkyl- or C₁-C₆-alkoxy- group.

Preferably, R⁴ represents a hydrogen atom.

In another preferred embodiment, the invention relates to compounds of formula (I), wherein :
R³ and R⁴ represent a hydrogen atom.

In another preferred embodiment, the invention relates to compounds of formula (I), wherein :
- R^{5a}: represents a group selected from: halo-, C₁-C₆-alkyl-,C₁-C₆-alkoxy-, halo-C₁-C₆-alkoxy-, hydroxy-C₁-C₆-alkyl-, C₁-C₆-alkoxy-C₁-C₆-alkyl-, halo-C₁-C₆-alkoxy-C₁-C₆-alkyl-, R⁸-(C₁-C₆-alkoxy)-, R⁸-O-, R⁸-S-, R⁸-S(=O)₂-, (C₃-C₆-cycloalkyl)-(CH₂)ₙ-O-.

Preferably, R^{5a} is selected from:
halo-, C₁-C₆-alkyl-, C₁-C₆-alkoxy-, halo-C₁-C₆-alkoxy-, C₁-C₆-alkoxy-C₁-C₆-alkyl-,
(C₃-C₆-cycloalkyl)-(CH₂)ₙ-O-.

More preferably, R^{5a} is selected from:
F-, methyl-, methoxy-, ethoxy-, n-propoxy-, iso-propoxy-, cyclopropyl-O-, cyclopropyl-CH₂-O-, CH₃-O-CH₂CH₂-O-, CHF₂-O-, CF₃-O-, CF₃CH₂-O-.

In another preferred embodiment, the invention relates to compounds of formula (I), wherein :
- R^{5a}: represents a C₁-C₆-alkoxy-, group.

In another preferred embodiment, the invention relates to compounds of formula (I), wherein :
- R^{5a}: represents a C₁-C₃-alkoxy-, group.

In another preferred embodiment, the invention relates to compounds of formula (I), wherein :
- R^{5a}: represents a halo-C₁-C₆-alkoxy-, group.

In another preferred embodiment, the invention relates to compounds of formula (I), wherein :
- R^{5a}: represents a halo-C₁-C₃-alkoxy-, group.

In another preferred embodiment, the invention relates to compounds of formula (I), wherein :
- R^{5a}: represents a (C₃-C₆-cycloalkyl)-(CH₂)ₙ-O- group.

In another preferred embodiment, the invention relates to compounds of formula (I), wherein :
- R^{5b}: represents a group selected from: halo-, cyano-, nitro-, C₁-C₆-alkyl-, halo-C₁-C₆-alkyl-, hydroxy-C₁-C₆-alkyl-, C₁-C₆-alkoxy-, halo-C₁-C₆-alkoxy-, R⁸-O-, -C(=O)R⁸, -C(=O)O-R⁸, -N(H)C(=O)R⁸, -N(R⁷)C(=O)R⁸, -N(H)S(=O)₂R⁸, -NR⁸R⁷, -NR⁷R⁷, -C(=O)N(H)R⁸, -C(=O)NR⁸R⁷, R⁸-S(=O)-, R⁸-S(=O)₂-, -S(=O)(=NR⁷)R⁸.

Preferably, R^{5b} is selected from:
halo-, cyano-, -NR⁷R⁷, C₁-C₆-alkoxy-, -N(H)C(=O)R⁸,-N(R⁷)C(=O)R⁸, -C(=O)N(H)R⁸, -C(=O)NR⁸R⁷, R⁸-S(=O)-, R⁸-S(=O)₂-, -S(=O)(=NR⁷)R⁸, hydroxy-C₁-C₆-alkyl-, -N(H)S(=O)₂R₈.

More preferably, R^{5b} is selected from:
fluoro-, cyano-, methoxy-, -CH₂-OH, -C(OH)(CH₃)₂, -N(CH₃)₂, -C(=O)NH₂, -C(=O)N(CH₃)₂, -C(=O)N(C₂H₅)₂, -C(=O)N(CH₃)(C₂H₅), -C(=O)NH(C₂H₅),
-C(=O)NH(CH₃), -C(=O)NH(C(CH₃)₃), -C(=O)NH(CH₂CH₂OH), -C(=O)NH(CH₂CH₂F), -C(=O)NH(CH₂CH₂OCH₃), -C(=O)NH(CH₂CH₂OCH₂CH₃), -C(=O)NH(C(CH₃)₂CH₂OH), -C(=O)NH(CH₂C(CH₃)₂OH), -C(=O)NH(CH₂CF₃), -S(=O)CH₃, (CH₃)S(=O)₂-, (C₂H₅)S(=O)₂-, -N(H)C(=O)CH₃, 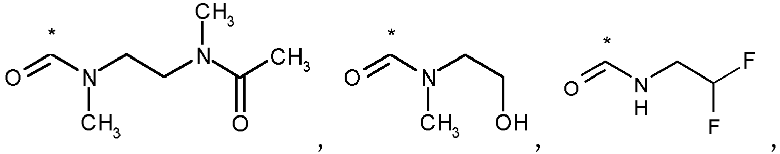 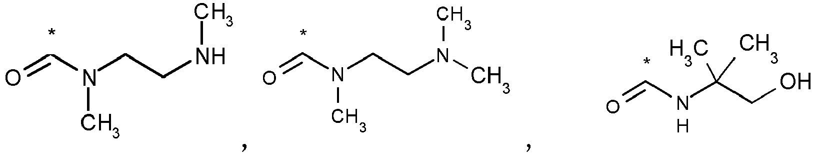 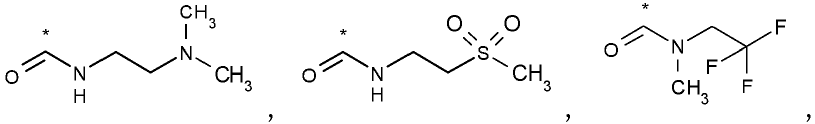 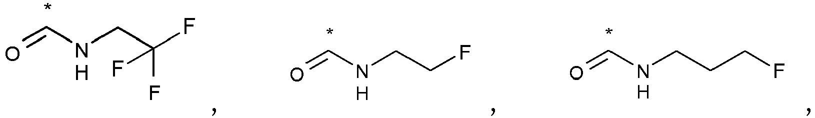 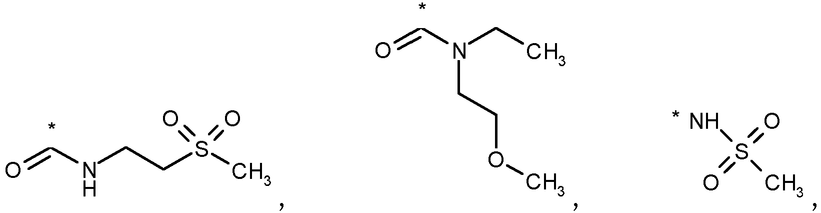 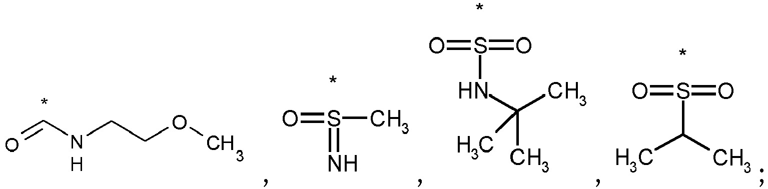
wherein * indicates the point of attachment of said group with the rest of the molecule.

In another preferred embodiment, the invention relates to compounds of formula (I), wherein :
- R^{5b}: represents a group selected from:
-C(=O)N(H)R⁸, -C(=O)NR⁸R⁷, R⁸-S(=O)-, R⁸-S(=O)₂-, -S(=O)(=NR⁷)R⁸.

In another preferred embodiment, the invention relates to compounds of formula (I), wherein :
- R^{5b}: represents a -C(=O)N(H)R⁸ group.

In another preferred embodiment, the invention relates to compounds of formula (I), wherein :
- R^{5b}: represents a -C(=O)NR⁸R⁷ group.

In another preferred embodiment, the invention relates to compounds of formula (I), wherein :
- R^{5b}: represents a R⁸-S(=O)- group.

In another preferred embodiment, the invention relates to compounds of formula (I), wherein :
- R^{5b}: represents a R⁸-S(=O)₂-, group.

In another preferred embodiment, the invention relates to compounds of formula (I), wherein :
- R^{5b}: represents a -S(=O)(=NR⁷)R⁸ group.

In another preferred embodiment, the invention relates to compounds of formula (I), wherein :
- R^{5b}: represents a -C(=O)N(H)R⁷ group.

In another preferred embodiment, the invention relates to compounds of formula (I), wherein :
- R^{5b}: represents a R⁷-S(=O)₂-, group.

In another preferred embodiment, the invention relates to compounds of formula (I), wherein :
- R⁶: represents a group selected from:
C₃-C₆-cycloalkyl-, 3- to 10-membered heterocyclyl-, aryl-, heteroaryl-, -(CH₂)_{q}-(C₃-C₆-cycloalkyl), -(CH₂)_{q}-(3- to 10-membered heterocyclyl), -(CH₂)_{q}-aryl, or -(CH₂)_{q}-heteroaryl;
wherein said group is optionally substituted, one or more times, identically or differently, with a substituent selected from:
halo-, hydroxy-, cyano-, nitro-, C₁-C₆-alkyl-, halo-C₁-C₆-alkyl-, C₁-C₆-alkoxy-, halo-C₁-C₆-alkoxy-, hydroxy-C₁-C₆-alkyl-, C₁-C₆-alkoxy-C₁-C₆-alkyl-, halo-C₁-C₆-alkoxy-C₁-C₆-alkyl-; wherein q is 1 or 2.

In another preferred embodiment, the invention relates to compounds of formula (I), wherein :
- R⁶: represents a group selected from:
-(CH₂)_{q}-(C₃-C₆-cycloalkyl), -(CH₂)_{q}-(3- to 10-membered heterocyclyl), -(CH₂)_{q}-aryl, or -(CH₂)_{q}-heteroaryl;
wherein said group is optionally substituted, one or more times, identically or differently, with a substituent selected from: halo-, C₁-C₆-alkyl-;
wherein q is 0 or 1.
The C₃-C₆-cycloalkyl- group preferably is a cyclopropyl- group; the aryl-group is preferably a phenyl- group; the heteroaryl- group is preferably a pyridyl- group.

In another preferred embodiment, the invention relates to compounds of formula (I), wherein :
- R⁶: represents a group selected from:
-(CH₂)-(C₃-C₆-cycloalkyl), -(CH₂)-aryl;
wherein said group is optionally substituted, one or more times, identically or differently, with a substituent selected from:
halo-, hydroxy-, cyano-, nitro-, C₁-C₆-alkyl-, halo-C₁-C₆-alkyl-, C₁-C₆-alkoxy-, halo-C₁-C₆-alkoxy-, hydroxy-C₁-C₆-alkyl-, C₁-C₆-alkoxy-C₁-C₆-alkyl-, halo-C₁-C₆-alkoxy-C₁-C₆-alkyl-.
The C₃-C₆-cycloalkyl- group preferably is a cyclopropyl- group; the aryl-group is preferably a phenyl- group.

In another preferred embodiment, the invention relates to compounds of formula (I), wherein :
- R⁶: represents a group selected from:
-CH₂-(C₃-C₆-cycloalkyl) or -CH₂-aryl;
wherein said group is optionally substituted, one or more times, identically or differently, with a substituent selected from:
halo-, C₁-C₆-alkyl-, halo-C₁-C₆-alkyl-, halo-C₁-C₆-alkoxy-.
The C₃-C₆-cycloalkyl- group preferably is a cyclopropyl- group; the aryl-group is preferably a phenyl- group.

In another preferred embodiment, the invention relates to compounds of formula (I), wherein :
- R⁶: represents a group selected from:
-(CH₂)-(C₃-C₆-cycloalkyl), -(CH₂)-aryl;
wherein said group is optionally substituted, one or more times, identically or differently, with a substituent selected from:
halo-, C₁-C₆-alkyl-.
The C₃-C₆-cycloalkyl- group preferably is a cyclopropyl- group; the aryl-group is preferably a phenyl- group.

In another preferred embodiment, the invention relates to compounds of formula (I), wherein :
- R⁶: represents a group selected from:
-(CH₂)-aryl;
wherein said group is optionally substituted, one or more times, identically or differently, with a substituent selected from: halo-, C₁-C₆-alkyl-.
The aryl- group is preferably a phenyl- group.

In another preferred embodiment, the invention relates to compounds of formula (I), wherein :
- R⁶: represents a group selected from:
-(CH₂)-(C₃-C₆-cycloalkyl);
wherein said group is optionally substituted, one or more times, identically or differently, with a substituent selected from:
halo-, C₁-C₆-alkyl-
The C₃-C₆-cycloalkyl- group preferably is a cyclopropyl- group.

In another preferred embodiment, the invention relates to compounds of formula (I), wherein :
- R⁶: represents a group selected from:
-(CH₂)-phenyl, -(CH₂)-cyclopropyl;
wherein said group is optionally substituted, one or more times, identically or differently, with a substituent selected from:
halo-, C₁-C₆-alkyl-;
wherein q is 0 or 1.

In another preferred embodiment, the invention relates to compounds of formula (I), wherein :
- R⁷: represents a hydrogen atom, or a C₁-C₆-alkyl-.

In another preferred embodiment, the invention relates to compounds of formula (I), wherein :
- R⁷: represents a hydrogen atom, or a C₃-C₆-cycloalkyl- group;

In another preferred embodiment, the invention relates to compounds of formula (I), wherein :
- R⁸: represents a hydrogen atom or a C₁-C₆-alkyl- group,
wherein said C₁-C₆-alkyl-group is optionally substituted, one or more times, identically or differently, with a substituent selected from:
halo-, hydroxy-, -NHR⁷, -NR⁷R⁷, -N(C₁-C₃-alkyl)-C(=O)R⁷, -N(C₁-C₃-alkyl)-C(=O)OR⁷, C₁-C₃-alkyl-, R⁷-S(=O)₂-, C₁-C₃-alkoxy-, halo-C₁-C₃-alkoxy-;

In another preferred embodiment, the invention relates to compounds of formula (I), wherein :
- R⁸: represents a hydrogen atom or C₃-C₆-cycloalkyl- group,
wherein said C₃-C₆-cycloalkyl- group is optionally substituted, one or more times, identically or differently, with a substituent selected from:
halo-, hydroxy-, -NHR⁷, -NR⁷R⁷, -N(C₁-C₃-alkyl)-C(=O)R⁷, -N(C₁-C₃-alkyl)-C(=O)OR⁷, C₁-C₃-alkyl-, R⁷-S(=O)₂-, C₁-C₃-alkoxy-, halo-C₁-C₃-alkoxy-;

In another preferred embodiment, the invention relates to compounds of formula (I), wherein :
- R⁸: represents a hydrogen atom or a C₁-C₆-alkyl- group,
wherein said C₁-C₆-alkyl-group is optionally substituted, one or more times, identically or differently, with a substituent selected from:
halo-, C₁-C₃-alkyl-, R⁷-S(=O)₂-, C₁-C₃-alkoxy-, halo-C₁-C₃-alkoxy-;

In another preferred embodiment, the invention relates to compounds of formula (I), wherein :
- R⁸: represents a hydrogen atom or C₃-C₆-cycloalkyl- group,
wherein said C₃-C₆-cycloalkyl- group is optionally substituted, one or more times, identically or differently, with a substituent selected from:
halo-, C₁-C₃-alkyl-, R⁷-S(=O)₂-, C₁-C₃-alkoxy-, halo-C₁-C₃-alkoxy-;

In another preferred embodiment, the invention relates to compounds of formula (I), wherein :
- R⁸: represents a hydrogen atom or a C₁-C₆-alkyl- group,
wherein said C₁-C₆-alkyl-group is optionally substituted, one or more times, identically or differently, with a substituent selected from:
halo-.

In another preferred embodiment, the invention relates to compounds of formula (I), wherein :
- n: represents an integer of 0, 1 or 2.

Preferably, n represent 0 or 1.

In another preferred embodiment, the invention relates to compounds of formula (I), wherein :
- q: represents an integer of 0, 1 or 2.

Preferably, q represents 1 or 2.

More preferably, q = 1.

It is to be understood that the present invention relates also to any combination of the preferred embodiments described above.

Some examples of combinations are given hereinafter. However, the invention is not limited to these combinations.

In another preferred embodiment, the invention relates to compounds of formula (I): 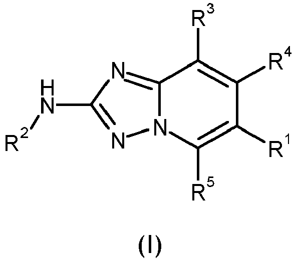 in which:
- R¹: represents a phenyl group
- which is substituted, one or more times, identically or differently, with a substituent selected from:
   R⁶-(C₁-C₆-alkoxy)-, R⁶-O-, -N(H)C(=O)R⁶, -N(H)C(=O)NR⁶R⁷, -C(=O)N(H)R⁶, - C(=O)NR⁶R⁷; and
- which is optionally substituted, one or more times, identically or differently, with a substituent selected from:
   halo-, C₁-C₆-alkyl-, C₁-C₆-alkoxy-, -N(H)C(=I)R⁸, -C(=O)N(H)R⁸;
- R²: represents a 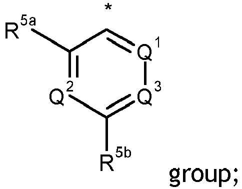 wherein * indicates the point of attachment of said group with the rest of the molecule;
R^{5a} represents a group selected from:
halo-, C₁-C₆-alkyl-,C₁-C₆-alkoxy-, halo-C₁-C₆-alkoxy-, hydroxy-C₁-C₆-alkyl-, C₁-C₆-alkoxy-C₁-C₆-alkyl-, halo-C₁-C₆-alkoxy-C₁-C₆-alkyl-, R⁸-(C₁-C₆-alkoxy)-, R⁸-O-, R⁸-S-, R⁸-S(=O)₂-, (C₃-C₆-cycloalkyl)-(CH₂)ₙ-O-;
R^{5b} represents a group selected from:
halo-, cyano-, nitro-, C₁-C₆-alkyl-, halo-C₁-C₆-alkyl-, C₁-C₆-alkoxy-, halo-C₁-C₆-alkoxy-, R⁸-O-, -C(=O)R⁸, -C(=O)O-R⁸, -N(H)C(=O)R⁸, -N(R⁷)C(=O)R⁸, -NR⁸R⁷, -NR⁷R⁷, -C(=O)N(H)R⁸, -C(=O)NR⁸R⁷, R⁸-S(=O)-, R⁸-S(=O)₂-, -S(=O)(=NR⁷)R⁸;
Q¹ represents a group selected from: CH, C-(C₁-C₆-alkyl), C-(C₁-C₆-alkoxy), C-halo;
Q² represents a group selected from: N, CH, C-R^{5c};
wherein R^{5c} is selected from the groups consisting of:
halo-, cyano-, C₁-C₆-alkyl-, C₁-C₆-alkoxy-, -N(H)C(=O)R⁷, -N(R⁷)C(=O)R⁷, -C(=O)N(H)R⁸, -C(=O)NR⁸R⁷, R⁸-S(=O)-, R⁸-S(=O)₂-, -S(=O)(=NR⁷)R⁸;
Q³ represents a group selected from: N, CH, C-R^{5c}, wherein R^{5c} is selected from the groups consisting of:
halo-, cyano-, C₁-C₆-alkyl-, C₁-C₆-alkoxy-, -N(H)C(=O)R⁷, -N(R⁷)C(=O)R⁷, -C(=O)N(H)R⁸, -C(=O)NR⁸R⁷, R⁸-S(=O)-, R⁸-S(=O)₂-, -S(=O)(=NR⁷)R⁸;
- R³: represents a hydrogen atom, halo-, hydroxy-, C₁-C₄-alkyl-, halo-C₁-C₄-alkyl-, or C₁-C₄-alkoxy- group;
- R⁴: represents a hydrogen atom, halo-, a C₁-C₆-alkyl-, halo-C₁-C₆-alkyl- or C₁-C₆-alkoxy- group;
- R⁵: represents a hydrogen atom.
- R⁶: represents a group selected from:
C₃-C₆-cycloalkyl-, 3- to 10-membered heterocyclyl-, aryl-, heteroaryl-, -(CH₂)_{q}-(C₃-C₆-cycloalkyl), -(CH₂)_{q}-(3- to 10-membered heterocyclyl), -(CH₂)_{q}-aryl, or -(CH₂)_{q}-heteroaryl;
wherein said group is optionally substituted, one or more times, identically or differently, with a substituent selected from:
halo-, hydroxy-, cyano-, nitro-, C₁-C₆-alkyl-, halo-C₁-C₆-alkyl-, C₁-C₆-alkoxy-, halo-C₁-C₆-alkoxy-, hydroxy-C₁-C₆-alkyl-,
C₁-C₆-alkoxy-C₁-C₆-alkyl-, halo-C₁-C₆-alkoxy-C₁-C₆-alkyl-;
- R⁷: represents a hydrogen atom, a C₁-C₆-alkyl- or a C₃-C₆-cycloalkyl- group;
- R⁸: represents a hydrogen atom or a C₁-C₆-alkyl- or C₃-C₆-cycloalkyl- group,
wherein said C₁-C₆-alkyl- or C₃-C₆-cycloalkyl- group is optionally substituted, one or more times, identically or differently, with a substituent selected from:
halo-, hydroxy-, -NHR⁷, -NR⁷R⁷, -N(C₁-C₃-alkyl)-C(=O)R⁷, -N(C₁-C₃-alkyl)-C(=O)OR⁷, C₁-C₃-alkyl-, R⁷-S(=O)₂-, C₁-C₃-alkoxy-, halo-C₁-C₃-alkoxy-;
- n: represents an integer of 0, 1 or 2; and
- q: represents an integer of 0, 1 or 2.

In another preferred embodiment, the invention relates to compounds of formula (I), wherein :
- R¹: represents a phenyl group
- which is substituted, one or more times, identically or differently, with a substituent selected from:
   -N(H)C(=O)R⁶, -C(=O)N(H)R⁶; and
- which is optionally substituted, one or more times, identically or differently, with a substituent selected from:
   halo-, C₁-C₆-alkyl-, C₁-C₆-alkoxy-;
- R²: represents a 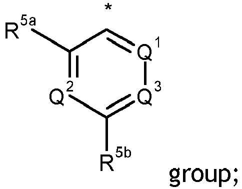 wherein * indicates the point of attachment of said group with the rest of the molecule;
Q¹ represents CH;
Q² represents CH;
Q³ represents CH or N;
R^{5a} represents a group selected from:
halo-, C₁-C₆-alkyl-,C₁-C₆-alkoxy-, halo-C₁-C₆-alkoxy-, (C₃-C₆-cycloalkyl)-(CH₂)ₙ-O-;
R^{5b} represents a group selected from:
halo-, cyano-, C₁-C₆-alkoxy-, -N(H)C(=O)R⁸, -N(R⁷)C(=O)R⁸, -C(=O)N(H)R⁸, -C(=O)NR⁸R⁷, R⁸-S(=O)-, R⁸-S(=O)₂-, -S(=O)(=NR⁷)R⁸;
- R³, R⁴, and R⁵: represent a hydrogen atom;
- R⁶: represents a group selected from:
-(CH₂)-(C₃-C₆-cycloalkyl) or -(CH₂)-aryl;
wherein said group is optionally substituted, one or more times, identically or differently, with a substituent selected from:
halo-, hydroxy-, cyano-, nitro-, C₁-C₆-alkyl-, halo-C₁-C₆-alkyl-, C₁-C₆-alkoxy-, halo-C₁-C₆-alkoxy-, hydroxy-C₁-C₆-alkyl-, C₁-C₆-alkoxy-C₁-C₆-alkyl-, halo-C₁-C₆-alkoxy-C₁-C₆-alkyl-;
- R⁷: represents a hydrogen atom, a C₁-C₆-alkyl- or a C₃-C₆-cycloalkyl- group;
- R⁸: represents a hydrogen atom or a C₁-C₆-alkyl- or C₃-C₆-cycloalkyl- group,
wherein said C₁-C₆-alkyl- or C₃-C₆-cycloalkyl- group is optionally substituted, one or more times, identically or differently, with a substituent selected from:
halo-, hydroxy-, -NHR⁷, -NR⁷R⁷, -N(C₁-C₃-alkyl)-C(=O)R⁷, -N(C₁-C₃-alkyl)-C(=O)OR⁷, C₁-C₃-alkyl-, R⁷-S(=O)₂-, C₁-C₃-alkoxy-, halo-C₁-C₃-alkoxy-;
- n: represents an integer of 0, 1 or 2;
- q: represents an integer of 1 or 2.

In another preferred embodiment, the invention relates to compounds of formula (I), wherein :
- R¹: represents a phenyl group
- which is substituted, one or more times, identically or differently, with a substituent selected from:
   -N(H)C(=O)R⁶, -C(=O)N(H)R⁶; and
- which is optionally substituted, one or more times, identically or differently, with a substituent selected from:
   halo-, C₁-C₆-alkyl-, C₁-C₆-alkoxy-;
- R²: represents a 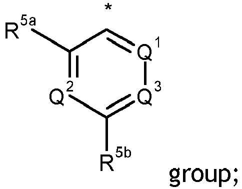 wherein * indicates the point of attachment of said group with the rest of the molecule;
Q¹ represents CH;
Q² represents CH;
Q³ represents CH or N;
R^{5a} represents a group selected from:
halo-, C₁-C₆-alkyl-,C₁-C₆-alkoxy-, halo-C₁-C₆-alkoxy-, (C₃-C₆-cycloalkyl)-(CH₂)ₙ-O-;
R^{5b} represents a group selected from:
halo-, cyano-, C₁-C₆-alkoxy-, -N(H)C(=O)R⁸, -N(R⁷)C(=O)R⁸, -C(=O)N(H)R⁸, -C(=O)NR⁸R⁷, R⁸-S(=O)-, R⁸-S(=O)₂-, -S(=O)(=NR⁷)R⁸;
- R³, R⁴ and R⁵: represent a hydrogen atom;
- R⁶: represents a group selected from:
-CH₂-(cyclopropyl) or -CH₂-(phenyl);
wherein said group is optionally substituted, one or more times, identically or differently, with a substituent selected from:
halo-, C₁-C₆-alkyl-, halo-C₁-C₆-alkyl-, halo-C₁-C₆-alkoxy-;
- R⁷: represents a hydrogen atom, a C₁-C₆-alkyl- or a C₃-C₆-cycloalkyl- group;
- R⁸: represents a hydrogen atom or a C₁-C₆-alkyl- or C₃-C₆-cycloalkyl- group, wherein said C₁-C₆-alkyl- or C₃-C₆-cycloalkyl- group is optionally substituted, one or more times, identically or differently, with a substituent selected from:
halo-, hydroxy-, -NHR⁷, -NR⁷R⁷, -N(C₁-C₃-alkyl)-C(=O)R⁷, -N(C₁-C₃-alkyl)-C(=O)OR⁷, C₁-C₃-alkyl-, R⁷-S(=O)₂-, C₁-C₃-alkoxy-, halo-C₁-C₃-alkoxy-;
- n: represents an integer of 0 or 1; and
- q: represents an integer of 1.

In another preferred embodiment, the invention relates to compounds of formula (I), wherein :
- R¹: represents a phenyl group
- which is substituted, one or more times, identically or differently, with a substituent selected from:
   -N(H)C(=O)R⁶, -C(=O)N(H)R⁶; and
- which is optionally substituted, one or more times, identically or differently, with a substituent selected from:
   halo-, C₁-C₆-alkyl-, C₁-C₆-alkoxy-;
- R²: represents a 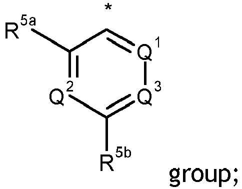 wherein * indicates the point of attachment of said group with the rest of the molecule;
Q¹ represents CH;
Q² represents CH;
Q³ represents CH or N;
R^{5a} represents a group selected from:
halo-, C₁-C₆-alkyl-,C₁-C₆-alkoxy-, halo-C₁-C₆-alkoxy-, (C₃-C₆-cycloalkyl)-(CH₂)ₙ-O-;
R^{5b} represents a group selected from:
halo-, cyano-, C₁-C₆-alkoxy-, -N(H)C(=O)R⁸, -N(R⁷)C(=O)R⁸, -C(=O)N(H)R⁸, -C(=O)NR⁸R⁷, R⁸-S(=O)-, R⁸-S(=O)₂-, -S(=O)(=NR⁷)R⁸;
- R³, R⁴ and R⁵: represent a hydrogen atom;
- R⁶: represents a group selected from:
-CH₂-(C₃-C₆-cycloalkyl) or -CH₂-aryl;
wherein said group is optionally substituted, one or more times, identically or differently, with a substituent selected from:
halo-, C₁-C₆-alkyl-, halo-C₁-C₆-alkyl-, halo-C₁-C₆-alkoxy-;
- R⁷: represents a hydrogen atom, a C₁-C₆-alkyl- or a C₃-C₆-cycloalkyl- group;
- R⁸: represents a hydrogen atom or a C₁-C₆-alkyl- or C₃-C₆-cycloalkyl- group,
wherein said C₁-C₆-alkyl- or C₃-C₆-cycloalkyl- group is optionally substituted, one or more times, identically or differently, with a substituent selected from:
halo-, hydroxy-, -NHR⁷, -NR⁷R⁷, -N(C₁-C₃-alkyl)-C(=O)R⁷, -N(C₁-C₃-alkyl)-C(=O)OR⁷, C₁-C₃-alkyl-, R⁷-S(=O)₂-, C₁-C₃-alkoxy-, halo-C₁-C₃-alkoxy-;
- n: represents an integer of 0 or 1; and
- q: represents an integer of 1.

It is to be understood that the present invention relates to any subcombination within any embodiment of the present invention of compounds of general formula (I), *supra.*

More particularly still, the present invention covers compounds of general formula (I) which are disclosed in the Example section of this text, *infra.*

In accordance with another aspect, the present invention covers methods of preparing compounds of the present invention, said methods comprising the steps as described in the Experimental Section herein.

In a preferred embodiment, the present invention relates to a method of preparing compounds of general formula (I) of the present invention, in which method an intermediate compound of general formula (5) : 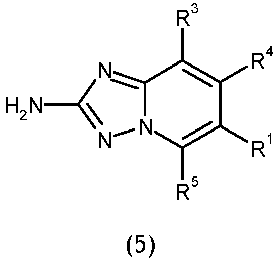 in which R¹, R³, R⁴, and R⁵ are as defined for the compounds of general formula (I), *supra,*
is allowed to react with an aryl halide of general formula (5a) :

R²-Y (5a)

in which R² is as defined for the compounds of general formula (I), *supra,* and Y represents a halogen atom,
thus providing a compound of general formula (I) :

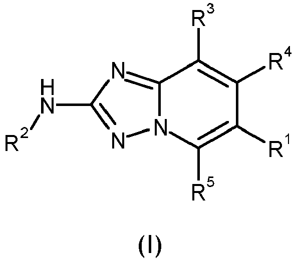

In another preferred embodiment, the present invention relates to a method of preparing compounds of general formula (I), *supra,* in which method an intermediate compound of general formula (7) : 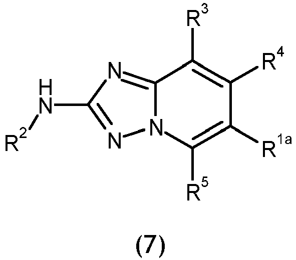 in which R², R³, R⁴, and R⁵ are as defined for the compounds of general formula (I), *supra,* and R^{1a} is an aryl group to which an -NH₂ substituent is bound, is allowed to react with a compound of general formula :

R^{1b}-X (7a)

in which R^{1b} is -C(=O)R⁶, -C(=O)NR⁶R⁷, -S(=O)R⁶, or -S(=O)₂R^{b}, (R⁶ and R⁷ being as defined for compounds of general formula (I), *supra*), and X is a suitable functional group (e.g. an -OH, -O-C₁-C₆-alkyl group, or a halogen atom), via which the R^{1b} of the R^{1b}-X compound (7a) can be coupled, via a coupling reaction, such as an amide coupling reaction for example, onto the -NH₂ substituent bound to the aryl group R^{1a} of compound (7), thereby replacing said X with said R^{1a},
thus providing a compound of general formula (I) :

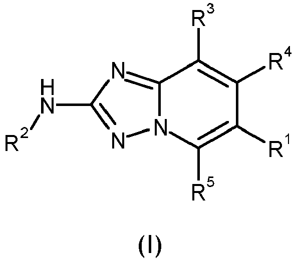

In accordance with a further aspect, the present invention covers intermediate compounds which are useful in the preparation of compounds of the present invention of general formula (I), particularly in the method described herein. The present invention covers :
compounds of general formula (7) : 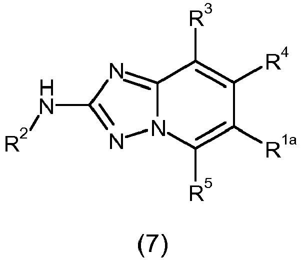 in which R², R³, R⁴, and R⁵ are as defined for the compound of general formula (I), *supra,* and R^{1a} is an aryl group to which an -NH₂ substituent is bound.

In accordance with yet another aspect, the present invention covers the use of an intermediate compound :
a) of general formula (5) : 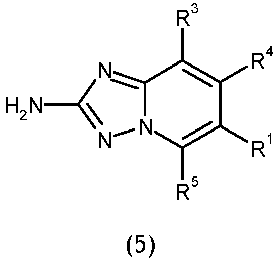 in which R¹, R³, R⁴, and R⁵ are as defined for the compound of general formula (I), *supra,* as defined in the claims,
   or
b) of general formula (7) : 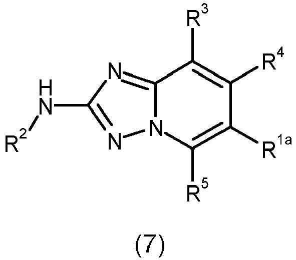 in which R², R³, R⁴, and R⁵ are as defined for the compound of general formula (I), *supra,* and R^{1a} is an aryl group to which an -NH₂ substituent is bound,
   for the preparation of a compound of general formula (I).

### EXPERIMENTAL SECTION

The following Table lists the abbreviations used in this paragraph, and in the Examples section. NMR peak forms are stated as they appear in the spectra, possible higher order effects have not been considered.

Names of compounds were generated using the Autonom 2000 add-in of ISIS/Draw [MDL Information Systems Inc. (Elsevier MDL)] or the ICS naming tool 12.01 of ACD labs. In some cases generally accepted names of commercially available reagents were used.

| **Abbreviation** | **Meaning** |
|---|---|
| Ac | acetyl |
| BINAP | 2,2'-bis(diphenylphosphino)-1,1'-binaphthyl |
| Boc | *tert*-butyloxycarbonyl |
| br | broad |
| Brett-Phos | 2-(dicyclohexylphosphino)-3,6-dimethoxy-2'-4'-6'-tri-i-propyl-1,1'-biphenyl |
| c- | cyclo- |
| D | doublet |
| Dd | doublet of doublets |
| DCM | dichloromethane |
| DMAP | *N,N*-dimethylpyridin-4-amine |
| DME | 1,2-dimethoxyethane |
| DIEA | *N,N*-diisopropylethylamine |
| DIPEA | *N,N*-diisopropylethylamine |
| DMF | *N,N*-dimethylformamide |
| DMSO | dimethyl sulfoxide |
| Dppf | 1,1'-bis(diphenylphosphino)ferrocene |
| Eq | equivalent |
| ESI | electrospray ionisation |
| h | hour or hours |
| hrs | hour or hours |
| HATU | *N*-[(dimethylamino)(3*H*-[1,2,3]triazolo[4,5-b]pyridin-3-yloxy)methylene]-N-methylmethanaminium hexafluorophosphate |
| Hünig Base | *N,N*-diisopropylethylamine |
| LiHMDS | lithium bis(trimethylsilyl)amide (alternative name: lithium hexamethyldisilazide) |
| M | multiplet |
| m.p. | melting point in °C |
| MS | mass spectrometry |
| MW | molecular weight |
| NaOtBu | sodium *tert*-butoxide; sodium 2-methylpropan-2-olate |
| NMP | *N*-methylpyrrolidinone |
| NMR | nuclear magnetic resonance spectroscopy: chemical shifts (δ) are given in ppm. |
| PdCl₂(PPh₃)₂ | dichlorobis(triphenylphosphine)palladium(II) |
| Pd(dba)₂ | bis-(dibenzylideneacetone)-palladium(0) complex |
| Pd₂(dba)₃ | tris-(dibenzylideneacetone)-dipalladium(0)-chloroform complex |
| Pd(dppf)Cl₂ | dichloro[1,1'-bis(diphenylphosphino)ferrocene]palladium(II) |
| Pd(dppf)Cl₂ CH₂Cl₂ | dichloro[1,1'-bis(diphenylphosphino)ferrocene]palladium(II) dichloromethane adduct |
| Pd-Brett-Phos-pre-cat | chloro[2-(dicyclohexylphosphino)-3,6-dimethoxy-2'-4'-6'-tri-i-propyl-1,1'-biphenyl][2-(2-aminoethyl)phenyl]palladium(II) |
| Pd-tBu-X-Phos-pre-cat | chloro(2-di-*tert*-butylphosphino-2',4',6'-tri-i-propyl-1,1'-biphenyl)[2-(2-aminoethyl)phenyl]palladium(II), |
| Pd-X-Phospre-cat | chloro(2-dicyclohexylphosphino-2',4',6'-tri-i-propyl-1,1'-biphenyl)[2-(2-aminoethyl)phenyl] palladium(II) methyl-tert-butylether adduct |
| PPh₃ | triphenylphosphine |
| P(oTol)₃ | tri-o-tolylphosphine |
| q | quartet |
| Quin | quintuplet |
| Rac | racemic |
| Rt | room temperature |
| r. t. | room temperature |
| RT | retention time in minutes |
| S | singlet |
| sept | septet |
| t | triplet |
| TBTU | *N*-[(1*H*-benzotriazol-1-yloxy)(dimethylamino)methylene]-*N-*methylmethanaminium tetrafluoroborate |
| TEA | triethylamine |
| TFA | trifluoroacetic acid |
| THF | tetrahydrofuran |
| Ts | para toluenesulfonyl; (tosyl) |
| UPLC | ultra performance liquid chromatography |
| X-Phos | 2-dicyclohexylphosphino-2',4',6'-triisopropylbiphenyl |

The schemes and procedures described below illustrate general synthetic routes to the compounds of general formula (I) of the invention and are not intended to be limiting. It is clear to the person skilled in the art that the order of transformations as exemplified in the Schemes can be modified in various ways. The order of transformations exemplified in the Schemes is therefore not intended to be limiting. In addition, interconversion of any of the substituents, R¹, R², R³, R⁴, R^{5a}, R^{5b}, R⁶ , R⁷ or R⁸ can be achieved before and/or after the exemplified transformations. These modifications can be such as the introduction of protecting groups, cleavage of protecting groups, reduction or oxidation of functional groups, halogenation, metallation, substitution or other reactions known to the person skilled in the art. These transformations include those which introduce a functionality which allows for further interconversion of substituents. Appropriate protecting groups and their introduction and cleavage are well-known to the person skilled in the art (see for example T.W. Greene and P.G.M. Wuts in Protective Groups in Organic Synthesis, 3rd edition, Wiley 1999). Specific examples are described in the subsequent paragraphs.

A first reaction scheme is outlined *infra* :

### Synthesis of compounds of general formula (I) of the present invention

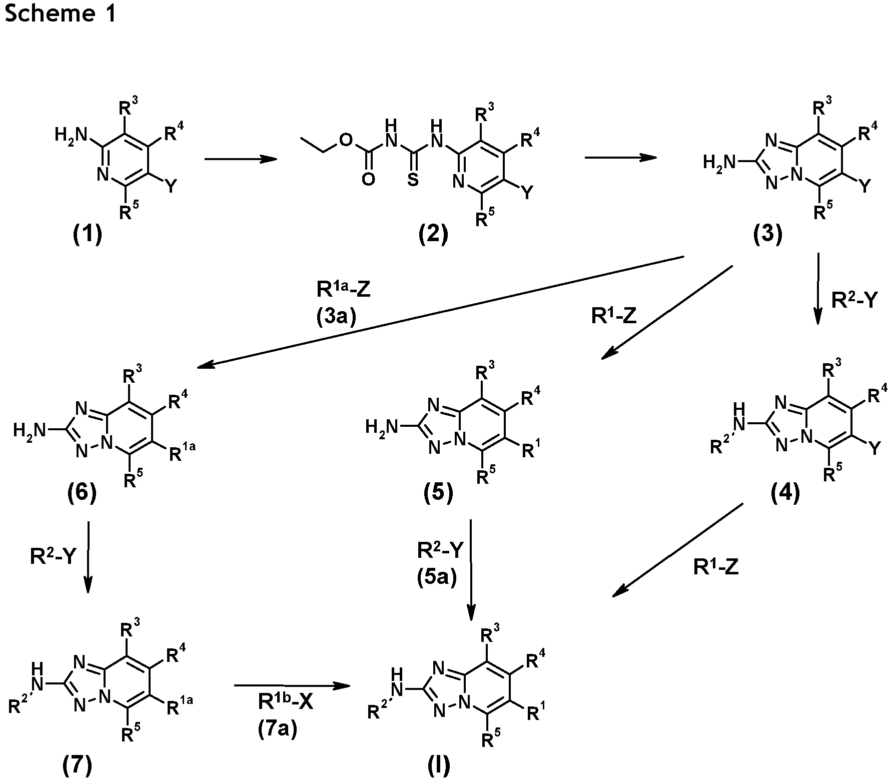

wherein R¹, R², R³, R⁴ and R⁵ are as defined for the compounds of formula (I), *supra,* Y is a halogen atom as defined *supra,* and Z represents a suitable functional group via which the R¹ of the R¹-Z compound can be coupled, by a coupling reaction, onto the Y-bearing carbon atom of a compound (4), thereby replacing said Y with said R¹ moiety. Many aryl halides of the formula R²-Y may be obtained commercially. Reagents of the general structure R^{1a}-Z and R¹-Z can for example be aryl boronic acids or aryl boronic esters. Many such reagents of the general structures R^{1a}-Z and R¹-Z are also commercially available. Reagents of the general structures R^{1a}-Z and R¹-Z can be prepared from aryl halides [see for example K.L. Billingslay, T.E. Barde, S.L Buchwald, Angew. Chem. 2007, 119, 5455 or T.Graening, Nachrichten aus der Chemie, Jan 2009, 57, 34].

R^{1a} can be converted to R¹ in one or several steps. Typically, R^{1a} can be a protected aryl-amine, especially -aryl-NH-Boc, or an aryl-carboxylic acid, [-aryl-C(O)OH] or an -aryl-carboxylic acid ester [-aryl-C(O)O-alkyl]. For example, when R^{1a} is an aryl group to which an -NH₂ substituent is bound, it may be allowed to react with a compound of general formula R^{1b}-X (7a), in which R^{1b} is -C(=O)R⁶, -C(=O)NR⁶R⁷, -S(=O)R⁶, or -S(=O)₂R⁶, (R⁶ and R⁷ being as defined as for compounds of general formula (I) of the present invention as defined in the claims), and X is a suitable functional group (*e.g*. an -OH, -O-C₁-C₆-alkyl group, or a halogen atom), via which the R^{1b} of the R^{1b}-X compound (7a) can be coupled, via a coupling reaction, such as an amide coupling reaction for example, onto the -NH₂ substituent bound to the aryl group R^{1a} of compound (7), thereby replacing said X with said R^{1a}, thus providing a compound of general formula (I) of the present invention.

Compounds of general formula (I) can be synthesised according to the procedures depicted in Scheme 1.

The person skilled in the art will recognise that there are many precedented methods for synthesising suitable 3,4,6-substituted 5-halo-pyridin-2-ylamines of general formula (1); some 3,4,6-substituted 5-halo-pyridin-2-ylamines may be obtained commercially.

A suitably substituted 5-halo-pyridin-2-ylamine intermediate of general formula (1) is converted to the corresponding intermediate of general formula (2) by reaction with a suitable oxycarbonylisothiocyanat, such as for example ethoxycarbonylisothiocyanat at temperatures ranging from room temperature to the boiling point of the solvent, preferably room temperature [see for example M. Nettekoven, B. Pullmann, S. Schmitt, Synthesis 2003, 1643 - 1652].

Intermediates of general formula (2) may be converted to 6-Halo-[1,2,4]triazolo[1,5-a]pyridin-2-ylamine intermediates of general formula (3) by reaction with a suitable reagent, for example hydroxylamine hydrochloride, in presence of a suitable base, such as, for example DIPEA in a suitable solvent system, such as, for example, methanol, ethanol, 1-propanol, 2-propanol or mixtures of these solvents at elevated temperatures, e.g. 60°C. [see for example M. Nettekoven, B. Pullmann, S. Schmitt, Synthesis 2003, 1643 - 1652].

Intermediates of general formula (3) can reacted with suitable aryl halides, preferably aryl bromides, in the presence of a suitable base, such as, for example NaOtBu or cesium carbonate, and a suitable catalyst/ligand system, such as for example Pd₂(dba)₃/rac-BINAP in a suitable solvent such as THF, toluene, DME, or NMP, or mixtures of these solvents at temperatures ranging from room temperature to the 200°C, to yield compounds of general formula (4). The person skilled in the art will recognise that the appropriate choice of reaction conditions, such as temperature, choice of solvent and catalyst system is critical for preferred derivatization at the amino group of intermediates of general formula (3). Intermediates of general formula (4) can be converted to compounds of general formula (I) by reaction with a suitable reagent, like for example a boronic acid derivative in the presence of a suitable catalyst system, like for example Pd(OAc)₂ and P(oTol)₃, or r PdCl₂(PPh₃)₂ and PPh₃ and a suitable base, like for example aqueous potassium carbonate in a suitable solvent, like for example THF, DME, ethanol or 1-propanol or mixtures of these solvents at temperatures ranging from room temperature to 200°C, prefereably the boiling point of the used solvent.

In an alternative route for the synthesis of compounds of general formula (I), Intermediates of general formula (3) can be reacted reacted with a suitable reagent, like for example a boronic acid derivative in the presence of a suitable catalyst system, like for example Pd(OAc)₂ and P(oTol)₃, or r PdCl₂(PPh₃)₂ and PPh₃ and a suitable base, like for example aqueous potassium carbonate in a suitable solvent, like for example THF, DME, ethanol or 1-propanol or mixtures of these solvents at temperatures ranging from room temperature to 200°C, prefereably the boiling point of the used solvent to furnish intermediates of the general formula (5).

Intermediates of general formula (5) can be converted to compounds of general formula (I) by reaction with with suitable aryl halides, of formula (5a) as defined herein, preferably aryl bromides, or aryl trifluoromethylsulphonates or aryl nonafluorobutylsulphonates, for example, optionally in the presence of a suitable base, such as, for example NaOtBu or cesium carbonate, and a suitable catalyst/ligand system, such as for example Pd₂(dba)₃/rac-BINAP in a suitable solvent such as for example THF, toluene, DME, or NMP, or mixtures of these solvents at temperatures ranging from room temperature to the 200°C.

Also as depicted in Scheme 1, is a further alternative route for the synthesis of compounds of general formula (I): Intermediates of general formula (3) can be converted to intermediates of general formula (6) by a coupling reaction as described supra for synthesis of intermediate of general formula (5), thereby replacing said Y with said R^{1a} moiety.

Intermediates of general formula (6) can then be converted to intermediates of general formula (7) by a coupling reaction as described supra for synthesis of of intermediates of general formula (4).

Intermediates of general formula (7) can then be converted to compounds of general formula (I) by one or more further transformations. These can be modifications such as cleavage of protecting groups, reduction or oxidation of functional groups, halogenation, metallation, substitution or other reactions known to the person skilled in the art, for example the formation of an amide bond, the formation of a urea, or the formation of a sulfonamide.

Each of the Schemes 2 - 7, *infra*, illustrates specific transformations for the synthesis of some selected compounds according to general formula (I).

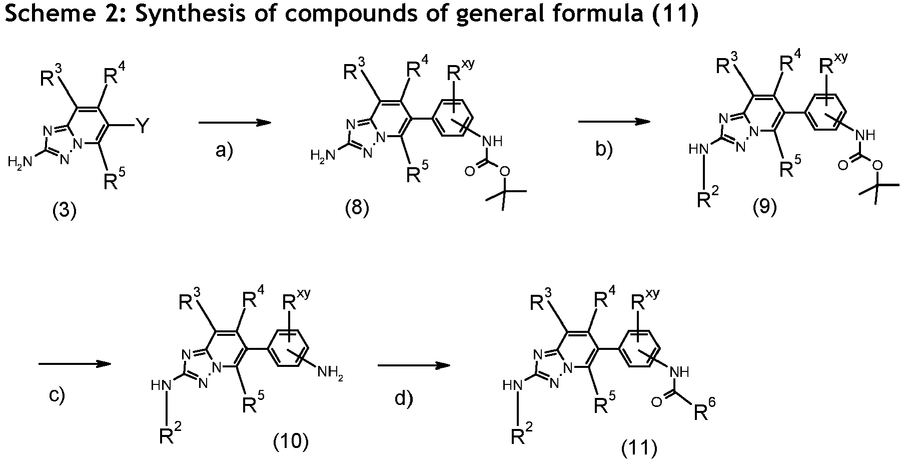

Scheme 2: Synthesis of compounds of general formula (11), wherein R², R³, R⁴, R⁵ and R⁶ are as defined for the compounds of general formula (I), *supra.* Y is a halogen as defined in the definitions. R^{xy} is halogen, hydroxy or C₁-C₆-alkyl. a) coupling reaction using conditions as described *supra* for synthesis of intermediates of general formula (5); b) coupling reaction using conditions as described *supra* for synthesis of intermediates of general formula (4); c) removal of a Boc-protecting group using conditions known to the person skilled in the art (see for example T.W. Greene and P.G.M. Wuts in Protective Groups in Organic Synthesis, 3rd edition, Wiley 1999); d) conditions for the formation of an amide bond, e.g. using coupling reagents like HATU or TBTU and a base like potassium carbonate or DIPEA in an inert solvent like THF, DMF, DCM or NMP.

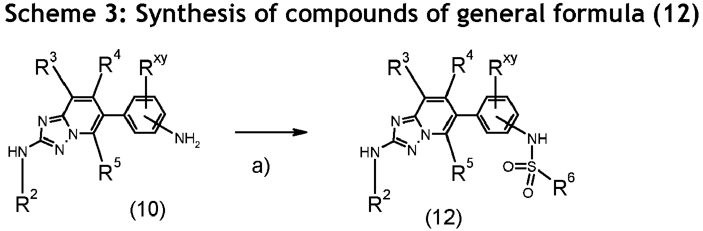

Scheme 3: Synthesis of compounds of general formula (12), wherein R², R³, R⁴, R⁵ and R⁶ are as defined for the compounds of general formula (I), *supra.* R^{xy} is halogen, hydroxy or C₁-C₆-alkyl. a) conditions for the formation of a sulfonamide, e.g. using a sulfonyl chloride and a base like DIPEA in an inert solvent like for example THF, DMF, DCM or NMP at temperatures ranging from room temperature to the 70°C.

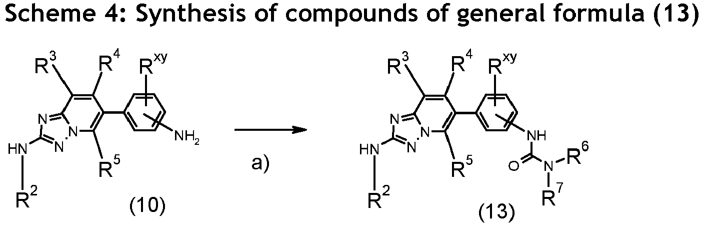

Scheme 4: Synthesis of compounds of general formula (13), wherein R², R³, R⁴, R⁵, R⁶ and R⁷ are as defined for the compounds of general formula (I), *supra.* R^{xy} is halogen, hydroxy or C₁-C₆-alkyl. a) Conditions for the formation of an urea, e.g. using an isocyanate in an inert solvent like for example THF, DMF, DCM or NMP at temperatures ranging from room temperature to 70°C. Alternatively, a two step procedure which involves reaction of 4-Nitrophenyl chloroformate in an inert solvent like for example THF or DCM and a base like pyridine at temperatures ranging from 0°C to room temperature, followed by reaction with an amine in an inert solvent like THF or DCM at temperatures ranging from 0°C to 40°C, can be used.

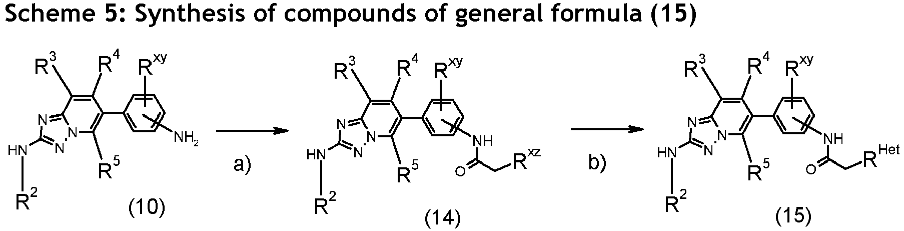

Scheme 5: Synthesis of compounds of general formula (15), wherein R², R³, R⁴, and R⁵ are as defined for the compounds of general formula (I), *supra.* R^{xy} is halogen, hydroxy or C₁-C₆-alkyl. R^{xz} is a leaving group, e.g. a halogen. R^{Het} is 3-to 10-membered heterocyclyl, as defined *supra.* a) Conditions for the formation of an amide bond, e.g. using coupling reagents like for example HATU or TBTU and a base like for example potassium carbonate or DIPEA in an inert solvent like for example THF, DMF, DCM or NMP. Alternatively, an acid chloride and a base like for example pyridine can be used in an inert solvent like for example THF or DCM. b) Reaction with a heterocyclic amine, like e.g. piperidine in a polar solvent like for example DMF or NMP using a base like for example potassium carbonate and optionally using a catalytic ammount of potassium iodide. 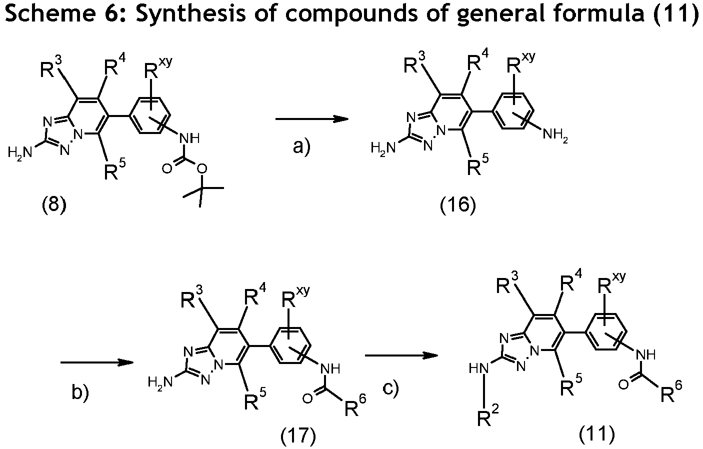 Scheme 6: Synthesis of compounds of general formula (11), wherein R², R³, R⁴, R⁵ and R⁶ are as defined for the compounds of general formula (I), *supra.* R^{xy} is halogen, hydroxy or C₁-C₆-alkyl. a) removal of a Boc-protecting group using conditions known to the person skilled in the art (see for example T.W. Greene and P.G.M. Wuts in Protective Groups in Organic Synthesis, 3rd edition, Wiley 1999); b) conditions for the formation of an amide bond, e.g. using coupling reagents like for example HATU or TBTU and a base like for example potassium carbonate or DIPEA in an inert solvent like for example THF, DMF, DCM or NMP; c) coupling reaction using conditions as described supra for synthesis of intermediates of general formula (4).

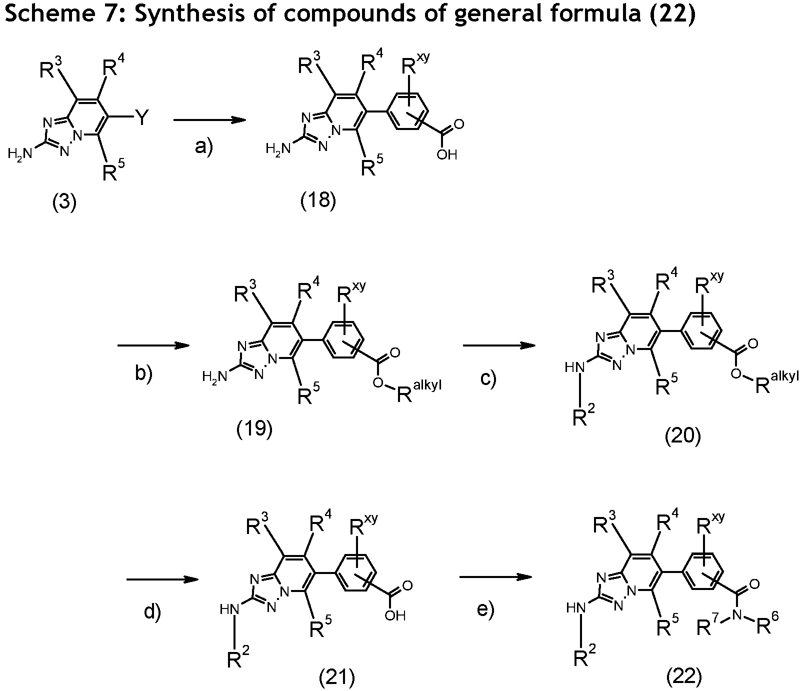

Scheme 7: Synthesis of compounds of general formula (21), wherein R², R³, R⁴, R⁵, R⁶ and R⁷ are as defined for the compounds of general formula (I), *supra.* R^{xy} is halogen, hydroxy or C₁-C₆-alkyl. R^{alkyl} is C₁-C₆-alkyl. a) coupling reaction using conditions as described supra for synthesis of intermediates of general formula (5); b) formation of an ester using conditions known to the person skilled in the art (see for example T.W. Greene and P.G.M. Wuts in Protective Groups in Organic Synthesis, 3rd edition, Wiley 1999), e.g. using thionyl chloride in the appropriate alcohol at temperatures ranging from room temperature to 100°C; c) coupling reaction using conditions as described supra for synthesis of intermediates of general formula (4); d) hydrolysis for an ester using conditions known to the person skilled in the art (see for example T.W. Greene and P.G.M. Wuts in Protective Groups in Organic Synthesis, 3rd edition, Wiley 1999), e.g. sodium hydroxide in a mixture of THF, methanol an water at r.t.; e) conditions for the formation of an amide bond, e.g. using coupling reagents like for example HATU or TBTU and a base like for example potassium carbonate or DIPEA in an inert solvent like for example THF, DMF, DCM or NMP.

The compounds and intermediates produced according to the methods of the invention may require purification. Purification of organic compounds is well known to the person skilled in the art and there may be several ways of purifying the same compound. In some cases, no purification may be necessary. In some cases, the compounds may be purified by crystallisation. In some cases, impurities may be stirred out using a suitable solvent. In some cases, the compounds may be purified by chromatography, particularly flash chromatography, using for example pre-packed silica gel cartridges, e.g. from Separtis such as Isolute® Flash silica gel (silica gel chromatography) or Isolute® Flash NH2 silica gel (aminophase-silica-gel chromatography) in combination with a suitable chromatographic system such as a Flashmaster II (Separtis) or an Isolera system (Biotage) and eluents such as, for example, gradients of hexane/ethyl acetate or DCM/methanol. In some cases, the compounds may be purified by preparative HPLC using, for example, a Waters autopurifier equipped with a diode array detector and/or on-line electrospray ionisation mass spectrometer in combination with a suitable pre-packed reverse phase column and eluants such as, for example, gradients of water and acetonitrile which may contain additives such as trifluoroacetic acid, formic acid or aqueous ammonia.

Analytical UPLC-MS was performed as follows:
Method A: System: UPLC Acquity (Waters) with PDA Detector und Waters ZQ mass spectrometer; Column: Acquity BEH C18 1.7 µm 2.1x50 mm; Temperature: 60° C; Solvent A: Water + 0.1% formic acid; Solvent B: acetonitrile; Gradient: 99 % A → 1 % A (1.6 min) → 1 % A (0.4 min) ; Flow: 0.8 mL/min; Injection Volume: 1.0 µL (0.1mg-1mg/mL sample concentration); Detection: PDA scan range 210-400 nm - Fixed and ESI (+),scan range 170-800 m/z
Names of compounds were generated using ACD/Name Batch ver. 12.00 or ACD/Name Batch ver. 12.01. Names of compounds in table format were generated using ACD/Name Batch ver. 12.00.

### Synthesis of Intermediate compounds

### Intermediate Example Int1.1

### ethyl [(5-bromopyridin-2-yl)carbamothioyl]carbamate

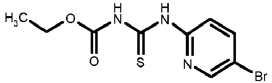

Ethoxycarbonylisothiocyanat (16.7 g) was added to a stirred solution of 2-amino-5-brompyridine (20 g) in dioxane (200 mL). The mixture was stirred for 2h at r.t. A white solid precipitated. Hexane (20 mL) was added and the white solid was collected by filtration.

Yield: 30.4 g of the title compound.

¹H-NMR (300MHz, DMSO-d₆): δ [ppm]= 1.22 (t, 3H), 4.19 (q, 2H), 8.08 (dd, 1H), 8.49 (d, 1H), 8.57 (br. d, 1H), 11.37 - 12.35 (m, 2H).

### Intermediate Example Int1.2

### 6-bromo[1,2,4]triazolo[1,5-a]pyridin-2-amine

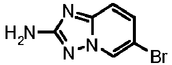

Hydroxylammoniumchlorid (39.8 g) was suspended in methanol (200 mL) and ethanol (190 mL) and Hünig Base (59 mL) was added at r.t. The mixture was heated to 60°C, **Int1.1** (30 g) was added portionwise, and the mixture was stirred at 60°C for 2h. The solvent was removed in vacuum and water (150 mL) was added. A solid was collected by filtration and was washed with water and dried in vacuum.

Yield: 19.3 g of the title compound.

¹H-NMR (300MHz, DMSO-d₆): δ [ppm]= 6.10 (s, 2H), 7.28 (dd, 1H), 7.51 (dd, 1H), 8.88 (dd, 1H).

### Intermediate Example Int2.1

### 4-(2-amino-[1,2,4]triazolo[1,5-a]pyridin-6-yl)-N-cyclopropyl-benzamide

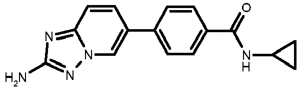

To a stirred solution of 426 mg (2 mmol) **Int1.2** in THF (30 mL) was added 615 mg (3 mmol, 1.5 eq) [4-[(cyclopropylamino)carbonyl]phenyl]-boronic acid, 163 mg (0.2 mmol, 0.1 eq) Pd(dppf)Cl₂ and 6 mL potassium carbonate solution (1M in water, 3 eq) at r.t. in a microwave reactor. The solution was heated at 150 °C for 120 min under microwave irradiation. After cooling, the solution was filtered and purified by preparative reverse phase HPLC to give 49.9 mg (8.5 %) of the title compound.

UPLC-MS: RT = 0.69 min; m/z (ES+) 294.3 [MH⁺]; required MW = 293.3.

### Intermediate Example Int3.1

### tert-butyl [4-(2-amino[1,2,4]triazolo[1,5-a]pyridin-6-yl)phenyl]carbamate

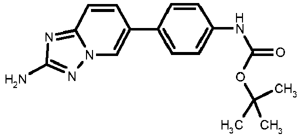

To a stirred solution of Int1.2 (5.82 g) in 1-propanol (400 mL) was added 2M potassium carbonate solution (41 mL), {4-[(tert-butoxycarbonyl) amino] phenyl} boronic acid (8.6 g), triphenylphosphine (150 mg) and PdCl₂(PPh₃)₂ (1.9 g). The mixture was heated to reflux for 4h, the solvent was removed in vacuum, water (150 mL) was added and the mixture was extracted with ethyl acetate (500 mL). The organic phase was dried (sodium sulfate), filtered through Celite and the solvent was removed in vacuum. The residue was titurated with DCM to give the title compound as a white solid. Yield: 7.2 g. ¹H-NMR (400MHz, DMSO-d₆): δ [ppm]= 1.37 - 1.55 (m, 9H), 5.99 (s, 2H), 7.36 (dd, 1 H), 7.48 - 7.55 (m, 2H), 7.55 - 7.62 (m, 2H), 7.69 (dd, 1 H), 8.78 (dd, 1 H), 9.44 (s, 1H).

### Intermediate Example Int3.2

### 6-(4-aminophenyl)[1,2,4]triazolo[1,5-a]pyridin-2-amine

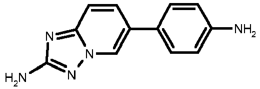

To a stirred suspension of **Int3.1** (7.05 g) in DCM (210 mL) was added TFA (66 mL). The mixture was stirred at r.t. for 1 h. The mixture was concentrated in vacuum. A saturated solution of potassium carbonate was added, until pH 10 was reached and the mixture was extracted for three times with DCM and methanol (10:1). The organic phase was dried (sodium sulfate) and the solvent was removed in vacuum to give 4.6 g of the title compound.

¹H-NMR (300MHz, DMSO-d₆): δ [ppm]= 5.26 (s, 2H), 5.95 (s, 2H), 6.64 (d, 2H), 7.29 - 7.45 (m, 3H), 7.64 (dd, 1 H), 8.60 - 8.70 (m, 1 H).

### Intermediate Example Int3.3

### N-[4-(2-amino[1,2,4]triazolo[1,5-a]pyridin-6-yl)phenyl]-2-phenylacetamide

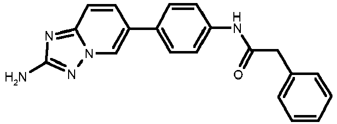

To a stirred suspension of **Int3.2** (1.09 g) in DMF (45 mL) was added potassium carbonate (3.3 g), phenylacetic acid (791 mg) and TBTU (3.1 g). The mixture was stirred at r.t. for 24 h. Water was added and the mixture was extracted with ethyl acetate. The organic phase was washed with saturated sodium chloride solution, dried (sodium sulfate) and the solvent was removed in vacuum. Silica gel chromatography gave 870 mg of the title compound.

¹H-NMR (400MHz, DMSO-d₆): δ [ppm]= 3.67 (s, 2H), 6.04 (s, 2H), 7.22 - 7.28 (m, 1H), 7.30 - 7.37 (m, 4H), 7.40 (dd, 1H), 7.63 - 7.72 (m, 4H), 7.74 (dd, 1H), 8.84 (dd, 1H), 10.31 (s, 1H).

### Intermediate Example Int3.4

### N-[4-(2-amino[1,2,4]triazolo[1,5-a]pyridin-6-yl)phenyl]-2-(4-fluorophenyl)-acetamide

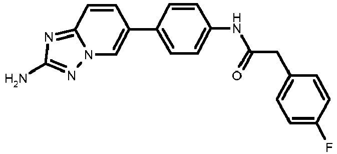

To a stirred solution of **Int3.2** (8.80 g) in THF (475 mL) was added Hünig Base (7.4 mL), (4-fluorophenyl)acetic acid (6.62 g), and HATU (16.3 g). The mixture was stirred at r.t. for 16 h. Water was added and the mixture was stirred at r.t. for 1 h. The precipitated solid was collected by filtration, was washed with methanol and diethyl ether and was dried in vacuum to give 10.6 g of the title compound.

¹H-NMR (300MHz, DMSO-d₆): δ [ppm]= 3.63 (s, 2H), 6.01 (s, 2H), 7.03 - 7.19 (m, 2H), 7.27 - 7.41 (m, 3H), 7.58 - 7.79 (m, 5H), 8.80 (dd, 1H), 10.26 (s, 1H).

### Intermediate Example Int3.5

### N-[4-(2-amino[1,2,4]triazolo[1,5-a]pyridin-6-yl)phenyl]-2-cyclopropyl-acetamide

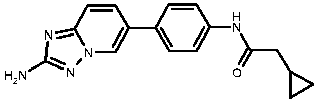

To a stirred solution of **Int3.2** (3.0 g) in THF (95 mL) was added Hünig Base (2.75 mL), cyclopropylacetic acid (1.65 g) and HATU (6.1 g). The mixture was stirred at r.t. for 16 h. Water was added and the mixture was stirred at r.t. for 20 minutes. The precipitated solid was collected by filtration, was washed with water and methanol and was dried in vacuum to give 3.08 g of the title compound.

¹H-NMR (300MHz, DMSO-d₆): δ [ppm]= 0.12 - 0.21 (m, 2H), 0.39 - 0.50 (m, 2H), 0.93 - 1.15 (m, 1H), 2.19 (d, 2H), 6.00 (br. s, 2H), 7.37 (d, 1H), 7.56 - 7.78 (m, 5H), 8.80 (d, 1 H), 9.88 (s, 1 H).

### Intermediate Example Int3.6

### N-[4-(2-amino[1,2,4]triazolo[1,5-a]pyridin-6-yl)phenyl]-2-(2,4-difluorophenyl)acetamide

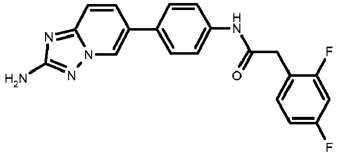

To a stirred solution of **Int3.2** (1.50 g) in DMF (135 mL) was added potassium carbonate (4.6 g), (2,4-difluorophenyl)acetic acid (2.29 g), and HATU (5.06 g). The mixture was stirred at r.t. for 72 h. Water was added and the mixture was stirred at room temperature for 1 h. The precipitated solid was collected by filtration, was washed with methanol and diethyl ether and was dried in vacuum. Aminophase-silica-gel chromatography followed by preparative reverse phase HPLC gave 692 mg of the title compound.

¹H-NMR (300MHz, DMSO-d₆): δ [ppm]= 3.71 (s, 2H), 6.00 (s, 2H), 7.03 (td, 1H), 7.19 (td, 1H), 7.31 - 7.50 (m, 2H), 7.57 - 7.77 (m, 5H), 8.81 (d, 1H), 10.29 (s, 1 H).

Starting with **Int3.2,** the intermediates **Int3.7** and **Int3.8** were prepared analogously to the procedures described above.

### Intermediate Example Int3.7.

### N-[4-(2-amino[1,2,4]triazolo[1,5-a]pyridin-6-yl)phenyl]-2-(4-fluoro-3-methylphenyl)acetamide

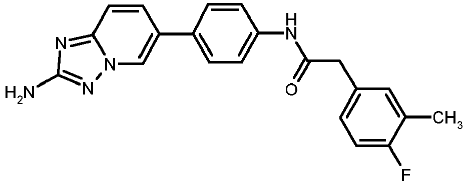

### Intermediate Example Int3.8.

### N-[4-(2-amino[1,2,4]triazolo[1,5-a]pyridin-6-yl)phenyl]-2-(4-chlorophenyl)-acetamide

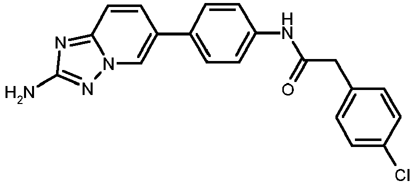

### Intermediate Example Int4.1

### 3-(2-amino[1,2,4]triazolo[1,5-a]pyridin-6-yl)benzoic acid

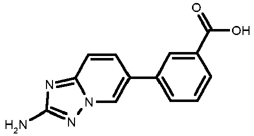

To a stirred solution of **Int1.2** (7.0 g) in 1-propanol (480 mL) was added 2M potassium carbonate solution (48 mL), 3-carboxyphenyl-boronic acid (10.7 g), triphenylphosphine (177 mg) and PdCl₂(PPh₃)₂ (2.26 g). The mixture was heated to reflux for 15h. Water (1000 mL) was added and the mixture was washed with ethyl acetate (1000 mL). A 4N solution of hydrochloric acid was added to the aqueous phase until pH 5 was reached. A white solid percipitated, was collected by filtration, washed with water and ethanol and dried in vacuum. Yield: 7.3 g of the title compound.

¹H-NMR (300MHz, DMSO-d₆): δ [ppm]= 6.11 (br. s., 2H), 7.45 (d, 1H), 7.56 - 7.68 (m, 1H), 7.79 (dd, 1H), 7.90 - 8.04 (m, 2H), 8.21 (s, 1H), 8.88 - 9.07 (m, 1H), 13.13 (br. s., 1 H).

### Intermediate Example Int5.1

### 4-(2-amino[1,2,4]triazolo[1,5-a]pyridin-6-yl)benzoic acid

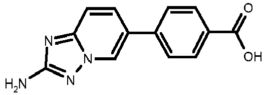

To a stirred solution of **Int1.2** (10.0 g) in 1-propanol (470 mL) was added 2M potassium carbonate solution (70 mL), 4-carboxyphenylboronic acid (10.3 g), triphenylphosphine (1.23 g) and PdCl₂(PPh₃)₂ (3.30 g). The mixture was heated to reflux for 1 h. Water (1.0 L) was added and the mixture was extracted with ethyl acetate (2 x 400 mL). 1 N hydrochloric acid was added to the aqueous phase until pH 5 was reached. The precipitated solid was collected by filtration, was washed with water and ethanol and was dried in vacuum to give 9.55 g of the title compound.

¹H-NMR (400MHz, DMSO-d₆): δ [ppm]= 6.12 (br. s., 2H), 7.42 (d, 1H), 7.76 - 7.87 (m, 3H), 7.98 (d, 2H), 8.98 (d, 1 H), 12.99 (br. s., 1 H).

### Intermediate Example Int5.2

### 4-(2-amino[1,2,4]triazolo[1,5-a]pyridin-6-yl)-N-(4-fluorobenzyl)benzamide

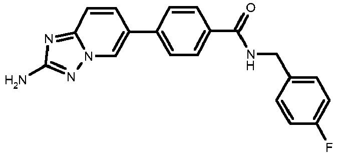

A suspension of Int5.1 (1.0 g) in DMSO (20 mL) and DMF (20 mL) was stirred at 50°C for 2h. At 50°C to the stirred suspension potassium carbonate (2.72 g), 1-(4-fluorophenyl)methanamine (0.66 g) and TBTU (1.77 g) was added. The mixture was stirred at 50°C for 1 h. Water (600 mL) was added and the mixture was stirred at room temperature for 20 minutes. The precipitated solid was collected by filtration, was washed with water and ethanol and hexane and was dried in vacuum to give 1.29 g of the title compound.

¹H-NMR (400MHz, DMSO-d₆): δ [ppm]= 4.44 (d, 2H), 6.08 (s, 2H), 7.08 - 7.16 (m, 2H), 7.34 (dd, 2H), 7.41 (dd, 1H), 7.78 - 7.86 (m, 3H), 7.97 (d, 2H), 8.91 - 8.99 (m, 1H), 9.16 (t, 1H).

### Intermediate Example Int5.3

### 4-(2-amino[1,2,4]triazolo[1,5-a]pyridin-6-yl)-N-benzylbenzamide

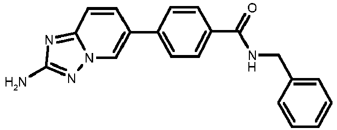

To a stirred solution of **Int5.1** (4.0 g) in 1-propanol (280 mL) was added 2M potassium carbonate solution (28 mL), [4-(benzylcarbamoyl)phenyl]boronic acid (6.2 g), triphenylphosphine (0.25 g) and PdCl₂(PPh₃)₂ (0.66 g). The mixture was heated to reflux for 1 h. The mixture was concentrated in vacuum, water (100 mL) and ethyl acetate (100 mL) was added and the mixture was stirred at r.t. for 15 minutes. The precipitated solid was collected by filtration, was washed with water and ethanol and was dried in vacuum to give 6.09 g of the title compound.

¹H-NMR (300MHz, DMSO-d₆): δ [ppm]= 4.47 (d, 2H), 6.08 (s, 2H), 7.16 - 7.25 (m, 1H), 7.26 - 7.33 (m, 4H), 7.41 (dd, 1H), 7.75 - 8.02 (m, 5H), 8.97 (dd, 1H), 9.10 (t, 1H).

### Intermediate Example Int5.4

### 4-(2-amino[1,2,4]triazolo[1,5-a]pyridin-6-yl)-N-(cyclopropylmethyl)benzamide

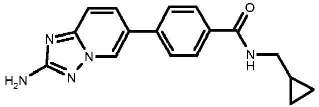

To a stirred suspension of **Int5.1** (3.0 g) in THF (80 mL) was added Hünig Base (2.42 mL), 1-cyclopropylmethanamine (1.03 g) and TBTU (4.55 g). The mixture was stirred at r.t. for 72 h. Water was added and the mixture was stirred at r.t. for 20 minutes. The precipitated solid was collected by filtration, was washed with water and methanol and was dried in vacuum to give 1.45 g of the title compound.

¹H-NMR (300MHz, DMSO-d₆): δ [ppm]= 0.14 - 0.26 (m, 2H), 0.32 - 0.47 (m, 2H), 0.91 - 1.10 (m, 1H), 3.13 (t, 2H), 6.02 - 6.13 (m, 2H), 7.41 (d, 1H), 7.76 - 7.85 (m, 3H), 7.87 - 7.99 (m, 2H), 8.59 (t, 1 H), 8.96 (d, 1 H).

### Intermediate Example Int5.5

### 4-(2-amino[1,2,4]triazolo[1,5-a]pyridin-6-yl)-N-(cyclopentylmethyl)benzamide

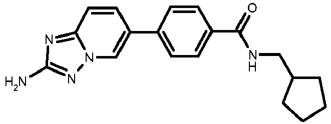

To a stirred suspension of **Int5.1** (5.0 g) in DMF (100 mL) was added potassium carbonate (13.6 g), 1-cyclopentylmethanamine hydrochloride (5.4 g) and HATU (12.7 g). The mixture was stirred at r.t. for 16 h. The mixture was concentrated in vacuum. Water was added and the reaction mixture was extracted with a mixture of DCM and methanol (10:1). The organic phase was dried (sodium sulfate) and the solvent was removed in vacuum. Silica gel chromatography gave a solid that was recrystallized from ethyl acetate. Yield: 3.9 g of the title compound.

¹H-NMR (300MHz, DMSO-d₆): δ [ppm]= 1.22 (dd, 2H), 1.36 - 1.74 (m, 6H), 2.03 - 2.21 (m, 1H), 3.17 (t, 2H), 6.08 (s, 2H), 7.41 (d, 1H), 7.71 - 7.96 (m, 5H), 8.51 (t, 1H), 8.96 (s, 1H).

### Intermediate Example Int5.6

### 4-(2-amino[1,2,4]triazolo[1,5-a]pyridin-6-yl)-N-(2,4-difluorobenzyl)benzamide

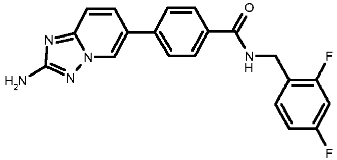

Starting with **Int5.1** and 2,4-difluorobenzylamine, **intermediate example Int5.6** was prepared analogously to the procedure for the preparation of **intermediate example Int5.5.**

¹H-NMR (400 MHz, DMSO-d₆): δ [ppm] = 4.50 (d, 13H), 6.12 (s, 2H), 7.07 (td, 1 H), 7.19 - 7.28 (m, 1 H), 7.39 - 7.47 (m, 2H), 7.82 - 7.90 (m, 3H), 7.99 (d, 2H), 9.01 (d, 1H), 9.11 (t, 1H).

### Intermediate Example Int5.7

### 4-(2-amino[1,2,4]triazolo[1,5-a]pyridin-6-yl)-N-(3,4-difluorobenzyl)benzamide

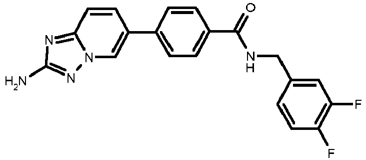

Starting with **Int5.1** and 3,4-difluorobenzylamine, **intermediate example Int5.7** was prepared analogously to the procedure for the preparation of

### intermediate example Int5.5.

¹H-NMR (400 MHz, DMSO-d₆): δ [ppm] = 4.48 (d, 2H), 6.08 - 6.14 (m, 2H), 7.18 (ddd, 1H), 7.34 - 7.48 (m, 3H), 7.81 - 7.92 (m, 3H), 7.99 (d, 2H), 9.01 (d, 1H), 9.16 (t, 1H).

### Intermediate Example Int5.8

### 4-(2-amino[1,2,4]triazolo[1,5-a]pyridin-6-yl)-N-(3-fluorobenzyl)benzamide

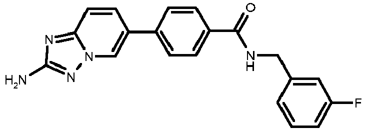

Starting with **Int5.1** and 3-fluorobenzylamine, **intermediate example Int5.8** was prepared analogously to the procedure for the preparation of

### intermediate example Int5.5.

¹H-NMR (400 MHz, DMSO-d₆): δ [ppm] = 4.51 (d, 6H), 6.12 (s, 2H), 7.07 (t, 1H), 7.11 - 7.22 (m, 2H), 7.33 - 7.42 (m, 1 H), 7.45 (d, 1 H), 7.81 - 7.92 (m, 3H), 8.03 (d, 2H), 9.01 (s, 1 H), 9.29 (t, 1 H).

Starting with **Int5.1** the intermediates **Int5.9** and **Int5.10** were prepared analogously to the procedures described above.

### Intermediate Example Int5.9.

### 4-(2-amino[1,2,4]triazolo[1,5-a]]pyridin-6-yl)-N-(4-chlorobenzyl)benzamide

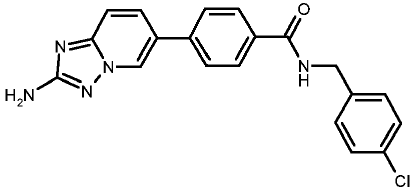

### Intermediate Example Int5.10.

### 4-(2-amino[1,2,4]triazolo[1,5-a]pyridin-6-yl)-N-(4-methylbenzyl)benzamide

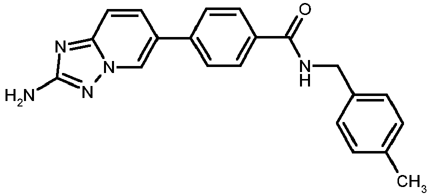

### Intermediate Example Int6.1

### 4-(2-amino[1,2,4]triazolo[1,5-a]pyridin-6-yl)-2-fluorobenzoic acid

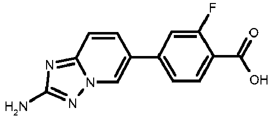

To a stirred solution of **Int1.2** (4.45 g) in 1-propanol (150 mL) was added 2M potassium carbonate solution (31 mL), 4-carboxy-3-fluorophenylboronic acid (3.92 g), triphenylphosphine (0.55 g) and PdCl₂(PPh₃)₂ (1.50 g). The mixture was heated to reflux for 2 h. Water (800 mL) was added and the mixture was extracted with ethyl acetate (2 x 500 mL). 1 N hydrochloric acid was added to the aqueous phase until pH 3 was reached. The precipitated solid was collected by filtration, was washed with water, ethanol and ether and was dried in vacuum to give 3.10 g of the title compound.

¹H-NMR (300MHz, DMSO-d₆): δ [ppm]= 6.13 (s, 2H), 7.41 (d, 1H), 7.59 - 7.96 (m, 4H), 9.06 (s, 1 H), 13.24 (br. s., 1 H).

### Intermediate Example Int6.2

### 4-(2-amino[1,2,4]triazolo[1,5-a]pyridin-6-yl)-2-fluoro-N-(4-fluorobenzyl)-benzamide

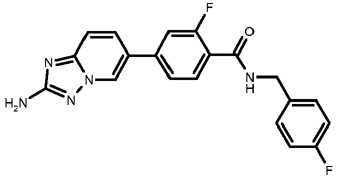

To a stirred suspension of **Int6.1** (1.6 g) in DMF (60 mL) was added molecular sives (4A) and the mixture was stirred for 1.5 h. To the stirred mixture was added potassium carbonate (4.1 g), 1-(4-fluorophenyl)methanamine (1.14 g) and HATU (3.35 g). The mixture was stirred at r.t. for 2 h. Solids were removed by filtration. The mixture was concentrated in vacuum. Water was added and the reaction mixture was extracted with a mixture of DCM and methanol (10:1). The organic phase was washed with a saturated solution of sodium bicarbonate, dried (sodium sulfate) and the solvent was removed in vacuum. Trituration of the residue with warm ethanol gave 0.55 g of the title compound.

¹H-NMR (400MHz, DMSO-d₆): δ [ppm]= 4.43 (d, 2H), 6.11 (s, 2H), 7.10 - 7.18 (m, 2H), 7.32 - 7.38 (m, 2H), 7.41 (dd, 1H), 7.64 - 7.78 (m, 3H), 7.83 (dd, 1H), 8.84 - 8.91 (m, 1 H), 9.03 (d, 1 H).

### Intermediate Example Int7.1

### 4-(2-amino[1,2,4]triazolo[1,5-a]pyridin-6-yl)-2-methylbenzoic acid

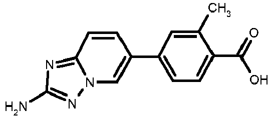

To a stirred solution of **Int1.2** (1.45 g) in 1-propanol (65 mL) was added 2M potassium carbonate solution (10 mL), 4-carboxy-3-methylphenylboronic acid (1.22 g), triphenylphosphine (0.17 g) and PdCl₂(PPh₃)₂ (0.35 g). The mixture was heated to reflux for 1 h. Water (200 mL) was added and the mixture was extracted with ethyl acetate (2 x 100 mL). 1 N hydrochloric acid was added to the aqueous phase until pH 5 was reached. The precipitated solid was collected by filtration, was washed with water, ethanol and ether and was dried in vacuum to give 0.8 g of the title compound.

¹H-NMR (400MHz, DMSO-d₆): δ [ppm]= 2.57 (s, 3H), 6.37 (br. s., 2H), 7.52 (d, 1H), 7.65 (dd, 1H), 7.70 (s, 1H), 7.89 (d, 1H), 7.95 (dd, 1H), 9.06 (d, 1H), 12.87 (br. s., 1 H).

### Intermediate Example Int7.2

### 4-(2-amino[1,2,4]triazolo[1,5-a]pyridin-6-yl)-N-(4-fluorobenzyl)-2-methylbenzamide

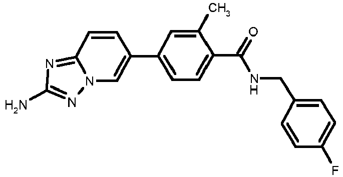

To a stirred suspension of **Int7.1** (0.425 g) in DMF (12 mL) was added potassium carbonate (1.1 g), 1-(4-fluorophenyl)methanamine (0.31 g) and HATU (1.21 g). The mixture was stirred at r.t. for 60 h. A saturated solution of sodium bicarbonate was added, the mixture was stirred at r.t. for 20 minutes and the mixture was extracted with ethyl acetate. The organic phase was dried (sodium sulfate) and the solvent was removed in vacuum. Trituration of the residue with warm ethanol gave 0.58 g of the title compound.

¹H-NMR (300MHz, DMSO-d₆): δ [ppm]= 2.37 (s, 3H), 4.39 (d, 2H), 6.05 (s, 2H), 7.14 (t, 2H), 7.28 - 7.46 (m, 4H), 7.50 - 7.66 (m, 2H), 7.76 (d, 1H), 8.74 - 8.99 (m, 2H).

Starting with Int7.1 the intermediate **Int7.3** was prepared analogously to the procedures described above.

### Intermediate Example Int7.3.

### 4-(2-amino[1,2,4]triazolo[1,5-a]pyridin-6-yl)-N-(2,4-difluorobenzyl)-2-methylbenzamide

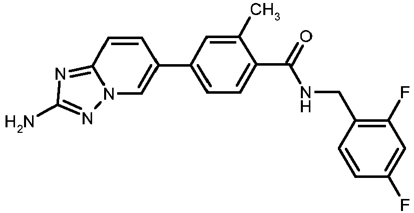

### Intermediate Example Int8.1

### 4-(2-amino[1,2,4]triazolo[1,5-a]pyridin-6-yl)-2-chlorobenzoic acid

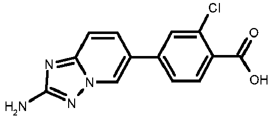

To a stirred solution of **Int1.2** (2.0 g) in 1-propanol (100 mL) was added 2M potassium carbonate solution (15 mL), 4-carboxy-3-chlorophenylboronic acid (1.92 g), triphenylphosphine (0.25 g) and PdCl₂(PPh₃)₂ (0.66 g). The mixture was heated to reflux for 1 h. Water (200 mL) was added and the mixture was extracted with ethyl acetate (2 x 100 mL). 1 N hydrochloric acid was added to the aqueous phase until pH 5 was reached. The precipitated solid was collected by filtration, was washed with water, ethanol and ether and was dried in vacuum to give 1.6 g of the title compound.

¹H-NMR (300MHz, DMSO-d₆): δ [ppm]= 6.13 (br. s., 2H), 7.41 (d, 1H), 7.73 - 7.88 (m, 3H), 7.92 (d, 1 H), 9.04 (d, 1 H), 13.37 (br. s., 1 H).

### Intermediate Example Int8.2

### 4-(2-amino[1,2,4]triazolo[1,5-a]pyridin-6-yl)-2-chloro-N-(4-fluorobenzyl)-benzamide

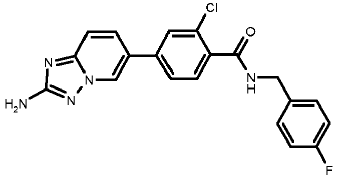

To a stirred suspension of **Int8.1** (0.425 g) in DMF (12 mL) was added potassium carbonate (1.1 g), 1-(4-fluorophenyl)methanamine (0.38 g) and HATU (1.40 g). The mixture was stirred at r.t. for 24 h. Further 1-(4-fluorophenyl)methanamine (95 mg) and HATU (280 g) was added and the mixture was stirred at r.t. for 16 h. Solids were removed by filtration. A half-saturated solution of sodium bicarbonate was added, the mixture was stirred at r.t. for 20 minutes and the mixture was extracted with a mixture of DCM and methanol (10:1). The organic phase was dried (sodium sulfate) and the solvent was removed in vacuum. Trituration of the residue with warm ethanol gave 0.38 g of the title compound.

¹H-NMR (300MHz, DMSO-d₆): δ [ppm]= 4.42 (d, 2H), 6.10 (br. s, 2H), 7.09 - 7.20 (m, 2H), 7.33 - 7.43 (m, 3H), 7.50 (d, 1 H), 7.77 (ddd, 2H), 7.89 (d, 1 H), 8.94 - 9.06 (m, 2H).

### Intermediate Example Int9.1

### 4-(2-amino[1,2,4]triazolo[1,5-a]pyridin-6-yl)-2-methoxybenzoic acid

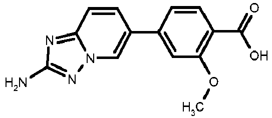

To a stirred solution of **Int1.2** (0.52 g) in 1-propanol (24 m) was added 2M potassium carbonate solution (3.6 mL), 4-carboxy-3-methoxyphenylboronic acid (0.48 g), triphenylphosphine (63 mg) and PdCl₂(PPh₃)₂ (170 mg). The mixture was heated to reflux for 1 h. Water (200 mL) was added and the mixture was extracted with ethyl acetate (2 x 100 mL). 2N hydrochloric acid was added to the aqueous phase until pH 4 was reached. The precipitated solid was collected by filtration, was washed with water, ethanol and ether and was dried in vacuum to give 466 mg of the title compound.

¹H-NMR (300MHz, DMSO-d₆, detected signals): δ [ppm]= 3.98 (s, 3H), 4.46 (d, 2H), 6.08 (s, 2H), 7.07 - 7.18 (m, 2H), 7.29 - 7.47 (m, 5H), 7.75 - 7.88 (m, 2H), 8.72 (t, 1H), 9.03 (dd, 1H).

### Intermediate Example Int9.2

### 4-(2-amino[1,2,4]triazolo[1,5-a]pyridin-6-yl)-N-(4-fluorobenzyl)-2-methoxybenzamide

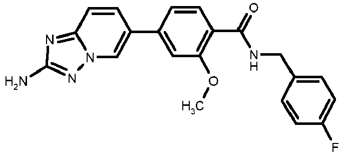

To a stirred suspension of **Int9.1** (0.30 g) in DMF (8.5 mL) was added potassium carbonate (0.73 g), 1-(4-fluorophenyl)methanamine (0.20 g) and HATU (0.80 g). The mixture was stirred at r.t. for 16 h. Solids were removed by filtration. The solvent was removed in vaccuum. Dichloromethane and methanol (10:1) and a half-saturated solution of sodium bicarbonate was added and the mixture was stirred at r.t. for 20 minutes. The mixture was extracted with a mixture of DCM and methanol (10:1). The organic phase was dried (sodium sulfate) and the solvent was removed in vacuum. Trituration of the residue with warm ethanol gave 0.36 g of the title compound.

¹H-NMR (300MHz, DMSO-d₆): δ [ppm]= 3.98 (s, 3H), 4.46 (d, 2H), 6.08 (s, 2H), 7.07 - 7.18 (m, 2H), 7.29 - 7.47 (m, 5H), 7.75 - 7.88 (m, 2H), 8.72 (t, 1H), 9.03 (dd, 1H).

### Intermediate Example Int10.1

### methyl 4-bromo-3-methoxybenzoate

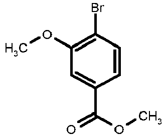

To a stirred solution of methyl 4-bromo-3-hydroxybenzoate (10.0 g) in DMF (50 mL) was added potassium carbonate (17.9 g) and iodomethane (9.2 mg). The mixture was stirred at r.t. for 2 h. Ethyl acetate was added and the mixture was washed with water. The organic phase was washed with saturated sodium chloride solution, dried (sodium sulfate) and the solvent was removed in vacuum to give 10 g of the title compound, that was used without further purification.

¹H-NMR (400MHz, DMSO-d₆): δ [ppm]= 3.82 (s, 3H), 3.87 (s, 3H), 7.41 (dd, 1H), 7.47 (d, 1H), 7.67 (d, 1H).

### Intermediate Example Int10.2

### 4-bromo-3-methoxybenzoic acid

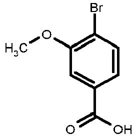

To a stirred solution of methyl 4-bromo-3-methoxybenzoate (11.2 g) in THF (130 mL), methanol (45 mL) and water (45 mL) was added a 1M solution of lithium hydroxide in water (140 mL). The mixture was stirred at r.t. for 1 h. The solvent was removed in vacuum. Water was added and 1 N hydrochloric acid was added with ice bath cooling until pH 4 was reached. The precipitated solid was collected by filtration, washed with water and dried in vacuum to give 10.1 g of the title compound, that was used without further purification.

¹H-NMR (300MHz, DMSO-d₆): δ [ppm]= 3.87 (s, 3H), 7.42 (dd, 1 H), 7.50 (d, 1 H), 7.68 (d, 1 H), 13.21 (br. s., 1 H).

### Intermediate Example Int10.3

### 4-bromo-N-(2-hydroxyethyl)-3-methoxybenzamide

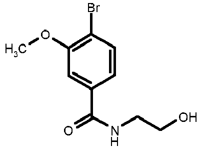

To a stirred suspension of 4-bromo-3-methoxybenzoic acid (1.65 g) in DCM (50 mL) was added DMF (0.5 mL) and oxalyl choride (0.98 g). The mixture was stirred at r.t. for 1 h. The solvent was removed in vacuum, and the residue was dissolved in THF (40 mL). Hünig Base (3.7 mL) and 2-aminoethanol (0.65 g) was added and the mixture was stirred at r.t. for 1 h. The solvent was removed in vacuum, a mixture of ethyl acetate and methanol (100:1) was added and the mixture was washed with a half-saturated solution of ammonium chloride. The organic phase was washed with saturated sodium chloride solution, dried (sodium sulfate) and the solvent was removed in vacuum. Silica gel chromatography gave 1.87 g of the title compound.

¹H-NMR (300MHz, CHLOROFORM-d): δ [ppm]= 2.75 (br. s., 1H), 3.56 - 3.67 (m, 2H), 3.75 - 3.89 (m, 2H), 3.93 (s, 3H), 6.74 (br. s., 1 H), 7.12 (dd, 1 H), 7.40 (d, 1H), 7.55 (d, 1H).

### Intermediate Example Int10.4

### 4-bromo-N-(2-hydroxy-2-methylpropyl)-3-methoxybenzamide

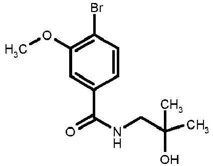

To a stirred suspension of 4-bromo-3-methoxybenzoic acid (1.5 g) in DCM (30 mL) was added DMF (0.5 mL) and oxalyl choride (1.05 g). The mixture was stirred at r.t. for 1 h. The solvent was removed in vacuum, and the residue was dissolved in DCM (40 mL). 1-Amino-2-methylpropan-2-ol (1.7 g) was added and the mixture was stirred for 3 h at r.t.. Water was added and the reaction mixture was extracted with ethyl acetate. The organic phase was washed with saturated sodium chloride solution, dried (sodium sulfate) and the solvent was removed in vacuum. Aminophase-silica-gel chromatography gave 1.45 g of the title compound.

¹H-NMR (300MHz, DMSO-d₆): δ [ppm]= 1.06 (s, 6H), 3.17 - 3.24 (m, 2H), 3.88 (s, 3H), 4.52 (s, 1H), 7.36 (dd, 1H), 7.50 (d, 1H), 7.63 (d, 1H), 8.34 (t, 1H).

### Intermediate Example Int10.5

### 4-bromo-N-(1-hydroxy-2-methylpropan-2-yl)-3-methoxybenzamide

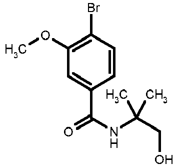

To a stirred solution of 4-bromo-3-methoxybenzoic acid (1.7 g) in DCM (22 mL) and DMF (1.0 mL) was added oxalyl chloride (1.19 g) at 0°C. The mixture was stirred at r.t. for 0.5 h. The solvent was removed in vacuum. The residue was dissolved in DCM (30 mL) and 2-amino-2-methylpropan-1-ol (1.93 g) were added. The mixture was stirred at r.t. for 3 h. A half-saturated solution of sodium bicarbonate was added and the mixture was extracted with ethyl acetate. The organic phase was washed with saturated sodium chloride solution, dried (sodium sulfate) and the solvent was removed in vacuum. Aminophase-silica-gel chromatography gave 1.90 g of the title compound.

¹H-NMR (400MHz, DMSO-d₆): δ [ppm]= 1.27 (s, 6H), 3.48 (d, 2H), 3.87 (s, 3H), 4.85 (t, 1H), 7.30 (dd, 1H), 7.40 (d, 1H), 7.57 - 7.62 (m, 2H).

### Intermediate Example Int10.6

### 4-bromo-N-(2-ethoxyethyl)-3-methoxybenzamide

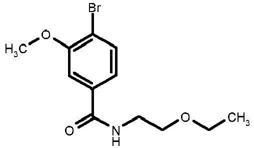

To a stirred suspension of 4-bromo-3-methoxybenzoic acid (1.55 g) in DCM (40 mL) was added DMF (0.5 mL) and oxalyl choride (0.92 g). The mixture was stirred at r.t. for 1 h. The solvent was removed in vacuum, and the residue was dissolved in THF (40 mL). Hünig Base (3.4 mL) and 2-ethoxyethanamine (0.89 g) was added and the mixture was stirred at r.t. for 1 h. The solvent was removed in vacuum, ethyl acetate was added and the mixture was washed with a half-saturated solution of ammonium chloride. The organic phase was washed with saturated sodium chloride solution, dried (sodium sulfate) and the solvent was removed in vacuum. Recrystallization of the residue from diisopropyl ether and cyclohexane gave 1.8 g of the title compound.

¹H-NMR (400MHz, CHLOROFORM-d): δ [ppm]= 1.22 (t, 3H), 3.54 (q, 2H), 3.58 - 3.68 (m, 4H), 3.95 (s, 3H), 6.53 (br. s., 1H), 7.12 (dd, 1H), 7.43 (d, 1H), 7.58 (d, 1H).

### Intermediate Example Int10.7

### 4-bromo-3-methoxy-N-methyl-N-[2-(methylamino)ethyl]benzamide

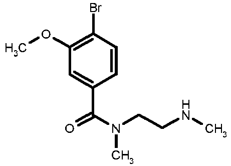

To a stirred suspension of 4-bromo-3-methoxybenzoic acid (2.0 g) in DCM (150 mL) was added DMF (0.5 mL) and oxalyl choride (1.4 g). The mixture was stirred at r.t. for 1 h. The solvent was removed in vacuum, and the residue was dissolved in DCM (40 mL). This mixture was slowly added to a solution of *N,N'-*dimethylethane-1,2-diamine (2.24 g) in DCM (40 mL) at 0°C and the mixture was stirred at r.t. for 1 h. 4N aqueous hydrochloric acid was added (100 mL) and the mixture was washed with DCM. Aqueous sodium hydroxide solution was added to the aqueous phase until pH8 was reached and the mixture was extracted with DCM. The organic phase was dried (sodium sulfate) and the solvent was removed in vacuum. Silica gel chromatography gave 1.25 g of the title compound.

¹H-NMR (500MHz, DMSO-d₆): δ [ppm]= 2.28 (br. s., 3H), 2.69 (br. s., 2H), 2.96 (s, 3H), 3.05 (br. s., 1H), 3.39 (br. s., 2H), 3.90 (s, 3H), 6.91 (dd, 1H), 7.12 (d, 1H), 7.61 (d, 1H).

### Intermediate Example Int10.8

### N-{2-[acetyl(methyl)amino]ethyl}-4-bromo-3-methoxy-N-methylbenzamide

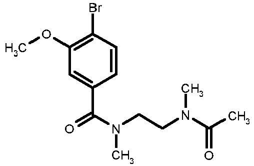

To a stirred solution of Int10.7 (720 mg) in DCM (15 mL) was added pyridine (580 µL) and acetyl chloride (340 µL). The mixture was stirred at r.t. for 1 h. Ethyl acetate was added and the mixture was washed with a half-saturated solution of ammonium chloride. The organic phase was washed with saturated sodium chloride solution, dried (sodium sulfate) and the solvent was removed in vacuum. Silica gel chromatography gave 1.94 g of the title compound.

¹H-NMR (500MHz, DMSO-d₆, 80°C, selected signals): δ [ppm]= 3.90 (s, 3H), 6.87 (dd, 1 H), 7.07 (br. s., 1 H), 7.62 (d, 1 H).

### Intermediate Example Int10.9

### tert-butyl {2-[(4-bromo-3-methoxybenzoyl)(methyl)amino]ethyl}methylcarbamate

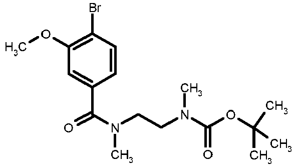

To a stirred solution of **Int10.7** (890 mg) in DCM (35 mL) was added Hünig Base (1.5 mL) and di-*tert*-butyl dicarbonate (775 mg). The mixture was stirred at r.t. for 16 h. A half-saturated solution of sodium bicarbonate was added and the mixture was extracted with ethyl acetate. The organic phase was washed with saturated sodium chloride solution, dried (sodium sulfate) and the solvent was removed in vacuum. Silica gel chromatography gave 1.0 g of the title compound.

¹H-NMR (500MHz, DMSO-d₆, 80°C): δ [ppm]= 1.37 (s, 9H), 2.70 (br. s., 3H), 2.95 (s, 3H), 3.35 (br. s., 2H), 3.48 (br. s., 2H), 3.88 (s, 3H), 6.85 (dd, 1 H), 7.02 (s, 1H), 7.59 (d, 1H).

### Intermediate Example Int10.10

### 1-bromo-2-methoxy-4-(methylsulfanyl)benzene

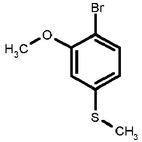

To a stirred solution of 1-bromo-4-fluoro-2-methoxybenzene (4.0 g) in DMF (40 mL) was added sodium methanethiolate (2.76 g). The mixture was stirred at r.t. for 30 minutes and at 85°C for 2 h. Water was added and the mixture was extracted with ethyl acetate. The organic phase was washed with saturated sodium chloride solution, dried (sodium sulfate) and the solvent was removed in vacuum. Silica gel chromatography gave 280 mg of the title compound.

¹H-NMR (400MHz, DMSO-d₆): δ [ppm]= 2.46 (s, 3H), 3.82 (s, 3H), 6.74 (dd, 1H), 6.91 (d, 1H), 7.44 (d, 1H).

### 1-bromo-2-methoxy-4-(methylsulfanyl)benzene

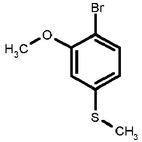

To a stirred solution of 1-bromo-4-fluoro-2-methoxybenzene (10.0 g) in DMF (100 mL) was added sodium methanethiolate (4.44 g). The mixture was stirred at 65°C for 2 h. The mixture was cooled to 0°C and methyl iodide (4.55 mL) was added. The mixture was stirred at r.t. for 1 h and further sodium methanethiolate (4.44 g) was added. The mixture was stirred at 65°C for 1 h. The mixture was cooled to 0°C and methyl iodide (4.55 mL) was added. The mixture was stirred at r.t. for 1 h. Water was added and the mixture was extracted with ethyl acetate. The organic phase was washed with saturated sodium chloride solution, dried (sodium sulfate) and the solvent was removed in vacuum. Silica gel chromatography gave 6.2 g of the title compound as a 2:1 mixture with the starting material. The mixture was used for the next step without purification.

### Intermediate Example Int10.11

### 1-bromo-2-methoxy-4-(methylsulfonyl)benzene

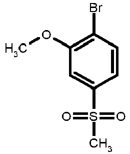

To a stirred solution of **Int10.10** (265 mg) in chloroform (10 mL) was added 3-chlorobenzenecarboperoxoic acid (*m*CPBA) (890 mg). The mixture was stirred at r.t. for 1 h. A half-saturated solution of sodium bicarbonate was added and the mixture was extracted with DCM. The organic phase was washed with saturated sodium chloride solution, dried (sodium sulfate) and the solvent was removed in vacuum. Silica gel chromatography gave 252 mg of the title compound.

¹H-NMR (300MHz, DMSO-d₆): δ [ppm]= 3.22 (s, 3H), 3.93 (s, 3H), 7.39 (dd, 1H), 7.50 (d, 1H), 7.84 (d, 1H).

### Intermediate Example Int10.12

### 4-bromo-3-methoxy-N-(2,2,2-trifluoroethyl)benzamide

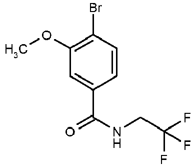

To a stirred suspension of 4-bromo-3-methoxybenzoic acid (2.0 g) in THF (100 mL) was added 2,2,2-trifluoroethylamine (1.26 g), HATU (3,87 g), and DIEA (1.7 mL). The mixture was stirred at r.t. for 12 h. Water (350 mL) and saturated sodium bicarbonate solution (350 mL) were added. The organic phase was separated and the aqueous phase was extracted with ethyl acetate. The combined organic extracts were dried (sodium sulfate) and the solvent was removed in vacuum. Silica gel chromatography gave 2.57 g of the title compound.

¹H-NMR (400 MHz, DMSO-d₆): δ [ppm] = 3.92 (s, 3H), 4.11 (qd, 2H), 7.43 (dd, 1H), 7.56 (d, 1H), 7.72 (d, 1H), 9.19 (t, 1H).

### Intermediate Example Int10.13

### 4-bromo-3-methoxy-N-[2-(methylsulfonyl)ethyl]benzamide

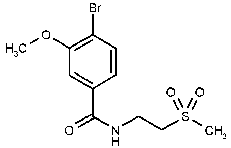

To a stirred suspension of 4-bromo-3-methoxybenzoic acid (1.0 g) in THF (50 mL) was added 2-(methylsulfonyl)ethanamine hydrochloride (1.09 g), HATU (1.97 g), and DIEA (0.89 mL). The mixture was stirred at r.t. for 12 h. Water (350 mL) and saturated sodium bicarbonate solution (350 mL) were added. The organic phase was separated and the aqueous phase was extracted with ethyl acetate. The combined organic extracts were dried (sodium sulfate) and the solvent was removed in vacuum. Silica gel chromatography gave 809 mg of the title compound.

¹H-NMR (400 MHz, DMSO-d₆): δ [ppm] = 3.04 (s, 3H), 3.38 (t, 2H), 3.63 - 3.72 (m, 2H), 3.90 (s, 3H), 7.36 (dd, 1 H), 7.51 (d, 1 H), 7.69 (d, 1 H), 8.84 (t, 1 H).

### Intermediate Example Int11.1

### methyl 4-bromo-3-ethoxybenzoate

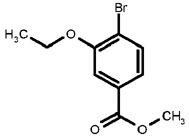

To a stirred solution of methyl 4-bromo-3-hydroxybenzoate (6.4 g) in DMF (35 mL) was added potassium carbonate (11.5 g and iodoethane (6.48 mg). The mixture was stirred at r.t. for 16 h. The solvent was removed in vacuum, ethyl acetate was added and the mixture was washed with water. The organic phase was washed with saturated sodium chloride solution, dried (sodium sulfate) and the solvent was removed in vacuum. Trituration of the residue with warm ethanol gave 5.7 g of the title compound.

¹H-NMR (300MHz, DMSO-d₆): δ [ppm]= 1.34 (t, 3H), 3.82 (s, 3H), 4.14 (q, 2H), 7.42 (dd, 1H), 7.48 (d, 1H), 7.70 (d, 1H).

### Intermediate Example Int11.2

### 4-bromo-3-ethoxybenzoic acid

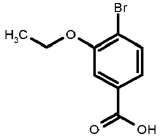

To a stirred solution of methyl 4-bromo-3-ethoxybenzoate (17.3 g) in THF (180 mL), methanol (60 mL) and water (60 mL) was added a 1M solution of lithium hydroxide in water (200 mL). The mixture was stirred at r.t. for 1 h. Water was added and 1 N hydrochloric acid was added until pH 4 was reached. The precipitated solid was collected by filtration, was washed with water. The solid was dissolved in DCM and methanol (10:1). The solution was washed with saturated sodium chloride solution, dried (sodium sulfate) and the solvent was removed in vacuum. Trituration of the residue with ether gave 15.4 g of the title compound.

¹H-NMR (400MHz, DMSO-d₆): δ [ppm]= 1.34 (t, 3H), 4.13 (q, 2H), 7.40 (dd, 1H), 7.48 (d, 1 H), 7.67 (d, 1 H), 13.17 (br. s., 1 H).

### Intermediate Example Int11.3

### 4-bromo-N-tert-butyl-3-ethoxybenzamide

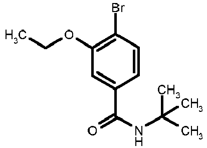

To a stirred suspension of 4-bromo-3-ethoxybenzoic acid (2.5 g) in DCM (25 mL) was added DMF (0.8 mL) and oxalyl choride (1.42 g). The mixture was stirred at r.t. for 1 h. The solvent was removed in vacuum, and the residue was dissolved in THF (50 mL). Hünig Base (5.3 mL) and 2-methylpropan-2-amine (1.13 g) was added and the mixture was stirred at r.t. for 1 h. The solvent was removed in vacuum, a mixture of DCM and methanol (100:1) was added and the mixture was washed with a half-saturated solution of ammonium chloride. The organic phase was washed with a half-saturated solution of sodium bicarbonate, dried (sodium sulfate) and the solvent was removed in vacuum. Recrystallization of the residue from isopropanol gave 2.4 g of the title compound.

¹H-NMR (300MHz, CHLOROFORM-d): δ [ppm]= 1.38 - 1.54 (m, 12H), 4.15 (q, 2H), 5.94 (br. s., 1H), 7.01 (dd, 1H), 7.37 (d, 1H), 7.52 (d, 1H).

### Intermediate Example Int11.4

### 4-bromo-3-ethoxy-N-ethylbenzamide 101,3

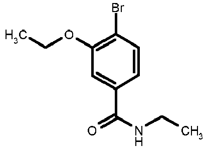

To a stirred suspension of 4-bromo-3-ethoxybenzoic acid (3.0 g) in DCM (35 mL) was added DMF (0.1 mL) and oxalyl choride (2.0 g). The mixture was stirred at r.t. for 1 h. The solvent was removed in vacuum, and the residue was dissolved in DCM (30 mL). Ethanamine (1.6 g) was added and the mixture was stirred at r.t. for 3 h. Ethyl acetate was added and the mixture was washed with a half-saturated solution of sodium bicarbonate. The organic phase was washed with saturated sodium chloride solution, dried (sodium sulfate) and the solvent was removed in vacuum. Silica gel chromatography gave 2.7 g of the title compound.

¹H-NMR (300MHz, DMSO-d₆): δ [ppm]= 1.08 (t, 3H), 1.34 (t, 3H), 3.17 - 3.29 (m, 2H), 4.13 (q, 2H), 7.32 (dd, 1 H), 7.45 (d, 1 H), 7.62 (d, 1 H), 8.51 (t, 1 H).

### Intermediate Example Int11.5

### 4-bromo-3-ethoxy-N-(2-hydroxyethyl)benzamide

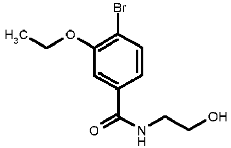

To a stirred suspension of 4-bromo-3-ethoxybenzoic acid (1.65 g) in DCM (50 mL) was added DMF (0.5 mL) and oxalyl choride (1.45 g). The mixture was stirred at r.t. for 1 h. The solvent was removed in vacuum, and the residue was dissolved in THF (50 mL). Hünig Base (3.5 mL) and 2-aminoethanol (0.62 g) was added and the mixture was stirred at r.t. for 16 h. The solvent was removed in vacuum, ethyl acetate was added and the mixture was washed with a half-saturated solution of sodium bicarbonate and with a saturated solution of ammonium chloride. The organic phase was dried (sodium sulfate) and the solvent was removed in vacuum. Silica gel chromatography gave 1.3 g of the title compound.

¹H-NMR (300MHz, DMSO-d₆): δ [ppm]= 1.34 (t, 3H), 3.20 - 3.33 (m, 2H), 3.40 - 3.53 (m, 2H), 4.13 (q, 2H), 4.71 (t, 1H), 7.33 (dd, 1 H), 7.48 (d, 1 H), 7.62 (d, 1H), 8.50 (t, 1H).

### Intermediate Example Int11.6

### 4-bromo-3-ethoxy-N-(2-hydroxy-2-methylpropyl)benzamide

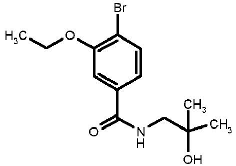

To a stirred suspension of 4-bromo-3-ethoxybenzoic acid (1.8 g) in DCM (25 mL) was added DMF (0.1 mL) and oxalyl choride (1.19 g). The mixture was stirred at r.t. for 1 h. The solvent was removed in vacuum, and the residue was dissolved in DCM (25 mL). 1-Amino-2-methylpropan-2-ol (1.93 g) was added and the mixture was stirred at r.t. for 3 h. Ethyl acetate was added and the mixture was washed with a half-saturated solution of sodium bicarbonate. The organic phase was washed with saturated sodium chloride solution, dried (sodium sulfate) and the solvent was removed in vacuum. Silica gel chromatography gave 1.8 g of the title compound.

¹H-NMR (300MHz, DMSO-d₆): δ [ppm]= 1.06 (s, 6H), 1.34 (t, 3H), 3.21 (d, 2H), 4.14 (q, 2H), 4.51 (s, 1 H), 7.35 (dd, 1 H), 7.48 (d, 1 H), 7.62 (d, 1 H), 8.32 (t, 1 H).

### Intermediate Example Int11.7

### 4-bromo-3-ethoxy-N-(1-hydroxy-2-methylpropan-2-yl)benzamide

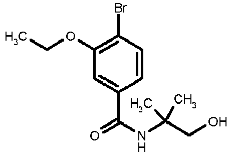

To a stirred suspension of 4-bromo-3-ethoxybenzoic acid (1.7 g) in DCM (50 mL) was added DMF (0.5 mL) and oxalyl choride (1.50g). The mixture was stirred at r.t. for 1 h. The solvent was removed in vacuum, and the residue was dissolved in THF (50 mL). Hünig Base (3.6 mL) and 2-Amino-2-methylpropan-1-ol (0.98 g) was added and the mixture was stirred at r.t. for 16 h. The solvent was removed in vacuum, ethyl acetate and methanol (100:1) were added and the mixture was washed with a half-saturated solution of sodium bicarbonate and with a saturated solution of ammonium chloride. The organic phase was dried (sodium sulfate) and the solvent was removed in vacuum. Silica gel chromatography gave 1.66 g of the title compound.

¹H-NMR (400MHz, DMSO-d₆): δ [ppm]= 1.27 (s, 6H), 1.34 (t, 3H), 3.44 - 3.51 (m, 2H), 4.13 (q, 2H), 4.84 (t, 1 H), 7.29 (dd, 1 H), 7.39 (d, 1 H), 7.57 (br. s, 1 H), 7.59 (d, 1H).

### Intermediate Example Int11.8

### 4-Bromo-3-ethoxy-N,N-diethylbenzamide

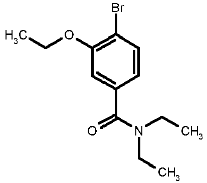

To a stirred solution of 4-bromo-3-ethoxybenzoic acid (1.0 g) in THF (50 mL) was added Hünig Base (0.82 mL), diethylamine (0.50 mL) and HATU (1.83 g). The mixture was stirred at r.t. for 16 h. A mixture of ethyl acetate and hexane (3:1) was added and the mixture was washed with a half-saturated solution of sodium bicarbonate and with a saturated solution of ammonium chloride. The organic phase was washed with saturated sodium chloride solution, dried (sodium sulfate) and the solvent was removed in vacuum. Silica gel chromatography gave 850 mg of the title compound.

¹H-NMR (300MHz, CHLOROFORM-d): δ [ppm]= 0.98 - 1.34 (m, 6H), 1.47 (t, 3H), 3.10 - 3.67 (m, 4H), 4.11 (q, 2H), 6.80 (dd, 1H), 6.89 (d, 1H), 7.54 (d, 1H).

### Intermediate Example Int11.9

### 4-bromo-3-ethoxy-N-ethyl-N-(2-methoxyethyl)benzamide

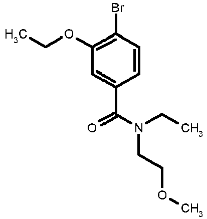

To a stirred solution of 4-bromo-3-ethoxybenzoic acid (0.83 g) in THF (25 mL) was added Hünig Base (0.68 mL), 2-ethoxy-*N*-ethylethanamine (0.50 mL) and HATU (1.51 g). The mixture was stirred at r.t. for 16 h. Ethyl acetate was added and the mixture was washed with a half-saturated solution of sodium bicarbonate and with a saturated solution of ammonium chloride. The organic phase was washed with saturated sodium chloride solution, dried (sodium sulfate) and the solvent was removed in vacuum. Aminophase-silica-gel chromatography gave 870 mg of the title compound.

¹H-NMR (300MHz, DMSO-d₆): δ [ppm]= 0.90 - 1.15 (m, 3H), 1.31 (t, 3H), 3.09 - 3.59 (m, 9H), 4.09 (q, 2H), 6.80 (d, 1 H), 7.02 (br. d, 1 H), 7.57 (d, 1 H).

### Intermediate Example Int11.10

### 4-bromo-3-ethoxy-N-(2-hydroxyethyl)-N-methylbenzamide

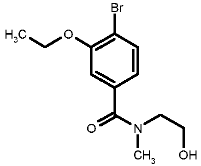

To a stirred suspension of 4-bromo-3-ethoxybenzoic acid (1.5 g) in DCM (45 mL) was added DMF (0.1 mL) and oxalyl choride (1.01 g). The mixture was stirred at r.t. for 1 h. The solvent was removed in vacuum, and the residue was dissolved in DCM (25 mL). 2-(methylamino)ethanol (1.38 g) was added and the mixture was stirred at r.t. for 2 h. Ethyl acetate was added and the mixture was washed with a half-saturated solution of sodium bicarbonate. The organic phase was washed with saturated sodium chloride solution, dried (sodium sulfate) and the solvent was removed in vacuum. Silica gel chromatography followed by aminophase-silica-gel chromatography gave 1.3 g of the title compound.

¹H-NMR (400MHz, DMSO-d₆): δ [ppm]= 1.32 (t, 3H), 2.82 - 2.98 (m, 3H), 3.15 - 3.63 (m, 4H), 4.02 - 4.14 (m, 2H), 4.71 - 4.84 (m, 1H), 6.85 (dd, 1H), 6.98 - 7.13 (m, 1 H), 7.51 - 7.63 (m, 1 H).

### Intermediate Example Int11.11

### 4-bromo-N-(2-{[tert-butyl(dimethyl)silyl]oxy}ethyl)-3-ethoxy-N-methyl-benzamide

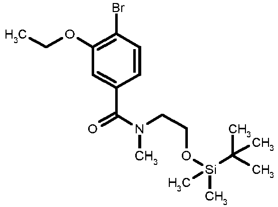

To a stirred solution of **Int10.10** (1.3 g) in THF (15 mL) was added Hünig Base (0.88 mL), imidazole (30 mg) and *tert*-butyl(chloro)dimethylsilane (0.78 g). The mixture was stirred at r.t. for 16 h. Water was added and the mixture extracted with ethyl acetate. The organic phase was washed with saturated sodium chloride solution, dried (sodium sulfate) and the solvent was removed in vacuum. Silica gel chromatography gave 1.45 g of the title compound.

¹H-NMR (300MHz, DMSO-d₆): δ [ppm]= -0.13 - 0.11 (m, 6H), 0.81 (d, 9H), 1.31 (t, 3H), 2.92 (br. s., 3H), 3.29 - 3.82 (m, 4H), 4.08 (q, 2H), 6.75 - 6.90 (m, 1H), 7.00 (d, 1H), 7.48 - 7.66 (m, 1H).

### Intermediate Example Int11.12

### 4-bromo-3-ethoxy-N-[2-(methylsulfonyl)ethyl]benzamide

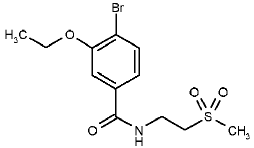

To a stirred suspension of 4-bromo-3-ethoxybenzoic acid (1.1 g) in THF (53 mL) was added 2-(methylsulfonyl)ethanamine hydrochloride (1.13 g), HATU (2.05 g), and DIEA (0.92 mL). The mixture was stirred at r.t. for 12 h. Water (350 mL) and saturated sodium bicarbonate solution (350 mL) were added. The organic phase was separated and the aqueous phase was extracted with ethyl acetate. The combined organic extracts were dried (sodium sulfate) and the solvent was removed in vacuum. Silica gel chromatography gave 808 mg of the title compound.

¹H-NMR (400 MHz, DMSO-d₆): δ [ppm] = 1.38 (t, 3H), 3.03 (s, 3H), 3.38 (t, 2H), 3.61 - 3.71 (m, 2H), 4.16 (q, 2H), 7.35 (dd, 1 H), 7.49 (d, 1 H), 7.69 (d, 1 H), 8.82 (t, 1H).

### Intermediate Example Int11.13

### 1-bromo-2-ethoxy-4-fluorobenzene

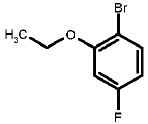

To a stirred solution of 2-bromo-5-fluorophenol (5.0 g) in DMF (30 mL) was added potassium carbonate (10.8 g) and iodoethane (6.12 g). The mixture was stirred at r.t. for 16 h. The solvent was removed in vaccuum. Water was added and the mixture was extracted with a mixture of ethyl acetate and hexane (3:1). The organic phase was washed with saturated sodium chloride solution, dried (sodium sulfate) and the solvent was removed in vacuum, to give 5.06 g of th title compound as a crude product, that was used for the next step without purification.

¹H-NMR (400MHz, DMSO-d₆): δ [ppm]= 1.31 (t, 3H), 4.08 (q, 2H), 6.71 (td, 1H), 7.00 (dd, 1H), 7.55 (dd, 1H).

### Intermediate Example Int11.14

### 1-bromo-2-ethoxy-4-(methylsulfanyl)benzene

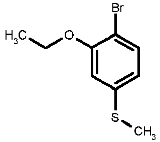

To a stirred solution of 1-bromo-2-ethoxy-4-fluorobenzene (2.0 g) in DMF (20 mL) was added sodium methanethiolate (1.66 g). The mixture was stirred for 2 h at 65°C. The mixture was cooled to r.t. and ethyl iodide (1.3 mL) was added. The mixture was stirred at r.t. for 1 h. Water was added and the mixture was extracted with ethyl acetate. The organic phase was washed with saturated sodium chloride solution, dried (sodium sulfate) and the solvent was removed in vacuum. Silica gel chromatography gave 1.65 g of the title compound.

¹H-NMR (400MHz, DMSO-d₆): δ [ppm]= 1.24 - 1.36 (m, 3H), 2.45 (s, 3H), 4.08 (q, 2H), 6.73 (dd, 1H), 6.89 (d, 1H), 7.43 (d, 1H).

### Intermediate Example Int11.15

### 1-bromo-2-ethoxy-4-(methylsulfonyl)benzene

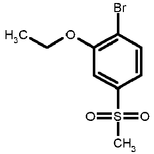

To a stirred solution of **Int11.14** (1.65 g) in chloroform (65 mL) was added 3-chlorobenzenecarboperoxoic acid (mCPBA) (4.49 g). The mixture was stirred at r.t. for 16 h. With ice bath cooling, a half-saturated solution of sodium bicarbonate and and a 0.2M solution of sodium thiosulfate was added, the mixture was stirred for 30 minutes and the mixture was extracted with DCM. The organic phase was washed with saturated sodium chloride solution, dried (sodium sulfate) and the solvent was removed in vacuum. Silica gel chromatography gave 1.35 g of the title compound.

¹H-NMR (300MHz, DMSO-d₆): δ [ppm]= 1.35 (t, 3H), 3.22 (s, 3H), 4.20 (q, 2H), 7.37 (dd, 1H), 7.48 (d, 1H), 7.84 (d, 1H).

### Intermediate Example Int11.16

### 1-bromo-2-ethoxy-4-(ethylsulfanyl)benzene

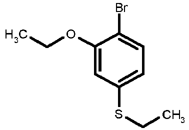

To a stirred solution of 1-bromo-4-fluoro-2-ethoxybenzene (2.0 g) in DMF (19 mL) was added sodium ethanethiolate (1.66 g). The mixture was stirred at 65°C for 2 h. Water was added and the mixture was extracted with ethyl acetate. The organic phase was washed with saturated sodium chloride solution, dried (sodium sulfate) and the solvent was removed in vacuum. Silica gel chromatography gave 2.02 g of the title compound.

¹H-NMR (300MHz, DMSO-d₆): δ [ppm]= 1.20 (t, 3H), 1.30 (t, 3H), 2.96 (q, 2H), 4.08 (q, 2H), 6.77 (dd, 1H), 6.92 (d, 1H), 7.44 (d, 1H).

### Intermediate Example Int11.17

### 1-bromo-2-ethoxy-4-(ethytsulfonyl)benzene

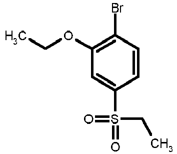

To a stirred solution of **Int11.16** (2.0 g) in chloroform (75 mL) was added 3-chlorobenzenecarboperoxoic acid (*m*CPBA) (5.15 g). The mixture was stirred at r.t. for 2 h. With ice bath cooling, a half-saturated solution of sodium bicarbonate and and a 0.2M solution of sodium thiosulfate was added, the mixture was stirred for 30 minutes and the mixture was extracted with DCM. The organic phase was washed with saturated sodium chloride solution, dried (sodium sulfate) and the solvent was removed in vacuum. Silica gel chromatography gave 1.71 g of the title compound.

¹H-NMR (400MHz, DMSO-d₆): δ [ppm]= 1.07 (t, 3H), 1.35 (t, 3H), 3.31 (q, 2H), 4.20 (q, 2H), 7.33 (dd, 1H), 7.41 (d, 1H), 7.85 (d, 1H).

### Intermediate Example Int12.1

### methyl 4-bromo-3-(2,2,2-trifluoroethoxy)benzoate

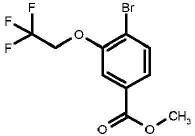

To a stirred solution of methyl 4-bromo-3-hydroxybenzoate (2.5 g) in acetonitrile (0.5 mL) and DMF (10 mL) in a microwave tube was added potassium carbonate (2.93 g) and 2,2,2-trifluoroethyl trifluoromethanesulfonate (2.79 g). The mixture was heated to 150°C in a microwave oven for 30 minutes. The solvent was removed in vacuum, ethyl acetate was added and the mixture was washed with water. The organic phase was washed with saturated sodium chloride solution, dried (sodium sulfate) and the solvent was removed in vacuum. Recrystallization of the residue from ethanol gave 1.2 g of the title compound. The mother liquor was concentrated in vacuum and purified by aminophase-silica-gel chromatography followed by recrystallized from methanol and water to give further 0.64 g of the title compound.

¹H-NMR (300MHz, CHLOROFORM-d): δ [ppm]= 3.93 (s, 3H), 4.47 (q, 2H), 7.56 (d, 1 H), 7.58 - 7.70 (m, 2H).

### Intermediate Example Int12.2

### 4-bromo-3-(2,2,2-trifluoroethoxy)benzoic acid

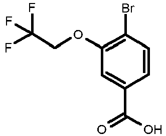

To a stirred solution of **Int12.1** (1.83 g) in THF (30 mL), methanol (10 mL) and water (10 mL) was added a 1M solution of lithium hydroxide in water (18 mL). The mixture was stirred at r.t. for 1 h. Water was added and 2N hydrochloric acid was added until pH 4 was reached. The precipitated solid was collected by filtration, was washed with water. The solid was suspended with toluene and concentrated in vacuum. Trituration of the residue with hexane gave 1.6 g of the title compound.

¹H-NMR (300MHz, DMSO-d₆): δ [ppm]= 4.95 (q, 2H), 7.51 (dd, 1H), 7.65 (d, 1H), 7.74 (d, 1H), 13.29 (br. s., 1H).

### Intermediate Example Int12.3

### 4-bromo-N-(2-hydroxyethyl)-3-(2,2,2-trifluoroethoxy)benzamide

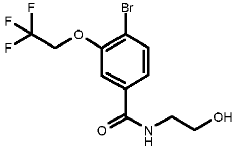

To a stirred suspension of **Int12.2** (1.53 g) in DCM (35 mL) was added DMF (0.4 mL) and oxalyl choride (0.97 g). The mixture was stirred at r.t. for 1 h. The solvent was removed in vacuum, and the residue was dissolved in THF (25 mL). Hünig Base (2.7 mL) and 2-aminoethanol (0.47 g) was added and the mixture was stirred at r.t. for 16 h. The solvent was removed in vacuum, ethyl acetate was added and the mixture was washed with a half-saturated solution of sodium bicarbonate and with a saturated solution of ammonium chloride. The organic phase was dried (sodium sulfate) and the solvent was removed in vacuum. Silica gel chromatography gave 1.50 g of the title compound.

¹H-NMR (300MHz, DMSO-d₆): δ [ppm]= 3.24 - 3.35 (m, 2H), 3.42 - 3.52 (m, 2H), 4.73 (t, 1H), 4.89 (q, 2H), 7.44 (dd, 1H), 7.62 (d, 1H), 7.70 (d, 1H), 8.50 (t, 1 H).

### Intermediate Example Int12.4

### 4-bromo-3-(2,2,2-trifluoroethoxy)-N-(2,2,2-trifluoroethyl)benzamide

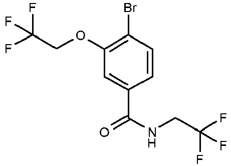

To a stirred suspension of 4-bromo-3-(2,2,2-trifluoroethoxy)benzoic acid (2.0 g) in THF (100 mL) was added 2,2,2-trifluoroethylamine (0.99 g), HATU (3.05 g), and DIEA (1.03 mL). The mixture was stirred at r.t. for 12 h. Water (350 mL) and saturated sodium bicarbonate solution (350 mL) were added. The organic phase was separated and the aqueous phase was extracted with ethyl acetate. The combined organic extracts were dried (sodium sulfate) and the solvent was removed in vacuum. Silica gel chromatography gave 2.42 g of the title compound as colourless solid.

¹H-NMR (400 MHz, DMSO-d₆): δ [ppm] = 4.13 (qd, 2H), 4.95 (q, 2H), 7.52 (dd, 1H), 7.69 (d, 1H), 7.79 (d, 1H), 9.17 (t, 1H).

### Intermediate Example Int12.5

### 4-bromo-N-(1-hydroxy-2-methylpropan-2-yl)-3-(2,2,2-trifluoroethoxy)benzamide

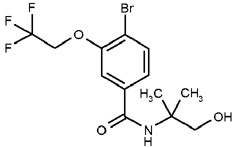

To a stirred suspension of 4-bromo-3-(2,2,2-trifluoroethoxy)benzoic acid (1.8 g) in THF (70 mL) was added 1-amino-2-methylpropan-2-ol (0.85 g), HATU (2.75 g), and DIEA (1.23 mL). The mixture was stirred at r.t. for 12 h. Water (350 mL) and saturated sodium bicarbonate solution (350 mL) were added. The organic phase was separated and the aqueous phase was extracted with ethyl acetate. The combined organic extracts were dried (sodium sulfate) and the solvent was removed in vacuum. Silica gel chromatography gave 1.97 g of the title compound.

¹H-NMR (400 MHz, DMSO-d₆): δ [ppm] = 1.31 (s, 6H), 3.52 (d, 2H), 4.88 (t, 1H), 4.94 (q, 2H), 7.43 (dd, 1H), 7.57 (d, 1H), 7.61 (s, 1H), 7.70 (d, 1H).

### Intermediate Example Int12.6

### 4-bromo-N-(2-hydroxy-2-methylpropyl)-3-(2,2,2-trifluoroethoxy)benzamide

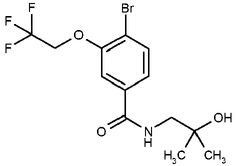

To a stirred suspension of 4-bromo-3-(2,2,2-trifluoroethoxy)benzoic acid (1.8 g) in THF (70 mL) was added 2-amino-2-methylpropan-1-ol (0.85 g), HATU (2.75 g), and DIEA (1.23 mL). The mixture was stirred at r.t. for 12 h. Water (350 mL) and saturated sodium bicarbonate solution (350 mL) were added. The organic phase was separated and the aqueous phase was extracted with ethyl acetate. The combined organic extracts were dried (sodium sulfate) and the solvent was removed in vacuum. Silica gel chromatography gave 2.2 g of the title compound.

¹H-NMR (400 MHz, DMSO-d₆): δ [ppm] = 1.10 (s, 6H), 3.26 (d, 2H), 4.56 (s, 1H), 4.95 (q, 2H), 7.49 (dd, 1H), 7.67 (d, 1H), 7.73 (d, 1H), 8.36 (t, 1H).

### Intermediate Example Int12.7

### 1-bromo-4-fluoro-2-(2,2,2-trifluoroethoxy)benzene

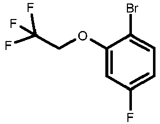

To a stirred solution of 2-bromo-5-fluorophenol (1.5 g) in acetonitrile (0.5 mL) and DMF (8.5 mL) in a microwave tube was added potassium carbonate (2.1 g) and 2,2,2-trifluoroethyl trifluoromethanesulfonate (2.37 g). The mixture was heated to 150°C in a microwave oven for 30 minutes. In a second microwave tube the same reaction was repeated. Both mixtures were combined. The solvent was removed in vacuum, ethyl acetate and hexane (1:1) was added and the mixture was washed with water. The organic phase was washed with saturated sodium chloride solution, dried (sodium sulfate) and the solvent was removed in vacuum. Silica gel chromatography gave 4.0 g of the title compound.

¹H-NMR (300MHz, CHLOROFORM-d): δ [ppm]= 4.39 (q, 2H), 6.62 - 6.78 (m, 2H), 7.53 (dd, 1H).

### Intermediate Example Int12.8

### 1-bromo-4-(methylsulfanyl)-2-(2,2,2-trifluoroethoxy)benzene

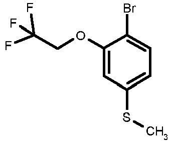

To a stirred solution of **Int12.7** (4.0 g) in DMF (15 mL) was added sodium methanethiolate (1.0 g). The mixture was stirred for 2 h at 60 °C. The mixture was cooled to r.t.. Water was added and the mixture was extracted with ethyl acetate. The organic phase was washed with saturated sodium chloride solution, dried (sodium sulfate) and the solvent was removed in vacuum to give 3.8 g of the crude title compound, that was used for the next step without purification.

¹H-NMR (300MHz, CHLOROFORM-d): δ [ppm]= 2.48 (s, 3H), 4.39 (q, 2H), 6.78 - 6.88 (m, 2H), 7.46 (d, 1 H).

### Intermediate Example Int12.9

### 1-bromo-4-(methylsulfonyl)-2-(2,2,2-trifluoroethoxy)benzene

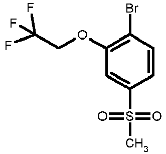

To a stirred solution of **Int12.8** (3.8 g) in chloroform (100 mL) was added 3-chlorobenzenecarboperoxoic acid (mCPBA) (8.48 g). The mixture was stirred at r.t. for 16 h. With ice bath cooling, a half-saturated solution of sodium bicarbonate and and a 0.2M solution of sodium thiosulfate was added, the mixture was stirred for 30 minutes and the mixture was extracted with DCM. The organic phase was washed with a 0.2M solution of sodium thiosulfate and a saturated sodium chloride solution, dried (sodium sulfate) and the solvent was removed in vacuum. Silica gel chromatography gave a solid that was triturated with ether to give 2.1 g of the title compound.

¹H-NMR (400MHz, CHLOROFORM-d): δ [ppm]= 3.06 (s, 3H), 4.50 (q, 2H), 7.45 (d, 1H), 7.52 (dd, 1H), 7.81 (d, 1H).

### Intermediate Example Int13.1

### N-tert-butyl-3-hydroxy-4-iodobenzamide

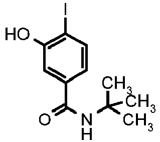

3-Hydroxy-4-iodobenzoic acid (WO2006/18325) (3.00 g) was dissolved in THF (50 mL) and tert-butylamine (997 mg), N-ethyl-diisopropylamine (1.76 g), and HATU (5.18 g) were added. The mixture was stirred overnight at rt. Subsequently, it was diluted with ethyl acetate (400 mL) and washed with satd. aqueous sodium bicarbonate solution, ammonium chloride solution, buffer solution (pH 2), and brine. The organic layer was dried over sodium sulfate, and the solvent was evaporated. The residue was purified by column chromatography on silica gel (eluent: cyclohexane / ethyl acetate gradient 4:1 to 1:1) to yield 2.5 g (60% yield, 88% purity) of the title compound.

¹H-NMR (400MHz, DMSO-d₆): δ [ppm]= 1.35 (s, 9H), 7.00 (dd, 1H), 7.24 (d, 1H), 7.68 - 7.72 (m, 2H), 10.49 (br. s, 1 H).

### Intermediate Example Int13.2

### N-tert-butyl-4-iodo-3-(2,2,2-trifluoroethoxy)benzamide

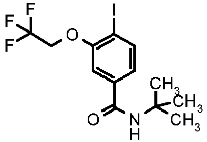

*N*-*tert*-butyl-3-hydroxy-4-iodobenzamide (**Int13.1**) (1.20 g) was dissolved in DMF (7.8 mL) and acetonitrile (0.3 mL), and potassium carbonate (1.04 g) and 2,2,2-trifluoroethyl trifluoromethanesulfonate (916 mg) were added. The mixture was heated for 30 min in a microwave oven to 150 °C. Subsequently, the reaction mixture was diluted with water and three times extracted with ethyl acetate. The combined organic layers were washed three times with aqueous ammonium chloride solution, then with satd. aqueous sodium bicarbonate solution and with brine. It was dried over sodium sulfate, and the solvent was evaporated to yield 1.43 g (93%) of the title compound as white crystals.

¹H-NMR (400MHz, DMSO-d₆): δ [ppm]= 1.38 (s, 9H), 4.91 (q, 2H), 7.28 (dd, 1H), 7.45 (d, 1H), 7.78 (br. s, 1H), 7.87 (d, 1H).

### Intermediate Example Int14.1

### N-ethyl-3-hydroxy-4-iodobenzamide

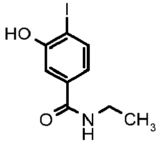

3-Hydroxy-4-iodobenzoic acid (WO2006/18325) (3.00 g) was dissolved in THF (50 mL) and ethylamine (2M solution in THF, 6.8 mL), *N*-ethyl-diisopropylamine (1.76 g), and HATU (5.18 g) were added. The mixture was stirred overnight at rt. Subsequently, it was diluted with ethyl acetate (200 mL) and washed with ½ satd. aqueous ammonium chloride solution, satd. aqueous sodium bicarbonate solution, and brine. The organic layer was dried over sodium sulfate, and the solvent was evaporated. The residue was titurated with DCM, and the precipitate was collected by suction filtration to yield 1.67 g (49% yield) of the title compound.

¹H-NMR (400MHz, DMSO-d₆): δ [ppm]= 1.10 (t, 3H), 3.20 - 3.28 (m, 2H), 7.04 (dd, 1H), 7.32 (d, 1H), 7.74 (d, 1H), 8.42 (t, 1H), 10.52 (br. s, 1H).

### Intermediate Example Int14.2

### N-ethyl-4-iodo-3-(2,2,2-trifluoroethoxy)benzamide

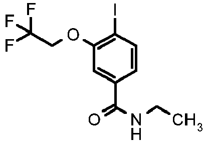

N-ethyl-3-hydroxy-4-iodobenzamide (**Int14.1**) (1.00 g) was dissolved in DMF (7.1 mL) and acetonitrile (0.29 mL), and potassium carbonate (950 mg) and 2,2,2-trifluoroethyl trifluoromethanesulfonate (837 mg) were added. The mixture was heated for 30 min in a microwave oven to 150 °C. Subsequently, the reaction mixture was diluted with water and three times extracted with ethyl acetate. The combined organic layers were washed three times with aqueous ammonium chloride solution, then with satd. aqueous sodium bicarbonate solution and with brine. The organic layer was dried over sodium sulfate, and the solvent was evaporated to yield 1.25 g (98%) of the title compound.

¹H-NMR (400MHz, DMSO-d₆): δ [ppm]= 1.13 (t, 3H), 3.25 - 3.33 (m, 2H), 4.89 (q, 2H), 7.30 (dd, 1 H), 7.51 (d, 1 H), 7.91 (d, 1 H), 8.51 (t, 1 H).

### Intermediate Example Int15.1

### N,N-diethyl-3-hydroxy-4-iodobenzamide

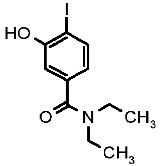

3-Hydroxy-4-iodobenzoic acid (WO2006/18325) (10.0 g) was dissolved in a mixture of DCM (75 mL) and DMF (50 mL) and cooled to 0 °C. Oxalyl chloride (7.21 g) was added, and the mixture was stirred for 10 min. Subsequently, diethylamine (6.93 g) was added, and the mixture was warmed to r.t. and stirred for 1.5 h. The reaction mixture was then diluted with water and three times extracted with ethyl acetate. The combined organic layers were washed with aqueous buffer solution (pH 2), satd. sodium bicarbonate solution, and brine, then dried over sodium sulfate, and the solvent was evaporated. The title compound (8.40 g, 66%) was obtained as an oil.

¹H-NMR (400MHz, DMSO-d₆): δ [ppm]= 1.07 (br. s, 6H), 3.16 (br. s, 2H), 3.39 (br. s, 2H), 6.55 (dd, 1 H), 6.80 (d, 1 H), 7.71 (d, 1 H), 10.56 (br. s, 1 H).

### Intermediate Example Int15.2

### N,N-diethyl-4-iodo-3-(2,2,2-trifluoroethoxy)benzamide

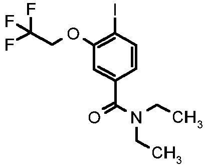

*N,N*-Diethyl-3-hydroxy-4-iodobenzamide (**Int15.1**) (1.00 g) was dissolved in DMF (6.5 mL) and acetonitrile (0.26 mL), and potassium carbonate (866 mg) and 2,2,2-trifluoroethyl trifluoromethanesulfonate (764 mg) were added. The mixture was heated for 30 min in a microwave oven to 150 °C. Subsequently, the reaction mixture was diluted with water and three times extracted with ethyl acetate. The combined organic layers were washed three times with aqueous ammonium chloride solution, then with satd. aqueous sodium bicarbonate solution and with brine. It was dried over sodium sulfate, and the solvent was evaporated to yield 1.25 g (99%) of the title compound as an oil. ¹H-NMR (400MHz, DMSO-d₆): δ [ppm]= 0.99 - 1.18 (m, 6H), 3.11 - 3.22 (m, 2H), 3.36 - 3.47 (m, 2H), 4.89 (q, 2H), 6.79 (dd, 1H), 7.11 (d, 1H), 7.86 (d, 1H).

### Intermediate Example Int15.3

### N,N-diethyl-4-iodo-3-propoxybenzamide

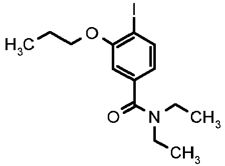

*N,N*-Diethyl-3-hydroxy-4-iodobenzamide (**Int15.1**) (630 mg) was dissolved in DMF (4.2 mL) and acetonitrile (0.17 mL), and potassium carbonate (546 mg) and 1-iodopropane (352 mg) were added. The mixture was heated for 30 min in a microwave oven to 150 °C. Subsequently, the reaction mixture was diluted with water and three times extracted with ethyl acetate. The combined organic layers were washed three times with aqueous ammonium chloride solution, then with satd. aqueous sodium bicarbonate solution and with brine. It was dried over sodium sulfate, and the solvent was evaporated to yield 670 mg (94%) of the title compound as an oil.

¹H-NMR (400MHz, DMSO-d₆): δ [ppm]= 1.00 - 1.18 (m, 6H), 1.03 (t, 3H), 1.69 - 1.80 (m, 2H), 3.10 - 3.25 (m, 2H), 3.35 - 3.47 (m, 2H), 4.02 (t, 2H), 6.69 (dd, 1H), 6.90 (d, 1H), 7.80 (d, 1H).

### Intermediate Example Int15.4

### 3-(cyclopropylmethoxy)-N,N-diethyl-4-iodobenzamide

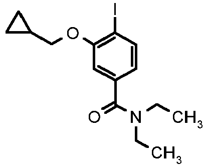

*N,N*-Diethyl-3-hydroxy-4-iodobenzamide (**Int15.1**) (618 mg) was dissolved in DMF (2.7 mL) and acetonitrile (0.1 mL), and potassium carbonate (535 mg) and 1-(bromomethyl)cyclopropane (275 g) were added. The mixture was heated for 30 min in a microwave oven to 150 °C. Subsequently, the reaction mixture was diluted with water and extracted three times with ethyl acetate. The combined organic layers were washed three times with aqueous ammonium chloride solution, then with satd. aqueous sodium bicarbonate solution and with brine. It was dried over sodium sulfate, and the solvent was evaporated to yield 498 mg (63%) of the title compound as an oil.

¹H-NMR (400MHz, DMSO-d₆): δ [ppm]= 0.34 - 0.40 (m, 2H), 0.54 - 0.60 (m, 2H), 0.99 - 1.08 (m, 3H), 1.09 - 1.16 (m, 3H), 1.17 - 1.28 (m, 1H), 3.09 - 3.23 (m, 2H), 3.35 - 3.45 (m, 2H), 3.94 (d, 2H), 6.68 (dd, 1H), 6.89 (d, 1H), 7.80 (d, 1H).

### Intermediate Example Int15.5

### N,N-diethyl-4-iodo-3-isopropoxybenzamide

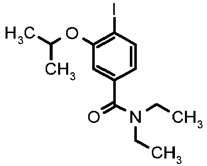

*N,N*-Diethyl-3-hydroxy-4-iodobenzamide (**Int15.1**) (400 mg) was dissolved in DMF (2.7 mL) and acetonitrile (0.10 mL), and potassium carbonate (346 mg) and 2-iodopropane (224 mg) were added. The mixture was heated for 30 min in a microwave oven to 150 °C. Subsequently, the reaction mixture was diluted with water and three times extracted with ethyl acetate. The combined organic layers were washed three times with aqueous ammonium chloride solution, then with satd. aqueous sodium bicarbonate solution and with brine. It was dried over sodium sulfate, and the solvent was evaporated to yield 425 mg (92%) of the title compound as an oil.

¹H-NMR (400MHz, DMSO-d₆): δ [ppm]= 1.00 - 1.18 (m, 6H), 1.29 (d, 6H), 3.11 - 3.25 (m, 2H), 3.34 - 3.47 (m, 2H), 4.72 (spt, 1 H), 6.67 (dd, 1 H), 6.94 (d, 1 H), 7.80 (d, 1H).

### Intermediate Example Int15.6

### N,N-diethyl-4-iodo-3-(2-methoxyethoxy)benzamide

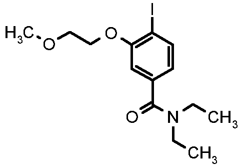

*N,N*-Diethyl-3-hydroxy-4-iodobenzamide (**Int15.1**) (423 mg) was dissolved in DMF (2.8 mL) and acetonitrile (0.11 mL), and potassium carbonate (266 mg) and 1-bromo-2-methoxyethane (193 mg) were added. The mixture was heated for 30 min in a microwave oven to 150 °C. Thereafter, further 1-bromo-2-methoxyethane (193 mg) was added, and the mixture was heated for additional 30 min. Subsequently, the reaction mixture was diluted with water and three times extracted with ethyl acetate. The combined organic layers were washed three times with aqueous ammonium chloride solution, then with satd. aqueous sodium bicarbonate solution and with brine. It was dried over sodium sulfate, and the solvent was evaporated to yield 470 mg (94%) of the title compound as an oil.

¹H-NMR (400MHz, DMSO-d₆): δ [ppm]= 0.98 - 1.19 (m, 6H), 3.10 - 3.24 (m, 2H), 3.35 (s, 3H), 3.36 - 3.47 (m, 2H), 3.68 - 3.71 (m, 2H), 4.17 - 4.20 (m, 2H), 6.70 (dd, 1H), 6.94 (d, 1H), 7.81 (d, 1H).

### Intermediate Example Int16.1

### tert-butyl {2-[(4-{[6-(4-{[(4-fluorophenyl)acetyl]amino}phenyl)[1,2,4]-triazolo[1,5-a]pyridin-2-yl]amino}-3-methoxybenzoyl)(methyl)amino]-ethyl}methylcarbamate

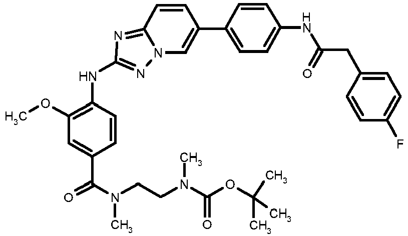

Starting with Intermediate **Int3.4** and *tert*-butyl {2-[(4-bromo-3-methoxybenzoyl)(methyl)amino]ethyl}methylcarbamate (**Int10.9**), **Intermediate Example Int16.1 was** prepared analogously to the procedure for the preparation of **Example11.2.**

¹H-NMR (500MHz, DMSO-d₆): δ [ppm]= 1.34 - 1.43 (m, 9H), 2.71 (br. s., 3H), 3.00 (s, 3H), 3.34 - 3.43 (m, 2H), 3.54 (t, 2H), 3.68 (s, 2H), 3.93 (s, 3H), 6.97 - 7.04 (m, 2H), 7.09 - 7.16 (m, 2H), 7.38 (dd, 2H), 7.62 (dd, 1 H), 7.71 (s, 4H), 7.86 - 7.95 (m, 2H), 8.30 (d, 1H), 9.00 (d, 1H), 10.03 (s, 1H).

### Intermediate Example Int16.2

### tert-butyl {2-[{4-[(6-{4-[(4-fluorobenzyl)carbamoyl]phenyl}[1,2,4]triazolo-[1,5-a]pyridin-2-yl)amino]-3-methoxybenzoyl}(methyl)amino]ethyl}methylcarbamate

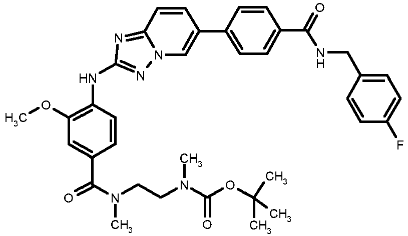

Starting with Intermediate **Int5.2** and *tert*-butyl {2-[(4-bromo-3-methoxybenzoyl)(methyl)amino]ethyl}methylcarbamate (**Int10**.9), **Intermediate Example Int16.2 was** prepared analogously to the procedure for the preparation of **Example2.5.**

¹H-NMR (500MHz, DMSO-d₆): δ [ppm]= 1.32 - 1.43 (m, 9H), 2.72 (br. s., 3H), 3.00 (s, 3H), 3.34 - 3.42 (m, 2H), 3.54 (t, 2H), 3.94 (s, 3H), 4.50 (d, 2H), 6.97 - 7.05 (m, 2H), 7.08 - 7.16 (m, 2H), 7.35 - 7.43 (m, 2H), 7.66 (dd, 1 H), 7.85 - 7.92 (m, 2H), 7.94 - 8.04 (m, 4H), 8.30 (d, 1 H), 8.87 (t, 1 H), 9.15 (d, 1 H).

### Intermediate Example Int16.3

### N-(cyclopropylmethyl)-4-{2-[(2-ethoxy-4-nitrophenyl)amino][1,2,4]-triazolo[1,5-a]pyridin-6-yl}benzamide

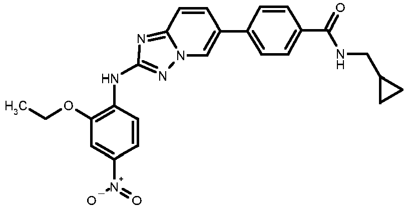

To a stirred suspension of **Int5.4** (103 mg) in toluene (3.0 mL) and NMP (0.3 mL) in a sealed tube was added 1-bromo-2-ethoxy-4-nitrobenzene (124 mg), chloro(2-dicyclohexylphosphino-2',4',6'-tri-i-propyl-1,1'-biphenyl)[2-(2-aminoethyl)phenyl] palladium(II) methyl-*tert*-butylether adduct (28 mg), X-Phos (16 mg), and sodium *tert*-butoxide (161 mg). The flask was twice degased and backfilled with argon. The mixture was heated to 130°C with an oil bath for 2 h. Water was added and the reaction mixture was extracted with a mixture of DCM and methanol (100:1). The organic phase was washed with saturated sodium chloride solution, dried (sodium sulfate) and the solvent was removed in vacuum. Silica gel chromatography gave 150 mg of the title compound.

¹H-NMR (300MHz, DMSO-d₆): δ [ppm]= 0.11 - 0.28 (m, 2H), 0.33 - 0.47 (m, 2H), 0.93 - 1.10 (m, 1 H), 1.44 (t, 3H), 3.14 (t, 2H), 4.25 (q, 2H), 7.74 (dd, 2H), 7.85 - 8.00 (m, 5H), 8.05 (dd, 1 H), 8.50 (d, 1 H), 8.63 (t, 1 H), 8.94 (s, 1 H), 9.29 (s, 1 H).

### Intermediate Example Int16.4

### 4-{2-[(4-amino-2-ethoxyphenyl)amino][1,2,4]triazolo[1,5-a]pyridin-6-yl}-N-(cyclopropylmethyl)benzamide

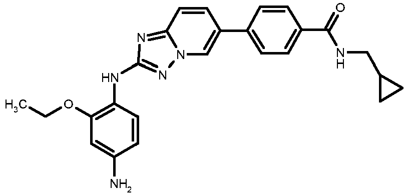

To a stirred solution of **Int16.3** (210 mg) in ethanol (100 mL) and DCM (60 mL) was added Raney Nickel (3.0 mg) and the mixture was stirred under a hydrogen atmosphere at r.t. for 20 h. The catalyst were removed by filtration and the solvent was removed in vacuum. Aminophase-silica-gel chromatography gave 31 mg of the title compound.

MW: 442,52; MS (ESI) found: [M+1] 443.

### Intermediate Example Int17.1

### 1-Bromo-2-(difluoromethoxy)-4-fluorobenzene

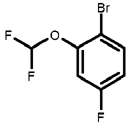

To a stirred solution of 2-bromo-5-fluorophenol (21.0 g) in DMF (600 mL) was added cesium carbonate (107.5 g), sodium chlorodifluoroacetate (52.3 g) and water (29.4 mL). The mixture was stirred at 100 °C for 2 h. The mixture was filtrated and the resulting solution was extracted with pentane (4 x 400 mL). The combined organic layers were washed with saturated sodium chloride solution, dried (sodium sulfate) and the solvent was removed in vacuum, to give 7.14 g of the title compound as a crude product. Silica gel chromatography afforded 3.2 g of the title compound.

¹H-NMR (400MHz, DMSO-d₆): δ [ppm] = 7.10 - 7.16 (m, 1 H), 7.34 (t, 1 H), 7.33 - 7.36 (m, 1H), 7.79 (dd, 1H).

### Intermediate Example Int17.2

### 1-bromo-2-(difluoromethoxy)-4-(methylsulfanyl)benzene

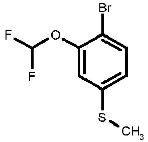

To a stirred solution of **Int17.1** (1.0 g) in DMF (9.5 mL) was added sodium methanethiolate (460 mg). The mixture was stirred for 1 h at 60 °C. The mixture was cooled to r.t., water (150 mL) was added and the mixture was extracted twice with ethyl acetate. The organic layer was washed with saturated sodium chloride solution, dried (sodium sulfate) and the solvent was removed in vacuum. Silica gel chromatography gave 850 mg of the title compound.

### Intermediate Example Int17.3

### 1-bromo-2-(difluoromethoxy)-4-(methylsulfonyl)benzene

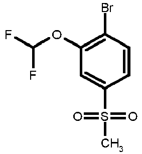

To a stirred solution of **Int17.2** (812 mg) in DCM (10 mL) was added 3-chlorobenzenecarboperoxoic acid (*m*CPBA) (2.23 g) at 0 °C. The mixture was stirred at r.t. for 2 h. Subsequently, the reaction mixture was diluted with DCM (20 mL), washed twice with aqueous sodium sulfite solution (10 %), and sodium bicarbonate solution. The organic layer was dried (sodium sulfate) and the solvent was removed in vacuum. Silica gel chromatography gave 633 mg of the title compound.

¹H-NMR (400 MHz, DMSO-d₆): δ [ppm] = 3.29 (s, 3H), 7.46 (t, 1H), 7.74 (dd, 1H), 7.81 (d, 1H), 8.06 (d, 1H).

### Intermediate Example Int17.4

### 1-bromo-2-(difluoromethoxy)-4-(ethylsulfanyl)benzene

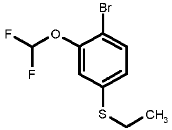

To a stirred solution of Int17.1 (500 mg) in DMF (4.75 mL) was added sodium ethanethiolate (276 mg). The mixture was stirred for 1 h at 60 °C. The mixture was cooled to r.t., water (150 mL) was added and the mixture was extracted twice with ethyl acetate. The organic layer was washed with saturated sodium chloride solution, dried (sodium sulfate) and the solvent was removed in vacuum. Silica gel chromatography gave 302 mg of the title compound.

### Intermediate Example Int17.5

### 1-bromo-2-(difluoromethoxy)-4-(ethylsulfonyl)benzene

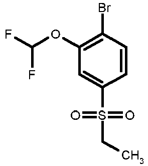

To a stirred solution of **Int17.4** (285 mg) in DCM (2.8 mL) was added 3-chlorobenzenecarboperoxoic acid (*m*CPBA) (625 g) at 0 °C. The mixture was stirred at r.t. for 2 h. Subsequently, the reaction mixture was diluted with DCM (20 mL), washed twice with aqueous sodium sulfite solution (10 %), and sodium bicarbonate solution. The organic layer was dried (sodium sulfate) and the solvent was removed in vacuum. Silica gel chromatography gave 275 mg of the title compound.

¹H-NMR (400MHz, DMSO-d₆): δ [ppm]= 1.12 (t, 3H), 3.38 (q, 2H), 7.26 - 7.66 (m, 1H), 7.69 (dd, 1H), 7.75 (d, 1H), 8.07 (d, 1H).

### Intermediate Example Int18.1

### 1-bromo-2-(cyclopropyloxy)-4-fluorobenzene

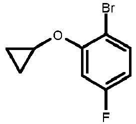

To a stirred solution of 2-bromo-5-fluorophenol (1.0 g) in DMF (15 mL) in a microwave tube was added cesium carbonate (5.0 g), potassium iodide (130 mg) and bromocyclopropane (1.82 g). The mixture was heated in a microwave oven to 180° C for 1 h, to 200° C for 1 h and to 220° C for 1 h. Ethyl acetate was added and the mixture was washed with water. The organic phase was washed with saturated sodium chloride solution, dried (sodium sulfate) and the solvent was removed in vacuum. Silica gel chromatography gave 1.14 g of the title compound.

¹H-NMR (300MHz, DMSO-d₆): δ [ppm]= 0.62 - 0.88 (m, 4H), 3.90 - 4.00 (m, 1H), 6.77 (td, 1H), 7.23 (dd, 1H), 7.48 - 7.63 (m, 1H).

### Intermediate Example Int18.2

### 1-bromo-2-(cyclopropyloxy)-4-(methylsulfanyl)benzene

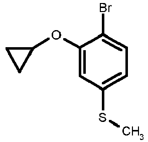

To a stirred solution of **Int18.1** (1.4 g) in DMF (12 mL) was added sodium methanethiolate (546 mg). The mixture was for 2 h at 90 °C. The mixture was cooled to r.t., water was added and the mixture was extracted with ethyl acetate. The organic phase was washed with saturated sodium chloride solution, dried (sodium sulfate) and the solvent was removed in vacuum. Silica gel chromatography gave 1.17 g of the title compound.

¹H-NMR (300MHz, DMSO-d₆): δ [ppm]= 0.59 - 0.85 (m, 4H), 2.46 (s, 3H), 3.95 (tt, 1H), 6.77 (dd, 1H), 7.18 (d, 1H), 7.43 (d, 1H).

### Intermediate Example Int18.3

### 1-bromo-2-(cyclopropyloxy)-4-(methylsulfonyl)benzene

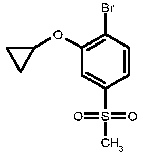

To a stirred solution of **Int18.2** (1.15 g) in chloroform (45 mL) was added 3-chlorobenzenecarboperoxoic acid (mCPBA) (2.98 g). The mixture was stirred at r.t. for 2 h. With ice bath cooling, a half-saturated solution of sodium bicarbonate and and a 0.2M solution of sodium thiosulfate was added, the mixture was stirred for 30 minutes and the mixture was extracted with DCM. The organic phase was washed with saturated sodium chloride solution, dried (sodium sulfate) and the solvent was removed in vacuum. Silica gel chromatography gave 0.91 g of the title compound.

¹H-NMR (300MHz, DMSO-d₆): δ [ppm]= 0.66 - 0.93 (m, 4H), 3.23 (s, 3H), 4.09 (tt, 1H), 7.43 (dd, 1H), 7.77 (d, 1H), 7.84 (d, 1H).

Starting with **Int10.2** the following intermediates were prepared analogously to the procedures described above.

| **Intermediate** | Structure | Name |
|---|---|---|
| **Int10.14** | 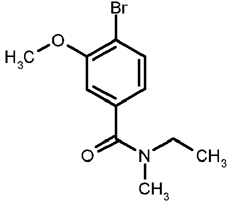 | 4-bromo-*N*-ethyl-3-methoxy-*N-*methylbenzamide |
| **Int10.15** | 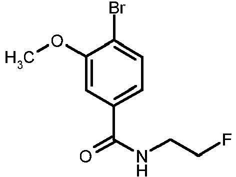 | 4-bromo-*N*-(2-fluoroethyl)-3-methoxybenzamide |
| **Int10.16** | 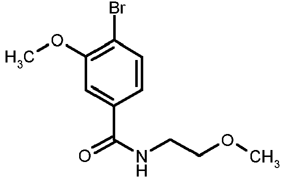 | 4-bromo-3-methoxy-*N*-(2-methoxyethyl)benzamide |
| **Int10.17** | 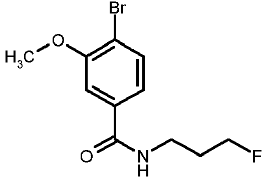 | 4-bromo-*N*-(3-fluoropropyl)-3-methoxybenzamide |
| **Int10.18** | 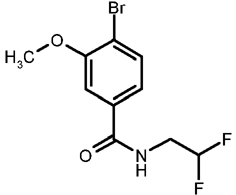 | 4-bromo-*N*-(2,2-difluoroethyl)-3-methoxybenzamide |
| **Int10.19** | 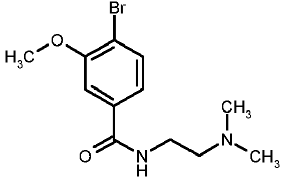 | 4-bromo-*N*-[2-(dimethylamino)ethyl]-3-methoxybenzamide |
| **Int10.20** | 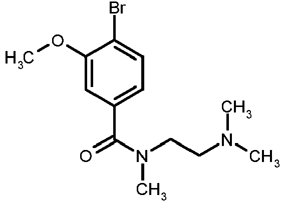 | 4-bromo-*N*-[2-(dimethylamino)ethyl]-3-methoxy-*N*-methylbenzamide |
| **Int10.21** | 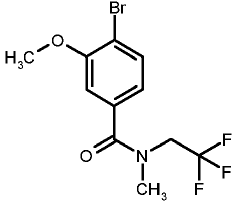 | 4-bromo-3-methoxy-*N*-methyl-*N-*(2,2,2-trifluoroethyl)benzamide |

### Intermediate Example Int10.22.01

### 1-bromo-5-fluoro-2-methoxy-4-(methylsulfanyl)benzene

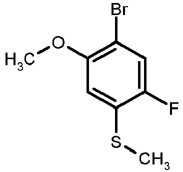

To a stirred solution of sodium methanethiolate (0.47 g) in DMF (15 mL) at 0 °C was added 1-bromo-4,5-difluoro-2-methoxybenzene (1.5 g). The mixture was stirred at r.t. for 2 h. Water was added and the mixture was extracted with ethyl acetate. The organic phase was washed with saturated sodium chloride solution, dried (sodium sulfate) and the solvent was removed in vacuum. Silica gel chromatography gave 1.46 g of the title compound.

### Intermediate Example Int10.22.02

### 1-bromo-5-fluoro-2-methoxy-4-(methylsulfonyl)benzene

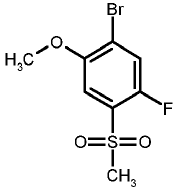

To a stirred solution of **Int10.22.01** (485 mg) in chloroform (10 mL) at 0 °C was added 3-chlorobenzenecarboperoxoic acid (*m*CPBA) (1.30 g). The mixture was stirred at r.t. for 16 h. The mixture was cooled to at 0 °C and a half-saturated solution of sodium bicarbonate and a solution of disodium sulfurothioate was added, the mixture was stirred for 30 minutes at r.t. and the mixture was extracted with DCM. The organic phase was washed with saturated sodium chloride solution, dried (sodium sulfate) and the solvent was removed in vacuum. Silica gel chromatography gave 311 mg of the title compound.

### Intermediate Example Int10.23

### 1-bromo-2-methoxy-4-(methylsulfinyl)benzene

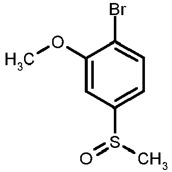

To a stirred solution of **Int10.10** (4.30 g) in DCM (150 mL) was added 3-chlorobenzenecarboperoxoic acid (*m*CPBA) (4.13 g; purity: 77% w/w) at 0 °C. The mixture was stirred at r.t. for 1 h. A half-saturated solution of sodium bicarbonate was added and the mixture was extracted with DCM. The organic phase was washed with saturated sodium chloride solution, dried (sodium sulfate) and the solvent was removed in vacuum. Silica gel chromatography gave 2.43 g of the title compound.

### Intermediate Example Int10.24

### 1-bromo-5-fluoro-2-methoxy-4-(methylsulfinyl)benzene

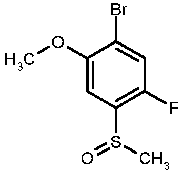

To a stirred solution of **Int10.22.01** (1.45 g) in chloroform (60 mL) was added 3-chlorobenzenecarboperoxoic acid (*m*CPBA) (1.29 g; purity: 77% w/w) at 0 °C. The mixture was stirred at r.t. for 1 h. The mixture was cooled to at 0 °C and a half-saturated solution of sodium bicarbonate and a solution of disodium sulfurothioate was added, the mixture was stirred for 30 minutes at r.t. and the mixture was extracted with DCM. The organic phase was washed with saturated sodium chloride solution, dried (sodium sulfate) and the solvent was removed in vacuum. Silica gel chromatography gave 950 mg of the title compound.

### Intermediate Example Int10.25.01

### N-[(4-bromo-3-methoxyphenyl)(methyl)oxido-λ⁶-sulfanylidene]-2,2,2-trifluoroacetamide

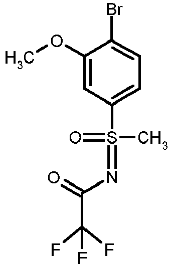

To a stirred solution of **Int10.23** (1.6 g) in DCM (80 mL) was added 2,2,2-trifluoroacetamide (1.60 g), magnesiumioide (1.14 g) and diacetoxy(phenyl)-λ³-iodane (3.41 g) and the flask was twice degased and backfilled with argon. The mixture was stirred for 5 minutes at r.t.. Rhodium(II)acetat dimer (142 mg) was added and the mixture mixture was stirred at r.t. for 1 h. The reaction mixture was filtered through celite and the solvent was removed in vaccuum. Silica gel chromatography gave 1.97 g of the title compound.

### Intermediate Example Int10.25.02

### 1-bromo-2-methoxy-4-(S-methylsulfonimidoyl)benzene

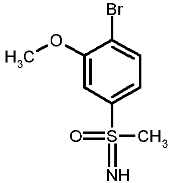

To a stirred solution of **Int10.25.01** (1.95 g) in methanol (50 mL) was added potassium carbonate (1.50 g) and the mixture mixture was stirred at r.t. for 30 minutes. Water was added and the reaction mixture was extracted with ethyl acetate. The organic phase was dried (sodium sulfate) and the solvent was removed in vacuum to give 1.36 g of the title compound that was used in the next step without further purification.

### Intermediate Example Int10.25.03

### tert-butyl [(4-bromo-3-methoxyphenyl)(methyl)oxido-λ⁶-sulfanylidene]-carbamate

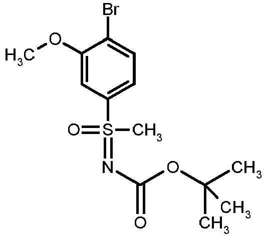

To a stirred suspension of **Int10.25.02** (850 mg) in THF (25 mL) was added sodium hydride (60 % w/w in oil; 193 mg) at 0 °C and the mixture was stirred at 0 °C for 30 minutes. Di-*tert*-butyl dicarbonate (1.40 g) was added and the mixture was stirred at r.t. for 16 hours. A half-saturated solution of sodium bicarbonate was added and the mixture was extracted with ethyl acetate. The organic phase was washed with saturated sodium chloride solution, dried (sodium sulfate) and the solvent was removed in vacuum. Aminophase-silica-gel chromatography gave 890 mg of the title compound.

### Intermediate Example Int10.25.04

### tert-butyl [(4-{[6-(4-{[(4-fluorophenyl)acetyl]amino}phenyl)[1,2,4]triazolo-[1,5-a]pyridin-2-yl]amino}-3-methoxyphenyl)(methyl)oxido-λ6-sulfanylidene]carbamate

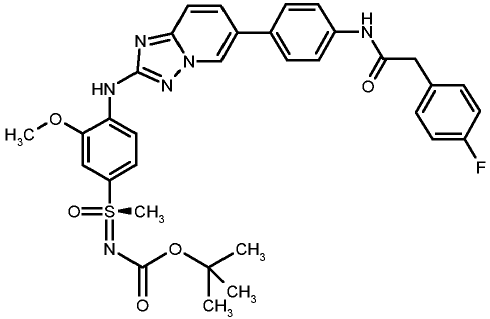

To a stirred suspension of **Int3.4** (350 mg) in toluene (10 mL) and NMP (5 mL) was added **Int10.25.03** (529 mg), chloro(2-dicyclohexylphosphino-2',4',6'-tri-i-propyl-1,1'-biphenyl)[2-(2-aminoethyl)phenyl] palladium(II) methyl-*tert*-butylether adduct (80 mg) and X-Phos (47 mg) and the flask was twice degased and backfilled with argon. The mixture was stirred for 5 minutes at r.t.. Powdered potassium phosphate (1.03 g) was added and the flask was twice degased and backfilled with argon. The mixture was heated to reflux for 1 h. The reaction mixture was filtered through an aminophase-silica-gel column and the solvent was removed in vaccuum. Aminophase-silica-gel chromatography gave a solid that was triturated with ethanol to give 438 mg of the title compound.

### Intermediate Example Int10.25.05

### tert-butyl [{4-[(6-{4-[(4-fluorobenzyl)carbamoyl]phenyl}[1,2,4]triazolo-[1,5-a]pyridin-2-yl)amino]-3-methoxyphenyl}(methyl)oxido-λ6-sulfanylidene]carbamate

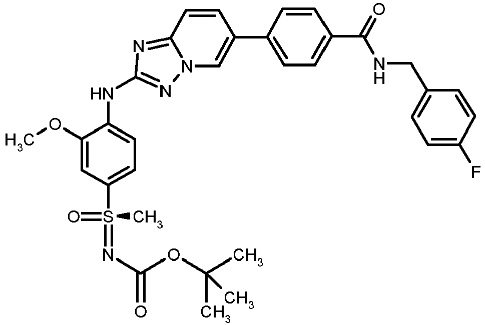

To a stirred suspension of **Int5.2** (100 mg) in toluene (3 mL) and NMP (1.5 mL) was added **Int10.25.03** (151 mg), chloro(2-dicyclohexylphosphino-2',4',6'-tri-i-propyl-1,1'-biphenyl)[2-(2-aminoethyl)phenyl] palladium(II) methyl-*tert*-butylether adduct (23 mg) and X-Phos (13 mg) and the flask was twice degased and backfilled with argon. The mixture was stirred for 5 minutes at r.t.. Powdered potassium phosphate (293 mg) was added and the flask was twice degased and backfilled with argon. The mixture was heated to reflux for 1 h. The reaction mixture was filtered through an aminophase-silica-gel column and the solvent was removed in vaccuum. Aminophase-silica-gel chromatography gave a solid that was triturated with ethanol to give 153 mg of the title compound.

### Intermediate Example Int10.26

### 4-bromo-N-tert-butyl-3-methoxybenzenesulfonamide

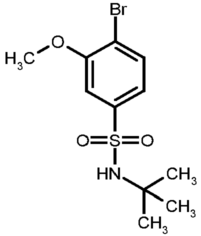

To a stirred solution of 4-bromo-3-methoxybenzenesulfonyl chloride (350 mg) in DMF (4.6 mL) was added *N,N*-diisopropylethylamine (0.18 mL) and *tert-*butylamine (0.26 mL). The mixture was stirred for 16 h. After evaporation of the reaction mixture the resulting residue was partitioned between water (50 mL) and ethyl acetate (30 mL). The organic phase was separated, dried (sodium sulfate) and the solvent was removed in vacuum. Silicagel chromatography afforded 315 mg of the title compound.

### Intermediate Example Int10.27.01

### 1-bromo-4-(chloromethyl)-2-methoxybenzene

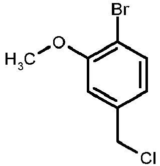

To a stirred solution (4-bromo-3-methoxyphenyl)methanol (660 mg) in DCM (20 mL) was added *N,N*-Diisopropylethylamin (1.59 mL) and methanesulfonyl chloride (0.36 mL) and the mixture was stirred at r.t. for 16 h. A half-saturated solution of sodium bicarbonate was added and the mixture was extracted with DCM. The organic phase was washed with saturated sodium chloride solution, dried (sodium sulfate) and the solvent was removed in vacuum. Silicagel chromatography gave 700 mg of the title compound.

### Intermediate Example Int10.27.02

### 2-[(4-bromo-3-methoxybenzyl)(ethyl)amino]-2-methylpropan-1-ol

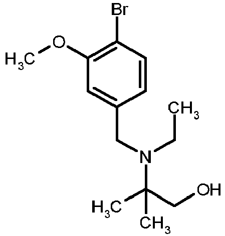

To a stirred solution of **Int10.27.01** (700 mg) in DMF (17 mL) was added potassium carbonate (1.23 g), potassium iodide (50 mg) and 2-(ethylamino)-2-methylpropan-1-ol hydrochloride (0.69 g). The mixture was stirred at 60 °C for 30 minutes and at r.t. for 60 h. The solvent was removed in vaccuum. Water was added, and the mixture was extracted with DCM and methanol (100 : 1 mixture). The organic phase was washed with saturated sodium chloride solution, dried (sodium sulfate) and the solvent was removed in vacuum. Silicagel chromatography gave 900 mg of the title compound.

### Intermediate Example Int10.28.01

### [(4-bromo-3-methoxybenzyl)oxy](tert-butyl)dimethylsilane

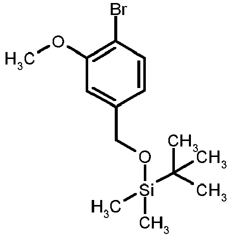

To a stirred solution of (4-bromo-3-methoxyphenyl)methanol (1.8 g) in THF (25 mL), *N,N*-Diisopropylethylamin (1.7 mL), imidazole (56 mg) and *tert-*butyl(chloro)diphenylsilane (1.5 g) were added. The mixture was stirred at r.t. for 3 h. A half-saturated solution of sodium bicarbonate was added and the mixture was extracted with ethyl acetate and hexane (1:1 mixture). The organic phase was washed with saturated sodium chloride solution, dried (sodium sulfate) and the solvent was removed in vacuum. Silicagel chromatography gave 2.7 g of the title compound.

### Intermediate Example Int10.28.02

### N-[4-(2-{[4-({[tert-butyl(dimethyl)silyl]oxy}methyl)-2-methoxyphenyl]-amino}[1,2,4]triazolo[1,5-a]pyridin-6-yl)phenyl]-2-(4-fluorophenyl)-acetamide

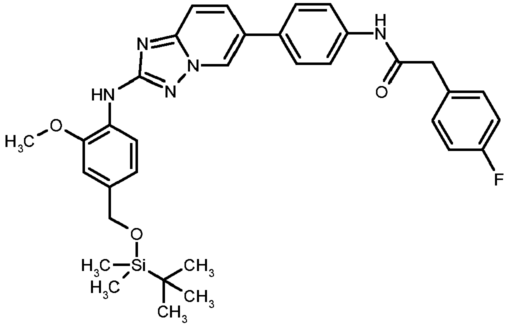

To a stirred suspension of **Int3.4** (100 mg) in toluene (4 mL) and NMP (1 mL) was added **Int10.28.01** (183 mg), chloro(2-dicyclohexylphosphino-2',4',6'-tri-i-propyl-1,1'-biphenyl)[2-(2-aminoethyl)phenyl] palladium(II) methyl-*tert*-butylether adduct (23 mg), X-Phos (13 mg) and powdered potassium phosphate (293 mg) and the flask was twice degased and backfilled with argon. The mixture was heated to reflux for 2 h. The solvent was removed in vaccuum. Silica-gel chromatography gave the title compound as crude product (100 mg) that was used for the next step (deprotection) without purification.

### Intermediate Example Int10.28.03

### 4-(2-{[4-({[tert-butyl(dimethyl)silyl]oxy}methyl)-2-methoxyphenyl]amino}-[1,2,4]triazolo[1,5-a]pyridin-6-yl)-N-(4-fluorobenzyl)benzamide

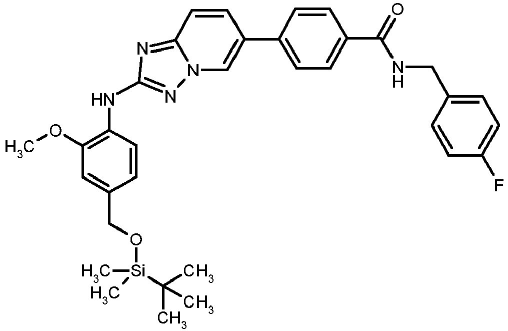

To a stirred suspension of **Int5.2** (100 mg) in toluene (4 mL) and NMP (1 mL) was added **Int10.28.01** (183 mg), chloro(2-dicyclohexylphosphino-2',4',6'-tri-i-propyl-1,1'-biphenyl)[2-(2-aminoethyl)phenyl] palladium(II) methyl-*tert*-butylether adduct (23 mg), X-Phos (13 mg) and powdered potassium phosphate (293 mg) and the flask was twice degased and backfilled with argon. The mixture was heated to reflux for 2 h. The solvent was removed in vaccuum. Silica-gel chromatography gave the title compound as crude product (120 mg) that was used for the next step (deprotection) without purification.

### Intermediate Example Int10.29

### 2-(4-bromo-3-methoxyphenyl)propan-2-ol

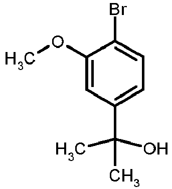

To a stirred solution of **Int10.1** (5.3 g) in THF (250 mL) was added methyl magnesium bromide (21.5 mL; c = 3.0 M) at r.t. and the mixture was heated to reflux for 1 h. A half-saturated aqueous solution of ammounim chloride was added and the mixture was extracted with ethyl acetate. The organic phase was washed with saturated sodium chloride solution, dried (sodium sulfate) and the solvent was removed in vacuum. Silicagel chromatography gave 3.09 g of the title compound.

Starting with **Int11.2** the following intermediates were prepared analogously to the procedures described above.

| **Intermediate** | Structure | Name |
|---|---|---|
| **Int11.18** | 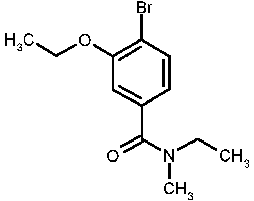 | 4-bromo-3-ethoxy-*N*-ethyl-*N-*methylbenzamide |
| **Int11.19** | 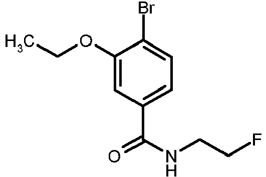 | 4-bromo-3-ethoxy-*N*-(2-fluoroethyl)benzamide |
| **Int11.20** | 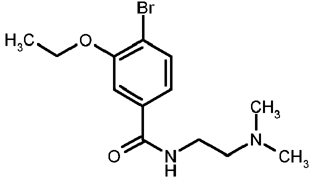 | 4-bromo-*N*-[2-(dimethylamino)ethyl]-3-ethoxybenzamide |

Starting with **Int12.2** the following intermediates were prepared analogously to the procedures described above.

| **Intermediate** | Structure | Name |
|---|---|---|
| **Int 12.10** | 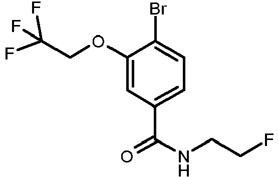 | 4-bromo-*N*-(2-fluoroethyl)-3-(2,2,2-trifluoroethoxy)benzamide |
| **Int12.11** | 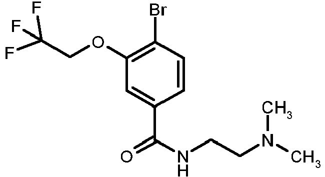 | 4-bromo-*N*-[2-(dimethylamino)-ethyl)-3-(2,2,2-trifluoroethoxy)-benzamide |
| **Int12.12** | 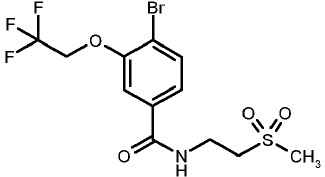 | 4-bromo-*N*-[2-(methylsulfonyl)-ethyl]-3-(2,2,2-trifluoroethoxy)-benzamide |

Starting with **Int12.8** the following intermediate was prepared analogously to the procedures described above.

| **Intermediate** | Structure | Name |
|---|---|---|
| **Int12.13** | 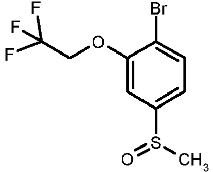 | 4-bromo-3-(2,2,2-trifluoro-ethoxy)phenyl methyl sulfoxide |

### Intermediate Example Int15.07.01

### ethyl 4-amino-3-(trifluoromethoxy)benzoate

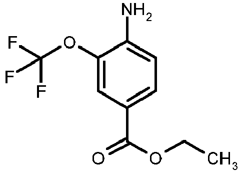

To a stirred solution of 4-amino-3-(trifluoromethoxy)benzoic acid (5.0 g) in ethanol (100 mL) was added thionyl chloride (2.47 mL) at 0 °C. The mixture was heated to reflux for 3 h. The solvent was removed in vacuum. The residue was dissolved in ethyl acetate and the mixture was washed with a half-saturated solution of sodium bicarbonate and with saturated sodium chloride solution, dried (sodium sulfate) and the solvent was removed in vacuum, to give 4.81 g of the title compound, that was used for the next step without purification.

### Intermediate Example Int15.07.02

### ethyl 4-bromo-3-(trifluoromethoxy)benzoate

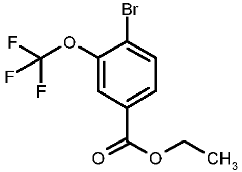

To a stirred solution of **Int15.07.01** (4.8 g) in concentrated aqueous hydrobromic acid (53 mL) was added at 5 °C 9.0 mL of a 3M solution of sodium nitrite. The mixture was stirred for 10 minutes and copper(1) bromide (2.76 g) was added. The mixture was stirred for 5 minutes and water (125 mL) was added and the mixture was stirred for 1h. The mixture was extracted with ethyl acetate. The organic phase was washed with saturated sodium chloride solution, dried (sodium sulfate) and the solvent was removed in vacuum. Silica gel chromatography gave 4.1 g of the title compound.

### Intermediate Example Int15.07.03

### 4-bromo-3-(trifluoromethoxy)benzoic acid

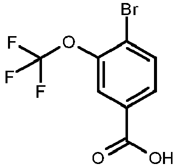

To a stirred solution of **Int15.07.02** (4.1 g) in ethanol (150 mL) was added 9.8 mL of a 2M solution of sodium hydroxide and the solution was stirred for 2 h at r.t.. An aqueous solution of hydrochloric acid was added until pH 3 was reached. About 100 mL of solvent were removed in vaccuum, water was added and the residue was extracted with ethyl acetate. The solution was dried (sodium sulfate) and the solvent was removed in vacuum, to give 3.4 g of the title compound, that was used for the next step without purification.

### Intermediate Example Int15.07.04

### 4-bromo-3-(trifluoromethoxy)benzamide

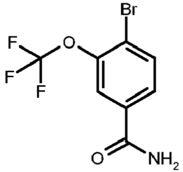

To a stirred solution of **Int15.07.03** (1.50 g) in DCM (80 mL) was added DMF (0.05 mL) and oxalyl choride (0.85 g). The mixture was stirred at r.t. for 0.5 h. The solvent was removed in vacuum, and the residue was dissolved in DCM (40 mL). A concentrated solution of ammonia in water (2.0 mL) was added and the mixture was stirred at r.t. for 1 h. Water was added and the reaction mixture was extracted with DCM. The organic phase was washed with saturated sodium chloride solution, dried (sodium sulfate) and the solvent was removed in vacuum. Silica gel chromatography gave 1.32 g of the title compound.

Starting with **Int17.1** the following intermediate was prepared analogously to the procedures described above.

| **Intermediate** | Structure | Name |
|---|---|---|
| **Int15.08** | 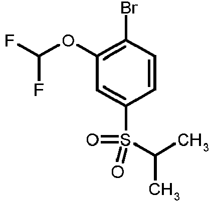 | 4-bromo-3-(difluoromethoxy)-phenyl isopropyl sulfone |

Starting with 4-bromo-3-methylbenzoic acid the following intermediates were prepared analogously to the procedures described above.

| **Intermediate** | Structure | Name |
|---|---|---|
| **Int15.11** | 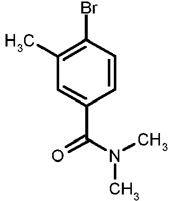 | 4-bromo-*N,N*,3-trimethyl-benzamide |
| **Int15.12** | 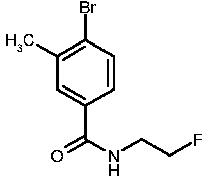 | 4-bromo-*N*-(2-fluoroethyl)-3-methylbenzamide |
| **Int15.13** | 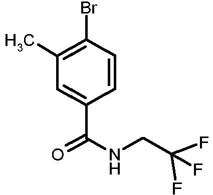 | 4-bromo-3-methyl-*N*-(2,2,2-trifluoroethyl)benzamide |

Starting with 1-bromo-2-methyl-4-(methylsulfanyl)benzene the following intermediates were prepared analogously to the procedures described above.

| **Intermediate** | Structure | Name |
|---|---|---|
| **Int15.14** | 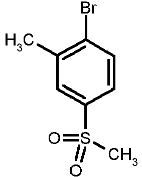 | 4-bromo-3-methylphenyl methyl sulfone |
| **Int15.15** | 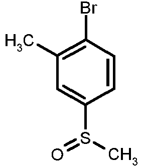 | 4-bromo-3-methylphenyl methyl sulfoxide |

### Intermediate Example Int15.17.01

### 5-bromo-2-ethenyl-4-methylpyridine

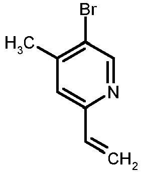

To a stirred solution of 2,5-dibromo-4-methylpyridine (5.1 g) in 1-propanol (200 mL) was added 2M potassium carbonate solution (30 mL), 2,4,6-trivinylboroxin-pyridine complex (2.0 g), triphenylphosphine (219 mg) and PdCl₂(PPh₃)₂ (1.40 g). The mixture was heated to reflux for 2 h. The reaction mixture was filtered and the solvent was removed in vaccuum. Water (100 mL) was added and the mixture was extracted with ethyl acetate and hexanes (1:1 mixture). The organic phase was washed with saturated sodium chloride solution, dried (sodium sulfate) and the solvent was removed in vacuum. Silicagel chromatography gave 1.66 g of the title compound.

### Intermediate Example Int15.17.02

### potassium 5-bromo-4-methylpyridine-2-carboxylate

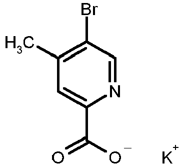

To a stirred solution of **Int15.17.01** (1.66 g) in acetone (65 mL) and water (65 mL) was added a potassium permanganate (2.65 g). The mixture was stirred at r.t. for 60 h. The reaction mixture was filtered and the solvent was removed in vaccuum to give 2.4 g of the title compound as a crude product, that was used for the next step without purification.

### Intermediate Example Int15.17.03

### methyl 5-bromo-4-methylpyridine-2-carboxylate

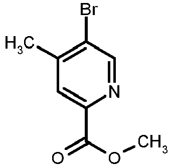

To a stirred suspension of the crude product **Int15.17.02** (2.4 g) in methanol (90 mL) was added thionyl dichloride (2.01 mL). The mixture was heated to reflux for 2 h. The solvent was removed in vaccuum. A half-saturated solution of sodium bicarbonate was added and the mixture was extracted with ethyl acetate. The organic phase was washed with saturated sodium chloride solution, dried (sodium sulfate) and the solvent was removed in vacuum. Silicagel chromatography gave 1.0 g of the title compound.

### Intermediate Example Int15.17.04

### 5-bromo-4-methylpyridine-2-carboxylic acid

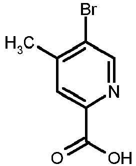

To a stirred solution of **Int15.17.03** (1.0 g) in THF (20 mL), methanol (5 mL) and water (5 mL) was added an aqueous solution of lithium hydroxide (6.1 mL; c = 1M). The mixture was stirred at r.t. for 1 h. Aqueous hydrochloric acid was added, until pH 4 was reached. The mixture was extracted with chloroform using a continous liquid/liquid extractor (from Normag Labor- und Prozesstechnik GmbH, Ilmenau, Germany) for 16 h. The solvent was removed in vacuum to give 870 mg of the title compound.

Starting with **Int15.17.04** the following intermediates were prepared analogously to the procedures described above.

| **Intermediate** | Structure | Name |
|---|---|---|
| **Int15.18** | 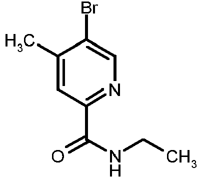 | 5-bromo-*N*-ethyl-4-methyl-pyridine-2-carboxamide |
| **Int15.19** | 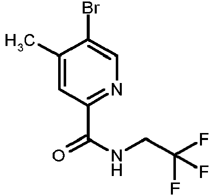 | 5-bromo-4-methyl-*N*-(2,2,2-trifluoroethyl)pyridine-2-carboxamide |

Starting with 1-bromo-2-fluoro-4-(methylsulfanyl)benzene the following intermediates was prepared analogously to the procedures described above.

| **Intermediate** | Structure | Name |
|---|---|---|
| **Int15.20** | 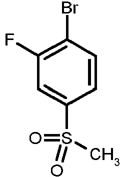 | 4-bromo-3-fluorophenyl methyl sulfone |

### EXAMPLES

### Compounds of the present invention

### Example01.1

### N,N-diethyl-4-{[6-(4-{[(4-fluorophenyl)acetyl]amino}phenyl)[1,2,4]-triazolo[1,5-a]pyridin-2-yl]amino}-3-methoxybenzamide

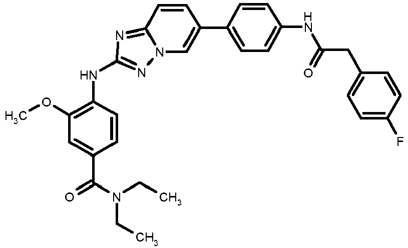

To a stirred suspension of **Int3.4** (150 mg) in toluene (3.5 mL) and NMP (0.5 mL) was added 4-bromo-*N,N*-diethyl-3-methoxybenzamide (237 mg), Pd₂dba₃ (19 mg) and rac-BINAP (26 mg). The flask was twice degased and backfilled with argon. The mixture was stirred at r.t. for 5 minutes. Caesium carbonate (405 mg) was added, the flask was twice degased and backfilled with argon and the mixture was heated to reflux for 20 h. Water was added and the reaction mixture was extracted with ethyl acetate. The organic phase was washed with saturated sodium chloride solution, dried (sodium sulfate) and the solvent was removed in vacuum. Silica gel chromatography gave a solid that was titurated with cyclohexane to give 27 mg of the title compound.

¹H-NMR (300MHz, DMSO-d₆): δ [ppm]= 1.10 (t, 6H), 3.33 (br. s, 4H), 3.64 (s, 2H), 3.87 (s, 3H), 6.91 - 7.00 (m, 2H), 7.13 (t, 2H), 7.34 (dd, 2H), 7.57 - 7.77 (m, 5H), 7.90 (dd, 1 H), 8.17 (s, 1 H), 8.27 (d, 1 H), 9.09 (s, 1 H), 10.27 (s, 1 H).

Starting with Intermediate **Int3.4,** the Examples **Example01.2** to **Example01.5** were prepared analogously to the procedure for the preparation of **Example01.1.**

### Example01.2

### N-(4-{2-[(4-cyano-2-methoxyphenyl)amino][1,2,4]triazolo[1,5-a]pyridin-6-yl}phenyl)-2-(4-fluorophenyl)acetamide

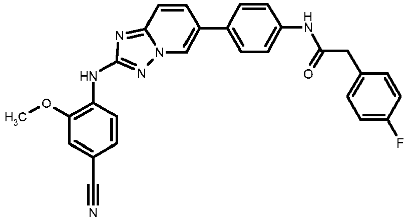

¹H-NMR (300MHz, DMSO-d₆): δ [ppm]= 3.64 (s, 2H), 3.90 (s, 3H), 7.13 (s, 2H), 7.34 (dd, 2H), 7.39 - 7.49 (m, 2H), 7.63 - 7.77 (m, 5H), 7.93 (dd, 1 H), 8.45 (d, 1H), 8.69 (s, 1H), 9.12 (d, 1H), 10.28 (s, 1H).
Starting materials: Intermediate **Int3.4;** 4-bromo-3-methoxybenzonitrile

### Example01.3

### N-(4-{2-[(2-ethoxy-4-fluorophenyl)amino][1,2,4]triazolo[1,5-a]pyridin-6-yl}phenyl)-2-(4-fluorophenyl)acetamide

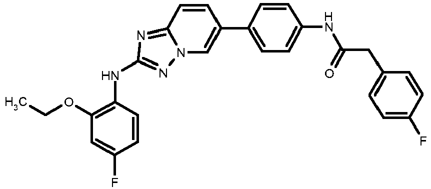

¹H-NMR (400MHz, DMSO-d₆): δ [ppm]= 1.37 (t, 3H), 3.64 (s, 2H), 4.10 (q, 2H), 6.76 (td, 1H), 6.93 (dd, 1H), 7.13 (t, 2H), 7.34 (dd, 2H), 7.58 (d, 1H), 7.64 - 7.74 (m, 4H), 7.87 (dd, 1H), 7.90 (s, 1H), 8.08 - 8.18 (m, 1H), 9.05 (s, 1H), 10.28 (s, 1H).
Starting materials: Intermediate **Int3.4;** 1-bromo-2-ethoxy-4-fluorobenzene

### Example01.4

### N-ethyl-4-{[6-(4-{[(4-fluorophenyl)acetyl]amino}phenyl)[1,2,4]triazolo[1,5-a]pyridin-2-yl]amino}-3-methoxybenzamide

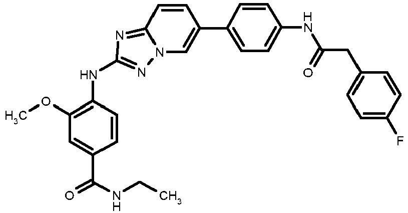

¹H-NMR (300MHz, DMSO-d₆): δ [ppm]= 1.10 (t, 3H), 3.19 - 3.29 (m, 2H), 3.64 (s, 2H), 3.91 (s, 3H), 7.13 (t, 2H), 7.34 (dd, 2H), 7.44 - 7.52 (m, 2H), 7.59 - 7.78 (m, 5H), 7.91 (dd, 1H), 8.21 - 8.35 (m, 3H), 9.11 (d, 1H), 10.28 (s, 1H). Starting materials: Intermediate **Int3.4;** commercial 4-bromo-*N*-ethyl-3-methoxybenzamide

### Example01.5

### N-tert-butyl-4-{[6-(4-{[(4-fluorophenyl)acetyl]amino}phenyl)[1,2,4]-triazolo[1,5-a]pyridin-2-yl]amino}-3-methoxybenzamide

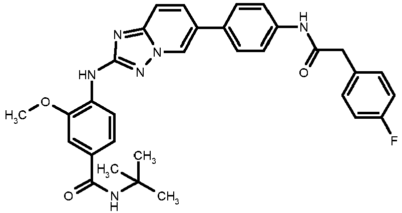

¹H-NMR (300MHz, DMSO-d₆): δ [ppm]= 1.36 (s, 9H), 3.64 (s, 2H), 3.91 (s, 3H), 7.13 (t, 2H), 7.34 (dd, 2H), 7.39 - 7.50 (m, 2H), 7.55 (s, 1 H), 7.60 - 7.78 (m, 5H), 7.91 (dd, 1 H), 8.13 - 8.39 (m, 2H), 9.10 (d, 1 H), 10.28 (s, 1 H).
Starting materials: Intermediate **Int3.4;** commercial 4-bromo-*N*-*tert*-butyl-3-methoxybenzamide

### Example01.6

### 4-{[6-(4-{[(4-fluorophenyl)acetyl]amino}phenyl)[1,2,4]triazolo[1,5-a]-pyridin-2-yl]amino}-N-(2-hydroxyethyl)-3-methoxybenzamide

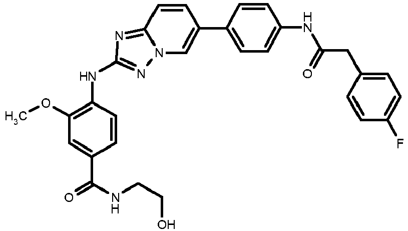

To a stirred suspension of **Int3.4** (100 mg) in toluene (4.0 mL) and NMP (0.4 mL) in a sealed tube was added **Int10.3** (114 mg), chloro(2-dicyclohexylphosphino-2',4',6'-tri-i-propyl-1,1'-biphenyl)[2-(2-aminoethyl)-phenyl] palladium(II) methyl-*tert*-butylether adduct (23 mg), X-Phos (13 mg), and powdered potassium phosphate (294 mg). The flask was twice degased and backfilled with argon. The mixture was heated to 130°C with an oil bath for 2 h. Water was added and the reaction mixture was extracted with a mixture of DCM and methanol (100:1). The organic phase was washed with saturated sodium chloride solution, dried (sodium sulfate) and the solvent was removed in vacuum. Aminophase-silica-gel chromatography gave a solid that was titurated with warm ethanol to give 40 mg of the title compound.

¹H-NMR (300MHz, DMSO-d₆): δ [ppm]= 3.30 - 3.36 (m, 2H), 3.41 - 3.54 (m, 2H), 3.64 (s, 2H), 3.91 (s, 3H), 4.72 (t, 1 H), 7.06 - 7.19 (m, 2H), 7.34 (dd, 2H), 7.46 - 7.55 (m, 2H), 7.59 - 7.78 (m, 5H), 7.91 (dd, 1H), 8.21 - 8.37 (m, 3H), 9.11 (s, 1H), 10.28 (s, 1H).

Starting with Intermediate **Int3.4,** the Examples **Example01.7** to **Example01.11** were prepared analogously to the procedure for the preparation of **Example01.6.**

### Example01.7

### N-(2-ethoxyethyl)-4-{[6-(4-{[(4-fluorophenyl)acetyl]amino}phenyl)[1,2,4]-triazolo[1,5-a]pyridin-2-yl]amino}-3-methoxybenzamide

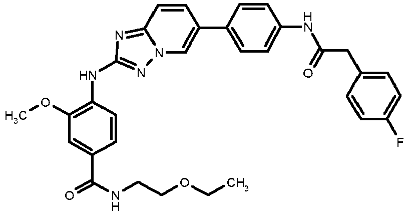

¹H-NMR (400MHz, DMSO-d₆): δ [ppm]= 1.09 (t, 3H), 3.34 - 3.50 (m, 6H), 3.64 (s, 2H), 3.91 (s, 3H), 7.13 (t, 2H), 7.35 (dd, 2H), 7.47 - 7.54 (m, 2H), 7.61 - 7.76 (m, 5H), 7.91 (dd, 1H), 8.23 - 8.34 (m, 2H), 8.39 (t, 1H), 9.12 (s, 1H), 10.29 (s, 1 H).
Starting materials: Intermediate **Int3.4;** 4-bromo-N-(2-ethoxyethyl)-3-methoxybenzamide **(Int10.6)**

### Example01.8

### 3-ethoxy-4-{[6-(4-{[(4-fluorophenyl)acetyl]amino}phenyl)[1,2,4]triazolo-[1,5-a]pyridin-2-yl]amino}-N-(2-hydroxyethyl)benzamide

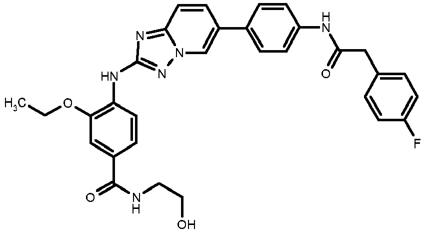

¹H-NMR (300MHz, DMSO-d₆): δ [ppm]= 1.42 (t, 3H), 3.24 - 3.35 (m, 2H), 3.42 - 3.53 (m, 2H), 3.64 (s, 2H), 4.17 (q, 2H), 4.70 (t, 1 H), 7.13 (t, 2H), 7.34 (dd, 2H), 7.45 - 7.54 (m, 2H), 7.59 - 7.78 (m, 5H), 7.91 (dd, 1H), 8.14 (s, 1H), 8.23 - 8.36 (m, 2H), 9.11 (s, 1 H), 10.27 (s, 1 H).
Starting materials: Intermediate **Int3.4;** 4-bromo-3-ethoxy-N-(2-hydroxyethyl)-benzamide **(Int11.5)**

### Example01.9

### 3-ethoxy-4-{[6-(4-{[(4-fluorophenyl)acetyl]amino}phenyl)[1,2,4]triazolo-[1,5-a]pyridin-2-yl]amino}-N-(1-hydroxy-2-methylpropan-2-yl)benzamide

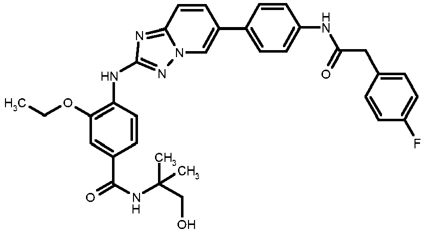

¹H-NMR (300MHz, DMSO-d₆): δ [ppm]= 1.29 (s, 6H), 1.42 (t, 3H), 3.49 (d, 2H), 3.64 (s, 2H), 4.17 (q, 2H), 4.92 (t, 1 H), 7.06 - 7.19 (m, 2H), 7.27 - 7.50 (m, 5H), 7.58 - 7.77 (m, 5H), 7.91 (dd, 1 H), 8.13 (s, 1 H), 8.29 (d, 1 H), 9.09 (s, 1 H), 10.28 (s, 1H).
Starting materials: Intermediate **Int3.4;** 4-bromo-3-ethoxy-N-(l-hydroxy-2-methylpropan-2-yl)benzamide **(Int11.7)**

### Example01.10

### 3-ethoxy-N,N-diethyl-4-{[6-(4-{[(4-fluorophenyl)acetyl]amino}phenyl)-[1,2,4]triazolo[1,5-a]pyridin-2-yl]amino}benzamide

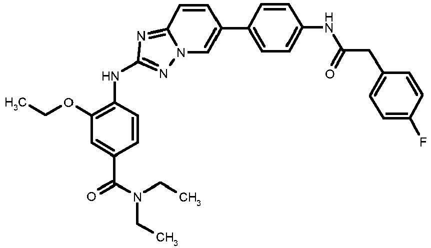

¹H-NMR (300MHz, DMSO-d₆, detected signals): δ [ppm]= 1.09 (t, 6H), 1.39 (t, 3H), 3.64 (s, 2H), 4.13 (q, 2H), 6.89 - 6.99 (m, 2H), 7.13 (t, 2H), 7.29 - 7.39 (m, 2H), 7.58 - 7.76 (m, 5H), 7.90 (dd, 1 H), 8.07 (s, 1 H), 8.28 (d, 1 H), 9.09 (s, 1H), 10.28 (s, 1H).
Starting materials: Intermediate **Int3.4;** 4-bromo-3-ethoxy-*N,N*-diethylbenzamide **(Int11.8)**

### Example01.11

### 4-{[6-(4-{[(4-fluorophenyl)acetyl]amino}phenyl)[1,2,4]triazolo[1,5-a]-pyridin-2-yl]amino}-N-(2-hydroxyethyl)-3-(2,2,2-trifluoroethoxy)benzamide

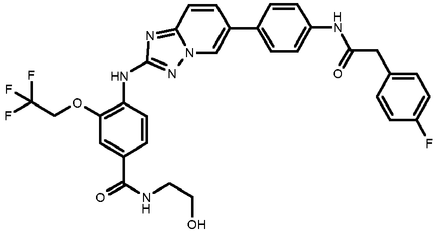

¹H-NMR (300MHz, DMSO-d₆): δ [ppm]= 3.25 - 3.37 (m, 2H), 3.49 (q, 2H), 3.64 (s, 2H), 4.73 (t, 1 H), 4.89 (q, 2H), 7.13 (t, 2H), 7.27 - 7.42 (m, 2H), 7.54 - 7.78 (m, 7H), 7.92 (dd, 1H), 8.22 (s, 1H), 8.25 - 8.40 (m, 2H), 9.12 (d, 1H), 10.28 (s, 1 H).
Starting materials: Intermediate **Int3.4;** 4-bromo-*N*-(2-hydroxyethyl)-3-(2,2,2-trifluoroethoxy)benzamide **(Int12.3)**

### Example01.12

### 4-{[6-(4-{[(4-fluorophenyl)acetyl]amino}phenyl)[1,2,4]triazolo[1,5-a]-pyridin-2-yl]amino}-N-(2-hydroxy-2-methylpropyl)-3-methoxybenzamide

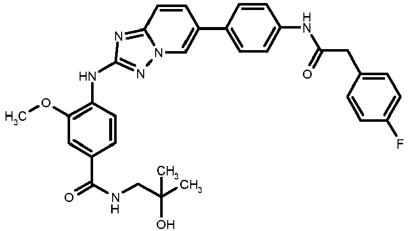

To a stirred suspension of **Int3.4** (100 mg) in toluene (3.0 mL) and NMP (1.0 mL) was added **Int10.4** (167 mg), chloro(2-dicyclohexylphosphino-2',4',6'-tri-i-propyl-1,1'-biphenyl)[2-(2-aminoethyl)phenyl] palladium(II) methyl-*tert*-butylether adduct (23 mg) and X-Phos (13 mg) and the flask was twice degased and backfilled with argon. The mixture was stirred for 5 minutes at r.t.. Powdered potassium phosphate (294 mg) was added and the flask was twice degased and backfilled with argon. The mixture was heated to reflux for 2 h. Water was added and the reaction mixture was extracted with ethyl acetate. The organic phase was washed with saturated sodium chloride solution, dried (sodium sulfate) and the solvent was removed in vacuum. Aminophase-silica-gel chromatography gave a solid that was recrystallized from ethanol to give 106 mg of the title compound.

¹H-NMR (400MHz, DMSO-d₆): δ [ppm]= 1.08 (s, 6H), 3.23 (d, 2H), 3.64 (s, 2H), 3.92 (s, 3H), 4.58 (s, 1 H), 7.13 (t, 2H), 7.35 (dd, 2H), 7.48 - 7.57 (m, 2H), 7.60 - 7.77 (m, 5H), 7.91 (dd, 1H), 8.16 (t, 1H), 8.23 - 8.39 (m, 2H), 9.11 (d, 1H), 10.30 (s, 1H).

Starting with Intermediate **Int3.4,** the Examples **Example01.13** to **Example01.18** were prepared analogously to the procedure for the preparation of **Example01.12.**

### Example01.13

### 4-{[6-(4-{[(4-fluorophenyl)acetyl]amino}phenyl)[1,2,4]triazolo[1,5-a]-pyridin-2-yl]amino}-N-(1-hydroxy-2-methylpropan-2-yl)-3-methoxybenzamide

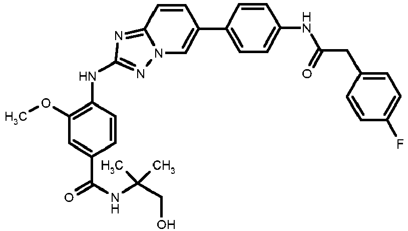

¹H-NMR (300MHz, DMSO-d₆): δ [ppm]= 1.29 (s, 6H), 3.49 (d, 2H), 3.64 (s, 2H), 3.91 (s, 3H), 4.93 (t, 1 H), 7.06 - 7.18 (m, 2H), 7.29 - 7.49 (m, 5H), 7.60 - 7.77 (m, 5H), 7.90 (dd, 1H), 8.19 - 8.35 (m, 2H), 9.09 (d, 1H), 10.28 (s, 1H). Starting materials: Intermediate **Int3.4;** 4-bromo-*N*-(1-hydroxy-2-methyl-propan-2-yl)-3-methoxybenzamide **(Int10.5)**

### Example01.14

### N-{2-[acetyl(methyl)amino]ethyl}-4-{[6-(4-{[(4-fluorophenyl)acetyl]amino}-phenyl)[1,2,4]triazolo[1,5-a]pyridin-2-yl]amino}-3-methoxy-N-methylbenzamide

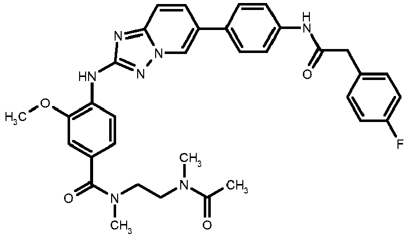

¹H-NMR (500MHz, DMSO-d₆): δ [ppm]= 1.97 (s, 3H), 2.71 - 3.13 (m, 6H), 3.55 (br. d, 4H), 3.70 (s, 2H), 3.95 (s, 3H), 6.99 - 7.07 (m, 2H), 7.10 - 7.19 (m, 2H), 7.35 - 7.45 (m, 2H), 7.64 (dd, 1H), 7.73 (s, 4H), 7.91 (dd, 1H), 7.95 (br. s., 1H), 8.32 (d, 1H), 9.02 (s, 1H), 10.05 (s, 1H).
Starting materials: Intermediate **Int3.4;** *N*-{2-[acetyl(methyl)amino]ethyl}-4-bromo-3-methoxy-*N*-methylbenzamide **(Int10.8)**

### Example01.15

### 2-(4-fluorophenyl)-N-[4-(2-{[2-methoxy-4-(methylsulfonyl)phenyl]amino}-[1,2,4]triazolo[1,5-a]pyridin-6-yl)phenyl]acetamide

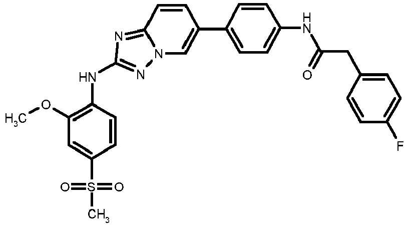

¹H-NMR (400MHz, DMSO-d₆): δ [ppm]= 3.16 (s, 3H), 3.64 (s, 2H), 3.96 (s, 3H), 7.08 - 7.17 (m, 2H), 7.30 - 7.37 (m, 2H), 7.42 (d, 1 H), 7.52 (dd, 1 H), 7.65 - 7.76 (m, 5H), 7.93 (dd, 1 H), 8.48 (d, 1 H), 8.64 (s, 1 H), 9.12 (dd, 1 H), 10.29 (s, 1 H). Starting materials: Intermediate **Int3.4;** 1-bromo-2-methoxy-4-(methyl-sulfonyl)benzene **(Int10.11)**

### Example01.16

### 3-ethoxy-N-ethyl-4-{[6-(4-{[(4-fluorophenyl)acetyl]amino}phenyl)[1,2,4]-triazolo[1,5-a]pyridin-2-yl]amino}benzamide

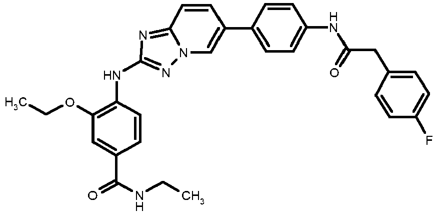

¹H-NMR (400MHz, DMSO-d₆): δ [ppm]= 1.10 (t, 3H), 1.42 (t, 3H), 3.21 - 3.27 (m, 2H), 3.64 (s, 2H), 4.17 (q, 2H), 7.13 (t, 2H), 7.35 (dd, 2H), 7.44 - 7.51 (m, 2H), 7.59 - 7.77 (m, 5H), 7.91 (d, 1H), 8.14 (s, 1H), 8.25 - 8.34 (m, 2H), 9.11 (s, 1H), 10.29 (s, 1H).
Starting materials: Intermediate **Int3.4;** 4-bromo-3-ethoxy-*N*-ethylbenzamide **(Int11.4)**

### Example01.17

### 3-ethoxy-4-{[6-(4-{[(4-fluorophenyl)acetyl]amino}phenyl)[1,2,4]triazolo-[1,5-a]pyridin-2-yl]amino}-N-(2-hydroxy-2-methylpropyl)benzamide

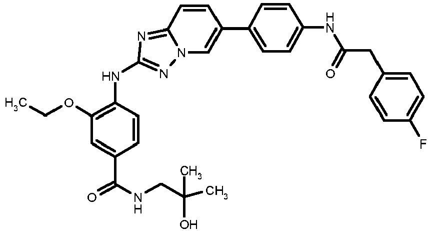

¹H-NMR (400MHz, DMSO-d₆): δ [ppm]= 1.08 (s, 6H), 1.42 (t, 3H), 3.23 (d, 2H), 3.64 (s, 2H), 4.18 (q, 2H), 4.57 (s, 1 H), 7.09 - 7.17 (m, 2H), 7.31 - 7.38 (m, 2H), 7.48 - 7.55 (m, 2H), 7.64 (d, 1 H), 7.66 - 7.76 (m, 4H), 7.91 (dd, 1 H), 8.10 - 8.18 (m, 2H), 8.32 (d, 1 H), 9.05 - 9.17 (m, 1 H), 10.29 (s, 1 H).
Starting materials: Intermediate **Int3.4;** 4-bromo-3-ethoxy-*N*-(2-hydroxy-2-methylpropyl)benzamide **(Int11.6)**

### Example01.18

### 3-ethoxy-N-ethyl-4-{[6-(4-{[(4-fluorophenyl)acetyl]amino}phenyl)[1,2,4]-triazolo[1,5-a]pyridin-2-yl]amino}-N-(2-methoxyethyl)benzamide

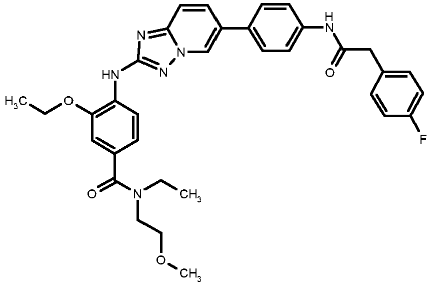

¹H-NMR (300MHz, DMSO-d₆): δ [ppm]= 1.08 (t, 3H), 1.39 (t, 3H), 3.22 (s, 3H), 3.46 (br. s., 6H), 3.64 (s, 2H), 4.12 (d, 2H), 6.92 - 7.03 (m, 2H), 7.13 (t, 2H), 7.34 (dd, 2H), 7.56 - 7.78 (m, 5H), 7.90 (dd, 1H), 8.07 (s, 1H), 8.28 (d, 1H), 9.09 (s, 1H), 10.28 (s, 1H).
Starting materials: Intermediate **Int3.4;** 4-bromo-3-ethoxy-*N*-ethyl-*N*-(2-methoxyethyl)benzamide **(Int11.9)**

### Example01.19

### 3-ethoxy-4-{[6-(4-{[(4-fluorophenyl)acetyl]amino}phenyl)[1,2,4]triazolo-[1,5-a]pyridin-2-yl]amino}-N-(2-hydroxyethyl)-N-methylbenzamide

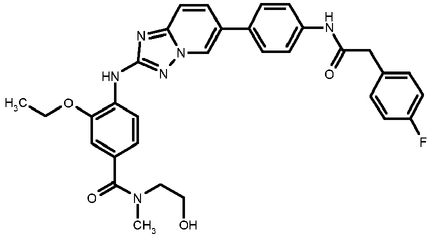

To a stirred suspension of **Int3.4** (100 mg) in toluene (3.0 mL) and NMP (0.5 mL) was added **Int11.11** (176 mg), chloro(2-dicyclohexylphosphino-2',4',6'-tri-i-propyl-1,1'-biphenyl)[2-(2-aminoethyl)phenyl] palladium(II) methyl-*tert*-butylether adduct (16 mg) and X-Phos (9 mg) and the flask was twice degased and backfilled with argon. The mixture was stirred for 5 minutes at r.t.. Powdered potassium phosphate (294 mg) was added and the flask was twice degased and backfilled with argon. The mixture was heated to reflux for 1 h. Further chloro(2-dicyclohexylphosphino-2',4',6'-tri-i-propyl-1,1'-biphenyl)[2-(2-aminoethyl)phenyl] palladium(II) methyl-*tert*-butylether adduct (16 mg) and X-Phos (9 mg) was added, the flask was twice degased and backfilled with argon and the mixture was heated to reflux for 2h. Water was added and the reaction mixture was extracted with ethyl acetate and methanol (10:1). The organic phase was washed with saturated sodium chloride solution, dried (sodium sulfate) and the solvent was removed in vacuum. Ethanol (5 mL) and 2N hydrochloric acid (1 mL) was added to the residue and the mixture was stirred for 15 minutes. A half-saturated solution of sodium bicarbonate was added and the mixture was extracted with ethyl acetate. The organic phase was washed with saturated sodium chloride solution, dried (sodium sulfate) and the solvent was removed in vacuum. Aminophase-silica-gel chromatography gave a solid that was titurated with diisopropyl ether to give 84 mg of the title compound.

¹H-NMR (400MHz, DMSO-d₆): δ [ppm]= 1.40 (t, 3H), 2.95 (br. s., 3H), 3.32 - 3.60 (m, 4H), 3.64 (s, 2H), 4.12 (q, 2H), 4.79 (br. s., 1H), 6.98 - 7.08 (m, 2H), 7.13 (t, 2H), 7.35 (dd, 2H), 7.62 (d, 1 H), 7.65 - 7.76 (m, 4H), 7.90 (dd, 1 H), 8.07 (s, 1 H), 8.28 (d, 1 H), 9.09 (br. d, 1 H), 10.28 (s, 1 H).

### Example01.20

### 4-{[6-(4-{[(4-fluorophenyl)acetyl]amino}phenyl)[1,2,4]triazolo[1,5-a]-pyridin-2-yl]amino}-3-methoxy-N-methyl-N-[2-(methylamino)ethyl]-benzamide

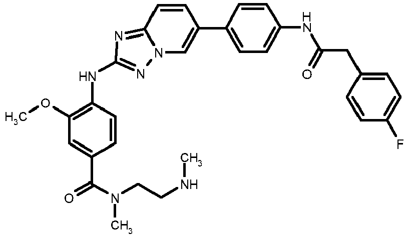

To a stirred suspension of **Intermediate Example Int16.1** (125 mg) in DCM (3 mL) was added TFA (1.5 mL). The mixture was stirred at r.t. for 2 h. A half-saturated solution of sodium bicarbonate was added until pH 9 was reached. The precipitated solid was collected by filtration. Purification by aminophase-silica-gel chromatography gave 78 mg of the title compound.

¹H-NMR (500MHz, DMSO-d₆, detected signals): δ [ppm]= 2.30 (s, 3H), 2.72 (t, 2H), 3.01 (s, 3H), 3.45 (t, 2H), 3.70 (s, 2H), 3.95 (s, 3H), 7.06 (dd, 1 H), 7.10 (d, 1 H), 7.12 - 7.18 (m, 2H), 7.36 - 7.44 (m, 2H), 7.64 (dd, 1 H), 7.73 (s, 4H), 7.91 (dd, 1H), 7.93 (br. s, 1H), 8.31 (d, 1H), 8.96 - 9.07 (m, 1H), 10.05 (s, 1H).

### Example01.21

### N-tert-butyl-4-{[6-(4-{[(4-fluorophenyl)acetyl]amino}phenyl)[1,2,4]-triazolo[1,5-a]pyridin-2-yl]amino}-3-(2,2,2-trifluoroethoxy)benzamide

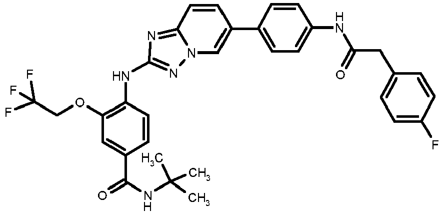

*N-*[4-(2-Amino[1,2,4]triazolo[1,5-*a*]pyridin-6-yl)phenyl]-2-(4-fluorophenyl)acetamide **(Int3.4)** (100 mg), *N*-*tert*-butyl-4-iodo-3-(2,2,2-trifluoroethoxy)benzamide **Int13.2** (133 mg), chloro(dicyclohexyl(2',4',6'-triisopropyl-3,6-di-methoxybiphenyl-2-yl)phosphine- [2-(2-aminoethyl)phenyl] palladium(II) (8.8 mg), Brett-Phos (5.9 mg), and sodium *tert*-butoxide (48.6 mg) were pre-mixed, and degassed toluene (2.0 mL) was added. The mixture was heated for 12h to 130 °C, then diluted with ethyl acetate and washed with satd. aqueous sodium carbonate solution. The organic layer was dried over sodium sulfate, and the solvent was evaporated. The crude product was purified by flash chromatography on silica gel (20 g, eluent: ethyl acetate/ cyclohexane 5:1) to yield 34 mg (18%) of the title compound.

¹H-NMR (400MHz, DMSO-d₆): δ [ppm]= 1.40 (s, 9H), 3.68 (s, 2H), 4.95 (q, 2H), 7.13 - 7.20 (m, 2H), 7.35 - 7.41 (m, 2H), 7.56 - 7.63 (m, 3H), 7.68 (d, 1H), 7.70 - 7.79 (m, 4H), 7.96 (dd, 1H), 8.19 (s, 1H), 8.34 (d, 1H), 9.14 (s, 1H), 10.31 (s, 1 H).

### Example01.22

### N,N-diethyl-4-{[6-(4-{[(4-fluorophenyl)acetyl]amino}phenyl)[1,2,4]triazolo-[1,5-a]pyridin-2-yl]amino}-3-(2,2,2-trifluoroethoxy)benzamide

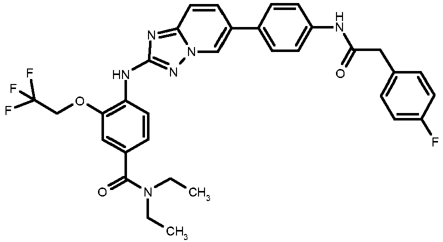

*N*-[4-(2-Amino[1,2,4]triazolo[1,5-a]pyridin-6-yl)phenyl]-2-(4-fluorophenyl)acetamide **(Int3.4)** (40.5 mg), *N,N*-diethyl-4-iodo-3-(2,2,2-trifluoroethoxy)benzamide **Int15.2** (54 mg), chloro(2-dicyclohexylphosphino-2',4',6'-tri-i-propyl-1,1'-biphenyl)[2-(2-aminoethyl)phenyl] palladium(II) *tert* butyl methylether adduct (3.7 mg), X-Phos (2.1 mg), and sodium *tert*-butoxide (19.7 mg) were pre-mixed, and degassed toluene (1.3 mL) was added. The mixture was heated for 8 h to 130 °C, then diluted with DCM and washed with satd. aqueous sodium carbonate solution. The organic layer was dried over sodium sulfate, and the solvent was evaporated. The crude product was purified by flash chromatography on silica gel (20 g, eluent: ethyl acetate/ cyclohexane gradient 2:1 to 4:1) to yield 25 mg (35%) of the title compound.

¹H-NMR (400MHz, DMSO-d₆): δ [ppm]= 1.13 (t, 6H), (4H under water), 3.68 (s, 2H), 4.92 (q, 2H), 7.10 (dd, 1H), 7.13 - 7.19 (m, 2H), 7.19 - 7.21 (m, 1H), 7.35-7.41 (m, 2H), 7.67 (d, 1H), 7.69 - 7.78 (m, 4H), 7.94 (dd, 1H), 8.13 (s, 1H), 8.33 (d, 1 H), 9.12 (br. s, 1 H), 10.30 (s, 1 H).

### Example01.23

### N,N-diethyl-4-{[6-(4-{[(4-fluorophenyl)acetyl]amino}phenyl)[1,2,4]triazolo-[1,5-a]pyridin-2-yl]amino}-3-propoxybenzamide

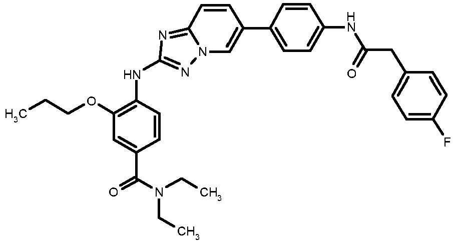

*N*-[4-(2-Amino[1,2,4]triazolo[1,5-*a*]pyridin-6-yl)phenyl]-2-(4-fluorophenyl)acetamide **(Int3.4)** (79 mg), *N,N*-diethyl-4-iodo-3-propoxybenzamide **Int15.3** (95 mg), chloro(2-dicyclohexylphosphino-2',4',6'-tri-i-propyl-1,1'-biphenyl)[2-(2-aminoethyl)phenyl] palladium(II) *tert* butyl methylether adduct (7.2 mg), X-Phos (4.2 mg), and sodium *tert*-butoxide (38.5 mg) were pre-mixed, and degassed toluene (1.6 mL) was added. The mixture was heated for 8 h to 130 °C, then diluted with DCM and washed with satd. aqueous sodium carbonate solution. The organic layer was dried over sodium sulfate, and the solvent was evaporated. The crude product was purified by flash chromatography on silica gel (20 g, eluent: ethyl acetate/ cyclohexane gradient 2:1 to 8:1) to yield 45 mg (35%) of the title compound.

¹H-NMR (400MHz, DMSO-d₆): δ [ppm]= 1.04 (t, 3H), 1.13 (t, 6H), 1.79 - 1.90 (m, 2H), 3.30 - 3.40 (m, 4H), 3.68 (s, 2H), 4.07 (t, 2H), 6.97 - 7.01 (m, 2H), 7.13 - 7.20 (m, 2H), 7.36 - 7.41 (m, 2H), 7.66 (d, 1H), 7.74 (d, 4H), 7.94 (dd, 1H), 8.05 (s, 1H), 8.31 (d, 1H), 9.13 (s, 1H), 10.31 (s, 1H).

### Example01.24

### 3-(cyclopropylmethoxy)-N,N-diethyl-4-{[6-(4-{[(4-fluorophenyl)acetyl]-amino}phenyl)[1,2,4]triazolo[1,5-a]pyridin-2-yl]amino}benzamide

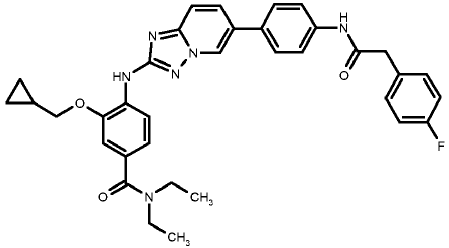

*N*-[4-(2-Amino[1,2,4]triazolo[1,5-*a*]pyridin-6-yl)phenyl]-2-(4-fluorophenyl)acetamide **(Int3.4)** (61 mg), 3-(cyclopropylmethoxy)-*N,N*-diethyl-4-iodobenzamide **Int15.4** (76 mg), chloro(2-dicyclohexylphosphino-2',4',6'-tri-i-propyl-1,1'-biphenyl)[2-(2-aminoethyl)phenyl] palladium(II) *tert* butyl methylether adduct (5.6 mg), X-Phos (3.2 mg), and sodium *tert*-butoxide (29.8 mg) were pre-mixed, and degassed toluene (1.9 mL) was added. The mixture was heated for 8 h to 130 °C, then diluted with satd. sodium carbonate solution and extracted with DCM. The organic layer was dried over sodium sulfate, and the solvent was evaporated. The crude product was purified by flash chromatography on silica gel (20 g, eluent: ethyl acetate/ cyclohexane gradient 2:1 to 4:1) to yield 30 mg (28%) of the title compound.

¹H-NMR (400MHz, DMSO-d₆): δ [ppm]= 0.39 (q, 2H), 0.58 - 0.64 (m, 2H), 1.12 (t, 6H), 1.30 - 1.40 (m, 1H), 3.31 - 3.39 (m, 4H), 3.68 (s, 2H), 3.98 (d, 2H), 6.97 - 7.01 (m, 2H), 7.16 (t, 1H), 7.38 (dd, 1H), 7.66 (d, 1H), 7.70 - 7.77 (m, 4H), 7.93 (d, 1H), 7.95 - 7.97 (m, 1H), 8.31 (d, 1H), 9.13 (br. s, 1H), 10.30 (br. s, 1 H).

### Example01.25

### N,N-diethyl-4-{[6-(4-{[(4-fluorophenyl)acetyl]amino}phenyl)[1,2,4]triazolo-[1,5-a]pyridin-2-yl]amino}-3-isopropoxybenzamide

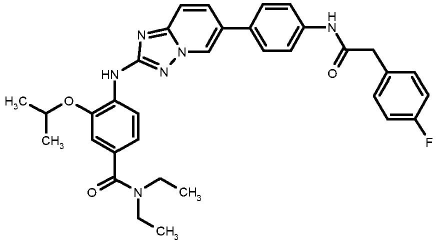

*N*-[4-(2-Amino[1,2,4]triazolo[1,5-*a*]pyridin-6-yl)phenyl]-2-(4-fluorophenyl)acetamide **(Int3.4)** (79 mg), *N,N*-diethyl-4-iodo-3-isopropoxybenzamide **Int15.5** (95 mg), chloro(2-dicyclohexylphosphino-2',4',6'-tri-i-propyl-1,1'-biphenyl)[2-(2-aminoethyl)phenyl] palladium(II) *tert* butyl methylether adduct (7.2 mg), X-Phos (4.2 mg), and sodium **tert**-butoxide (38.5 mg) were pre-mixed, and degassed toluene (1.6 mL) was added. The mixture was heated for 8 h to 130 °C, then diluted with ethyl acetate and washed with satd. aqueous sodium carbonate solution. The organic layer was dried over sodium sulfate, and the solvent was evaporated. The crude product was purified by flash chromatography on silica gel (20 g, eluent: ethyl acetate/ cyclohexane gradient 2:1 to 8:1) to yield 27 mg (20%) of the title compound.

¹H-NMR (400MHz, DMSO-d₆): δ [ppm]= 1.13 (t, 6H), 1.36 (d, 6H), 3.30 - 3.40 (m, 4H), 3.68 (s, 2H), 4.72 - 4.80 (m, 1 H), 6.98 (dd, 1 H), 7.00 - 7.02 (m, 1 H), 7.13 - 7.20 (m, 2H), 7.35 - 7.41 (m, 2H), 7.66 (d, 1 H), 7.70 - 7.77 (m, 4H), 7.94 (dd, 1H), 8.01 (s, 1H), 8.33 (d, 1H), 9.13 (br. s, 1H), 10.31 (s, 1H).

### Example01.26

### N,N-diethyl-4-{[6-(4-{[(4-fluorophenyl)acetyl]amino}phenyl)[1,2,4]triazolo-[1,5-a]pyridin-2-yl]amino}-3-(2-methoxyethoxy)benzamide

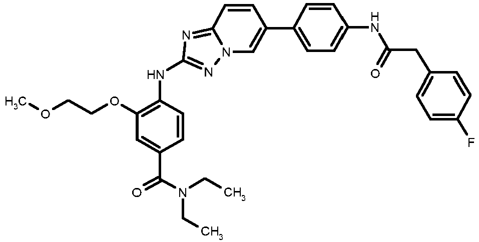

*N*-[4-(2-Amino[1,2,4]triazolo[1,5-α]pyridin-6-yl)phenyl]-2-(4-fluorophenyl)acetamide **(Int3.4)** (60 mg), *N,N*-diethyl-4-iodo-3-(2-methoxyethoxy)benzamide **Int15.6** (75 mg), chloro(2-dicyclohexylphosphino-2',4',6'-tri-i-propyl-1,1'-biphenyl)[2-(2-aminoethyl)phenyl] palladium(II) *tert* butyl methylether adduct (5.5 mg), X-Phos (3.2 mg), and sodium *tert*-butoxide (29 mg) were pre-mixed, and degassed toluene (0.9 mL) was added. The mixture was heated for 8 h to 130 °C, then diluted with DCM and washed with satd. aqueous sodium carbonate solution. The organic layer was dried over sodium sulfate, and the solvent was evaporated. The crude product was purified by flash chromatography on silica gel (20 g, eluent: ethyl acetate/ cyclohexane gradient 2:1 to 1:0) to yield 12 mg (12%) of the title compound.

¹H-NMR (400MHz, DMSO-d₆): δ [ppm]= 1.13 (t, 6H), 3.31 - 3.44 (m, 4H), 3.38 (s, 3H), 3.68 (s, 2H), 3.74 - 3.78 (m, 2H), 4.22 - 4.26 (m, 2H), 7.03 (dd, 1 H), 7.05 (d, 1H), 7.13 - 7.20 (m, 2H), 7.35 - 7.41 (m, 2H), 7.67 (d, 1H), 7.70 - 7.78 (m, 4H), 7.94 (dd, 1H), 8.12 (s, 1H), 8.34 (d, 1H), 9.13 - 9.14 (m, 1H), 10.31 (s, 1H).

### Example01.27

### 4-{[6-(4-{[(4-fluorophenyl)acetyl]amino}phenyl)[1,2,4]triazolo[1,5-a]-pyridin-2-yl]amino}-3-(2,2,2-trifluoroethoxy)-N-(2,2,2-trifluoroethyl)-benzamide

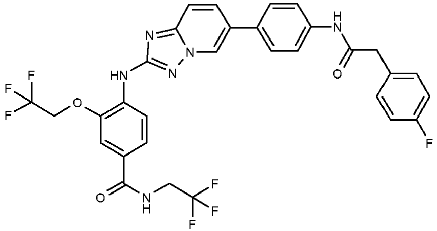

To a stirred suspension of **Int3.4** (85 mg) in toluene (3.0 mL) and NMP (0.4 mL) was added **Int12.4** (156 mg), chloro(2-dicyclohexylphosphino-2',4',6'-tri-i-propyl-1,1'-biphenyl)[2-(2-aminoethyl)phenyl] palladium(II) methyl-*tert*-butylether adduct (14 mg) and X-Phos (8 mg) and the flask was twice degased and backfilled with argon. The mixture was stirred for 5 minutes at r.t.. Powdered potassium phosphate (250 mg) was added and the flask was twice degased and backfilled with argon. The mixture was heated to reflux for 3 h. Water was added and the reaction mixture was extracted with ethyl acetate. The organic phase was washed with saturated sodium chloride solution, dried (sodium sulfate) and the solvent was removed in vacuum. Silicagel chromatography gave a solid that was triturated with warm ethanol to give 122 mg of the title compound.

¹H-NMR (400 MHz, DMSO-d₆): δ [ppm] = 3.68 (s, 2H), 4.06 - 4.20 (m, 2H), 4.95 (q, 2H), 7.10 - 7.23 (m, 2H), 7.38 (dd, 2H), 7.65 - 7.81 (m, 7H), 7.96 (dd, 1 H), 8.36 (s, 1H), 8.41 (d, 1H), 8.93 (t, 1H), 9.16 (d, 1H), 10.32 (s, 1H).

### Example01.28

### 3-ethoxy-4-{[6-(4-{[(4-fluorophenyl)acetyl]amino}phenyl)[1,2,4]triazolo-[1,5-a]pyridin-2-yl]amino}-N-[2-(methylsulfonyl)ethyl]benzamide

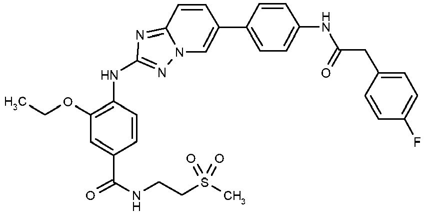

Starting with **Int3.4** and **Int11.12, Example01.28** was prepared analogously to the procedure for the preparation of **Example01.27.**

¹H-NMR (400 MHz, DMSO-d₆): δ [ppm] = 1.46 (t, 3H), 3.04 (s, 3H), 3.39 (t, 2H), 3.60 - 3.74 (m, 4H), 4.20 (q, 2H), 7.10 - 7.22 (m, 2H), 7.38 (dd, 2H), 7.47 - 7.56 (m, 2H), 7.63 - 7.81 (m, 5H), 7.95 (dd, 1 H), 8.24 (s, 1 H), 8.37 (d, 1 H), 8.62 (t, 1H), 9.15 (s, 1H), 10.33 (s, 1H).

### Example01.29

### 4-{[6-(4-{[(4-fluorophenyl)acetyl]amino}phenyl)[1,2,4]triazolo[1,5-a]-pyridin-2-yl]amino}-N-(2-hydroxy-2-methylpropyl)-3-(2,2,2-trifluoroethoxy)benzamide

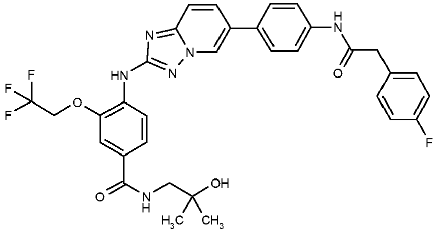

Starting with **Int3.4** and **Int12.6, Example01.29** was prepared analogously to the procedure for the preparation of **Example01.27.**

¹H-NMR (400 MHz, DMSO-d₆): δ [ppm] = 1.12 (s, 6H), 3.27 (d, 2H), 3.68 (s, 2H), 4.60 (s, 1H), 4.95 (q, 2H), 7.11 - 7.21 (m, 2H), 7.32 - 7.43 (m, 2H), 7.63 - 7.81 (m, 7H), 7.96 (dd, 1H), 8.18 (t, 1H), 8.25 (s, 1H), 8.37 (d, 1H), 9.15 (d, 1H), 10.32 (s, 1H).

### Example01.30

### 4-{[6-(4-{[(4-fluorophenyl)acetyl]amino}phenyl)[1,2,4]triazolo[1,5-a]-pyridin-2-yl]amino}-3-methoxy-N-methylbenzamide

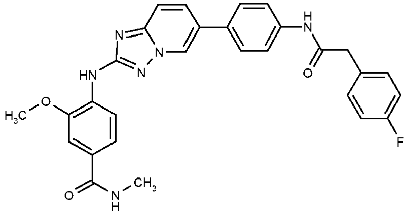

Starting with **Int3.4** and 4-bromo-3-methoxy-*N*-methylbenzamide, **Example01.30** was prepared analogously to the procedure for the preparation of **Example01.27.**

¹H-NMR (400 MHz, DMSO-d₆): δ [ppm] = 2.79 (d, 3H), 3.68 (s, 2H), 3.94 (s, 3H), 7.12 - 7.20 (m, 2H), 7.34 - 7.42 (m, 2H), 7.47 - 7.54 (m, 2H), 7.64 - 7.80 (m, 5H), 7.94 (dd, 1H), 8.25 - 8.38 (m, 3H), 9.14 (d, 1H), 10.31 (s, 1H).

### Example01.31

### 4-{[6-(4-{[(4-fluorophenyl)acetyl]amino}phenyl)[1,2,4]triazolo[1,5-a]-pyridin-2-yl]amino}-3-methoxy-N-(2,2,2-trifluoroethyl)benzamide

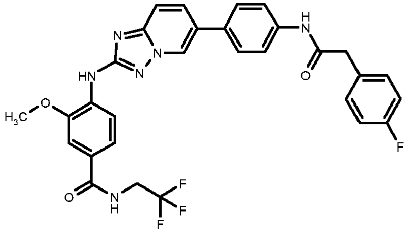

Starting with **Int3.4** and **Int10.12, Example01.31** was prepared analogously to the procedure for the preparation of **Example01.27.**

¹H-NMR (400 MHz, DMSO-d₆): δ [ppm] = 3.70 (s, 2H), 3.96 (s, 3H), 4.10 (dd, 2H), 7.12 - 7.20 (m, 2H), 7.36 - 7.43 (m, 2H), 7.57 - 7.65 (m, 2H), 7.66 - 7.71 (m, 1 H), 7.75 (s, 4H), 7.95 (dd, 1 H), 8.35 - 8.42 (m, 2H), 8.98 (t, 1 H), 9.12 - 9.18 (m, 1H), 10.47 (s, 1H).

### Example01.32

### N-[4-(2-{[2-ethoxy-4-(methylsulfonyl)phenyl]amino}[1,2,4]triazolo[1,5-a]-pyridin-6-yl)phenyl]-2-(4-fluorophenyl)acetamide

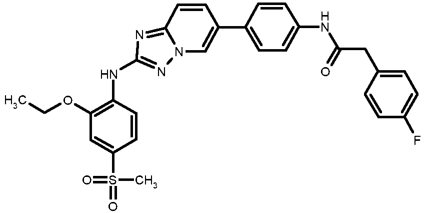

To a stirred suspension of **Int3.4** (200 mg) in toluene (5.0 mL) and NMP (2.5 mL) was added **Int11.15** (232 mg), chloro(2-dicyclohexylphosphino-2',4',6'-tri-i-propyl-1,1'-biphenyl)[2-(2-aminoethyl)phenyl] palladium(II) methyl-*tert*-butylether adduct (46 mg) and X-Phos (27 mg) and the flask was twice degased and backfilled with argon. The mixture was stirred for 5 minutes at r.t.. Powdered potassium phosphate (587 mg) was added and the flask was twice degased and backfilled with argon. The mixture was heated to reflux for 2 h. The reaction mixture was filtered through an aminophase-silica-gel column and the solvent was removed in vaccuum. Aminophase-silica-gel chromatography gave a solid that was triturated with warm DCM to give 150 mg of the title compound.

¹H-NMR (400MHz, DMSO-d₆): δ [ppm]= 1.43 (t, 3H), 3.15 (s, 3H), 3.64 (s, 2H), 4.22 (q, 2H), 7.08 - 7.16 (m, 2H), 7.31 - 7.38 (m, 2H), 7.41 (d, 1 H), 7.51 (dd, 1H), 7.62 - 7.78 (m, 5H), 7.93 (dd, 1H), 8.41 - 8.54 (m, 2H), 9.10 (dd, 1H), 10.27 (s, 1H).

### Example01.33

### N-[4-(2-{[2-ethoxy-4-(ethylsulfonyl)phenyl]amino}[1,2,4]triazolo[1,5-a]-pyridin-6-yl)phenyl]-2-(4-fluorophenyl)acetamide

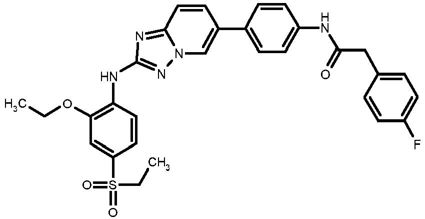

To a stirred suspension of **Int3.4** (100 mg) in toluene (2.5 mL) and NMP (1.4 mL) was added **Int11.17** (122 mg), chloro(2-dicyclohexylphosphino-2',4',6'-tri-i-propyl-1,1'-biphenyl)[2-(2-aminoethyl)phenyl] palladium(II) methyl-*tert*-butylether adduct (23 mg) and X-Phos (13 mg) and the flask was twice degased and backfilled with argon. The mixture was stirred for 5 minutes at r.t.. Powdered potassium phosphate (293 mg) was added and the flask was twice degased and backfilled with argon. The mixture was heated to reflux for 35 minutes. The reaction mixture was filtered through an aminophase-silica-gel column and the solvent was removed in vaccuum. Aminophase-silica-gel chromatography gave a solid that was triturated with warm DCM to give 68 mg of the title compound.

¹H-NMR (400MHz, DMSO-d₆): δ [ppm]= 1.08 (t, 3H), 1.43 (t, 3H), 3.22 (q, 2H), 3.64 (s, 2H), 4.21 (q, 2H), 7.09 - 7.17 (m, 2H), 7.31 - 7.38 (m, 3H), 7.47 (dd, 1H), 7.64 - 7.76 (m, 5H), 7.93 (dd, 1H), 8.45 - 8.55 (m, 2H), 9.11 (dd, 1H), 10.27 (s, 1H).

### Example01.34

### 2-(4-fluorophenyl)-N-[4-(2-{[4-(methylsulfonyl)-2-(2,2,2-trifluoroethoxy)-phenyl]amino}[1,2,4]triazolo[1,5-a]pyridin-6-yl)phenyl]acetamide

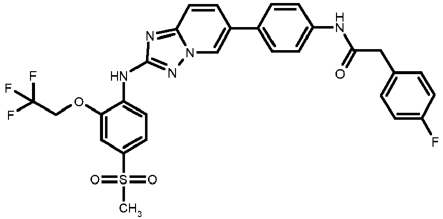

To a stirred suspension of **Int3.4** (500 mg) in toluene (15 mL) and NMP (5 mL) in a sealed tube was added **Int12.9** (600 mg), chloro(2-dicyclohexylphosphino-2',4',6'-tri-i-propyl-1,1'-biphenyl)[2-(2-aminoethyl)phenyl] palladium(II) methyl-*tert*-butylether adduct (114 mg), X-Phos (67 mg), and powdered potassium phosphate (1.03 g). The tube was twice degased and backfilled with argon. The mixture was heated to 120°C with an oil bath for 16 h. The solvent was removed in vaccuum. Aminophase-silica-gel chromatography gave a solid that was triturated with ethanol to give 600 mg of the title compound.

¹H-NMR (400MHz, DMSO-d₆): δ [ppm]= 3.17 (s, 3H), 3.64 (s, 2H), 5.00 (q, 2H), 7.07 - 7.19 (m, 2H), 7.30 - 7.39 (m, 2H), 7.57 - 7.77 (m, 7H), 7.94 (dd, 1 H), 8.50 (d, 1H), 8.56 (s, 1H), 9.12 (d, 1H), 10.27 (s, 1H).

### Example01.35

### N-[4-(2-{[2-(difluoromethoxy)-4-(methylsulfonyl)phenyl]amino}[1,2,4]-triazolo[1,5-pyridin-6-yl)phenyl]-2-(4-fluorophenyl)acetamide

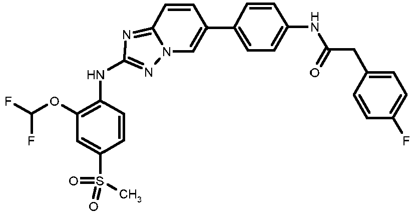

Starting with **Int3.4** and **Int17.3**, **Example01.35** was prepared analogously to the procedure for the preparation of **Example01.27**.

¹H-NMR (40 0MHz, DMSO-d₆): δ [ppm] = 3.22 (s, 3H), 3.68 (s, 2H), 7.16 (t, 2H), 7.27 (t, 1 H), 7.38 (dd, 2H), 7.68 - 7.80 (m, 6H), 7.82 (dd, 1 H), 7.98 (dd, 1 H), 8.65 (d, 1H), 9.13 - 9.16 (m, 1H), 9.46 (s, 1H), 10.31 (s, 1H).

### Example01.36

### N-[4-(2-{[2-(difluoromethoxy)-4-(ethylsulfonyl)phenyl]amino}[1,2,4]-triazolo[1,5-a]pyridin-6-yl)phenyl]-2-(4-fluorophenyl)acetamide

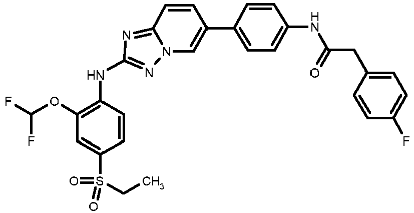

Starting with **Int3.4** and **Int17.5, Example01.36** was prepared analogously to the procedure for the preparation of **Example01.27**.

¹H-NMR (400 MHz, DMSO-d₆): δ [ppm] = 1.13 (t, 3H), 3.28 (q, 2H), 3.68 (s, 2H), 7.16 (t, 2H), 7.27 (t, 1 H), 7.38 (dd, 2H), 7.65 (s, 1 H), 7.69 - 7.81 (m, 6H), 7.98 (dd, 1H), 8.67 (d, 1H), 9.15 (s, 1H), 9.48 (s, 1H), 10.30 (s, 1H).

### Example01.37

### N-[4-(2-{[2-(cyclopropyloxy)-4-(methylsulfonyl)phenyl]amino}[1,2,4]-triazolo[1,5-a]pyridin-6-yl)phenyl]-2-(4-fluorophenyl)acetamide

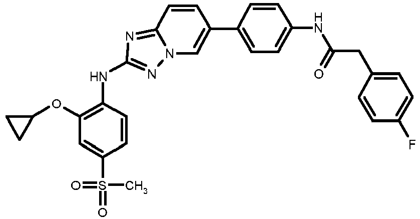

To a stirred suspension of **Int3.4** (98 mg) in toluene (2.5 mL) and NMP (1.4 mL) was added **Int18.3** (118 mg), chloro(2-dicyclohexylphosphino-2',4',6'-tri-i-propyl-1,1'-biphenyl)[2-(2-aminoethyl)phenyl] palladium(II) methyl-*tert*-butylether adduct (22 mg) and X-Phos (13 mg) and the flask was twice degased and backfilled with argon. The mixture was stirred for 5 minutes at r.t.. Powdered potassium phosphate (288 mg) was added and the flask was twice degased and backfilled with argon. The mixture was heated to 85° C with an oil bath for for 35 minutes. The reaction mixture was filtered through an aminophase-silica-gel column and the solvent was removed in vaccuum. Aminophase-silica-gel chromatography gave a solid that was triturated with warm DCM to give 65 mg of the title compound.

¹H-NMR (300MHz, DMSO-d₆): δ [ppm]= 0.72 - 0.94 (m, 4H), 3.16 (s, 3H), 3.64 (s, 2H), 4.03 - 4.14 (m, 1H), 7.06 - 7.19 (m, 2H), 7.27 - 7.40 (m, 2H), 7.53 (dd, 1 H), 7.61 - 7.78 (m, 6H), 7.92 (dd, 1 H), 8.48 (d, 1 H), 8.55 (s, 1 H), 9.09 (d, 1 H), 10.26 (s, 1H).

### Example02.1

### 4-[(6-{4-[(4-fluorobenzyl)carbamoyl]phenyl}[1,2,4]triazolo[1,5-a]pyridin-2-yl)amino]-N-(2-hydroxyethyl)-3-methoxybenzamide

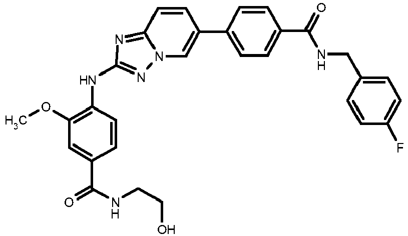

To a stirred suspension of **Int5.2** (100 mg) in toluene (4.0 mL) and NMP (1.0 mL) in a sealed tube was added **Int10.3** (114 mg), chloro(2-dicyclohexylphosphino-2',4',6'-tri-i-propyl-1,1'-biphenyl)[2-(2-aminoethyl)-phenyl] palladium(II) methyl-*tert*-butylether adduct (23 mg), X-Phos (13 mg), and powdered potassium phosphate (294 mg). The flask was twice degased and backfilled with argon. The mixture was heated to 130°C with an oil bath for 2 h. A mixture of DCM and methanol (100:1) added, solids were removed by filtration and the solvent was removed in vacuum. Aminophase-silica-gel chromatography gave a solid that was titurated with warm ethanol to give 70 mg of the title compound.

¹H-NMR (300MHz, DMSO-d₆): δ [ppm]= 3.30 - 3.35 (m, 2H), 3.42 - 3.54 (m, 2H), 3.91 (s, 3H), 4.45 (d, 2H), 4.71 (t, 1 H), 7.13 (t, 2H), 7.28 - 7.39 (m, 2H), 7.45 - 7.55 (m, 2H), 7.68 (d, 1H), 7.83 - 8.05 (m, 5H), 8.22 - 8.40 (m, 3H), 9.11 (t, 1H), 9.27 (s, 1H).

Starting with Intermediate **Int5.2**, the Examples **Example02.2** to **Example02.4** were prepared analogously to the procedure for the preparation of **Example02.1.**

### Example02.2

### 3-ethoxy-4-[(6-{4-[(4-fluorobenzyl)carbamoyl]phenyl}[1,2,4]triazolo[1,5-a]pyridin-2-yl)amino]-N-(2-hydroxyethyl)benzamide

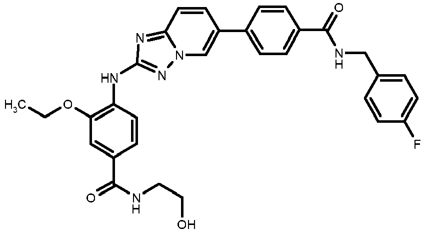

¹H-NMR (400MHz, DMSO-d₆): δ [ppm]= 1.43 (t, 3H), 3.27 - 3.34 (m, 2H), 3.44 - 3.52 (m, 2H), 4.17 (q, 2H), 4.46 (d, 2H), 4.71 (t, 1 H), 7.08 - 7.17 (m, 2H), 7.30 - 7.38 (m, 2H), 7.47 - 7.54 (m, 2H), 7.69 (d, 1 H), 7.88 - 8.04 (m, 5H), 8.21 (s, 1 H), 8.28 - 8.35 (m, 2H), 9.12 (t, 1 H), 9.28 (d, 1 H).
Starting materials: Intermediate **Int5.2**; 4-bromo-3-ethoxy-*N*-(2-hydroxy-ethyl)benzamide (**Int11.5**)

### Example02.3

### 3-ethoxy-4-[(6-{4-[(4-fluorobenzyl)carbamoyl]phenyl}[1,2,4]triazolo[1,5-a]pyridin-2-yl)amino]-N-(1-hydroxy-2-methylpropan-2-yl)benzamide

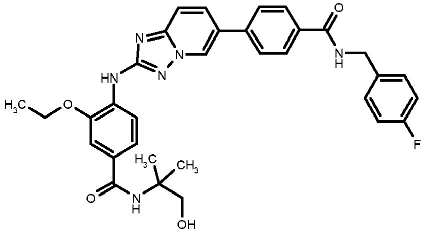

¹H-NMR (300MHz, DMSO-d₆): δ [ppm]= 1.29 (s, 6H), 1.42 (t, 3H), 3.43 - 3.53 (m, 2H), 4.18 (q, 2H), 4.45 (d, 2H), 4.87 - 4.96 (m, 1 H), 7.07 - 7.18 (m, 2H), 7.28 - 7.51 (m, 5H), 7.68 (d, 1 H), 7.87 - 8.05 (m, 5H), 8.19 (s, 1 H), 8.30 (d, 1 H), 9.12 (t, 1H), 9.26 (d, 1H).
Starting materials: Intermediate **Int5.2**; 4-bromo-3-ethoxy-*N*-(1-hydroxy-2-methylpropan-2-yl)benzamide (**Int11.7**)

### Example02.4

### 4-[(6-{4-[(4-fluorobenzyl)carbamoyl]phenyl}[1,2,4]triazolo[1,5-a]pyridin-2-yl)amino]-N-(2-hydroxyethyl)-3-(2,2,2-trifluoroethoxy)benzamide

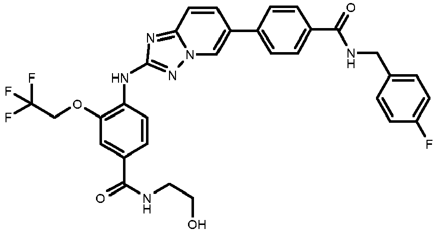

¹H-NMR (300MHz, DMSO-d₆): δ [ppm]= 3.29 - 3.36 (m, 2H), 3.43 - 3.53 (m, 2H), 4.45 (d, 2H), 4.68 - 4.77 (m, 1 H), 4.90 (q, 2H), 7.06 - 7.19 (m, 2H), 7.29 - 7.40 (m, 2H), 7.56 - 7.74 (m, 3H), 7.85 - 8.07 (m, 5H), 8.24 - 8.39 (m, 3H), 9.12 (t, 1 H), 9.24 - 9.33 (m, 1 H).
Starting materials: Intermediate **Int5.2;** 4-bromo-*N*-(2-hydroxyethyl)-3-(2,2,2-trifluoroethoxy)benzamide (**Int12.3**)

### Example02.5

### 4-[(6-{4-[(4-fluorobenzyl)carbamoyl]phenyl}[1,2,4]triazolo[1,5-a]pyridin-2-yl)amino]-N-(2-hydroxy-2-methylpropyl)-3-methoxybenzamide

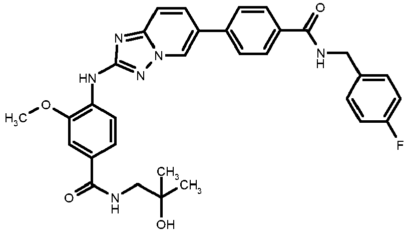

To a stirred suspension of **Int5.2** (100 mg) in toluene (3.0 mL) and NMP (1.5 mL) was added **Int10.4** (167 mg), chloro(2-dicyclohexylphosphino-2',4',6'-tri-i-propyl-1,1'-biphenyl)[2-(2-aminoethyl)phenyl] palladium(II) methyl-tert-butylether adduct (23 mg) and X-Phos (13 mg) and the flask was twice degased and backfilled with argon. The mixture was stirred for 5 minutes at r.t.. Powdered potassium phosphate (294 mg) was added and the flask was twice degased and backfilled with argon. The mixture was heated to reflux for 2 h. Water was added and the reaction mixture was extracted with ethyl acetate. The organic phase was washed with saturated sodium chloride solution, dried (sodium sulfate) and the solvent was removed in vacuum. Aminophase-silica-gel chromatography gave a solid that was recrystallized from ethyl acetate to give 72 mg of the title compound.

¹H-NMR (300MHz, DMSO-d₆): δ [ppm]= 1.08 (s, 6H), 3.23 (d, 2H), 3.92 (s, 3H), 4.45 (d, 2H), 4.58 (s, 1 H), 7.07 - 7.20 (m, 2H), 7.30 - 7.38 (m, 2H), 7.49 - 7.57 (m, 2H), 7.69 (d, 1H), 7.86 - 8.05 (m, 5H), 8.16 (t, 1H), 8.26 - 8.39 (m, 2H), 9.07 - 9.17 (m, 1H), 9.27 (d, 1H).

Starting with Intermediate **Int5.2,** the Examples **Example02.6** to **Example02.7** were prepared analogously to the procedure for the preparation of **Example02.5.**

### Example02.6

### N-(4-fluorobenzyl)-4-(2-{[2-methoxy-4-(methylsulfonyl)phenyl]amino}-[1,2,4]triazolo[1,5-a]pyridin-6-yl)benzamide

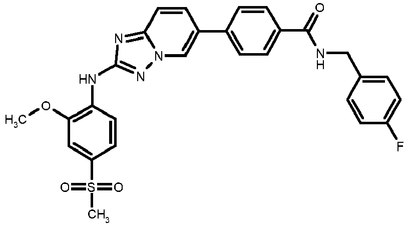

¹H-NMR (400MHz, DMSO-d₆): δ [ppm]= 3.17 (s, 3H), 3.96 (s, 3H), 4.45 (d, 2H), 7.08 - 7.18 (m, 2H), 7.31 - 7.38 (m, 2H), 7.43 (d, 1 H), 7.52 (dd, 1 H), 7.72 (dd, 1H), 7.88 - 8.00 (m, 4H), 8.03 (dd, 1H), 8.49 (d, 1H), 8.69 (s, 1H), 9.12 (t, 1H), 9.25 - 9.32 (m, 1 H).
Starting materials: Intermediate **Int5.2;** 1-bromo-2-methoxy-4-(methylsulfonyl)benzene **(Int10.11)**

### Example02.7

### 3-ethoxy-4-[(6-{4-[(4-fluorobenzyl)carbamoyl]phenyl}[1,2,4]triazolo[1,5-a]pyridin-2-yl)amino]-N-(2-hydroxy-2-methylpropyl)benzamide

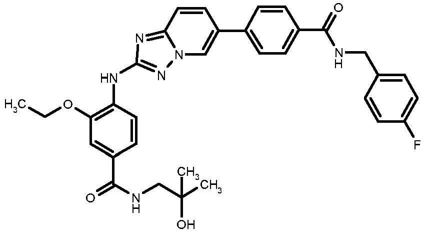

¹H-NMR (300MHz, DMSO-d₆): δ [ppm]= 1.08 (s, 6H), 1.43 (t, 3H), 3.23 (d, 2H), 4.18 (q, 2H), 4.45 (d, 2H), 4.57 (s, 1 H), 7.13 (t, 2H), 7.34 (dd, 2H), 7.48 - 7.57 (m, 2H), 7.68 (d, 1 H), 7.85 - 8.06 (m, 5H), 8.14 (t, 1 H), 8.21 (s, 1 H), 8.32 (d, 1H), 9.12 (t, 1H), 9.27 (d, 1H).
Starting materials: Intermediate **Int5.2**; 4-bromo-3-ethoxy-*N*-(2-hydroxy-2-methylpropyl)benzamide **(Int11.6)**

### Example02.8

### 4-[(6-{4-[(4-fluorobenzyl)carbamoyl]phenyl}[1,2,4]triazolo[1,5-a]pyridin-2-yl)amino]-N-(1-hydroxy-2-methylpropan-2-yl)-3-methoxybenzamide

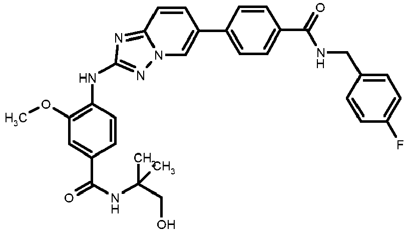

To a stirred suspension of **Int5.2** (250 mg) in toluene (6.5 mL) and NMP (3.0 mL) was added **Int10.5** (314 mg), chloro(2-dicyclohexylphosphino-2',4',6'-tri-i-propyl-1,1'-biphenyl)[2-(2-aminoethyl)phenyl] palladium(II) methyl-tert-butylether adduct (57 mg) and X-Phos (34 mg) and the flask was twice degased and backfilled with argon. The mixture was stirred for 5 minutes at r.t.. Powdered potassium phosphate (734 mg) was added and the flask was twice degased and backfilled with argon. The mixture was heated to reflux for 2 h. Water was added and the reaction mixture was extracted with ethyl acetate. The organic phase was washed with saturated sodium chloride solution, dried (sodium sulfate) and the solvent was removed in vacuum. Aminophase-silica-gel chromatography followed by silica gel chromatography gave 210 mg of the title compound.

¹H-NMR (400MHz, DMSO-d₆): δ [ppm]= 1.28 (br. s., 6H), 3.50 (d, 2H), 3.92 (s, 3H), 4.46 (d, 2H), 4.94 (t, 1 H), 7.09 - 7.17 (m, 2H), 7.31 - 7.37 (m, 2H), 7.38 - 7.43 (m, 2H), 7.47 (dd, 1 H), 7.68 (dd, 1 H), 7.87 - 8.05 (m, 5H), 8.24 - 8.34 (m, 2H), 9.12 (t, 1 H), 9.26 (dd, 1 H).

### Example02.9

### N-{2-[acetyl(methyl)amino]ethyl}-4-[(6-{4-[(4-fluorobenzyl)carbamoyl]-phenyl}[1,2,4]triazolo[1,5-a]pyridin-2-yl)amino]-3-methoxy-N-methylbenzamide

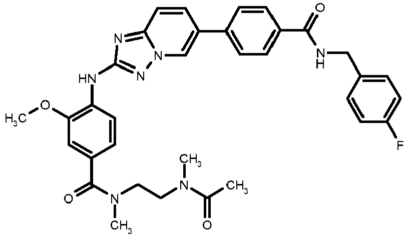

Starting with Intermediate **Int5.2** and *N*-{2-[acetyl(methyl)amino]ethyl}-4-bromo-3-methoxy-*N*-methylbenzamide **(Int10.8), Example02.9** was prepared analogously to the procedure for the preparation of **Example02.8.**

¹H-NMR (500MHz, DMSO-d₆): δ [ppm]= 1.97 (s, 3H), 2.72 - 3.15 (m, 6H), 3.55 (br. d, 4H), 3.96 (s, 3H), 4.52 (d, 2H), 7.00 - 7.07 (m, 2H), 7.10 - 7.18 (m, 2H), 7.36 - 7.44 (m, 2H), 7.68 (d, 1 H), 7.90 (d, 2H), 7.96 - 8.06 (m, 4H), 8.33 (d, 1H), 8.89 (t, 1H), 9.18 (s, 1H).

### Example02.10

### N-(2-ethoxyethyl)-4-[(6-{4-[(4-fluorobenzyl)carbamoyl]phenyl}[1,2,4]-triazolo[1,5-a]pyridin-2-yl)amino]-3-methoxybenzamide

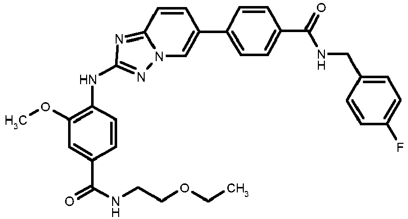

To a stirred suspension of **Int5.2** (100 mg) in toluene (3.0 mL) and NMP (0.3 mL) in a sealed tube was added **Int10.6** (125 mg), chloro(2-dicyclohexylphosphino-2',4',6'-tri-i-propyl-1,1'-biphenyl)[2-(2-aminoethyl)phenyl] palladium(II) methyl-*tert*-butylether adduct (23 mg), X-Phos (13 mg), and sodium *tert*-butoxide (133 mg). The flask was twice degased and backfilled with argon. The mixture was heated to 130°C with an oil bath for 2 h. Water was added and the reaction mixture was extracted with a mixture of DCM and methanol (100:1). The organic phase was washed with saturated sodium chloride solution, dried (sodium sulfate) and the solvent was removed in vacuum. Aminophase-silica-gel chromatography gave a solid that was titurated with warm ethanol to give 70 mg of the title compound.

¹H-NMR (400MHz, DMSO-d₆): δ [ppm]= 1.09 (t, 3H), 3.34 - 3.50 (m, 6H), 3.91 (s, 3H), 4.45 (d, 2H), 7.13 (t, 2H), 7.34 (dd, 2H), 7.47 - 7.56 (m, 2H), 7.69 (d, 1 H), 7.87 - 8.05 (m, 5H), 8.29 - 8.35 (m, 2H), 8.40 (t, 1 H), 9.12 (t, 1 H), 9.28 (s, 1 H).

### Example02.11

### 4-[(6-{4-[(4-fluorobenzyl)carbamoyl]phenyl}[1,2,4]triazolo[1,5-a]pyridin-2-yl)amino]-3-methoxy-N-methyl-N-[2-(methylamino)ethyl]benzamide

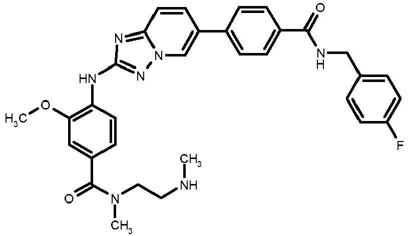

To a stirred suspension of **Intermediate Example Int16.2** (65 mg) in DCM (1 mL) was added TFA (0.5 mL). The mixture was stirred at r.t. for 2 h. A half-saturated solution of sodium bicarbonate was added until pH 9 was reached. The precipitated solid was collected by filtration. Purification by aminophase-silica-gel chromatography gave 38 mg of the title compound.

¹H-NMR (500MHz, DMSO-d₆, detected signals): δ [ppm]= 2.30 (s, 3H), 2.69 - 2.76 (m, 2H), 3.01 (s, 3H), 3.45 (t, 2H), 3.95 (s, 3H), 4.52 (d, 2H), 7.04 - 7.18 (m, 4H), 7.41 (dd, 2H), 7.68 (d, 1H), 7.90 (d, 2H), 7.96 - 8.06 (m, 4H), 8.31 (d, 1H), 8.84 - 8.91 (m, 1H), 9.18 (s, 1H).

### Example02.12

### 3-ethoxy-4-[(6-{4-[(4-fluorobenzyl)carbamoyl]phenyl}[1,2,4]triazolo[1,5-a]pyridin-2-yl)amino]-N-(2-hydroxyethyl)-N-methylbenzamide

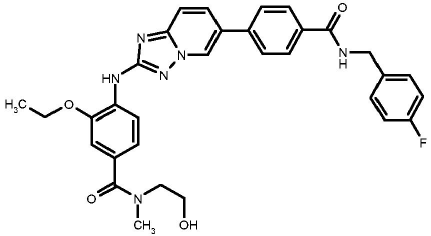

To a stirred suspension of **Int5.2** (100 mg) in toluene (3.0 mL) and NMP (1.3 mL) was added **Int11.11** (176 mg), chloro(2-dicyclohexylphosphino-2',4',6'-tri-i-propyl-1,1'-biphenyl)[2-(2-aminoethyl)phenyl] palladium(II) methyl-tert-butylether adduct (16 mg) and X-Phos (9 mg) and the flask was twice degased and backfilled with argon. The mixture was stirred for 5 minutes at r.t.. Powdered potassium phosphate (294 mg) was added and the flask was twice degased and backfilled with argon. The mixture was heated to reflux for 1 h. Water was added and the reaction mixture was extracted with ethyl acetate and methanol (10:1). The organic phase was washed with saturated sodium chloride solution, dried (sodium sulfate) and the solvent was removed in vacuum. Ethanol (5 mL) and 2N hydrochloric acid (1 mL) was added to the residue and the mixture was stirred for 15 minutes. A half-saturated solution of sodium bicarbonate was added and the mixture was extracted with ethyl acetate. The organic phase was washed with saturated sodium chloride solution, dried (sodium sulfate) and the solvent was removed in vacuum. Aminophase-silica-gel chromatography gave a solid that was titurated with diisopropyl ether to give 27 mg of the title compound.

¹H-NMR (300MHz, DMSO-d₆): δ [ppm]= 1.40 (t, 3H), 2.96 (br. s., 3H), 3.32 - 3.63 (m, 4H), 4.13 (q, 2H), 4.45 (d, 2H), 4.79 (br. s., 1H), 6.99 - 7.08 (m, 2H), 7.13 (t, 2H), 7.34 (dd, 2H), 7.67 (d, 1 H), 7.86 - 8.05 (m, 5H), 8.13 (s, 1 H), 8.28 (d, 1H), 9.12 (t, 1H), 9.26 (s, 1H).

### Example02.13

### 4-[(6-{4-[(4-fluorobenzyl)carbamoyl]phenyl}[1,2,4]triazolo[1,5-a]pyridin-2-yl)amino]-3-methoxy-N-methylbenzamide

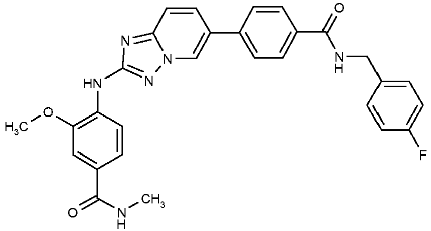

To a stirred suspension of **Int5.2** (90 mg) in toluene (3.0 mL) and NMP (0.4 mL) was added 4-bromo-3-methoxy-*N*-methylbenzamide (104 mg), chloro(2-dicyclohexylphosphino-2',4',6'-tri-i-propyl-1,1'-biphenyl)[2-(2-aminoethyl)phenyl] palladium(II) methyl-*tert*-butylether adduct (14 mg) and X-Phos (8 mg) and the flask was twice degased and backfilled with argon. The mixture was stirred for 5 minutes at r.t.. Powdered potassium phosphate (254 mg) was added and the flask was twice degased and backfilled with argon. The mixture was heated to reflux for 3 h.

Water was added and the reaction mixture was extracted with ethyl acetate. The organic phase was washed with saturated sodium chloride solution, dried (sodium sulfate) and the solvent was removed in vacuum.

Silicagel chromatography gave a solid that was triturated with warm ethanol to give 95 mg of the title compound.

¹H-NMR (400 MHz, DMSO-d₆): δ [ppm] = 2.80 (d, 3H), 3.94 (s, 3H), 4.49 (d, 2H), 7.12 - 7.21 (m, 2H), 7.38 (dd, 2H), 7.48 - 7.56 (m, 2H), 7.72 (d, 1H), 7.90 - 7.98 (m, 2H), 7.98 - 8.08 (m, 3H), 8.27 - 8.38 (m, 3H), 9.15 (t, 1 H), 9.31 (d, 1 H).

### Example02.14

### N-tert-butyl-4-[(6-{4-[(4-fluorobenzyl)carbamoyl]phenyl}[1,2,4]triazolo-[1,5-a]pyridin-2-yl)amino]-3-methoxybenzamide

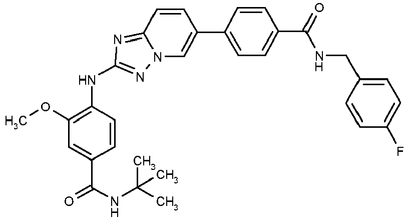

Starting with **Int5.2** and 4-bromo-*N*-*tert*-butyl-3-methoxybenzamide, **Example02.14** was prepared analogously to the procedure for the preparation of **Example02.13.**

¹H-NMR (400 MHz, DMSO-d₆): δ [ppm] = 1.40 (s, 9H), 3.95 (s, 3H), 4.49 (d, 2H), 7.12 - 7.21 (m, 2H), 7.38 (dd, 2H), 7.46 (d, 1H), 7.51 (dd, 1H), 7.60 (s, 1H), 7.72 (d, 1 H), 7.90 - 7.98 (m, 2H), 7.99 - 8.07 (m, 3H), 8.29 - 8.35 (m, 2H), 9.16 (t, 1H), 9.30 (d, 1H).

### Example02.15

### 4-[(6-{4-[(4-fluorobenzyl)carbamoyl]phenyl}[1,2,4]triazolo[1,5-a]pyridin-2-yl)amino]-3-methoxy-N-[2-(methylsulfonyl)ethyl]benzamide

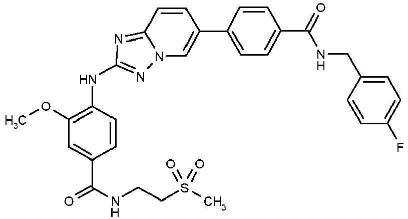

Starting with **Int5.2** and **Int10.13**, **Example02.15** was prepared analogously to the procedure for the preparation of **Example02.13**.

¹H-NMR (400 MHz, DMSO-d₆): δ [ppm] = 3.05 (s, 3H), 3.39 (t, 2H), 3.68 (q, 2H), 3.95 (s, 3H), 4.49 (d, 2H), 7.11 - 7.21 (m, 2H), 7.38 (dd, 2H), 7.49 - 7.57 (m, 2H), 7.72 (d, 1H), 7.91 - 7.98 (m, 2H), 7.98 - 8.08 (m, 3H), 8.37 (d, 1H), 8.41 (s, 1H), 8.64 (t, 1H), 9.16 (t, 1H), 9.31 (d, 1H).

### Example02.16

### 4-{2-[(2,4-dimethoxyphenyl)amino][1,2,4]triazolo[1,5-a]pyridin-6-yl}-N-(4-fluorobenzyl)benzamide

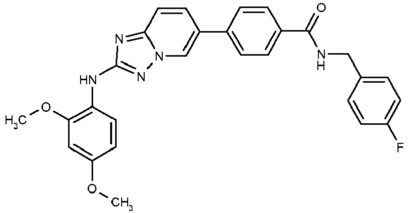

Starting with **Int5.2** and 1-bromo-2,4-dimethoxybenzene, **Example02.16** was prepared analogously to the procedure for the preparation of **Example02.13**. ¹H-NMR (400 MHz, DMSO-d₆): δ [ppm] = 3.76 (s, 3H), 3.84 (s, 3H), 4.48 (d, 2H), 6.55 (dd, 1 H), 6.65 (d, 1 H), 7.12 - 7.21 (m, 2H), 7.33 - 7.41 (m, 2H), 7.61 (d, 1H), 7.86 - 7.93 (m, 3H), 7.93 - 8.03 (m, 4H), 9.14 (t, 1H), 9.21 (d, 1H).

### Example02.17

### 4-[(6-{4-[(4-fluorobenzyl)carbamoyl]phenyl}[1,2,4]triazolo[1,5-a]pyridin-2-yl)amino]-3-methoxy-N-(2,2,2-trifluoroethyl)benzamide

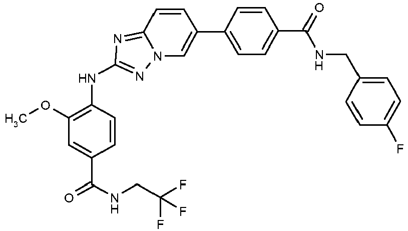

Starting with **Int5.2** and **Int10.12, Example02.17** was prepared analogously to the procedure for the preparation of **Example02.13**.

¹H-NMR (400 MHz, DMSO-d₆): δ [ppm] = 3.96 (s, 3H), 4.05 - 4.17 (m, 2H), 4.49 (d, 2H), 7.12 - 7.21 (m, 2H), 7.34 - 7.42 (m, 2H), 7.54 - 7.65 (m, 2H), 7.73 (d, 1 H), 7.91 - 7.98 (m, 2H), 7.99 - 8.08 (m, 3H), 8.39 (d, 1 H), 8.46 (s, 1 H), 8.95 (t, 1H), 9.16 (t, 1H), 9.32 (d, 1H).

### Example02.18

### 4-[(6-{4-[(4-fluorobenzyl)carbamoyl]phenyl}[1,2,4]triazolo[1,5-a]pyridin-2-yl)amino]-N-(1-hydroxy-2-methylpropan-2-yl)-3-(2,2,2-trifluoroethoxy)-benzamide

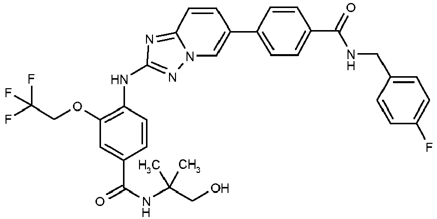

Starting with **Int5.2** and **Int12.5, Example02.18** was prepared analogously to the procedure for the preparation of **Example02.13**.

¹H-NMR (400 MHz, DMSO-d₆): δ [ppm] = 1.33 (s, 6H), 3.54 (d, 2H), 4.49 (d, 2H), 4.90 - 5.00 (m, 3H), 7.12 - 7.21 (m, 2H), 7.34 - 7.44 (m, 3H), 7.57 - 7.64 (m, 2H), 7.73 (d, 1 H), 7.91 - 7.98 (m, 2H), 7.99 - 8.09 (m, 3H), 8.28 (s, 1 H), 8.35 (d, 1H), 9.16 (t, 1H), 9.30 (d, 1H).

### Example02.19

### 3-ethoxy-4-[(6-{4-[(4-fluorobenzyl)carbamoyl]phenyl}[1,2,4]triazolo[1,5-a]pyridin-2-yl)amino]-N-[2-(methylsulfonyl)ethyl]benzamide

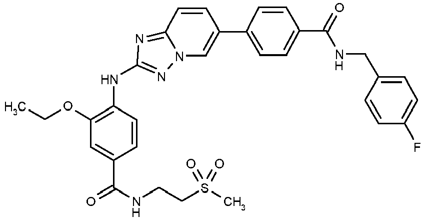

Starting with **Int5.2** and **Int11.12, Example02.19** was prepared analogously to the procedure for the preparation of **Example02.13.**

¹H-NMR (400 MHz, DMSO-d₆): δ [ppm] = 1.43 (t, 3H), 3.01 (s, 3H), 3.35 (t, 2H), 3.59 - 3.68 (m, 2H), 4.17 (q, 2H), 4.46 (d, 2H), 7.09 - 7.19 (m, 2H), 7.30 - 7.38 (m, 2H), 7.45 - 7.53 (m, 2H), 7.66 - 7.72 (m, 1 H), 7.87 - 7.93 (m, 2H), 7.96 - 8.05 (m, 3H), 8.26 (s, 1 H), 8.34 (d, 1 H), 8.59 (t, 1 H), 9.12 (t, 1 H), 9.27 (d, 1 H).

### Example02.20

### N-ethyl-4-[(6-{4-[(4-fluorobenzyl)carbamoyl]phenyl}[1,2,4]triazolo[1,5-a]-pyridin-2-yl)amino]-3-methoxybenzamide

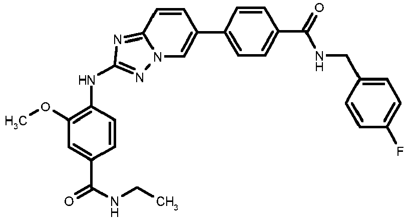

Starting with **Int5.2** and 4-bromo-*N*-ethyl-3-methoxy-benzamide, **Example013.21** was prepared analogously to the procedure for the preparation of **Example02.13.**

¹H-NMR (400 MHz, DMSO-d₆): δ [ppm] = 1.10 (t, 3H), 3.26 (q, 2H), 3.91 (s, 3H), 4.45 (d, 2H), 7.13 (t, 2H), 7.34 (dd, 2H), 7.46 - 7.52 (m, 2H), 7.68 (d, 1 H), 7.88 - 7.93 (m, 2H), 7.98 (d, 3H), 8.29 - 8.35 (m, 3H), 9.12 (t, 1 H), 9.28 (s, 1 H).

### Example02.21

### N-tert-butyl-3-ethoxy-4-[(6-{4-[(4-fluorobenzyl)carbamoyl]phenyl}[1,2,4]-triazolo[1,5-a]pyridin-2-yl)amino]benzamide

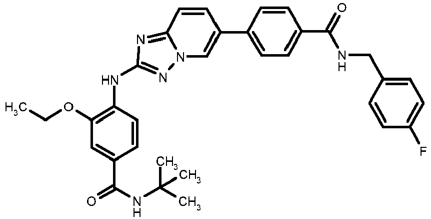

Starting with **Int5.2** and **Int11.3, Example02.21** was prepared analogously to the procedure for the preparation of **Example02.13.**

¹H-NMR (400 MHz, DMSO-d₆): δ [ppm] = 1.40 (s, 9H), 1.46 (t, 3H), 4.21 (q, 2H), 4.49 (d, 2H), 7.17 (t, 2H), 7.38 (dd, 2H), 7.46 (s, 1H), 7.50 (d, 1H), 7.59 (s, 1H), 7.71 (d, 1H), 7.90 - 7.98 (m, 2H), 7.99 - 8.08 (m, 3H), 8.21 (s, 1H), 8.33 (d, 1H), 9.16 (t, 1H), 9.30 (s, 1H).

### Example02.22

### 4-[(6-{4-[(4-fluorobenzyl)carbamoyl]phenyl}[1,2,4]triazolo[1,5-a]pyridin-2-yl)amino]-N-(2-hydroxy-2-methylpropyl)-3-(2,2,2-trifluoroethoxy)benzamide

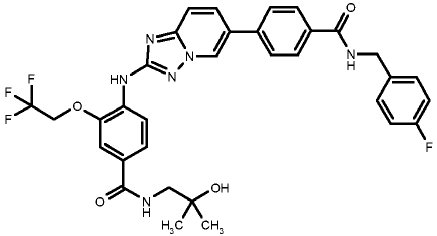

Starting with **Int5.2** and **Int12.6, Example02.22** was prepared analogously to the procedure for the preparation of **Example02.13.**

¹H-NMR (400 MHz, DMSO-d₆): δ [ppm] = 1.12 (s, 6H), 3.28 (d, 2H), 4.49 (d, 2H), 4.60 (s, 1 H), 4.95 (q, 2H), 7.17 (t, 2H), 7.38 (dd, 2H), 7.63 - 7.78 (m, 3H), 7.91 - 7.99 (m, 2H), 7.99 - 8.09 (m, 3H), 8.19 (t, 1 H), 8.31 (s, 1 H), 8.37 (d, 1 H), 9.15 (t, 1H), 9.31 (s, 1H).

### Example02.23

### 4-[(6-{4-[(4-fluorobenzyl)carbamoyl]phenyl}[1,2,4]triazolo[1,5-a]pyridin-2-yl)amino]-3-(2,2,2-trifluoroethoxy)-N-(2,2,2-trifluoroethyl)benzamide

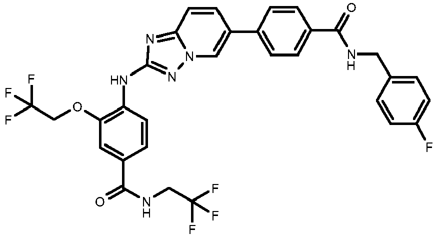

Starting with **Int5.2** and **Int12.4, Example02.23** was prepared analogously to the procedure for the preparation of **Example02.13.**

¹H-NMR (400 MHz, DMSO-d₆): δ [ppm] = 4.06 - 4.20 (m, 2H), 4.49 (d, 2H), 4.95 (q, 2H), 7.11 - 7.21 (m, 2H), 7.33 - 7.43 (m, 2H), 7.67 - 7.78 (m, 3H), 7.92 - 7.98 (m, 2H), 7.99 - 8.09 (m, 3H), 8.38 - 8.46 (m, 2H), 8.94 (t, 1H), 9.16 (t, 1 H), 9.29 - 9.35 (m, 1 H).

### Example02.24

### 4-(2-{[4-(dimethylamino)-2-methylphenyl]amino}[1,2,4]triazolo[1,5-a]-pyridin-6-yl)-N-(4-fluorobenzyl)benzamide

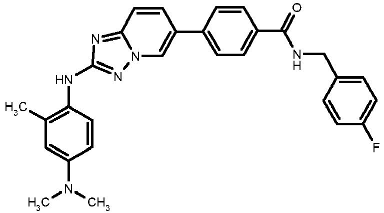

Starting with **Int5.2** and 4-bromo-*N,N*,3-trimethylaniline, **Example02.24** was prepared analogously to the procedure for the preparation of **Example02.13.** ¹H-NMR (400 MHz, DMSO-d₆): δ [ppm] = 2.22 (s, 3H), 2.86 (s, 6H), 4.48 (d, 2H), 6.53 - 6.64 (m, 2H), 7.11 - 7.20 (m, 2H), 7.33 - 7.41 (m, 3H), 7.51 (d, 1 H), 7.84 - 7.93 (m, 3H), 7.96 - 8.03 (m, 2H), 8.24 (s, 1 H), 9.10 (d, 1 H), 9.12 (t, 1 H).

### Example02.25

### 4-(2-{[2-ethoxy-4-(methylsulfonyl)phenyl]amino}[1,2,4]triazolo[1,5-a]-pyridin-6-yl)-N-(4-fluorobenzyl)benzamide

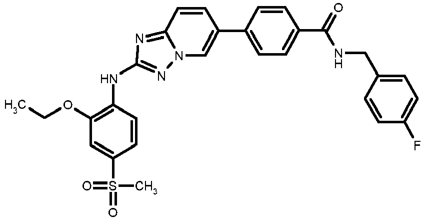

Starting with **Int5.2** and **Int11.15, Example02.25** was prepared analogously to the procedure for the preparation of **Example01.32.**

¹H-NMR (400MHz, DMSO-d₆): δ [ppm]= 1.44 (t, 3H), 3.16 (s, 3H), 4.22 (q, 2H), 4.46 (d, 2H), 7.05 - 7.17 (m, 2H), 7.31 - 7.38 (m, 2H), 7.42 (d, 1 H), 7.51 (dd, 1 H), 7.71 (dd, 1 H), 7.87 - 8.05 (m, 5H), 8.49 (d, 1 H), 8.53 (s, 1 H), 9.10 (t, 1 H), 9.27 (dd, 1H).

### Example02.26

### 4-(2-{[2-ethoxy-4-(ethylsulfonyl)phenyl]amino}[1,2,4]triazolo[1,5-a]-pyridin-6-yl)-N-(4-fluorobenzyl)benzamide

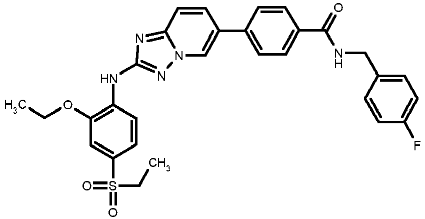

Starting with **Int5.2** and **Int11.17, Example02.26** was prepared analogously to the procedure for the preparation of **Example01.33.**

¹H-NMR (300MHz, DMSO-d₆): δ [ppm]= 1.08 (t, 3H), 1.43 (t, 3H), 3.23 (q, 2H), 4.21 (q, 2H), 4.45 (d, 2H), 7.06 - 7.19 (m, 2H), 7.28 - 7.39 (m, 3H), 7.47 (dd, 1 H), 7.72 (d, 1 H), 7.85 - 8.08 (m, 5H), 8.50 (d, 1 H), 8.59 (s, 1 H), 9.12 (t, 1 H), 9.28 (d, 1H).

### Example02.27

### N-(4-fluorobenzyl)-4-(2-{[4-(methylsulfonyl)-2-(2,2,2-trifluoroethoxy)-phenyl]amino}[1,2,4]triazolo[1,5-a]pyridin-6-yl)benzamide

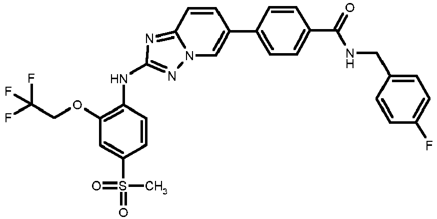

Starting with **Int5.2** and **Int12.9, Example02.27** was prepared analogously to the procedure for the preparation of **Example01.32**.

¹H-NMR (300MHz, DMSO-d₆): δ [ppm]= 3.17 (s, 3H), 4.46 (d, 2H), 5.00 (q, 2H), 7.06 - 7.19 (m, 2H), 7.34 (dd, 2H), 7.56 - 7.67 (m, 2H), 7.73 (d, 1 H), 7.85 - 8.09 (m, 5H), 8.50 (d, 1 H), 8.62 (s, 1 H), 9.10 (t, 1 H), 9.28 (s, 1 H).

### Example02.28

### 4-(2-{[2-(difluoromethoxy)-4-(methylsulfonyl)phenyl]amino}[1,2,4]triazolo-[1,5-a]pyridin-6-yl)-N-(4-fluorobenzyl)benzamide

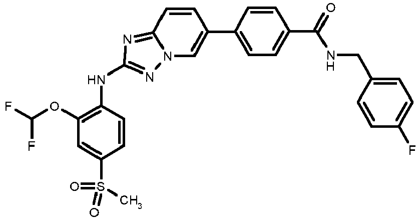

Starting with **Int5.2** and **Int17.3, Example02.28** was prepared analogously to the procedure for the preparation of **Example01.27**. ¹H-NMR (400 MHz, DMSO-d₆): δ [ppm] = 3.23 (s, 3H), 4.49 (d, 2H), 7.13 - 7.20 (m, 2H), 7.28 (t, 1H), 7.35 - 7.41 (m, 2H), 7.71 (d, 1H), 7.78 (dd, 1H), 7.83 (dd, 1H), 7.92 - 7.98 (m, 2H), 8.00 - 8.05 (m, 2H), 8.08 (dd, 1 H), 8.66 (d, 1 H), 9.14 (t, 1 H), 9.29 - 9.33 (m, 1H), 9.51 (s, 1H).

### Example02.29

### 4-(2-{[2-(cyclopropyloxy)-4-(methylsulfonyl)phenyl]amino}[1,2,4]triazolo-[1,5-a]pyridin-6-yl)-N-(4-fluorobenzyl)benzamide

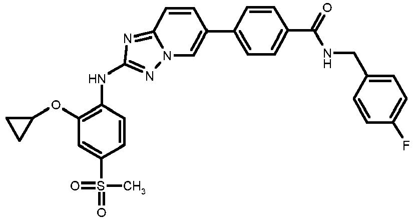

Starting with **Int5.2** and **Int18.3, Example02.29** was prepared analogously to the procedure for the preparation of **Example01.37**.

¹H-NMR (500MHz, DMSO-d₆): δ [ppm]= 0.83 - 0.96 (m, 4H), 3.21 (s, 3H), 4.13 (tt, 1 H), 4.50 (d, 2H), 7.13 - 7.21 (m, 2H), 7.39 (dd, 2H), 7.59 (dd, 1 H), 7.73 (d, 1H), 7.76 (d, 1H), 7.93 - 8.05 (m, 4H), 8.07 (dd, 1H), 8.53 (d, 1H), 8.68 (s, 1H), 9.16 (t, 1H), 9.32 (d, 1H).

### Example03.1

### 2-fluoro-N-(4-fluorobenzyl)-4-[2-({4-[(1-hydroxy-2-methylpropan-2-yl)-carbamoyl]-2-methoxyphenyl}amino)[1,2,4]triazolo[1,5-a]pyridin-6-yl]-benzamide

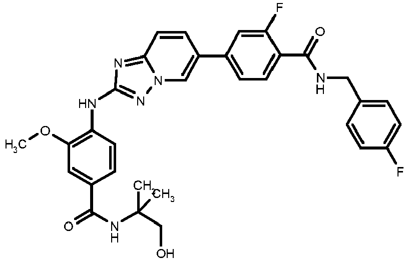

To a stirred suspension of **Int6.2** (100 mg) in toluene (4.0 mL) and NMP (1.0 mL) in a sealed tube was added **Int10.5** (119 mg), chloro(2-dicyclohexylphosphino-2',4',6'-tri-i-propyl-1,1'-biphenyl)[2-(2-aminoethyl)-phenyl] palladium(II) methyl-*tert*-butylether adduct (22 mg), X-Phos (13 mg), and powdered potassium phosphate (280 mg). The flask was twice degased and backfilled with argon. The mixture was heated to 130°C with an oil bath for 2 h. A mixture of DCM and methanol (100:1) added, solids were removed by filtration and the solvent was removed in vacuum. Aminophase-silica-gel chromatography gave a solid that was titurated with ethyl acetate to give 40 mg of the title compound.

¹H-NMR (300MHz, DMSO-d₆): δ [ppm]= 1.29 (s, 6H), 3.49 (d, 2H), 3.92 (s, 3H), 4.44 (d, 2H), 4.92 (t, 1H), 7.08 - 7.21 (m, 2H), 7.30 - 7.51 (m, 5H), 7.63 - 7.75 (m, 3H), 7.82 (d, 1 H), 8.02 (dd, 1 H), 8.24 - 8.37 (m, 2H), 8.85 - 8.96 (m, 1 H), 9.32 (d, 1H).

### Example04.1

### N-(4-fluorobenzyl)-4-[2-({4-[(1-hydroxy-2-methylpropan-2-yl)carbamoyl]-2-methoxyphenyl}amino)[1,2,4]triazolo[1,5-a]pyridin-6-yl]-2-methylbenzamide

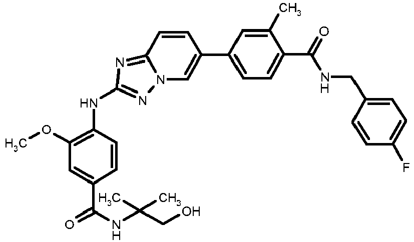

To a stirred suspension of **Int7.2** (100 mg) in toluene (4.0 mL) and NMP (1.0 mL) in a sealed tube was added **Int10.5** (120 mg), chloro(2-dicyclohexylphosphino-2',4',6'-tri-i-propyl-1,1'-biphenyl)[2-(2-aminoethyl)-phenyl] palladium(II) methyl-*tert*-butylether adduct (22 mg), X-Phos (13 mg), and powdered potassium phosphate (282 mg). The flask was twice degased and backfilled with argon. The mixture was heated to 130°C with an oil bath for 2 h. A mixture of DCM and methanol (100:1) added, solids were removed by filtration and the solvent was removed in vacuum. Aminophase-silica-gel chromatography gave 45 mg of the title compound.

¹H-NMR (300MHz, DMSO-d₆): δ [ppm]= 1.29 (s, 6H), 2.39 (s, 3H), 3.49 (d, 2H), 3.92 (s, 3H), 4.41 (d, 2H), 4.89 - 4.97 (m, 1H), 7.09 - 7.21 (m, 2H), 7.31 - 7.50 (m, 6H), 7.61 - 7.73 (m, 3H), 7.96 (dd, 1 H), 8.23 - 8.35 (m, 2H), 8.85 (t, 1 H), 9.19 (dd, 1H).

### Example04.2

### N-(4-fluorobenzyl)-2-methyl-4-(2-{[4-(methylsulfonyl)-2-(2,2,2-trifluoro-ethoxy)phenyl]amino}[1,2,4]triazolo[1,5-a]pyridin-6-yl)benzamide

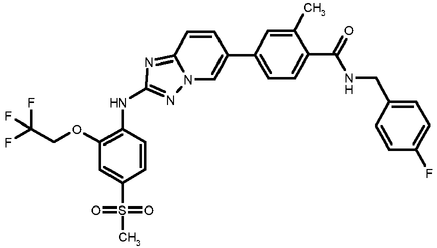

Starting with **Int7.2** and **Int12.9, Example04.2** was prepared analogously to the procedure for the preparation of **Example04.1**.

¹H-NMR (400MHz, DMSO-d₆): δ [ppm]= 2.39 (s, 3H), 3.17 (s, 3H), 4.41 (d, 2H), 5.00 (q, 2H), 7.09 - 7.21 (m, 2H), 7.32 - 7.40 (m, 2H), 7.46 (d, 1H), 7.58 - 7.76 (m, 5H), 8.00 (dd, 1H), 8.51 (d, 1H), 8.60 (s, 1H), 8.84 (t, 1H), 9.22 (dd, 1H).

### Example05.1

### 2-chloro-N-(4-fluorobenzyl)-4-[2-({4-[(1-hydroxy-2-methylpropan-2-yl)-carbamoyl]-2-methoxyphenyl}amino)[1,2,4]triazolo[1,5-a]pyridin-6-yl]-benzamide

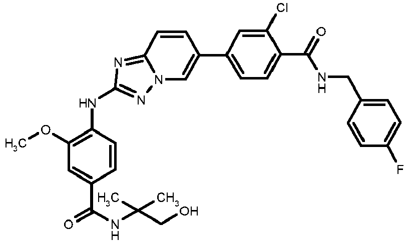

To a stirred suspension of **Int8.2** (100 mg) in toluene (3.0 mL) and NMP (0.3 mL) in a sealed tube was added **Int10.5** (115 mg), chloro(2-dicyclohexylphosphino-2',4',6'-tri-i-propyl-1,1'-biphenyl)[2-(2-aminoethyl)-phenyl] palladium(II) methyl-*tert*-butylether adduct (221 mg), X-Phos (12 mg), and powdered potassium phosphate (268 mg). The flask was twice degased and backfilled with argon. The mixture was heated to 100°C with an oil bath for 3 h. A mixture of DCM and methanol (100:1) added, solids were removed by filtration and the solvent was removed in vacuum. Aminophase-silica-gel chromatography followed by preparative reverse phase HPLC gave a solid that was recrystallized from DCM and diisopropyl ether to give 35 mg of the title compound.

¹H-NMR (400MHz, DMSO-d₆): δ [ppm]= 1.29 (s, 6H), 3.50 (d, 2H), 3.92 (s, 3H), 4.43 (d, 2H), 4.92 (t, 1H), 7.09 - 7.21 (m, 2H), 7.34 - 7.43 (m, 4H), 7.46 (dd, 1H), 7.53 (d, 1H), 7.68 (d, 1H), 7.82 (dd, 1H), 7.94 - 8.04 (m, 2H), 8.25 - 8.35 (m, 2H), 9.02 (t, 1 H), 9.30 (d, 1 H).

### Example06.1

### 4-[(6-{4-[(cyclopropylmethyl)carbamoyl]phenyl}[1,2,4]triazolo[1,5-a]-pyridin-2-yl)amino]-3-ethoxy-N-ethylbenzamide

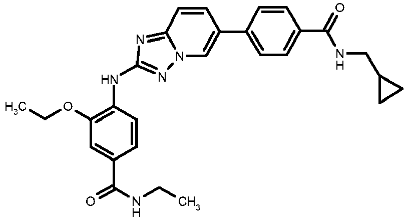

To a stirred suspension of **Int5.4** (1500 mg) in toluene (44.0 mL) and NMP (9.0 mL) was added 4-bromo-3-ethoxy-*N*-ethylbenzamide (**Int11.4**) (1762 mg), chloro(2-dicyclohexylphosphino-2',4',6'-tri-i-propyl-1,1'-biphenyl)[2-(2-amino-ethyl)phenyl] palladium(II) methyl-*tert*-butylether adduct (242 mg) and X-Phos (140 mg). The flask was twice degased and backfilled with argon. The mixture was stirred at r.t. for 5 minutes. Sodium *tert*-butoxide (2.35 g) was added, the flask was twice degased and backfilled with argon and the mixture was heated to reflux for 2 h. Water was added and the precipitated solid was isolated by filtration. Aminophase-silica-gel chromatography gave a solid that was triturated with DCM to give 2.2 g of the title compound.

¹H-NMR (300MHz, DMSO-d₆): δ [ppm]= 0.16 - 0.25 (m, 2H), 0.36 - 0.45 (m, 2H), 0.95 - 1.05 (m, 1H), 1.10 (t, 3H), 1.42 (t, 3H), 3.14 (t, 2H), 3.20 - 3.28 (m, 2H), 4.17 (q, 2H), 7.43 - 7.53 (m, 2H), 7.67 (d, 1H), 7.82 - 8.05 (m, 5H), 8.19 (s, 1 H), 8.31 (d, 2H), 8.62 (t, 1 H), 9.27 (d, 1 H).

### Example06.2

### 4-[(6-{4-[(cyclopropylmethyl)carbamoyl]phenyl}[1,2,4]triazolo[1,5-a]-pyridin-2-yl)amino]-3-ethoxy-N-ethyl-N-(2-methoxyethyl)benzamide

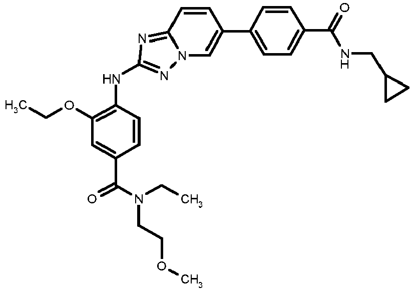

To a stirred suspension of **Int5.4** (96 mg) in toluene (3.0 mL) was 4-bromo-3-ethoxy-*N*-ethyl-*N*-(2-methoxyethyl)benzamide (**Int11.9**) (206 mg), chloro(2-dicyclohexylphosphino-2',4',6'-tri-i-propyl-1,1'-biphenyl)[2-(2-aminoethyl)-phenyl] palladium(II) methyl-*tert*-butylether adduct (13 mg) and X-Phos (8 mg) and the flask was twice degased and backfilled with argon. The mixture was stirred for 5 minutes at r.t.. Sodium *tert*-butoxide (150 mg) was added and the flask was twice degased and backfilled with argon. The mixture was heated to reflux for 2 h. Water was added and the reaction mixture was extracted with ethyl acetate. The organic phase was washed with saturated sodium chloride solution, dried (sodium sulfate) and the solvent was removed in vacuum. Silica-gel chromatography followed by Aminophase-silica gel chromatography gave a solid that was triturated with a mixture of ethyl acetate and hexane to give 62 mg of the title compound.

¹H-NMR (300MHz, DMSO-d₆): δ [ppm]= 0.17 - 0.25 (m, 2H), 0.36 - 0.47 (m, 2H), 0.92 - 1.14 (m, 4H), 1.40 (t, 3H), 3.14 (t, 2H), 3.23 (s, 3H), 3.31 - 3.58 (m, 6H), 4.13 (q, 2H), 6.93 - 7.05 (m, 2H), 7.66 (d, 1H), 7.85 - 7.97 (m, 4H), 8.00 (dd, 1H), 8.13 (s, 1H), 8.28 (d, 1H), 8.62 (t, 1H), 9.26 (s, 1H).

### Example06.3

### 4-[(6-{4-[(cyclopropylmethyl)carbamoyl]phenyl}[1,2,4]triazolo[1,5-a]-pyridin-2-yl)amino]-3-ethoxy-N-(2-hydroxyethyl)benzamide

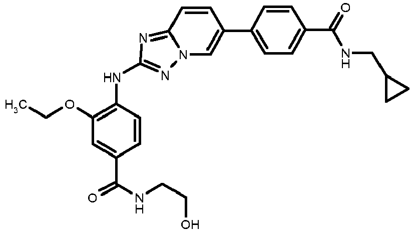

To a stirred suspension of **Int5.4** (80 mg) in toluene (4.0 mL) and NMP (1.0 mL) in a sealed tube was added **Int11.5** (112 mg), chloro(2-dicyclohexylphosphino-2',4',6'-tri-i-propyl-1,1'-biphenyl)[2-(2-aminoethyl)phenyl] palladium(II) methyl-*tert*-butylether adduct (22 mg), X-Phos (13 mg), and powdered potassium phosphate (276 mg). The flask was twice degased and backfilled with argon. The mixture was heated to 130°C with an oil bath for 2 h. A mixture of DCM and methanol (100:1) added, solids were removed by filtration and the solvent was removed in vacuum. Aminophase-silica-gel chromatography gave a solid that was titurated with warm ethanol to give 75 mg of the title compound.

¹H-NMR (400MHz, DMSO-d₆): δ [ppm]= 0.18 - 0.24 (m, 2H), 0.38 - 0.44 (m, 2H), 0.96 - 1.08 (m, 1H), 1.43 (t, 3H), 3.14 (t, 2H), 3.30 - 3.34 (m, 2H), 3.48 (q, 2H), 4.17 (q, 2H), 4.69 - 4.74 (m, 1 H), 7.47 - 7.54 (m, 2H), 7.68 (dd, 1 H), 7.86 - 7.98 (m, 4H), 8.01 (dd, 1H), 8.21 (s, 1H), 8.28 - 8.36 (m, 2H), 8.62 (t, 1H), 9.28 (dd, 1H).

### Example06.4

### 4-[(6-{4-[(cyclopropylmethyl)carbamoyl]phenyl}[1,2,4]triazolo[1,5-a]-pyridin-2-yl)amino]-N-(2-hydroxyethyl)-3-(2,2,2-trifluoroethoxy)benzamide

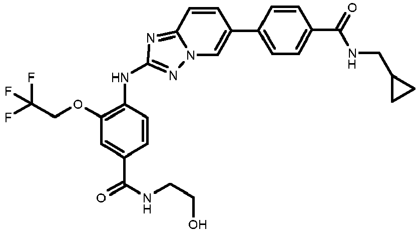

Starting with Intermediate **Int5.4** and ; 4-bromo*-*N-(2-hydroxyethyl)-3-(2,2,2-trifluoroethoxy)benzamide **(Int12.3); Example06.4** was prepared analogously to the procedure for the preparation of **Example06.3.**

¹H-NMR (400MHz, DMSO-d₆): δ [ppm]= 0.17 - 0.24 (m, 2H), 0.36 - 0.45 (m, 2H), 0.96 - 1.07 (m, 1H), 3.14 (t, 2H), 3.30 - 3.35 (m, 2H), 3.44 - 3.52 (m, 2H), 4.70 - 4.78 (m, 1 H), 4.90 (q, 2H), 7.58 - 7.73 (m, 3H), 7.87 - 7.98 (m, 4H), 8.02 (dd, 1 H), 8.26 - 8.37 (m, 3H), 8.63 (t, 1 H), 9.29 (dd, 1 H).
Starting materials: Intermediate **Int5.4;** 4-bromo-*N*-(2-hydroxyethyl)-3-(2,2,2-trifluoroethoxy)benzamide **(Int12.3)**

### Example06.5

### 4-[(6-{4-[(cyclopropylmethyl)carbamoyl]phenyl}[1,2,4]triazolo[1,5-a]-pyridin-2-yl)amino]-N-ethyl-3-(2,2,2-trifluoroethoxy)benzamide

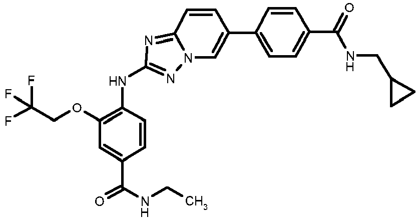

4-(2-Amino[1,2,4]triazolo[1,5-*a*]pyridin-6-yl)-*N*-(cyclopropylmethyl)benzamide **Int5.4** (100 mg, 76% purity), *N*-ethyl-4-iodo-3-(2,2,2-trifluoroethoxy)benzamide **Int14.2** (111 mg), chloro(2-dicyclohexylphosphino-2',4',6'-tri-i-propyl-1,1'-biphenyl)[2-(2-aminoethyl)phenyl] palladium(II) (18 mg), X-Phos (12 mg), and sodium *tert*-butoxide (43.4 mg) were pre-mixed, and degassed toluene (1.5 mL) was added. The mixture was heated for 6h to 130 °C. Subsequently, DCM was added, and the mixture was washed with satd. aqueous sodium carbonate solution. The organic layer was dried over sodium sulfate, and the solvent was evaporated. The crude product was purified by flash chromatography on silica gel (eluent: ethyl acetate/ cyclohexane gradient 4:1 to 8:1). The product was titurated with DCM/tert butyl methylether/pentane and collected by suction filtration to yield 30 mg (21%) of the title compound.

¹H-NMR (400MHz, DMSO-d₆): δ [ppm]= 0.22 - 0.28 (m, 2H), 0.41 - 0.49 (m, 2H), 1.00 - 1.11 (m, 1H), 1.15 (t, 3H), 3.18 (t, 2H), 3.26 - 3.37 (m, 2H), 4.93 (q, 2H), 7.63 (d, 1H), 7.66 (s, 1H), 7.73 (d, 1H), 7.90 - 8.02 (m, 4H), 8.06 (d, 1H), 8.31 (s, 1 H), 8.32 - 8.40 (m, 2H), 8.66 (t, 1 H), 9.32 (s, 1 H).

### Example07.1

### N-ethyl-4-[(6-{4-[(3-fluorobenzyl)carbamoyl]phenyl}[1,2,4]triazolo[1,5-a]-pyridin-2-yl)amino]-3-methoxybenzamide

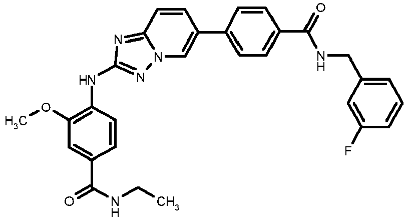

Starting with **Int5.8** and 4-bromo-*N*-ethyl-3-methoxy-benzamide, Example07.1 was prepared analogously to the procedure for the preparation of

### Example02.13.

¹H-NMR (400 MHz, DMSO-d₆): δ [ppm] = 1.14 (t, 3H), 3.25 - 3.32 (m, 2H), 3.95 (s, 3H), 4.53 (d, 2H), 7.04 - 7.12 (m, 1 H), 7.12 - 7.22 (m, 2H), 7.35 - 7.43 (m, 1 H), 7.48 - 7.57 (m, 2H), 7.72 (d, 1 H), 7.91 - 8.00 (m, 2H), 8.00 - 8.10 (m, 3H), 8.31 - 8.40 (m, 3H), 9.19 (t, 1 H), 9.32 (s, 1 H).

### Example08.1

### N-(4-fluorobenzyl)-4-[2-({4-[(1-hydroxy-2-methylpropan-2-yl)carbamoyl]-2-methoxyphenyl}amino)[1,2,4]triazolo[1,5-a]pyridin-6-yl]-2-methoxybenz-amide

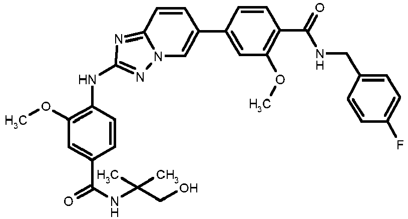

To a stirred suspension of **Int9.2** (100 mg) in toluene (3.0 mL) and NMP (1.0 mL) was added **Int10.5** (115 mg), chloro(2-dicyclohexylphosphino-2',4',6'-tri-i-propyl-1,1'-biphenyl)[2-(2-aminoethyl)phenyl] palladium(II) methyl-*tert*-butylether adduct (21 mg) and X-Phos (12 mg) and powdered potassium phosphate (271 mg). The flask was twice degased and backfilled with argon. The mixture was heated to reflux for 3 h. The solvent was removed in vacuum. Aminophase-silica-gel chromatography followed by preparative reverse phase HPLC gave 200 mg of the title compound.

¹H-NMR (300MHz, DMSO-d₆): δ [ppm]= 1.29 (s, 6H), 3.49 (d, 2H), 3.92 (s, 3H), 4.01 (s, 3H), 4.47 (d, 2H), 4.92 (t, 1H), 7.04 - 7.19 (m, 2H), 7.30 - 7.54 (m, 7H), 7.68 (d, 1 H), 7.82 (d, 1 H), 8.02 (dd, 1 H), 8.25 - 8.34 (m, 2H), 8.74 (t, 1 H), 9.33 (s, 1H).

### Example09.1

### N-(4-fluorobenzyl)-2-methoxy-4-(2-{[2-methoxy-4-(methylsulfonyl)phenyl]-amino}[1,2,4]triazolo[1,5-a]pyridin-6-yl)benzamide

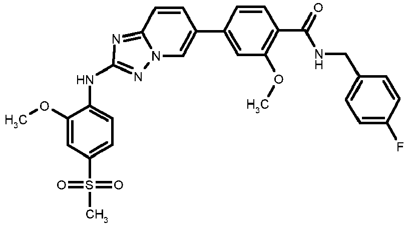

Starting with **Int9.2** and **Int10.11, Example09.1** was prepared analogously to the procedure for the preparation of **Example01.32**.

¹H-NMR (300MHz, DMSO-d₆): δ [ppm]= 3.17 (s, 3H), 3.96 (s, 3H), 4.01 (s, 3H), 4.47 (d, 2H), 7.08 - 7.18 (m, 2H), 7.30 - 7.39 (m, 2H), 7.40 - 7.47 (m, 2H), 7.49 - 7.55 (m, 2H), 7.72 (d, 1H), 7.82 (d, 1H), 8.05 (dd, 1H), 8.49 (d, 1H), 8.68 (s, 1H), 8.74 (t, 1H), 9.35 (d, 1H).

### Example10.1

### 2-(2,4-difluorophenyl)-N-[4-(2-{[2-methoxy-4-(methylsulfonyl)phenyl]-amino}[1,2,4]triazolo[1,5-a]pyridin-6-yl)phenyl]acetamide

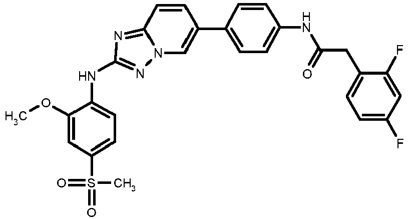

Starting with **Int3.6** and **Int10.11, Example10.1** was prepared analogously to the procedure for the preparation of **Example01.32.**

¹H-NMR (300MHz, DMSO-d₆): δ [ppm]= 3.16 (s, 3H), 3.72 (s, 2H), 3.96 (s, 3H), 6.98 - 7.08 (m, 1H), 7.19 (td, 1H), 7.37 - 7.47 (m, 2H), 7.52 (dd, 1H), 7.61 - 7.79 (m, 5H), 7.93 (dd, 1H), 8.48 (d, 1H), 8.59 (s, 1H), 9.11 (d, 1H), 10.31 (s, 1 H).

### Example 11.1

### N-(2,4-difluorobenzyl)-4-(2-{[2-methoxy-4-(methylsulfonyl)phenyl]amino}-[1,2,4]triazolo[1,5-a]pyridin-6-yl)benzamide

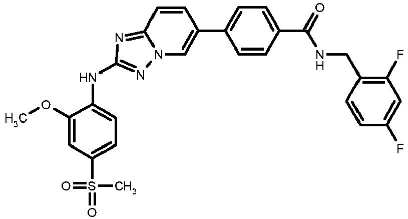

Starting with **Int5.7** and **Int10.11, Example11.1** was prepared analogously to the procedure for the preparation of **Example1.27**.

¹H-NMR (400M Hz, DMSO-d₆): δ [ppm] = 3.20 (s, 3H), 4.00 (s, 3H), 4.51 (d, 2H), 7.07 (t, 1H), 7.23 (t, 1H), 7.40 - 7.50 (m, 2H), 7.56 (d, 1H), 7.75 (d, 1H), 7.92 - 7.98 (m, 2H), 7.99 - 8.09 (m, 3H), 8.52 (d, 1H), 8.69 (s, 1H), 9.11 (t, 1H), 9.31 (s, 1H).

The following Examples wer prepared analogously to the procedures described above:

| Example No | Name | 1H NMR | Strukture | MW |
|---|---|---|---|---|
| Example 12.01 | 4-{[6-(4-{[(4-fluorophenyl)-acetyl]amino}phenyl)[1,2,4] triazolo[1, 5-*a*] pyridin-2-yl]amino}-3-methoxy-*N*-(2-methoxyethyl)benzamide | ¹H-NMR (400 MHz, DMSO-d₆): δ [ppm] = 3.28 (s, 3H), 3.39 - 3.53 (m, 4H), 3.68 (s, 2H), 3.95 (s, 3H), 7.12 - 7.22 (m, 2H), 7.33 - 7.43 (m, 2H), 7.50 - 7.58 (m, 2H), 7.67 (d, 1H), 7.74 (q, 4H), 7.94 (dd, 1H), 8.30 (s, 1H), 8.34 (d, 1H), 8.42 (t, 1H), 9.14 (d, 1H), 10.32 (s, 1H). | 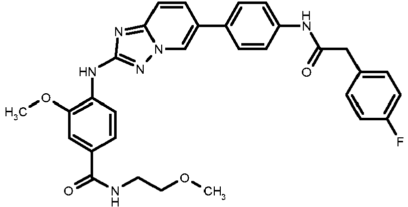 | 568.6 |
| Example 12.02 | 4-{[6-(4-{[(4-fluorophenyl)-acetyl]amino}phenyl)[1,2,4] triazolo[1, 5-*a*] pyridin-2-yl]amino}-3-methoxy-*N,N-*dimethylbenzamide | ¹H-NMR (400 MHz, DMSO-d₆): δ [ppm] = 2.99 (s, 6H), 3.68 (s, 2H), 3.91 (s, 3H), 7.01 - 7.11 (m, 2H), 7.12 - 7.22 (m, 2H), 7.31 - 7.43 (m, 2H), 7.66 (d, 1H), 7.69 - 7.80 (m, 4H), 7.93 (dd, 1H), 8.20 (s, 1H), 8.32 (d, 1H), 9.11 (d, 1H), 10.30 (s, 1H). | 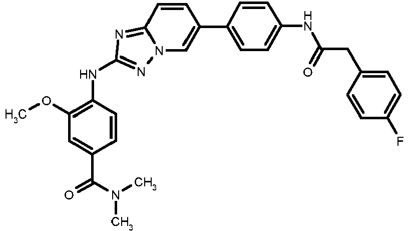 | 538.6 |
| Example 12.03 | 4-{[6-(4-{[(4-fluorophenyl)-acetyl]amino}phenyl)[1,2,4] triazolo[1, 5-*a*] pyridin-2-yl]amino}-3-methoxy-*N*-[2-(methylsulfonyl)ethyl]benza mide | ¹H-NMR (400 MHz, DMSO-d₆): δ [ppm] = 3.04 (s, 3H), 3.39 (t, 2H), 3.62 - 3.74 (m, 4H), 3.95 (s, 3H), 7.16 (t, 2H), 7.38 (dd, 2H), 7.48 - 7.57 (m, 2H), 7.63 - 7.80 (m, 5H), 7.94 (dd, 1H), 8.28 - 8.41 (m, 2H), 8.61 (t, 1H), 9.13 (s, 1H), 10.30 (s, 1H). | 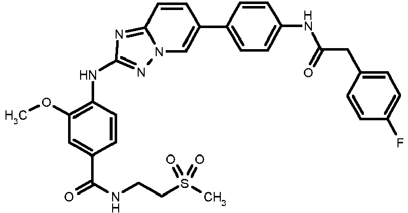 | 616.7 |
| Example 12.04 | 4-{[6-(4-{[(4-fluorophenyl)-acetyl]amino}phenyl)[1,2,4] triazolo[1, 5-*a*] pyridin-2-yl]amino}-3-methoxybenzamide | ¹H-NMR (400 MHz, DMSO-d₆): δ [ppm] = 3.68 (s, 2H), 3.94 (s, 3H), 7.11 - 7.20 (m, 2H), 7.23 (br. s., 1H), 7.38 (dd, 2H), 7.51 - 7.61 (m, 2H), 7.64 - 7.80 (m, 5H), 7.88 (br. s., 1H), 7.95 (dd, 1H), 8.28 - 8.38 (m, 2H), 9.16 (s, 1H), 10.32 (s, 1H). | 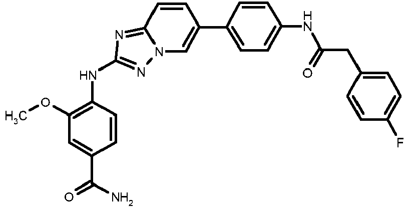 | 510.5 |
| Example 12.05 | *N*-(2-fluoroethyl)-4-{[6-(4-{[(4-fluorophenyl)acetyl]-amino}phenyl)[1,2,4]-triazolo[1,5-*a*]pyridin-2-yl]amino}-3-methoxybenzamide | ¹H-NMR (400 MHz, DMSO-d₆): δ [ppm] = 3.50 - 3.58 (m, 1H), 3.61 (d, 1H), 3.68 (s, 2H), 3.95 (s, 3H), 4.49 (t, 1H), 4.61 (t, 1H), 7.17 (t, 2H), 7.38 (dd, 2H), 7.50 - 7.61 (m, 2H), 7.64 - 7.81 (m, 5H), 7.95 (dd, 1H), 8.29 - 8.40 (m, 2H), 8.59 (t, 1H), 9.15 (s, 1H), 10.32 (s, 1H). | 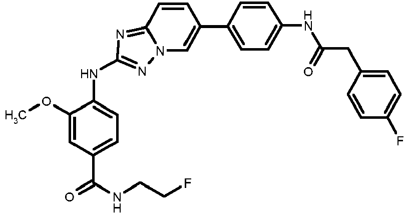 | 556.6 |
| Example 12.06 | *N*-ethyl-4-{[6-(4-{[(4-fluorophenyl)acetyl]amino }-phenyl)[1,2,4]triazolo[1,5-*a*]pyridin-2-yl]amino}-3-methoxy-*N*-methylbenzamide | ¹H-NMR (400 MHz, DMSO-d₆): δ [ppm] = 1.13 (t, 3H), 2.95 (s, 3H), 3.35 - 3.49 (m, 2H), 3.68 (s, 2H), 3.91 (s, 3H), 6.99 - 7.09 (m, 2H), 7.16 (t, 2H), 7.38 (dd, 2H), 7.66 (d, 1H), 7.69 - 7.81 (m, 4H), 7.93 (dd, 1H), 8.22 (s, 1H), 8.31 (d, 1H), 9.12 (s, 1H), 10.31 (s, 1H). | 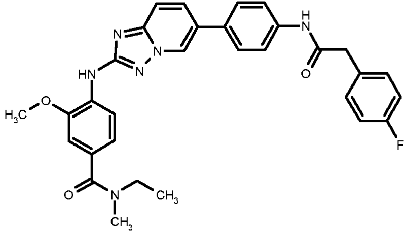 | 552.6 |
| Example 12.07 | *N*-(2,2-difluoroethyl)-4-{[6-(4-{[(4-fluorophenyl)-acetyl]amino}phenyl)[1,2,4] triazolo[1,5-*a*]pyridin-2-yl]amino}-3-methoxybenzamide | ¹H-NMR (400 MHz, DMSO-d₆): δ [ppm] = 3.68 (s, 4H), 3.95 (s, 3H), 5.95 - 6.30 (m, 1H), 7.12 - 7.21 (m, 2H), 7.32 - 7.42 (m, 2H), 7.50 - 7.62 (m, 2H), 7.65 - 7.80 (m, 5H), 7.95 (dd, 1H), 8.34 - 8.41 (m, 2H), 8.74 (t, 1H), 9.15 (d, 1H), 10.32 (s, 1H). | 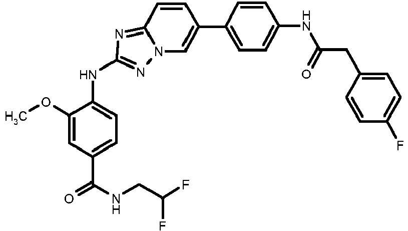 | 574.6 |
| Example 12.08 | *N*-[2-(dimethylamino)ethyl]-4-{[6-(4-{[(4-fluorophenyl)-acetyl]amino}phenyl)[1,2,4] triazolo[1,5-*a*]pyridin-2-yl]amino}-3-methoxy-*N-*methylbenzamide | ¹H-NMR (400 MHz, DMSO-d₆): δ [ppm] = 1.90 - 2.33 (m, 6H), 2.38 - 2.48 (m, 2H), 2.91 - 3.04 (m, 3H), 3.65 - 3.70 (m, 2H), 3.91 (s, 3H), 6.99 - 7.08 (m, 2H), 7.12 - 7.22 (m, 2H), 7.36 - 7.44 (m, 2H), 7.66 (d, 1H), 7.73 (q, 4H), 7.93 (dd, 1H), 8.21 - 8.26 (m, 1H), 8.23 (s, 1H), 8.31 (d, 1H), 9.12 (d, 1H), 10.32 (s, 1H). | 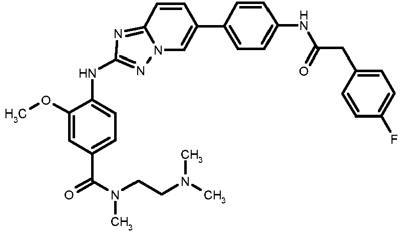 | 595.7 |
| Example 12.09 | 4-{[6-(4-{[(4-fluorophenyl)-acetyl]amino}phenyl)[1,2,4] triazolo[1, 5-*a*] pyridin-2-yl]amino}-3-methoxy-*N-*methyl-N-(2,2,2-trifluoro-ethyl)benzamide | ¹H-NMR (400 MHz, DMSO-d₆): δ [ppm] = 3.11 (s, 3H), 3.68 (s, 2H), 3.93 (s, 3H), 4.26 - 4.40 (m, 2H), 7.06 - 7.12 (m, 2H), 7.12 - 7.20 (m, 2H), 7.35 - 7.41 (m, 2H), 7.64 - 7.69 (m, 1H), 7.69 - 7.78 (m, 4H), 7.94 (dd, 1H), 8.29 (s, 1H), 8.36 (d, 1H), 9.07 - 9.16 (m, 1H), 10.30 (s, 1H). | 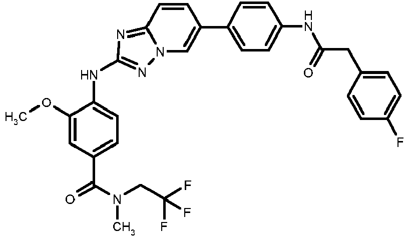 | 606.6 |
| Example 12.10 | *N*-[2-(dimethylamino)ethyl]-4-{[6-(4-{[(4-fluorophenyl)-acetyl]amino}phenyl)[1,2,4] triazolo[1,5-*a*]pyridin-2-yl]amino}-3-methoxybenzamide | ¹H-NMR (400 MHz, DMSO-d₆): δ [ppm] = 2.23 (s, 6H), 2.46 qt, 2H), 3.35 - 3.41 (q, 2H), 3.68 (s, 2H), 3.95 (s, 3H), 7.16 (t, 2H), 7.38 (dd, 2H), 7.48 - 7.56 (m, 2H), 7.67 (d, 1H), 7.69 - 7.79 (m, 4H), 7.94 (dd, 1H), 8.25 - 8.32 (m, 2H), 8.34 (d, 1H), 9.13 (s, 1H), 10.30 (s, 1H). | 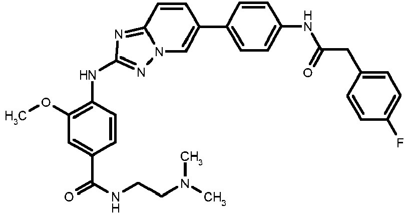 | 581.6 |
| Example 12.11 | 4-{[6-(4-{[(4-fluorophenyl)-acetyl]amino}phenyl)[1,2,4] triazolo[1, 5-*a*] pyridin-2-yl]amino}-*N*-(3-fluoro-propyl)-3-methoxybenzamide | ¹H-NMR (400 MHz, DMSO-d₆): δ [ppm] = 1.83 - 2.02 (m, 2H), 3.34 - 3.43 (m, 2H), 3.68 (s, 2H), 3.95 (s, 3H), 4.47 (t, 1H), 4.59 (t, 1H), 7.16 (t, 2H), 7.38 (dd, 2H), 7.50 - 7.56 (m, 2H), 7.67 (d, 1H), 7.74 (q, 4H), 7.94 (dd, 1H), 8.28 (s, 1H), 8.34 (d, 1H), 8.42 (t, 1H), 9.13 (d, 1H), 10.30 (s, 1H). | 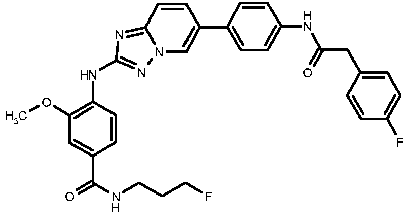 | 570.6 |
| Example 12.12 | *N*-[4-(2-{[5-fluoro-2-methoxy-4-(methylsulfonyl)-phenyl]amino}[1,2,4]-triazolo[1, 5-*a*] pyridin-6-yl)phenyl]-2-(4-fluoro-phenyl)acetamide | ¹H-NMR (300 MHz, DMSO-d₆): δ [ppm]= 3.21 - 3.27 (m, 3H), 3.64 (s, 2H), 3.91 (s, 3H), 7.08 - 7.17 (m, 2H), 7.22 (d, 1H), 7.34 (dd, 2H), 7.63 - 7.79 (m, 5H), 7.95 (dd, 1H), 8.42 (d, 1H), 9.06 (s, 1H), 9.17 (s, 1H), 10.28 (s, 1H). | 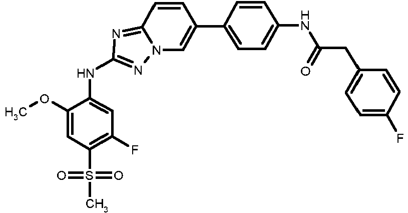 | 563.6 |
| Example 12.13 | 2-(4-fluorophenyl)-*N*-[4-(2-{[2-methoxy-4-(methylsulfinyl)phenyl]amino}-[1,2,4]triazolo[1,5-*a*]pyridin-6-yl)phenyl]acetamide | ¹H-NMR (300 MHz, DMSO-d₆): δ [ppm]= 2.71 (s, 3H), 3.64 (s, 2H), 3.93 (s, 3H), 7.07 - 7.17 (m, 2H), 7.21 - 7.40 (m, 4H), 7.59 - 7.77 (m, 5H), 7.89 (d, 1H), 8.33 (s, 1H), 8.41 (d, 1H), 9.09 (d, 1H), 10.28 (s, 1H). | 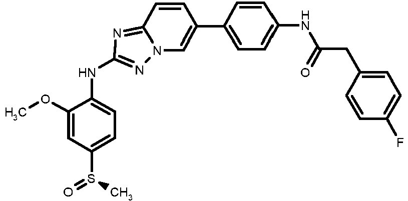 | 529.6 |
| Example 12.14 | *N*-[4-(2-{[5-fluoro-2-methoxy-4-(methylsulfinyl)-phenyl]amino}[1,2,4]-triazolo[1,5-*a*]pyridin-6-yl)-phenyl]-2-(4-fluorophenyl)-acetamide | ¹H-NMR (300 MHz, DMSO-d₆): δ [ppm]= 2.79 (s, 3H), 3.64 (s, 2H), 3.92 (s, 3H), 7.07 - 7.18 (m, 2H), 7.23 (d, 1H), 7.29 - 7.41 (m, 2H), 7.61 - 7.76 (m, 5H), 7.93 (dd, 1H), 8.32 (d, 1H), 8.72 (s, 1H), 9.14 (d, 1H), 10.28 (s, 1H). | 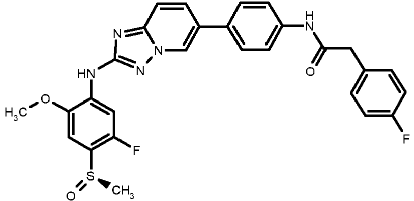 | 547.6 |
| Example 12.15 | *N-*[4-(2-{[4-(*tert*-butylsulfamoyl)-2-methoxy-phenyl]amino}[1,2,4]-triazolo[1, 5-*a*] pyridin-6-yl)phenyl]-2-(4-fluoro-phenyl)acetamide | ¹H-NMR (400 MHz, DMSO-d₆): δ [ppm] = 1.14 (s, 9H), 3.68 (s, 2H), 3.95 (s, 3H), 7.10 - 7.21 (m, 3H), 7.29 - 7.48 (m, 4H), 7.64 (d, 1H), 7.68 - 7.80 (m, 4H), 7.89 - 7.98 (m, 1H), 8.35 (s, 1H), 8.73 (d, 1H), 9.00 (s, 1H), 10.30 (s, 1H). | 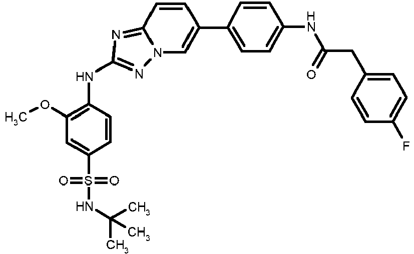 | 602.7 |
| Example 12.16 | *N*-(4-{2-[(4-{[ethyl(1-hydroxy-2-methylpropan-2-yl)amino]methyl}-2-methoxyphenyl)amino]-[1,2,4]triazolo[1,5-*a*]pyridin-6-yl}phenyl)-2-(4-fluorophenyl)acetamide | ¹H-NMR (300MHz, DMSO-d₆): δ [ppm]= 0.82 (t, 3H), 1.01 (br. s., 6H), 2.60 (br. s., 2H), 3.27 (s, 2H), 3.64 (s, 4H), 3.83 (s, 3H), 4.25 (br. s., 1H), 6.92 (br. s., 1H), 7.02 (br. s., 1H), 7.13 (t, 2H), 7.34 (dd, 2H), 7.57 (d, 1H), 7.62 - 7.76 (m, 4H), 7.79 - 7.92 (m, 2H), 8.06 (br. s., 1H), 9.04 (s, 1H), 10.28 (s, 1H). | 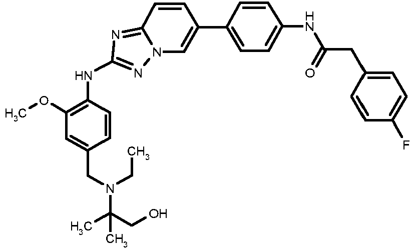 | 596.7 |
| Example 12.17 | 2-(4-fluorophenyl)-*N*-[4-(2-{[4-(2-hydroxypropan-2-yl)-2-methoxyphenyl]amino}-[1,2,4]triazolo[1,5-*a*]pyridin-6-yl)phenyl]acetamide | ¹H-NMR (400MHz, DMSO-d6): δ [ppm]= 1.41 (s, 6H), 3.61 - 3.68 (m, 2H), 3.85 (s, 3H), 4.89 (s, 1H), 6.98 (dd, 1H), 7.09 - 7.16 (m, 3H), 7.30 - 7.37 (m, 2H), 7.57 (d, 1H), 7.64 - 7.74 (m, 4H), 7.83 (s, 1H), 7.86 (dd, 1H), 8.05 (d, 1H), 9.03 (d, 1H), 10.25 (s, 1H). | 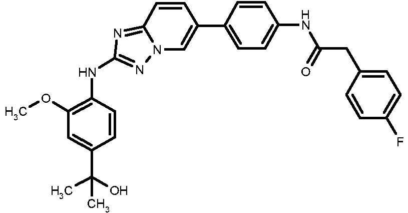 | 525.6 |
| Example 12.18 | *N-*(4-{2-[(2,4-dimethoxyphenyl)amino][1,2,4]-triazolo[1, 5-*a*] pyridin-6-yl}phenyl)-2-(4-fluoro-phenyl)acetamide | ¹H-NMR (400 MHz, DMSO-d₆): δ [ppm] = 3.67 (s, 2H), 3.75 (s, 3H), 3.84 (s, 3H), 6.54 (dd, 1H), 6.64 (d, 1H), 7.11 - 7.20 (m, 2H), 7.31 - 7.41 (m, 2H), 7.51 - 7.60 (m, 1H), 7.66 - 7.77 (m, 4H), 7.80 (s, 1H), 7.87 (dd, 1H), 7.96 (d, 1H), 9.03 (d, 1H), 10.30 (s, 1H). | 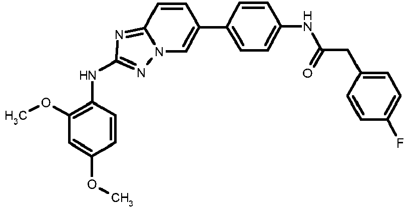 | 497.5 |
| Example 12.19 | 3-ethoxy-*N*-ethyl-4-{[6-(4-{[(4-fluorophenyl)acetyl]-amino}phenyl)[1,2,4]-triazolo[1,5-*a*]pyridin-2-yl]amino}-*N*-methylbenzamide | ¹H-NMR (400 MHz, DMSO-d₆): δ [ppm] = 1.12 (t, 3H), 1.43 (t, 3H), 2.94 (s, 3H), 3.35 - 3.49 (m, 1H), 3.68 (s, 2H), 4.17 (q, 2H), 6.98 - 7.08 (m, 2H), 7.11 - 7.22 (m, 2H), 7.34 - 7.43 (m, 2H), 7.65 (d, 1H), 7.69 - 7.79 (m, 4H), 7.94 (dd, 1H), 8.11 (s, 1H), 8.32 (d, 1H), 9.12 (d, 1H), 10.31 (s, 1H). | 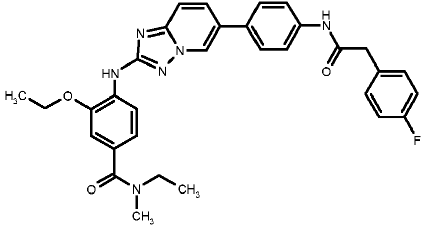 | 566.6 |
| Example 12.20 | *N*-[2-(dimethylamino)ethyl]-3-ethoxy-4-{[6-(4-{[(4-fluorophenyl)acetyl]amino}-phenyl)[1,2,4]triazolo[1,5-*a*]pyridin-2-yl]amino}-benzamide | ¹H-NMR (400 MHz, DMSO-d₆): δ [ppm] = 1.46 (t, 3H), 2.21 (s, 6H), 2.43 (t, 2H), 3.37 (q, 2H), 3.68 (q, 2H), 4.20 (q, 2H), 7.16 (t, 2H), 7.38 (dd, 2H), 7.48 - 7.54 (m, 2H), 7.67 (d, 1H), 7.70 - 7.81 (m, 4H), 7.95 (dd, 1H), 8.17 (s, 1H), 8.26 (t, 1H), 8.34 (d, 1H), 9.11 - 9.16 (m, 1H), 10.31 (s, 1H). | 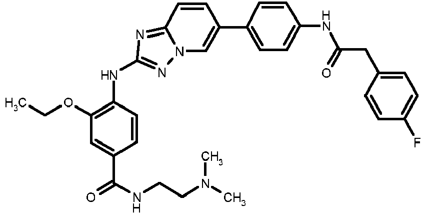 | 595.7 |
| Example 12.21 | 4-{[6-(4-{[(4-fluorophenyl)-acetyl]amino}phenyl)[1,2,4] triazolo[1, 5-*a*] pyridin-2-yl]amino}-*N*-[2-(methylsulfonyl)ethyl]-3-(2,2,2-trifluoroethoxy)benzamide | ¹H-NMR (400 MHz, DMSO-d₆): δ [ppm] = 3.05 (s, 3H), 3.39 (t, 2H), 3.63 - 3.76 (m, 4H), 4.92 (q, 2H), 7.16 (t, 2H), 7.38 (dd, 2H), 7.60 - 7.79 (m, 7H), 7.96 (dd, 1H), 8.30 (s, 1H), 8.39 (d, 1H), 8.62 (t, 1H), 9.14 (s, 1H), 10.30 (s, 1H). | 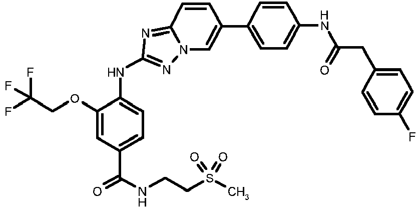 | 684.7 |
| Example 12.22 | 4-{[6-(4-{[(4-fluorophenyl)-acetyl]amino}phenyl)[1,2,4] triazolo[1,5-*a*] pyridin-2-yl]amino}-*N*-(1-hydroxy-2-methylpropan-2-yl)-3-(2,2,2-trifluoroethoxy)benzamide | ¹H-NMR (400 MHz, DMSO-d₆): δ [ppm] = 1.33 (s, 6H), 3.54 (d, 2H), 3.68 (s, 2H), 4.89 - 5.01 (m, 3H), 7.11 - 7.21 (m, 2H), 7.35 - 7.41 (m, 3H), 7.56 - 7.61 (m, 2H), 7.68 (d, 1H), 7.70 - 7.79 (m, 4H), 7.95 (dd, 1H), 8.19 (s, 1H), 8.34 (d, 1H), 9.13 (s, 1H), 10.30 (s, 1H). | 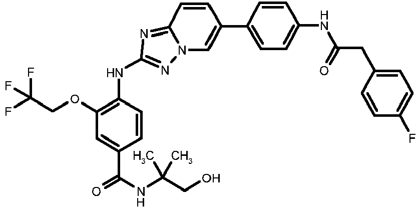 | 650.6 |
| Example 12.23 | *N*-[2-(dimethylamino)ethyl]-4-{[6-(4-{[(4-fluorophenyl)-acetyl]amino}phenyl)[1,2,4] triazolo[1,5-*a*]pyridin-2-yl]amino}-3-(2,2,2-trifluoro-ethoxy)benzamide | ¹H-NMR (400 MHz, DMSO-d₆): δ [ppm] = 2.21 (s, 6H), 2.44 (t, 2H), 3.38 (q, 2H), 3.68 (s, 2H), 4.92 (q, 2H), 7.09 - 7.24 (m, 2H), 7.38 (dd, 2H), 7.58 - 7.84 (m, 7H), 7.95 (dd, 1H), 8.22 - 8.33 (m, 2H), 8.37 (d, 1H), 9.14 (d, 1H), 10.31 (s, 1H). | 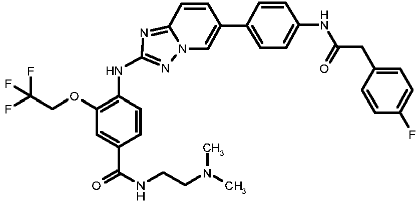 | 649.6 |
| Example 12.24 | 2-(4-fluorophenyl)-*N*-[4-(2-{[4-(methylsulfinyl)-2-(2,2,2-trifluoroethoxy)-phenyl]amino}[1,2,4]-triazolo[1,5-*a*]pyridin-6-yl)phenyl]acetamide | ¹H-NMR (300MHz, DMSO-d₆): δ [ppm]= 2.72 (s, 3H), 3.64 (s, 2H), 4.95 (q, 2H), 7.07 - 7.17 (m, 2H), 7.30 - 7.41 (m, 3H), 7.47 (d, 1H), 7.61 - 7.76 (m, 5H), 7.92 (dd, 1H), 8.29 (s, 1H), 8.43 (d, 1H), 9.10 (s, 1H), 10.28 (s, 1H). | 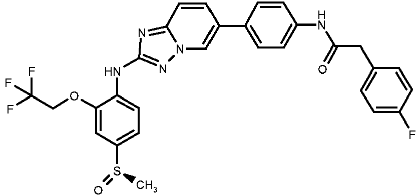 | 597.6 |
| Example 12.25 | 4-{[6-(4-{[(4-fluorophenyl)-acetyl]amino}phenyl)[1,2,4] triazolo[1, 5-*a*] pyridin-2-yl]amino}-3-(trifluoro-methoxy)benzamide | ¹H-NMR (400MHz, DMSO-d₆): δ [ppm]= 3.64 (s, 2H), 7.09 - 7.17 (m, 2H), 7.30 - 7.41 (m, 3H), 7.64 - 7.76 (m, 5H), 7.83 (br. s, 1H), 7.88 (dd, 1H), 7.93 (dd, 1H), 7.98 (br. s., 1H), 8.48 (d, 1H), 9.11 (d, 1H), 9.70 (s, 1H), 10.30 (s, 1H). | 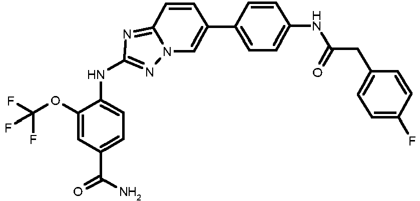 | 564.5 |
| Example 12.26 | *N*-[4-(2-{[2-(difluoro-methoxy)-4-(propan-2-ylsulfonyl)phenyl]amino}-[1,2,4]triazolo[1,5-*a*]pyridin-6-yl)phenyl]-2-(4-fluorophenyl)acetamide | ¹H-NMR (400 MHz, DMSO-d₆): δ [ppm] = 1.18 (d, 6H), 3.35 - 3.47 (m, 1H), 3.68 (s, 2H), 7.16 (t, 2H), 7.27 (t, 1H), 7.38 (dd, 2H), 7.60 (s, 1H), 7.68 - 7.81 (m, 6H), 7.98 (dd, 1H), 8.68 (d, 1H), 9.15 (s, 1H), 9.51 (s, 1H), 10.31 (s, 1H). | 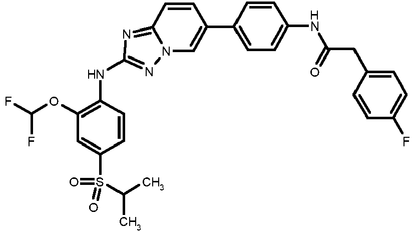 | 609.6 |
| Example 12.27 | *N*-[4-(2-{[2-(difluoro-methoxy)-4-fluorophenyl]-amino}[1,2,4]triazolo[1,5-*a*]pyridin-6-yl)phenyl]-2-(4-fluorophenyl)acetamide | ¹H-NMR (400 MHz, DMSO-d₆): δ [ppm] = 3.67 (s, 2H), 7.01 (s, 1H), 7.11 - 7.18 (m, 4H), 7.20 (1H, t), 7.34 - 7.43 (m, 2H), 7.62 (dd, 1H), 7.68 - 7.78 (m, 4H), 7.90 (dd, 1H), 8.17 - 8.28 (m, 1H), 8.70 (s, 1H), 9.06 (d, 1H), 10.29 (s, 1H). | 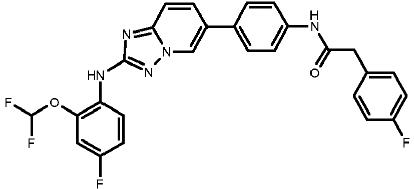 | 521.5 |
| Example 12.28 | 4-{[6-(4-{[(4-fluorophenyl)-acetyl]amino}phenyl)[1,2,4] triazolo[1, 5-*a*] pyridin-2-yl]amino}-3-methylbenzamide | ¹H-NMR (400 MHz, DMSO-d₆): δ [ppm] = 2.36 (s, 3H), 3.68 (s, 2H), 7.12 - 7.23 (m, 3H), 7.34 - 7.43 (m, 2H), 7.64 (d, 1H), 7.68 - 7.84 (m, 7H), 7.92 (dd, 1H), 8.14 (d, 1H), 8.74 (s, 1H), 9.10 (d, 1H), 10.31 (s, 1H). | 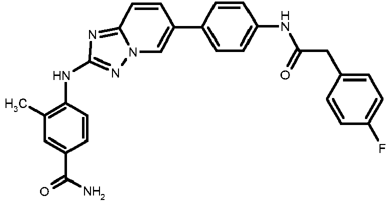 | 494.5 |
| Example 12.29 | 4-{[6-(4-{[(4-fluorophenyl)-acetyl]amino}phenyl)[1,2,4] triazolo[1, 5-*a*] pyridin-2-yl]amino}-3-methyl-*N-*(2,2,2-trifluoroethyl)-benzamide | ¹H-NMR (400 MHz, DMSO-d₆): δ [ppm] = 2.38 (s, 3H), 3.65 - 3.72 (m, 2H), 3.99 - 4.15 (m, 2H), 7.11 - 7.21 (m, 2H), 7.38 (dd, 2H), 7.65 (d, 1H), 7.69 - 7.80 (m, 6H), 7.93 (dd, 1H), 8.21 (d, 1H), 8.82 (s, 1H), 8.88 (t, 1H), 9.11 (s, 1H), 10.32 (s, 1H). | 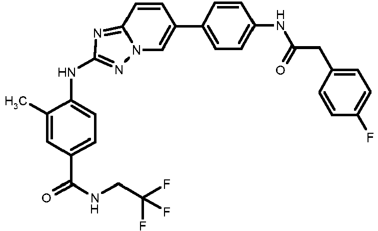 | 576.6 |
| Example 12.30 | *N*-(2-fluoroethyl)-4-{[6-(4-{[(4-fluorophenyl)acetyl]-amino}phenyl)[1,2,4]-triazolo[1,5-*a*]pyridin-2-yl]amino}-3-methylbenzamide | ¹H-NMR (400 MHz, DMSO-d₆): δ [ppm] = 2.37 (s, 3H), 3.52 (q, 1H), 3.59 (q, 1H), 3.68 (s, 2H), 4.48 (t, 1H), 4.60 (t, 1H), 7.16 (t, 2H), 7.38 (dd, 2H), 7.64 (d, 1H), 7.68 - 7.80 (m, 6H), 7.92 (d, 1H), 8.16 (d, 1H), 8.48 (t, 1H), 8.73 (s, 1H), 9.09 (s, 1H), 10.29 (s, 1H). | 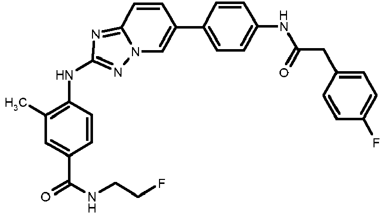 | 540.6 |
| Example 12.31 | 4-{[6-(4-{[(4-fluorophenyl)-acetyl]amino}phenyl)[1,2,4] triazolo[1, 5-*a*] pyridin-2-yl]amino}-*N,N*,3-trimethyl-benzamide | ¹H-NMR (400MHz, DMSO-d₆): δ [ppm] ¹H-NMR (400MHz, DMSO-d₆): d [ppm]= 2.31 (s, 3H), 2.94 (s, 6H), 3.64 (s, 2H), 7.09 - 7.16 (m, 2H), 7.18 - 7.24 (m, 2H), 7.30 - 7.38 (m, 2H), 7.58 (d, 1H), 7.64 - 7.74 (m, 4H), 7.86 (dd, 1H), 8.00 - 8.07 (m, 1H), 8.63 (s, 1H), 8.95 - 9.07 (m, 1H), 10.25 (s, 1H). | 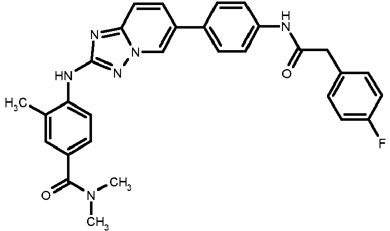 | 522.6 |
| Example 12.32 | 2-(4-fluorophenyl)-*N*-[4-(2-{[2-methyl-4-(methyl-sulfonyl)phenyl]amino}-[1,2,4]triazolo[1,5-*a*]pyridin-6-yl)phenyl]acetamide | ¹H-NMR (400 MHz, DMSO-d₆): δ [ppm] = 2.43 (s, 3H), 3.15 (s, 3H), 3.68 (s, 2H), 7.16 (t, 2H), 7.31 - 7.43 (m, 2H), 7.65 - 7.82 (m, 7H), 7.95 (dd, 1H), 8.37 (d, 1H), 9.03 (s, 1H), 9.12 (d, 1H), 10.32 (s, 1H). | 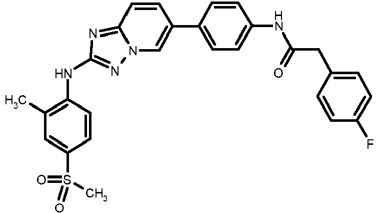 | 529.6 |
| Example 12.33 | 2-(4-fluorophenyl)-*N*-[4-(2-{[2-methyl-4-(methylsulfinyl)phenyl]amino}-[1,2,4]triazolo[1,5-*a*]pyridin-6-yl)phenyl]acetamide | ¹H-NMR (400 MHz, DMSO-d₆): δ [ppm] = 2.40 (s, 3H), 2.72 (s, 3H), 3.67 (s, 2H), 7.12 - 7.22 (m, 2H), 7.33 - 7.42 (m, 2H), 7.47 - 7.54 (m, 2H), 7.63 (d, 1H), 7.68 - 7.79 (m, 4H), 7.91 (dd, 1H), 8.19 - 8.26 (m, 1H), 8.81 (s, 1H), 9.08 (d, 1H), 10.31 (s, 1H). | 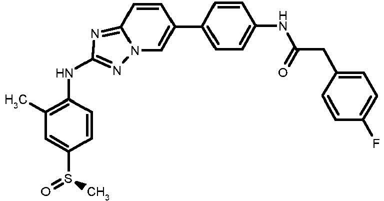 | 513.6 |
| Example 12.34 | 2-(4-fluorophenyl)-*N*-{4-[2-({2-methyl-4-[(methylsulfonyl)amino]phenyl}-amino)[1,2,4]triazolo[1,5-*a*]pyridin-6-yl]phenyl}-acetamide | ¹H-NMR (400 MHz, DMSO-d₆): δ [ppm] = 2.27 (s, 3H), 2.94 (s, 3H), 3.67 (s, 2H), 7.01 - 7.08 (m, 2H), 7.16 (t, 2H), 7.38 (dd, 2H), 7.55 (d, 1H), 7.71 (s, 4H), 7.77 (d, 1H), 7.85 (dd, 1H), 8.49 (s, 1H), 9.00 (d, 1H), 9.40 (br. s., 1H), 10.28 (s, 1H). | 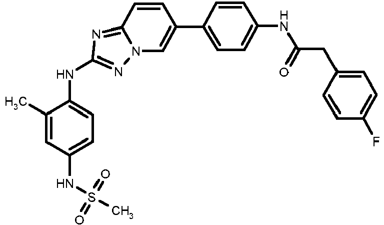 | 544.6 |
| Example 12.35 | 2-(4-fluorophenyl)-*N*-(4-{2-[(4-methoxy-2-methylphenyl)amino][1,2,4]-triazolo[1,5-*a*]pyridin-6-yl}-phenyl)acetamide | ¹H-NMR (400 MHz, DMSO-d₆): δ [ppm] = 2.24 (s, 3H), 3.67 (s, 2H), 3.73 (s, 3H), 6.75 (dd, 1H), 6.79 (d, 1H), 7.09 - 7.21 (m, 2H), 7.32 - 7.43 (m, 2H), 7.47 - 7.53 (m, 1H), 7.55 (d, 1H), 7.70 (s, 4H), 7.82 (dd, 1H), 8.33 (s, 1H), 8.97 (d, 1H), 10.30 (s, 1H). | 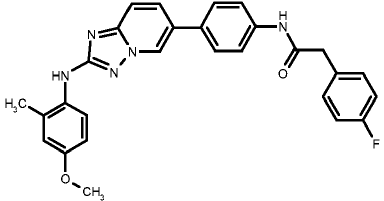 | 481.5 |
| Example 12.36 | *N*-[4-(2-{[4-(dimethylamino)-2-methylphenyl]-amino}[1,2,4]triazolo[1,5-*a*]pyridin-6-yl)phenyl]-2-(4-fluorophenyl)acetamide | ¹H-NMR (400 MHz, DMSO-d₆): δ [ppm] = 2.21 (s, 3H), 2.85 (s, 6H), 3.67 (s, 2H), 6.53 - 6.63 (m, 2H), 7.11 - 7.21 (m, 2H), 7.33 - 7.42 (m, 3H), 7.47 (dd, 1H), 7.69 (s, 4H), 7.80 (dd, 1H), 8.17 (s, 1H), 8.93 (d, 1H), 10.28 (s, 1H). | 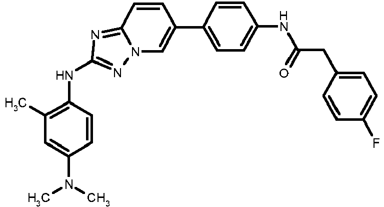 | 494.6 |
| Example 12.37 | *N*-ethyl-5-{[6-(4-{[(4-fluorophenyl)acetyl]amino}-phenyl)[1,2,4]triazolo[1,5-*a*]pyridin-2-yl]amino}-4-methylpyridine-2-carboxamide | ¹H-NMR (300MHz, DMSO-d₆): δ [ppm]= 1.09 (t, 3H), 2.37 (s, 3H), 3.19 - 3.35 (m, 2H), 3.64 (s, 2H), 7.06 - 7.19 (m, 2H), 7.30 - 7.39 (m, 2H), 7.61 (d, 1H), 7.64 - 7.75 (m, 4H), 7.82 (s, 1H), 7.89 (dd, 1H), 8.56 (t, 1H), 9.03 (d, 1H), 9.09 (s, 1 H), 9.19 (s, 1H), 10.27 (s, 1H). | 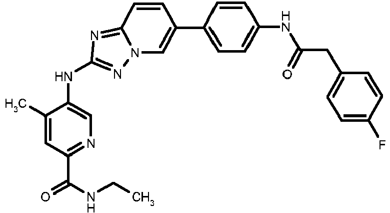 | 523.6 |
| Example 12.38 | 5-{[6-(4-{[(4-fluorophenyl)-acetyl]amino}phenyl)[1,2,4] triazolo[1, 5-*a*] pyridin-2-yl]amino}-4-methyl-*N-*(2,2,2-trifluoroethyl)-pyridine-2-carboxamide | ¹H-NMR (300MHz, DMSO-d₆): δ [ppm]= 2.39 (s, 3H), 3.64 (s, 2H), 3.94 - 4.15 (m, 2H), 7.07 - 7.19 (m, 2H), 7.28 - 7.38 (m, 2H), 7.63 (d, 1H), 7.65 - 7.75 (m, 4H), 7.86 - 7.94 (m, 2H), 9.02 - 9.12 (m, 2H), 9.18 (s, 1H), 9.27 (s, 1H), 10.28 (s, 1H). | 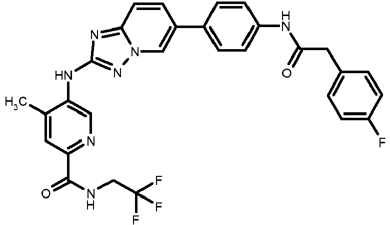 | 577.5 |
| Example 12.39 | *N*-[4-(2-{[2-fluoro-4-(methylsulfonyl)phenyl]-amino}[1,2,4]triazolo[1,5-*a*]pyridin-6-yl)phenyl]-2-(4-fluorophenyl)acetamide | ¹H-NMR (400 MHz, DMSO-d₆): δ [ppm] = 3.22 (s, 3H), 3.68 (s, 2H), 7.11 - 7.22 (m, 2H), 7.33 - 7.43 (m, 2H), 7.69 - 7.81 (m, 7H), 7.97 (dd, 1H), 8.60 (t, 1H), 9.15 (dd, 1H), 9.98 (s, 1H), 10.32 (s, 1H). | 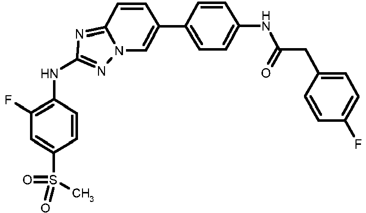 | 533.6 |
| Example 12.40 | 2-(4-fluoro-3-methylphenyl)-*N*-[4-(2-{[2-methoxy-4-(methylsulfonyl)phenyl]-amino}[1,2,4]triazolo[1,5-*a*]pyridin-6-yl)phenyl]-acetamide | ¹H-NMR (400MHz, DMSO-d₆): δ [ppm]= 2.22 (d, 3H), 3.18 (s, 3H), 3.62 (s, 2H), 3.99 (s, 3H), 7.04 - 7.12 (m, 1H), 7.14 - 7.20 (m, 1H), 7.23 (d, 1H), 7.45 (d, 1H), 7.54 (dd, 1H), 7.65 - 7.79 (m, 5H), 7.95 (dd, 1H), 8.51 (d, 1H), 8.62 (s, 1H), 9.13 (d, 1H), 10.26 (s, 1H). | 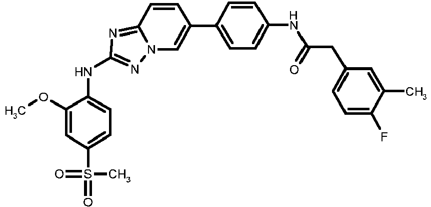 | 559.6 |
| Example 12.41 | 2-(4-chlorophenyl)-*N*-[4-(2-{[2-methoxy-4-(methyl-sulfonyl)phenyl]amino}-[1,2,4]triazolo[1,5-*a*]pyridin-6-yl)phenyl]acetamide | ¹H-NMR (300MHz, DMSO-d₆): δ [ppm]= 3.16 (s, 3H), 3.65 (s, 2H), 3.95 (s, 3H), 7.29 - 7.40 (m, 4H), 7.42 (d, 1H), 7.53 (s, 1H), 7.61 - 7.80 (m, 5H), 7.93 (dd, 1H), 8.48 (d, 1H), 8.62 (s, 1H), 9.11 (d, 1H), 10.29 (s, 1H). | 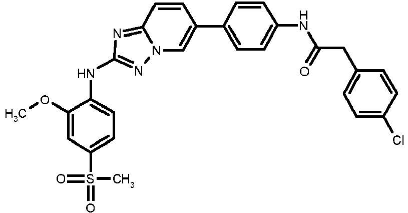 | 562.0 |
| Example 12.42 | *N*-[4-(2-{[2-methoxy-4-(methylsulfonyl)phenyl]-amino}[1,2,4]triazolo[1,5-*a*]pyridin-6-yl)phenyl]-2-phenylacetamide | ¹H-NMR (300MHz, DMSO-d₆): δ [ppm]= 3.16 (s, 3H), 3.64 (s, 2H), 3.95 (s, 3H), 7.17 - 7.35 (m, 5H), 7.42 (d, 1H), 7.51 (dd, 1H), 7.62 - 7.78 (m, 5H), 7.93 (dd, 1H), 8.48 (d, 1H), 8.62 (s, 1H), 9.11 (s, 1H), 10.28 (s, 1H). | 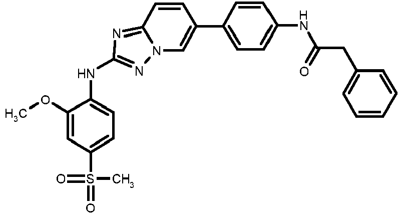 | 527.6 |
| Example 12.43 | 4-[(6-{4-[(4-fluorobenzyl)-carbamoyl]phenyl}[1,2,4]triazolo[1,5-*a*]pyridin-2-yl)-amino]-3-methoxy-benzamide | ¹H-NMR (400 MHz, DMSO-d₆): δ [ppm] = 3.94 (s, 3H), 4.49 (d, 2H), 7.09 - 7.20 (m, 6H), 7.23 (br. s., 1H), 7.38 (dd, 2H), 7.52 - 7.61 (m, 2H), 7.72 (d, 1H), 7.88 (br. s., 1H), 7.92 - 7.98 (m, 2H), 7.99 - 8.08 (m, 3H), 8.32 - 8.39 (m, 2H), 9.15 (t, 1H), 9.32 (d, 1H). | 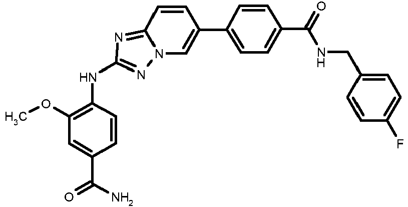 | 510.5 |
| Example 12.44 | 4-[(6-{4-[(4-fluorobenzyl)-carbamoyl]phenyl}[1,2,4]triazolo[1,5-*a*]pyridin-2-yl)-amino]-3-methoxy-*N*-(2-methoxyethyl)benzamide | ¹H-NMR (400 MHz, DMSO-d₆): δ [ppm] = 3.28 (s, 3H), 3.39 - 3.51 (m, 4H), 3.95 (s, 3H), 4.49 (d, 2H), 7.17 (t, 2H), 7.38 (dd, 2H), 7.50 - 7.59 (m, 2H), 7.72 (d, 1H), 7.90 - 7.98 (m, 2H), 7.99 - 8.10 (m, 3H), 8.31 - 8.40 (m, 2H), 8.43 (t, 1H), 9.15 (t, 1H), 9.31 (s, 1H). | 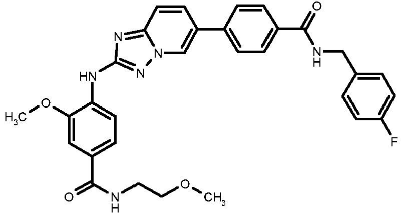 | 568.6 |
| Example 12.45 | 4-[(6-{4-[(4-fluorobenzyl)-carbamoyl]phenyl}[1,2,4]triazolo[1,5-*a*]pyridin-2-yl)-amino]-*N*-(2-fluoroethyl)-3-methoxybenzamide | ¹H-NMR (400 MHz, DMSO-d₆): δ [ppm] = 3.49 - 3.68 (m, 2H), 3.95 (s, 3H), 4.49 (d, 3H), 4.61 (t, 1H), 7.16 (t, 2H), 7.32 - 7.45 (m, 2H), 7.50 - 7.63 (m, 2H), 7.72 (d, 1H), 7.89 - 7.99 (m, 2H), 7.98 - 8.11 (m, 3H), 8.32 - 8.44 (m, 2H), 8.54 - 8.68 (m, 1H), 9.16 (t, 1H), 9.31 (s, 1H). | 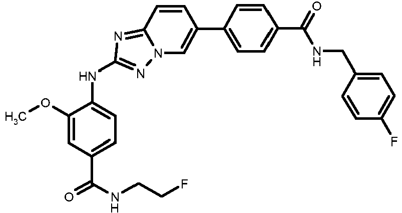 | 556.6 |
| Example 12.46 | 4-[(6-{4-[(4-fluorobenzyl)-carbamoyl]phenyl}[1,2,4]triazolo[1,5-*a*]pyridin-2-yl)-amino]-3-methoxy-*N,N-*dimethylbenzamide | ¹H-NMR (400 MHz, DMSO-d₆): δ [ppm] = 2.99 (s, 6H), 3.92 (s, 3H), 4.49 (d, 2H), 7.04 - 7.10 (m, 2H), 7.12 - 7.20 (m, 2H), 7.38 (dd, 2H), 7.66 - 7.74 (m, 1H), 7.89 - 7.97 (m, 2H), 7.97 - 8.07 (m, 3H), 8.27 (s, 1H), 8.32 (d, 1H), 9.13 (t, 1H), 9.25 - 9.31 (m, 1H). | 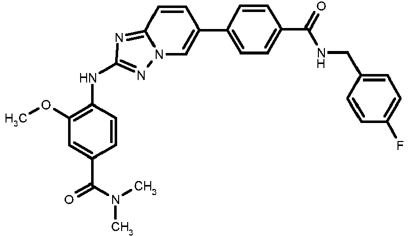 | 538.6 |
| Example 12.47 | *N*-(2,2-difluoroethyl)-4-[(6-{4-[(4-fluorobenzyl)-carbamoyl]phenyl}[1,2,4]tri-azolo[1,5-*a*]pyridin-2-yl)amino]-3-methoxybenzamide | ¹H-NMR (400 MHz, DMSO-d₆): δ [ppm] = 3.60 - 3.76 (m, 2H), 3.96 (s, 3H), 4.49 (d, 2H), 5.96 - 6.29 (m, 1H), 7.13 - 7.21 (m, 2H), 7.38 (dd, 2H), 7.54 - 7.62 (m, 2H), 7.73 (d, 1H), 7.92 - 7.98 (m, 2H), 7.99 - 8.08 (m, 3H), 8.38 (d, 1H), 8.42 (s, 1H), 8.74 (t, 1H), 9.15 (t, 1H), 9.31 (d, 1H). | 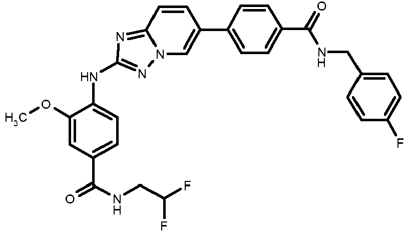 | 574.6 |
| Example 12.48 | *N*-[2-(dimethylamino)ethyl]-4-[(6-{4-[(4-fluorobenzyl)-carbamoyl]phenyl}[1,2,4]tri-azolo[1,5-*a*]pyridin-2-yl)amino]-3-methoxybenzamide | ¹H-NMR (400 MHz, DMSO-d₆): δ [ppm] = 2.29 (s, 6H), 2.52 - 2.58 (m, 5H), 3.41 (q, 3H), 3.95 (s, 3H), 4.49 (d, 2H), 7.12 - 7.21 (m, 2H), 7.38 (dd, 2H), 7.50 - 7.58 (m, 2H), 7.72 (d, 1H), 7.91 - 7.99 (m, 2H), 7.99 - 8.08 (m, 3H), 8.32 - 8.40 (m, 3H), 9.16 (t, 1H), 9.31 (d, 1H). | 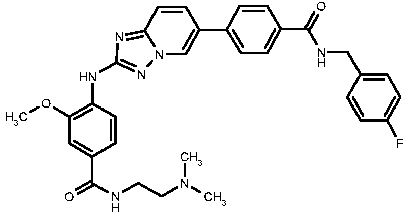 | 581.6 |
| Example 12.49 | N-[2-(dimethylamino)ethyl]-4-[(6-{4-[(4-fluorobenzyl)-carbamoyl]phenyl}[1,2,4]tri-azolo[1,5-*a*]pyridin-2-yl)-amino]-3-methoxy-*N-*methylbenzamide | ¹H-NMR (400 MHz, DMSO-d₆): δ [ppm] = 1.87 - 2.31 (m, 6H), 2.39 - 2.48 (m, 2H), 2.98 (s, 3H), 3.91 (s, 3H), 4.49 (d, 2H), 7.01 - 7.08 (m, 2H), 7.12 - 7.21 (m, 2H), 7.38 (dd, 2H), 7.70 (d, 1H), 7.90 - 7.97 (m, 2H), 7.98 - 8.06 (m, 3H), 8.26 (s, 1H), 8.32 (d, 1H), 9.13 (t, 1H), 9.28 (d, 1H). | 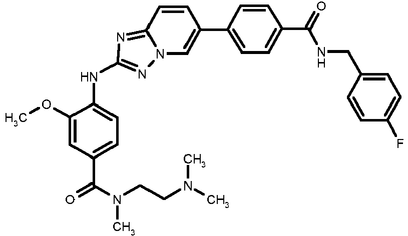 | 595.7 |
| Example 12.50 | 4-[(6-{4-[(4-fluorobenzyl)-carbamoyl]phenyl}[1,2,4]tri-azolo[1,5-*a*]pyridin-2-yl)-amino]-*N*-(3-fluoropropyl)-3-methoxybenzamide | ¹H-NMR (400 MHz, DMSO-d₆): δ [ppm] = 1.84 - 2.01 (m, 2H), 3.34 - 3.42 (m, 2H), 3.95 (s, 3H), 4.43 - 4.53 (m, 3H), 4.59 (t, 1H), 7.16 (t, 2H), 7.38 (dd, 2H), 7.51 - 7.57 (m, 2H), 7.72 (d, 1H), 7.91 - 7.97 (m, 2H), 7.99 - 8.07 (m, 3H), 8.32 - 8.37 (m, 2H), 8.42 (t, 1H), 9.14 (t, 1H), 9.30 (d, 1H). | 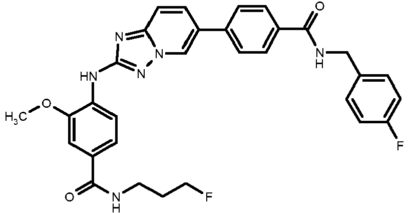 | 570.6 |
| Example 12.51 | *N*-(4-fluorobenzyl)-4-(2-{[2-methoxy-4-(methylsulfinyl)-phenyl]amino}[1,2,4]-triazolo[1,5-*a*]pyridin-6-yl)-benzamide | ¹H-NMR (300MHz, DMSO-d₆): δ [ppm]= 2.72 (s, 3H), 3.93 (s, 3H), 4.45 (d, 2H), 7.05 - 7.19 (m, 2H), 7.22 - 7.40 (m, 4H), 7.68 (d, 1H), 7.85 - 8.05 (m, 5H), 8.35 - 8.47 (m, 2H), 9.12 (t, 1H), 9.26 (s, 1H). | 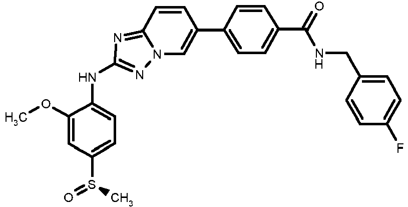 | 529.6 |
| Example 12.52 | 4-(2-{[4-(*tert*-butylsulfamoyl)-2-methoxy-phenyl]amino}[1,2,4]-triazolo[1,5-*a*]pyridin-6-yl)-*N*-(4-fluorobenzyl)-benzamide | ¹H-NMR (400 MHz, DMSO-d₆): δ [ppm] = 1.14 (s, 9H), 3.95 (s, 3H), 4.49 (d, 2H), 7.12 - 7.21 (m, 3H), 7.34 - 7.41 (m, 3H), 7.44 (dd, 1H), 7.69 (dd, 1H), 7.91 - 7.97 (m, 2H), 7.99 - 8.06 (m, 3H), 8.44 (s, 1H), 8.72 (d, 1H), 9.12 - 9.20 (m, 2H). | 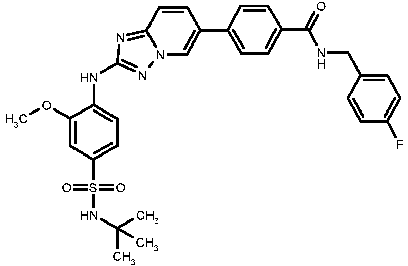 | 602.7 |
| Example 12.53 | 4-{2-[(4-{[ethyl(1-hydroxy-2-methylpropan-2-yl)amino]-methyl}-2-methoxyphenyl)-amino][1,2,4]triazolo[1,5-*a*]pyridin-6-yl}-*N*-(4-fluoro-benzyl)benzamide | ¹H-NMR (300MHz, DMSO-d₆): δ [ppm]= 0.82 (t, 3H), 1.01 (s, 6H), 2.60 (q, 2H), 3.28 (br. s., 2H), 3.65 (s, 2H), 3.82 (s, 3H), 4.27 (t, 1H), 4.45 (d, 2H), 6.91 (d, 1H), 7.03 (s, 1H), 7.06 - 7.19 (m, 2H), 7.34 (dd, 2H), 7.61 (d, 1H), 7.84 - 8.01 (m, 6H), 8.04 (d, 1H), 9.11 (t, 1H), 9.20 (s, 1H). | 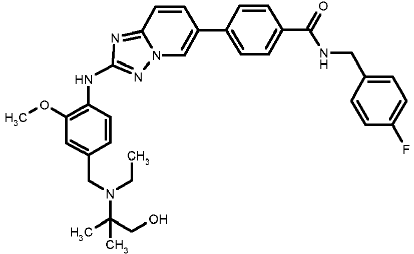 | 596.7 |
| Example 12.54 | *N*-(4-fluorobenzyl)-4-(2-{[4-(2-hydroxypropan-2-yl)-2-methoxyphenyl]amino}-[1,2,4]triazolo[1,5-*a*]pyridin-6-yl)benzamide | ¹H-NMR (300MHz, DMSO-d₆): δ [ppm]= 1.41 (s, 6H), 3.85 (s, 3H), 4.45 (d, 2H), 4.90 (s, 1H), 6.99 (dd, 1H), 7.05 - 7.18 (m, 3H), 7.34 (dd, 2H), 7.61 (d, 1H), 7.82 - 8.00 (m, 6H), 8.05 (d, 1H), 9.09 (t, 1H), 9.20 (s, 1H). | 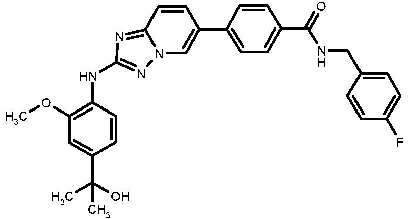 | 525.6 |
| Example 12.55 | *N*-[2-(dimethylamino)ethyl]-3-ethoxy-4-[(6-{4-[(4-fluorobenzyl)carbamoyl] phenyl}-[1,2,4]triazolo[1,5-*a*]pyridin-2-yl)amino]benzamide | ¹H-NMR (400 MHz, DMSO-d₆): δ [ppm] = 1.46 (t, 3H), 2.19 (s, 6H), 2.40 (t, 2H), 4.20 (q, 2H), 4.49 (d, 2H), 7.12 - 7.21 (m, 2H), 7.38 (dd, 2H), 7.48 - 7.54 (m, 2H), 7.72 (d, 1H), 7.90 - 7.98 (m, 2H), 7.98 - 8.08 (m, 3H), 8.25 (s, 1H), 8.26 - 8.32 (m, 1H), 8.35 (d, 1H), 9.16 (t, 1H), 9.31 (d, 1H). | 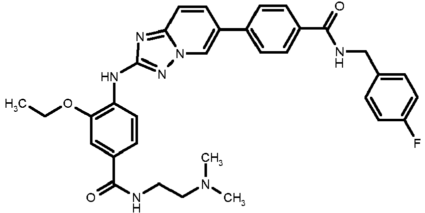 | 595.7 |
| Example 12.56 | 3-ethoxy-*N*-ethyl-4-[(6-{4-[(4-fluorobenzyl)carbamoyl]-phenyl}[1,2,4]triazolo[1,5-*a*]pyridin-2-yl)amino]-*N-*methylbenzamide | ¹H-NMR (400 MHz, DMSO-d₆): δ [ppm] = 1.12 (t, 3H), 1.43 (t, 3H), 2.94 (s, 3H), 3.36 - 3.49 (m, 2H), 4.17 (q, 2H), 4.49 (d, 2H), 7.03 (br. s., 2H), 7.16 (t, 2H), 7.38 (dd, 2H), 7.70 (d, 1H), 7.90 - 7.98 (m, 2H), 7.99 - 8.07 (m, 3H), 8.17 (s, 1H), 8.30 - 8.36 (m, 1H), 9.15 (t, 1H), 9.29 (s, 1H). | 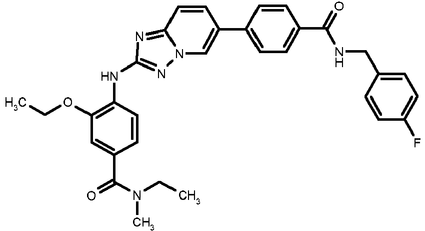 | 566.6 |
| Example 12.57 | 3-ethoxy-4-[(6-{4-[(4-fluoro-benzyl)carbamoyl]phenyl}-[1,2,4]triazolo[1,5-*a*]pyridin-2-yl)amino]-*N*-(2-fluoroethyl)benzamide | ¹H-NMR (400 MHz, DMSO-d₆): δ [ppm] = 1.47 (t, 3H), 3.54 (d, 1H), 3.61 (d, 1H), 4.21 (q, 2H), 4.44 - 4.53 (m, 3H), 4.61 (t, 1H), 7.11 - 7.22 (m, 2H), 7.38 (dd, 2H), 7.51 - 7.61 (m, 2H), 7.72 (d, 1H), 7.91 - 7.98 (m, 2H), 7.99 - 8.08 (m, 3H), 8.26 (s, 1H), 8.37 (d, 1H), 8.58 (t, 1H), 9.15 (t, 1H), 9.31 (d, 1H). | 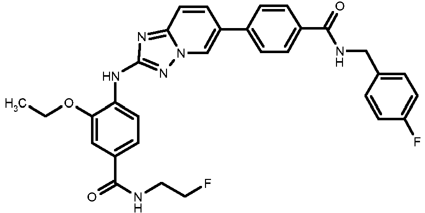 | 570.6 |
| Example 12.58 | *N*-[2-(dimethylamino)ethyl]-4-[(6-{4-[(4-fluorobenzyl)-carbamoyl]phenyl}[1,2,4]tri-azolo[1,5-*a*]pyridin-2-yl)-amino]-3-(2,2,2-trifluoroethoxy)benzamide | ¹H-NMR (400 MHz, DMSO-d₆): δ [ppm] = 2.21 (s, 6H), 2.43 (t, 2H), 3.34 - 3.42 (m, 3H), 4.49 (d, 2H), 4.93 (q, 2H), 7.16 (t, 2H), 7.38 (dd, 2H), 7.59 - 7.68 (m, 2H), 7.70 - 7.75 (m, 1H), 7.89 - 7.99 (m, 2H), 7.99 - 8.10 (m, 3H), 8.26 - 8.34 (m, 2H), 8.37 (d, 1H), 9.14 (t, 1H), 9.31 (d, 1H). | 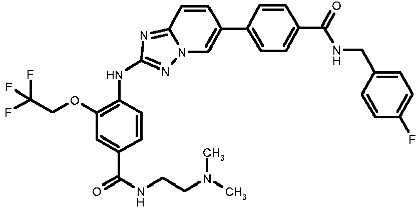 | 649.6 |
| Example 12.59 | 4-[(6-{4-[(4-fluorobenzyl)-carbamoyl]phenyl}[1,2,4]tri-azolo[1,5-*a*]pyridin-2-yl)-amino]-*N*-(2-fluoroethyl)-3-(2,2,2-trifluoroethoxy)-benzamide | ¹H-NMR (400 MHz, DMSO-d₆): δ [ppm] = 3.56 (d, 1H), 3.62 (d, 1H), 4.45 - 4.53 (m, 3H), 4.62 (t, 1H), 4.94 (q, 2H), 7.11 - 7.21 (m, 2H), 7.38 (dd, 2H), 7.64 - 7.77 (m, 3H), 7.91 - 7.98 (m, 2H), 7.99 - 8.09 (m, 3H), 8.35 (s, 1H), 8.39 (d, 1H), 8.60 (t, 1H), 9.16 (t, 1H), 9.32 (d, 1H). | 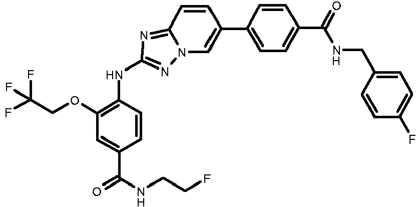 | 624.6 |
| Example 12.60 | 4-[(6-{4-[(4-fluorobenzyl)-carbamoyl]phenyl}[1,2,4]tri-azolo[1,5-K]pyridin-2-yl)-amino]-3-(trifluoromethoxy)benzamide | ¹H-NMR (300MHz, DMSO-d₆): δ [ppm]= 4.45 (d, 2H), 7.08 - 7.18 (m, 2H), 7.27 - 7.42 (m, 3H), 7.72 (d, 1H), 7.80 - 8.07 (m, 8H), 8.49 (d, 1H), 9.12 (t, 1H), 9.28 (s, 1H), 9.75 (s, 1H). | 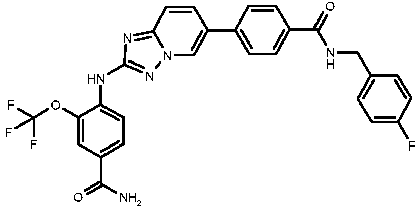 | 564.5 |
| Example 12.61 | 4-(2-{[2-(difluoromethoxy)-4-(ethylsulfonyl)phenyl]-amino}[1,2,4]triazolo[1,5-*a*]pyridin-6-yl)-N-(4-fluorobenzyl)benzamide | ¹H-NMR (400 MHz, DMSO-d₆): δ [ppm] = 1.13 (t, 3H), 3.28 (q, 2H), 4.49 (d, 2H), 7.16 (t, 2H), 7.28 (t, 1H), 7.38 (dd, 2H), 7.66 (s, 1H), 7.78 (d, 2H), 7.92 - 7.98 (m, 2H), 8.00 - 8.05 (m, 2H), 8.08 (dd, 1H), 8.67 (d, 1H), 9.14 (t, 1H), 9.31 (s, 1H), 9.54 (s, 1H). | 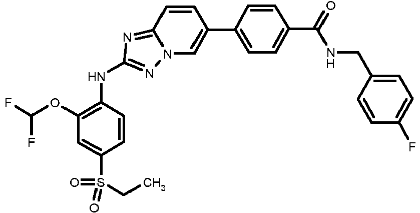 | 595.6 |
| Example 12.62 | 4-(2-{[2-(difluoromethoxy)-4-(propan-2-ylsulfonyl)-phenyl]amino}[1,2,4]-triazolo[1,5-*a*]pyridin-6-yl)-*N*-(4-fluorobenzyl)-benzamide | ¹H-NMR (400 MHz, DMSO-d₆): δ [ppm] = 1.18 (d, 6H), 3.41 (quin, 1H), 4.49 (d, 2H), 7.12 - 7.21 (m, 2H), 7.28 (t, 1H), 7.35 - 7.42 (m, 2H), 7.61 (d, 1H), 7.72 - 7.81 (m, 2H), 7.92 - 7.98 (m, 2H), 8.00 - 8.05 (m, 2H), 8.08 (dd, 1H), 8.68 (d, 1H), 9.14 (t, 1H), 9.29 - 9.34 (m, 1H), 9.57 (s, 1H). | 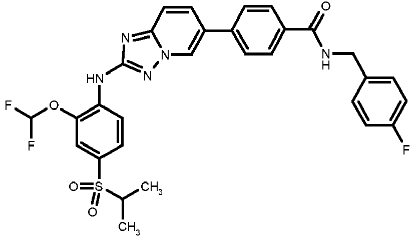 | 609.6 |
| Example 12.63 | 4-(2-{[2-(difluoromethoxy)-4-fluorophenyl]amino}-[1,2,4]triazolo[1,5-a]pyridin-6-yl)-*N*-(4-fluorobenzyl)benzamide | ¹H-NMR (400 MHz, DMSO-d₆): δ [ppm] = 4.49 (d, 2H), 7.13 - 7.20 (m, 4H), 7.20 (t, 1H), 7.33 - 7.41 (m, 2H), 7.61 - 7.69 (m, 1H), 7.88 - 7.96 (m, 2H), 7.98 - 8.05 (m, 3H), 8.18 - 8.25 (m, 1H), 8.77 (s, 1 H), 9.13 (t, 1H), 9.23 (d, 1H). | 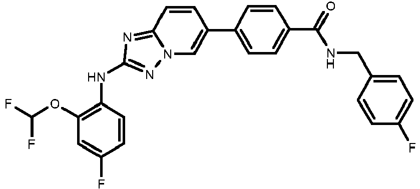 | 521.5 |
| Example 12.64 | *N*-ethyl-4-[(6-{4-[(4-fluorobenzyl)carbamoyl}-phenyl}-[1,2,4]triazolo[1,5-*a*]pyridin-2-yl)amino]-3-methylbenzamide | ¹H-NMR (400 MHz, DMSO-d₆): δ [ppm] = 1.12 (t, 3H), 2.37 (s, 3H), 3.24 - 3.30 (m, 2H), 4.49 (d, 2H), 7.16 (t, 2H), 7.38 (dd, 2H), 7.64 - 7.73 (m, 3H), 7.89 - 7.97 (m, 2H), 7.97 - 8.05 (m, 3H), 8.13 (d, 1H), 8.26 (t, 1H), 8.76 (s, 1H), 9.13 (t, 1H), 9.25 (s, 1H). | 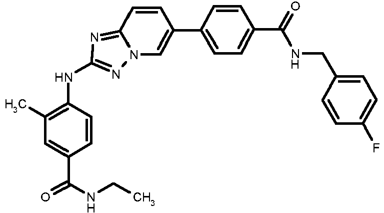 | 522.6 |
| Example 12.65 | 4-[(6-{4-[(4-fluorobenzyl)-carbamoyl]phenyl}[1,2,4]tri-azolo[1,5-*a*]pyridin-2-yl)-amino]-3-methylbenzamide | ¹H-NMR (400 MHz, DMSO-d₆): δ [ppm] = 2.37 (s, 3H), 4.49 (d, 2H), 7.10 - 7.22 (m, 3H), 7.38 (dd, 2H), 7.65 - 7.76 (m, 3H), 7.80 (br. s., 1H), 7.91 - 7.98 (m, 2H), 8.02 (d, 3H), 8.14 (d, 1H), 8.80 (s, 1H), 9.16 (t, 1H), 9.27 (d, 1H). | 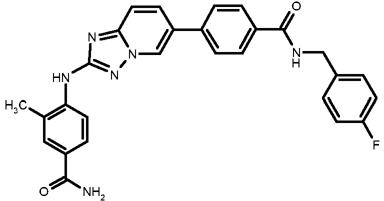 | 494.5 |
| Example 12.66 | 4-[(6-{4-[(4-fluorobenzyl)-carbamoyl]phenyl}[1,2,4]tri-azolo[1,5-*a*]pyridin-2-yl)-amino]-3-methyl-*N*-(2,2,2-trifluoroethyl)benzamide | ¹H-NMR (400 MHz, DMSO-d₆): δ [ppm] = 2.39 (s, 3H), 4.01 - 4.16 (m, 2H), 4.49 (d, 2H), 7.17 (t, 2H), 7.38 (dd, 2H), 7.70 (d, 1H), 7.73 - 7.79 (m, 2H), 7.90 - 7.98 (m, 2H), 7.99 - 8.08 (m, 3H), 8.21 (d, 1H), 8.84 - 8.92 (m, 2H), 9.16 (t, 1H), 9.27 (s, 1H). | 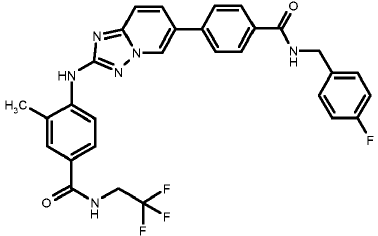 | 576.6 |
| Example 12.67 | 4-[(6-{4-[(4-fluorobenzyl)-carbamoyl]phenyl}[1,2,4]tri-azolo[1,5-*a*]pyridin-2-yl)-amino]-*N*-(2-fluoroethyl)-3-methylbenzamide | ¹H-NMR (400 MHz, DMSO-d₆): δ [ppm] = 2.38 (s, 3H), 3.53 (d, 1H), 3.59 (d, 1 H), 4.49 (d, 3H), 4.60 (t, 1H), 7.12 - 7.21 (m, 2H), 7.38 (t, 2H), 7.65 - 7.77 (m, 3H), 7.90 - 7.97 (m, 2H), 8.02 (d, 3H), 8.16 (d, 1H), 8.49 (br. s., 1H), 8.79 (s, 1H), 9.13 (t, 1H), 9.25 (s, 1H). | 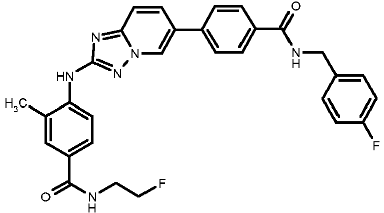 | 540.6 |
| Example 12.68 | *N*-(4-fluorobenzyl)-4-(2-{[2-methyl-4-(methylsulfonyl)-phenyl]amino}[1,2,4]triazolo[1,5-*a*]pyridin-6-yl)-benzamide | ¹H-NMR (400 MHz, DMSO-d₆): δ [ppm] = 2.43 (s, 3H), 3.16 (s, 3H), 4.49 (d, 2H), 7.12 - 7.21 (m, 2H), 7.38 (dd, 2H), 7.68 - 7.77 (m, 3H), 7.91 - 7.98 (m, 2H), 7.99 - 8.08 (m, 3H), 8.35 - 8.40 (m, 1H), 9.09 (s, 1H), 9.16 (s, 1H), 9.28 (d, 1H). | 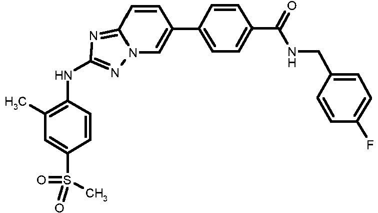 | 529.6 |
| Example 12.69 | *N*-(4-fluorobenzyl)-4-(2-{[2-methyl-4-(methylsulfinyl)-phenyl]amino}[1,2,4]tri-azolo[1,5-*a*]pyridin-6-yl)-benzamide | ¹H-NMR (400 MHz, DMSO-d₆): δ [ppm] = 2.40 (s, 3H), 2.72 (s, 3H), 4.49 (d, 2H), 7.12 - 7.21 (m, 2H), 7.38 (dd, 2H), 7.47 - 7.53 (m, 2H), 7.68 (d, 1H), 7.90 - 7.97 (m, 2H), 7.99 - 8.05 (m, 3H), 8.22 (d, 1H), 8.87 (s, 1H), 9.14 (t, 1H), 9.25 (d, 1H). | 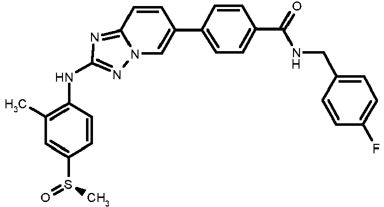 | 513.6 |
| Example 12.70 | *N*-(4-fluorobenzyl)-4-[2-({2-methyl-4-[(methylsulfonyl)-amino]phenyl}amino)[1,2,4]t riazolo[1,5-*a*]pyridin-6-yl]-benzamide | ¹H-NMR (400 MHz, DMSO-d₆): δ [ppm] = 2.28 (s, 3H), 2.94 (s, 3H), 4.48 (d, 2H), 7.09 (m, 2H), 7.16 (t, 2H), 7.37 (dd, 2H), 7.59 (d, 1H), 7.76 (d, 1H), 7.90 (d, 2H), 7.95 (dd, 1H), 7.98 - 8.04 (m, 2H), 8.55 (s, 1H), 9.12 (t, 1H), 9.16 (d, 1H), 9.42 (br. s., 1H). | 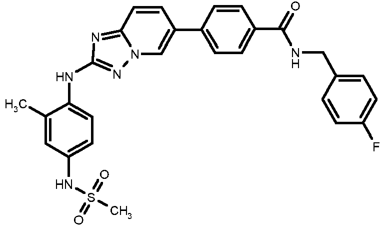 | 544.6 |
| Example 12.71 | *N*-(4-fluorobenzyl)-4-{2-[(4-methoxy-2-methylphenyl)-amino][1,2,4]triazolo[1,5-*a*]pyridin-6-yl}benzamide | ¹H-NMR (400 MHz, DMSO-d₆): δ [ppm] = 2.25 (s, 3H), 3.73 (s, 3H), 4.48 (d, 2H), 6.76 (dd, 1H), 6.80 (d, 1H), 7.12 - 7.20 (m, 2H), 7.33 - 7.41 (m, 2H), 7.55 (dd, 2H), 7.85 - 7.94 (m, 3H), 7.99 (d, 2H), 8.37 (s, 1H), 9.08 - 9.15 (m, 2H). | 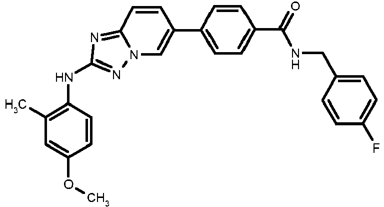 | 481.5 |
| Example 12.72 | 4-[(6-{4-[(4-chlorobenzyl)-carbamoyl]phenyl}[1,2,4]tri-azolo[1,5-*a*]pyridin-2-yl)-amino]-3-methoxy-*N*-(2,2,2-trifluoroethyl)benzamide | ¹H-NMR (400 MHz, DMSO-d₆): δ [ppm] = 3.97 (s, 3H), 4.05 - 4.17 (m, 2H), 4.50 (d, 2H), 7.32 - 7.43 (m, 4H), 7.55 - 7.65 (m, 2H), 7.73 (d, 1H), 7.91 - 7.98 (m, 2H), 7.99 - 8.08 (m, 3H), 8.39 (d, 1H), 8.43 (s, 1H), 8.93 (t, 1H), 9.16 (t, 1H), 9.31 (d, 1H). | 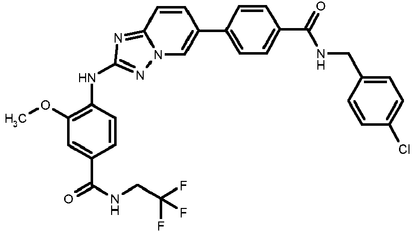 | 609.0 |
| Example 12.73 | *N*-(4-chlorobenzyl)-4-(2-{[2-methoxy-4-(methylsulfonyl)-phenyl]amino}[1,2,4]triazolo[1,5-*a*]pyridin-6-yl)-benzamide | ¹H-NMR (400 MHz, DMSO-d₆): δ [ppm] = 3.20 (s, 3H), 4.00 (s, 3H), 4.50 (d, 2H), 7.33 - 7.43 (m, 4H), 7.47 (d, 1H), 7.56 (dd, 1H), 7.76 (d, 1H), 7.92 - 7.97 (m, 2H), 8.00 - 8.08 (m, 3H), 8.52 (d, 1H), 8.69 (s, 1H), 9.16 (t, 1H), 9.31 (s, 1H). | 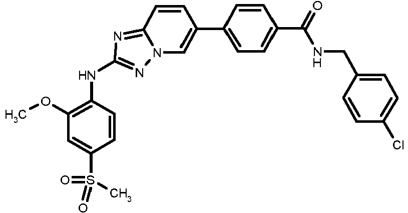 | 562.0 |
| Example 12.74 | *N*-(4-chlorobenzyl)-4-(2-{[2-methyl-4-(methylsulfonyl)-phenyl]amino}[1,2,4]tri-azolo[1,5-*a*]pyridin-6-yl)-benzamide | ¹H-NMR (400 MHz, DMSO-d₆): δ [ppm] = 2.43 (s, 3H), 3.16 (s, 3H), 4.49 (d, 2H), 7.32 - 7.44 (m, 4H), 7.69 - 7.77 (m, 3H), 7.92 - 7.98 (m, 2H), 8.00 - 8.08 (m, 3H), 8.33 - 8.40 (m, 1H), 9.09 (s, 1H), 9.18 (t, 1H), 9.28 (d, 1H). | 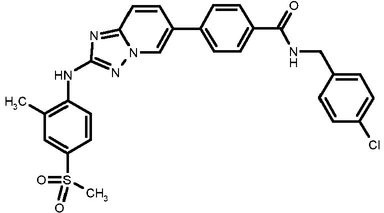 | 546.0 |
| Example 12.75 | *N*-(4-chlorobenzyl)-4-(2-{[2-methyl-4-(methylsulfinyl)-phenyl]amino}[1,2,4]tri-azolo[1,5-*a*]pyridin-6-yl)-benzamide | ¹H-NMR (400 MHz, DMSO-d₆): δ [ppm] = 2.40 (s, 3H), 2.72 (s, 3H), 4.49 (d, 2H), 7.33 - 7.44 (m, 4H), 7.47 - 7.53 (m, 2H), 7.68 (d, 1H), 7.90 - 7.97 (m, 2H), 7.99 - 8.05 (m, 3H), 8.20 - 8.25 (m, 1H), 8.88 (s, 1H), 9.17 (s, 1H), 9.25 (d, 1H). | 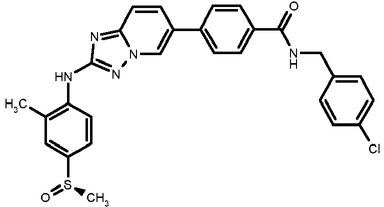 | 530.0 |
| Example 12.76 | 4-(2-{[2-methoxy-4-(methylsulfonyl)phenyl]amino}-[1,2,4]triazolo[1,5-*a*]pyridin-6-yl)-*N*-(4-methylbenzyl)benzamide | ¹H-NMR (400 MHz, DMSO-d₆): δ [ppm] = 2.28 (s, 3H), 3.20 (s, 3H), 4.00 (s, 3H), 4.46 (d, 2H), 7.10 - 7.19 (m, 2H), 7.19 - 7.26 (m, 2H), 7.47 (d, 1H), 7.56 (dd, 1H), 7.76 (d, 1H), 7.90 - 7.98 (m, 2H), 7.98 - 8.10 (m, 3H), 8.52 (d, 1H), 8.73 (s, 1H), 9.11 (t, 1H), 9.32 (d, 1H). | 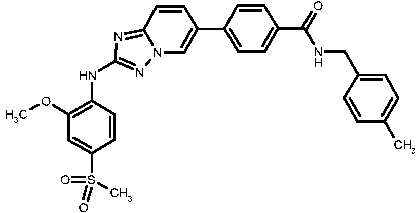 | 541.6 |
| Example 12.77 | *N*-(4-methylbenzyl)-4-{[2-{[2-methyl-4-(methylsulfonyl)-phenyl]amino{[1,2,4]triazolo[1,5-*a*]pyridin-6-yl)-benzamide | ¹H-NMR (400 MHz, DMSO-d₆): δ [ppm] = 2.28 (s, 3H), 2.43 (s, 3H), 3.16 (s, 3H), 4.46 (d, 2H), 7.11 - 7.17 (m, 2H), 7.19 - 7.25 (m, 2H), 7.73 (d, 3H), 7.91 - 7.96 (m, 2H), 8.00 - 8.06 (m, 3H), 8.37 (d, 1H), 9.04 - 9.12 (m, 2H), 9.27 (d, 1H). | 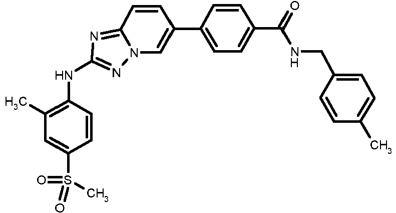 | 525.6 |
| Example 12.78 | *N*-(4-methylbenzyl)-4-(2-{[2-methyl-4-(methylsulfinyl)-phenyl]amino}[1,2,4]tri-azolo[1,5-*a*]pyridin-6-yl)-benzamide | ¹H-NMR (400 MHz, DMSO-d₆): δ [ppm] = 2.28 (s, 3H), 2.40 (s, 3H), 2.72 (s, 3H), 4.46 (d, 2H), 7.11 - 7.17 (m, 2H), 7.19 - 7.25 (m, 2H), 7.47 - 7.52 (m, 2H), 7.65 - 7.70 (m, 1H), 7.89 - 7.95 (m, 2H), 7.98 - 8.03 (m, 3H), 8.22 (d, 1H), 8.85 (s, 1H), 9.08 (t, 1H), 9.24 (d, 1H). | 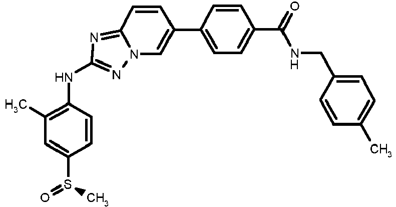 | 509.6 |
| Example 12.79 | 4-[(6-{4-[(2,4-difluorobenzyl)carbamoyl]phenyl}-[1,2,4]triazolo[1,5-*a*]pyridin-2-yl)amino]-3-methoxy-*N-*(2,2,2-trifluoro-ethyl)benzamide | ¹H-NMR (400 MHz, DMSO-d₆): δ [ppm] = 3.97 (s, 3H), 4.06 - 4.17 (m, 2H), 4.51 (d, 2H), 7.07 (td, 1H), 7.20 - 7.27 (m, 1H), 7.40 - 7.48 (m, 1H), 7.58 (d, 1H), 7.61 (dd, 1H), 7.73 (d, 1H), 7.91 - 7.98 (m, 2H), 7.98 - 8.07 (m, 3H), 8.39 (d, 1H), 8.42 (s, 1H), 8.93 (t, 1H), 9.11 (t, 1H), 9.31 (d, 1H). | 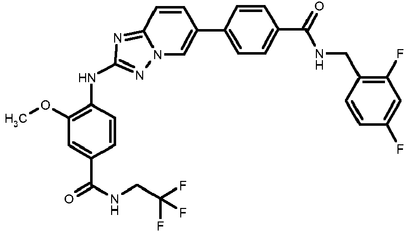 | 610.5 |
| Example 12.80 | *N*-(2,4-difluorobenzyl)-2-methyl-4-(2-{[4-(methyl-sulfonyl)-2-(2,2,2-trifluoro-ethoxy)phenyl]amino}[1,2,4] triazolo[1,5-*a*]pyridin-6-yl)-benzamide | ¹H-NMR (400MHz, DMSO-d₆): δ [ppm]= 2.39 (s, 3H), 3.17 (s, 3H), 4.43 (d, 2H), 5.00 (q, 2H), 7.07 (td, 1H), 7.16 - 7.25 (m, 1H), 7.40 - 7.50 (m, 2H), 7.58 - 7.76 (m, 5H), 8.00 (dd, 1H), 8.51 (d, 1H), 8.60 (s, 1H), 8.83 (t, 1H), 9.22 (d, 1H). | 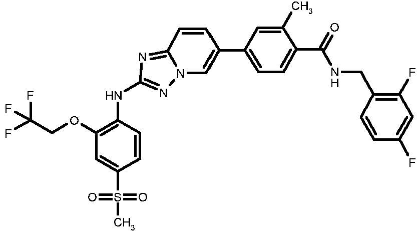 | 645.6 |
| Example 12.81 | *N*-(4-fluorobenzyl)-4-(2-{[4-(2-hydroxypropan-2-yl)-2-methoxyphenyl]amino}-[1,2,4]triazolo[1,5-*a*]pyridin-6-yl)-2-methoxybenzamide | ¹H-NMR (300MHz, DMSO-d₆): δ [ppm]= 1.41 (s, 6H), 3.85 (s, 3H), 4.00 (s, 3H), 4.46 (d, 2H), 4.93 (s, 1H), 6.98 (dd, 1H), 7.08 - 7.18 (m, 3H), 7.30 - 7.38 (m, 2H), 7.42 (dd, 1H), 7.49 (d, 1H), 7.61 (d, 1H), 7.81 (d, 1H), 7.92 (s, 1H), 7.98 (dd, 1H), 8.05 (d, 1H), 8.74 (t, 1H), 9.28 (d, 1H). | 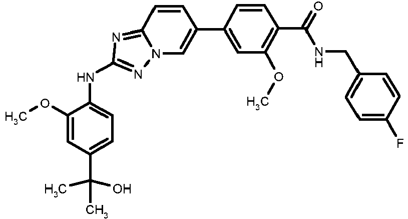 | 555.6 |

### Example 12.82

### 2-(4-fluorophenyl)-N-[4-(2-{[2-methoxy-4-(S-methylsulfonimidoyl)phenyl]-amino}[1,2,4]triazolo[1,5-a]pyridin-6-yl)phenyl]acetamide

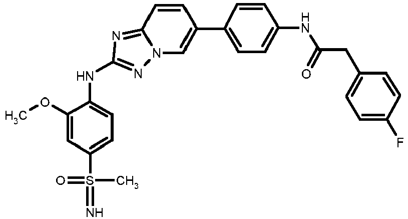

To a stirred suspension of **Int10.25.04** (430 mg) in DCM (5 mL) was added TFA (1 mL). The mixture was stirred at r.t. for 1 h. A saturated solution of potassium carbonate was added until pH 9 was reached. The mixture was extracted with DCM. The solution was dried (sodium sulfate) and the solvent was removed in vacuum. Aminophase-silica-gel chromatography gave a solid that was recrystallized from dicloromethane to give 302 mg of the title compound.

¹H-NMR (300MHz, DMSO-d₆): δ [ppm]= 3.03 (s, 3H), 3.64 (s, 2H), 3.93 (s, 3H), 4.01 (s, 1H), 7.07 - 7.18 (m, 2H), 7.30 - 7.39 (m, 2H), 7.45 (d, 1H), 7.52 (dd, 1H), 7.63 - 7.77 (m, 5H), 7.92 (dd, 1H), 8.43 (d, 1H), 8.51 (s, 1H), 9.11 (d, 1H), 10.29 (s, 1H).

### Example 12.83

### N-(4-fluorobenzyl)-4-(2-{[2-methoxy-4-(S-methylsulfonimidoyl)phenyl]-amino}[1,2,4]triazolo[1,5-a]pyridin-6-yl)benzamide

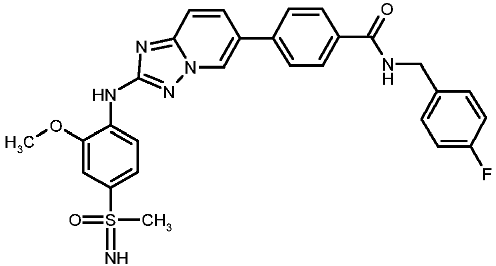

To a stirred suspension of **Int10.25.05** (132 mg) in DCM (1 mL) was added TFA (0.5 mL). The mixture was stirred at r.t. for 1 h. A saturated solution of potassium carbonate was added until pH 9 was reached. The mixture was extracted with DCM. The solution was dried (sodium sulfate) and the solvent was removed in vacuum. Aminophase-silica-gel chromatography gave a solid that was recrystallized from dicloromethane to give 51 mg of the title compound.

¹H-NMR (300MHz, DMSO-d₆): δ [ppm]= 3.03 (s, 3H), 3.94 (s, 3H), 4.02 (s, 1H), 4.45 (d, 2H), 7.05 - 7.19 (m, 2H), 7.26 - 7.38 (m, 2H), 7.46 (d, 1H), 7.52 (dd, 1H), 7.70 (d, 1H), 7.84 - 8.07 (m, 5H), 8.43 (d, 1H), 8.56 (s, 1H), 9.12 (t, 1H), 9.27 (d, 1H).

### Example 12.84

### 2-(4-fluorophenyl)-N-[4-(2-{[4-(hydroxymethyl)-2-methoxyphenyl]amino}-[1,2,4]triazolo[1,5-a]pyridin-6-yl)phenyl]acetamide

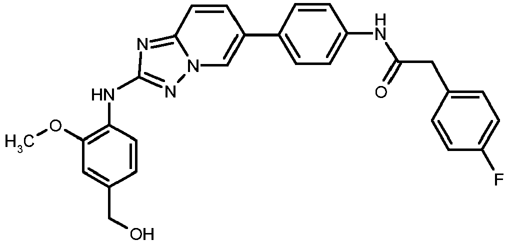

To a stirred solution of **Int10.28.02** (100 mg) in ethanol (5 mL) was added a hydrochloric acid (2.0 mL; c = 2N). The mixture was stirred at r.t. for 10 minutes. A saturated solution of potassium carbonate was added until pH 9 was reached. The mixture was extracted with DCM and methanol (10:1 mixture). The solution was dried (sodium sulfate) and the solvent was removed in vacuum. Aminophase-silica-gel chromatography gave a solid that was recrystallized from ethanol to give 50 mg of the title compound.

¹H-NMR (300MHz, DMSO-d₆): δ [ppm]= 3.64 (s, 2H), 3.84 (s, 3H), 4.43 (d, 2H), 5.07 (t, 1H), 6.88 (d, 1H), 6.97 (s, 1H), 7.07 - 7.18 (m, 2H), 7.30 - 7.39 (m, 2H), 7.58 (d, 1 H), 7.64 - 7.75 (m, 4H), 7.83 - 7.93 (m, 2H), 8.13 (d, 1 H), 9.05 (s, 1H), 10.27 (s, 1H).

### Example 12.85

### N-(4-fluorobenzyl)-4-(2-{[4-(hydroxymethyl)-2-methoxyphenyl]amino}-[1,2,4]triazolo[1,5-a]pyridin-6-yl)benzamide

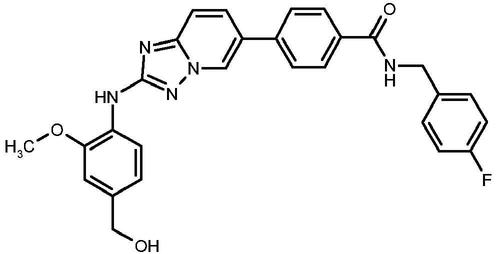

To a stirred solution of **Int10.28.03** (120 mg) in ethanol (5 mL) was added a hydrochloric acid (2.0 mL; c = 2N). The mixture was stirred at r.t. for 10 minutes. A saturated solution of potassium carbonate was added until pH 9 was reached. The mixture was extracted with DCM and methanol (10:1 mixture). The solution was dried (sodium sulfate) and the solvent was removed in vacuum. Aminophase-silica-gel chromatography gave a solid that was recrystallized from ethanol to give 70 mg of the title compound.

¹H-NMR (300MHz, DMSO-d₆): δ [ppm]= 3.84 (s, 3H), 4.39 - 4.50 (m, 4H), 5.08 (t, 1H), 6.88 (d, 1H), 6.97 (d, 1H), 7.07 - 7.19 (m, 2H), 7.34 (dd, 2H), 7.62 (d, 1H), 7.83 - 8.03 (m, 6H), 8.13 (d, 1H), 9.11 (t, 1H), 9.23 (d, 1H).

Further, the compounds of formula (I) of the present invention can be converted to any salt as described herein, by any method which is known to the person skilled in the art. Similarly, any salt of a compound of formula (I) of the present invention can be converted into the free compound, by any method which is known to the person skilled in the art.

### Pharmaceutical compositions of the compounds of the invention

This invention also relates to pharmaceutical compositions containing one or more compounds of the present invention. These compositions can be utilised to achieve the desired pharmacological effect by administration to a patient in need thereof. A patient, for the purpose of this invention, is a mammal, including a human, in need of treatment for the particular condition or disease. Therefore, the present invention includes pharmaceutical compositions that are comprised of a pharmaceutically acceptable carrier and a pharmaceutically effective amount of a compound, or salt thereof, of the present invention. A pharmaceutically acceptable carrier is preferably a carrier that is relatively non-toxic and innocuous to a patient at concentrations consistent with effective activity of the active ingredient so that any side effects ascribable to the carrier do not vitiate the beneficial effects of the active ingredient. A pharmaceutically effective amount of compound is preferably that amount which produces a result or exerts an influence on the particular condition being treated. The compounds of the present invention can be administered with pharmaceutically-acceptable carriers well known in the art using any effective conventional dosage unit forms, including immediate, slow and timed release preparations, orally, parenterally, topically, nasally, ophthalmically, optically, sublingually, rectally, vaginally, and the like.

For oral administration, the compounds can be formulated into solid or liquid preparations such as capsules, pills, tablets, troches, lozenges, melts, powders, solutions, suspensions, or emulsions, and may be prepared according to methods known to the art for the manufacture of pharmaceutical compositions. The solid unit dosage forms can be a capsule that can be of the ordinary hard- or soft-shelled gelatin type containing, for example, surfactants, lubricants, and inert fillers such as lactose, sucrose, calcium phosphate, and corn starch.

In another embodiment, the compounds of this invention may be tableted with conventional tablet bases such as lactose, sucrose and cornstarch in combination with binders such as acacia, corn starch or gelatin, disintegrating agents intended to assist the break-up and dissolution of the tablet following administration such as potato starch, alginic acid, corn starch, and guar gum, gum tragacanth, acacia, lubricants intended to improve the flow of tablet granulation and to prevent the adhesion of tablet material to the surfaces of the tablet dies and punches, for example talc, stearic acid, or magnesium, calcium or zinc stearate, dyes, coloring agents, and flavoring agents such as peppermint, oil of wintergreen, or cherry flavoring, intended to enhance the aesthetic qualities of the tablets and make them more acceptable to the patient. Suitable excipients for use in oral liquid dosage forms include dicalcium phosphate and diluents such as water and alcohols, for example, ethanol, benzyl alcohol, and polyethylene alcohols, either with or without the addition of a pharmaceutically acceptable surfactant, suspending agent or emulsifying agent. Various other materials may be present as coatings or to otherwise modify the physical form of the dosage unit. For instance tablets, pills or capsules may be coated with shellac, sugar or both.

Dispersible powders and granules are suitable for the preparation of an aqueous suspension. They provide the active ingredient in admixture with a dispersing or wetting agent, a suspending agent and one or more preservatives. Suitable dispersing or wetting agents and suspending agents are exemplified by those already mentioned above. Additional excipients, for example those sweetening, flavoring and coloring agents described above, may also be present.

The pharmaceutical compositions of this invention may also be in the form of oil-in-water emulsions. The oily phase may be a vegetable oil such as liquid paraffin or a mixture of vegetable oils. Suitable emulsifying agents may be (1) naturally occurring gums such as gum acacia and gum tragacanth, (2) naturally occurring phosphatides such as soy bean and lecithin, (3) esters or partial esters derived form fatty acids and hexitol anhydrides, for example, sorbitan monooleate, (4) condensation products of said partial esters with ethylene oxide, for example, polyoxyethylene sorbitan monooleate. The emulsions may also contain sweetening and flavoring agents.

Oily suspensions may be formulated by suspending the active ingredient in a vegetable oil such as, for example, arachis oil, olive oil, sesame oil or coconut oil, or in a mineral oil such as liquid paraffin. The oily suspensions may contain a thickening agent such as, for example, beeswax, hard paraffin, or cetyl alcohol. The suspensions may also contain one or more preservatives, for example, ethyl or n-propyl p-hydroxybenzoate ; one or more coloring agents ; one or more flavoring agents ; and one or more sweetening agents such as sucrose or saccharin.

Syrups and elixirs may be formulated with sweetening agents such as, for example, glycerol, propylene glycol, sorbitol or sucrose. Such formulations may also contain a demulcent, and preservative, such as methyl and propyl parabens and flavoring and coloring agents.

The compounds of this invention may also be administered parenterally, that is, subcutaneously, intravenously, intraocularly, intrasynovially, intramuscularly, or interperitoneally, as injectable dosages of the compound in preferably a physiologically acceptable diluent with a pharmaceutical carrier which can be a sterile liquid or mixture of liquids such as water, saline, aqueous dextrose and related sugar solutions, an alcohol such as ethanol, isopropanol, or hexadecyl alcohol, glycols such as propylene glycol or polyethylene glycol, glycerol ketals such as 2,2-dimethyl-1,1-dioxolane-4-methanol, ethers such as poly(ethylene glycol) 400, an oil, a fatty acid, a fatty acid ester or, a fatty acid glyceride, or an acetylated fatty acid glyceride, with or without the addition of a pharmaceutically acceptable surfactant such as a soap or a detergent, suspending agent such as pectin, carbomers, methycellulose, hydroxypropylmethylcellulose, or carboxymethylcellulose, or emulsifying agent and other pharmaceutical adjuvants.

Illustrative of oils which can be used in the parenteral formulations of this invention are those of petroleum, animal, vegetable, or synthetic origin, for example, peanut oil, soybean oil, sesame oil, cottonseed oil, corn oil, olive oil, petrolatum and mineral oil. Suitable fatty acids include oleic acid, stearic acid, isostearic acid and myristic acid. Suitable fatty acid esters are, for example, ethyl oleate and isopropyl myristate. Suitable soaps include fatty acid alkali metal, ammonium, and triethanolamine salts and suitable detergents include cationic detergents, for example dimethyl dialkyl ammonium halides, alkyl pyridinium halides, and alkylamine acetates ; anionic detergents, for example, alkyl, aryl, and olefin sulfonates, alkyl, olefin, ether, and monoglyceride sulfates, and sulfosuccinates ; non-ionic detergents, for example, fatty amine oxides, fatty acid alkanolamides, and poly(oxyethylene-oxypropylene)s or ethylene oxide or propylene oxide copolymers ; and amphoteric detergents, for example, alkyl-beta-aminopropionates, and 2-alkylimidazoline quarternary ammonium salts, as well as mixtures.

The parenteral compositions of this invention will typically contain from about 0.5% to about 25% by weight of the active ingredient in solution. Preservatives and buffers may also be used advantageously. In order to minimise or eliminate irritation at the site of injection, such compositions may contain a non-ionic surfactant having a hydrophile-lipophile balance (HLB) preferably of from about 12 to about 17. The quantity of surfactant in such formulation preferably ranges from about 5% to about 15% by weight. The surfactant can be a single component having the above HLB or can be a mixture of two or more components having the desired HLB.

Illustrative of surfactants used in parenteral formulations are the class of polyethylene sorbitan fatty acid esters, for example, sorbitan monooleate and the high molecular weight adducts of ethylene oxide with a hydrophobic base, formed by the condensation of propylene oxide with propylene glycol.

The pharmaceutical compositions may be in the form of sterile injectable aqueous suspensions. Such suspensions may be formulated according to known methods using suitable dispersing or wetting agents and suspending agents such as, for example, sodium carboxymethylcellulose, methylcellulose, hydroxypropylmethyl-cellulose, sodium alginate, polyvinylpyrrolidone, gum tragacanth and gum acacia ; dispersing or wetting agents which may be a naturally occurring phosphatide such as lecithin, a condensation product of an alkylene oxide with a fatty acid, for example, polyoxyethylene stearate, a condensation product of ethylene oxide with a long chain aliphatic alcohol, for example, heptadeca-ethyleneoxycetanol, a condensation product of ethylene oxide with a partial ester derived form a fatty acid and a hexitol such as polyoxyethylene sorbitol monooleate, or a condensation product of an ethylene oxide with a partial ester derived from a fatty acid and a hexitol anhydride, for example polyoxyethylene sorbitan monooleate.

The sterile injectable preparation may also be a sterile injectable solution or suspension in a non-toxic parenterally acceptable diluent or solvent. Diluents and solvents that may be employed are, for example, water, Ringer's solution, isotonic sodium chloride solutions and isotonic glucose solutions. In addition, sterile fixed oils are conventionally employed as solvents or suspending media. For this purpose, any bland, fixed oil may be employed including synthetic mono- or diglycerides. In addition, fatty acids such as oleic acid can be used in the preparation of injectables.

A composition of the invention may also be administered in the form of suppositories for rectal administration of the drug. These compositions can be prepared by mixing the drug with a suitable non-irritation excipient which is solid at ordinary temperatures but liquid at the rectal temperature and will therefore melt in the rectum to release the drug. Such materials are, for example, cocoa butter and polyethylene glycol.

Another formulation employed in the methods of the present invention employs transdermal delivery devices ("patches"). Such transdermal patches may be used to provide continuous or discontinuous infusion of the compounds of the present invention in controlled amounts. The construction and use of transdermal patches for the delivery of pharmaceutical agents is well known in the art (see, e.g., US Patent No. 5,023,252, issued June 11, 1991, incorporated herein by reference). Such patches may be constructed for continuous, pulsatile, or on demand delivery of pharmaceutical agents.

Controlled release formulations for parenteral administration include liposomal, polymeric microsphere and polymeric gel formulations that are known in the art.

It may be desirable or necessary to introduce the pharmaceutical composition to the patient via a mechanical delivery device. The construction and use of mechanical delivery devices for the delivery of pharmaceutical agents is well known in the art. Direct techniques for, for example, administering a drug directly to the brain usually involve placement of a drug delivery catheter into the patient's ventricular system to bypass the blood-brain barrier. One such implantable delivery system, used for the transport of agents to specific anatomical regions of the body, is described in US Patent No. 5,011,472, issued April 30, 1991.

The compositions of the invention can also contain other conventional pharmaceutically acceptable compounding ingredients, generally referred to as carriers or diluents, as necessary or desired. Conventional procedures for preparing such compositions in appropriate dosage forms can be utilized. Such ingredients and procedures include those described in the following references, each of which is incorporated herein by reference: Powell, M.F. et al., "Compendium of Excipients for Parenteral Formulations" PDA Journal of Pharmaceutical Science Technology 1998, 52(5), 238-311 ; Strickley, R.G "Parenteral Formulations of Small Molecule Therapeutics Marketed in the United States (1999)-Part-1" PDA Journal of Pharmaceutical Science & Technology 1999, 53(6), 324-349 ; and Nema, S. et al., "Excipients and Their Use in Injectable Products" PDA Journal of Pharmaceutical Science & Technology 1997, 51(4), 166-171.

Commonly used pharmaceutical ingredients that can be used as appropriate to formulate the composition for its intended route of administration include:
**acidifying agents** (examples include but are not limited to acetic acid, citric acid, fumaric acid, hydrochloric acid, nitric acid) ;
alkalinizing **agents** (examples include but are not limited to ammonia solution, ammonium carbonate, diethanolamine, monoethanolamine, potassium hydroxide, sodium borate, sodium carbonate, sodium hydroxide, triethanolamine, trolamine) ;
**adsorbents** (examples include but are not limited to powdered cellulose and activated charcoal) ;
**aerosol propellants** (examples include but are not limited to carbon dioxide, CCl₂F₂, F₂ClC-CClF₂ and CClF₃)
**air displacement agents** (examples include but are not limited to nitrogen and argon) ;
**antifungal preservatives** (examples include but are not limited to benzoic acid, butylparaben, ethylparaben, methylparaben, propylparaben, sodium benzoate);
**antimicrobial preservatives** (examples include but are not limited to benzalkonium chloride, benzethonium chloride, benzyl alcohol, cetylpyridinium chloride, chlorobutanol, phenol, phenylethyl alcohol, phenylmercuric nitrate and thimerosal) ;
**antioxidants** (examples include but are not limited to ascorbic acid, ascorbyl palmitate, butylated hydroxyanisole, butylated hydroxytoluene, hypophosphorus acid, monothioglycerol, propyl gallate, sodium ascorbate, sodium bisulfite, sodium formaldehyde sulfoxylate, sodium metabisulfite) ;
**binding materials** (examples include but are not limited to block polymers, natural and synthetic rubber, polyacrylates, polyurethanes, silicones, polysiloxanes and styrene-butadiene copolymers) ;
**buffering agents** (examples include but are not limited to potassium metaphosphate, dipotassium phosphate, sodium acetate, sodium citrate anhydrous and sodium citrate dihydrate)
**carrying agents** (examples include but are not limited to acacia syrup, aromatic syrup, aromatic elixir, cherry syrup, cocoa syrup, orange syrup, syrup, corn oil, mineral oil, peanut oil, sesame oil, bacteriostatic sodium chloride injection and bacteriostatic water for injection)
**chelating agents** (examples include but are not limited to edetate disodium and edetic acid)
**colorants** (examples include but are not limited to FD&C Red No. 3, FD&C Red No. 20, FD&C Yellow No. 6, FD&C Blue No. 2, D&C Green No. 5, D&C Orange No. 5, D&C Red No. 8, caramel and ferric oxide red) ;
**clarifying agents** (examples include but are not limited to bentonite) ;
**emulsifying agents** (examples include but are not limited to acacia, cetomacrogol, cetyl alcohol, glyceryl monostearate, lecithin, sorbitan monooleate, polyoxyethylene 50 monostearate) ;
**encapsulating agents** (examples include but are not limited to gelatin and cellulose acetate phthalate)
**flavorants** (examples include but are not limited to anise oil, cinnamon oil, cocoa, menthol, orange oil, peppermint oil and vanillin) ;
**humectants** (examples include but are not limited to glycerol, propylene glycol and sorbitol) ;
**levigating agents** (examples include but are not limited to mineral oil and glycerin) ;
oils (examples include but are not limited to arachis oil, mineral oil, olive oil, peanut oil, sesame oil and vegetable oil) ;
**ointment bases** (examples include but are not limited to lanolin, hydrophilic ointment, polyethylene glycol ointment, petrolatum, hydrophilic petrolatum, white ointment, yellow ointment, and rose water ointment) ;
**penetration enhancers (transdermal delivery)** (examples include but are not limited to monohydroxy or polyhydroxy alcohols, mono-or polyvalent alcohols, saturated or unsaturated fatty alcohols, saturated or unsaturated fatty esters, saturated or unsaturated dicarboxylic acids, essential oils, phosphatidyl derivatives, cephalin, terpenes, amides, ethers, ketones and ureas) **plasticizers** (examples include but are not limited to diethyl phthalate and glycerol) ;
**solvents** (examples include but are not limited to ethanol, corn oil, cottonseed oil, glycerol, isopropanol, mineral oil, oleic acid, peanut oil, purified water, water for injection, sterile water for injection and sterile water for irrigation) ;
**stiffening agents** (examples include but are not limited to cetyl alcohol, cetyl esters wax, microcrystalline wax, paraffin, stearyl alcohol, white wax and yellow wax);
**suppository bases** (examples include but are not limited to cocoa butter and polyethylene glycols (mixtures)) ;
**surfactants** (examples include but are not limited to benzalkonium chloride, nonoxynol 10, oxtoxynol 9, polysorbate 80, sodium lauryl sulfate and sorbitan mono-palmitate) ;
**suspending agents** (examples include but are not limited to agar, bentonite, carbomers, carboxymethylcellulose sodium, hydroxyethyl cellulose, hydroxypropyl cellulose, hydroxypropyl methylcellulose, kaolin, methylcellulose, tragacanth and veegum) ;
**sweetening agents** (examples include but are not limited to aspartame, dextrose, glycerol, mannitol, propylene glycol, saccharin sodium, sorbitol and sucrose);
**tablet anti-adherents** (examples include but are not limited to magnesium stearate and talc) ;
**tablet binders** (examples include but are not limited to acacia, alginic acid, carboxymethylcellulose sodium, compressible sugar, ethylcellulose, gelatin, liquid glucose, methylcellulose, non-crosslinked polyvinyl pyrrolidone, and pregelatinized starch) ;
**tablet and capsule diluents** (examples include but are not limited to dibasic calcium phosphate, kaolin, lactose, mannitol, microcrystalline cellulose, powdered cellulose, precipitated calcium carbonate, sodium carbonate, sodium phosphate, sorbitol and starch) ;
**tablet coating agents** (examples include but are not limited to liquid glucose, hydroxyethyl cellulose, hydroxypropyl cellulose, hydroxypropyl methylcellulose, methylcellulose, ethylcellulose, cellulose acetate phthalate and shellac) ;
**tablet direct compression excipients** (examples include but are not limited to dibasic calcium phosphate) ;
**tablet disintegrants** (examples include but are not limited to alginic acid, carboxymethylcellulose calcium, microcrystalline cellulose, polacrillin potassium, cross-linked polyvinylpyrrolidone, sodium alginate, sodium starch glycollate and starch) ;
**tablet glidants** (examples include but are not limited to colloidal silica, corn starch and talc) ;
**tablet lubricants** (examples include but are not limited to calcium stearate, magnesium stearate, mineral oil, stearic acid and zinc stearate) ;
**tablet/capsule opaquants** (examples include but are not limited to titanium dioxide) ;
**tablet polishing agents** (examples include but are not limited to carnuba wax and white wax) ;
**thickening agents** (examples include but are not limited to beeswax, cetyl alcohol and paraffin) ;
**tonicity agents** (examples include but are not limited to dextrose and sodium chloride) ;
**viscosity increasing agents** (examples include but are not limited to alginic acid, bentonite, carbomers, carboxymethylcellulose sodium, methylcellulose, polyvinyl pyrrolidone, sodium alginate and tragacanth) ; and
**wetting agents** (examples include but are not limited to heptadecaethylene oxycetanol, lecithins, sorbitol monooleate, polyoxyethylene sorbitol monooleate, and polyoxyethylene stearate).

Pharmaceutical compositions according to the present invention can be illustrated as follows:
Sterile IV Solution: A 5 mg/mL solution of the desired compound of this invention can be made using sterile, injectable water, and the pH is adjusted if necessary. The solution is diluted for administration to 1 - 2 mg/mL with sterile 5% dextrose and is administered as an IV infusion over about 60 minutes.
Lyophilised powder for IV administration: A sterile preparation can be prepared with (i) 100 - 1000 mg of the desired compound of this invention as a lyophilised powder, (ii) 32- 327 mg/mL sodium citrate, and (iii) 300 - 3000 mg Dextran 40. The formulation is reconstituted with sterile, injectable saline or dextrose 5% to a concentration of 10 to 20 mg/mL, which is further diluted with saline or dextrose 5% to 0.2 - 0.4 mg/mL, and is administered either IV bolus or by IV infusion over 15 - 60 minutes.
Intramuscular suspension: The following solution or suspension can be prepared, for intramuscular injection:
   50 mg/mL of the desired, water-insoluble compound of this invention
   5 mg/mL sodium carboxymethylcellulose
   4 mg/mL TWEEN 80
   9 mg/mL sodium chloride
   9 mg/mL benzyl alcohol
Hard Shell Capsules: A large number of unit capsules are prepared by filling standard two-piece hard galantine capsules each with 100 mg of powdered active ingredient, 150 mg of lactose, 50 mg of cellulose and 6 mg of magnesium stearate.
Soft Gelatin Capsules: A mixture of active ingredient in a digestible oil such as soybean oil, cottonseed oil or olive oil is prepared and injected by means of a positive displacement pump into molten gelatin to form soft gelatin capsules containing 100 mg of the active ingredient. The capsules are washed and dried. The active ingredient can be dissolved in a mixture of polyethylene glycol, glycerin and sorbitol to prepare a water miscible medicine mix.
Tablets: A large number of tablets are prepared by conventional procedures so that the dosage unit is 100 mg of active ingredient, 0.2 mg. of colloidal silicon dioxide, 5 mg of magnesium stearate, 275 mg of microcrystalline cellulose, 11 mg. of starch, and 98.8 mg of lactose. Appropriate aqueous and non-aqueous coatings may be applied to increase palatability, improve elegance and stability or delay absorption.
Immediate Release Tablets/Capsules: These are solid oral dosage forms made by conventional and novel processes. These units are taken orally without water for immediate dissolution and delivery of the medication. The active ingredient is mixed in a liquid containing ingredient such as sugar, gelatin, pectin and sweeteners. These liquids are solidified into solid tablets or caplets by freeze drying and solid state extraction techniques. The drug compounds may be compressed with viscoelastic and thermoelastic sugars and polymers or effervescent components to produce porous matrices intended for immediate release, without the need of water.

### Combination therapies

The compounds of this invention can be administered as the sole pharmaceutical agent or in combination with one or more other pharmaceutical agents where the combination causes no unacceptable adverse effects. The present invention relates also to such combinations. For example, the compounds of this invention can be combined with known anti-hyper-proliferative or other indication agents, and the like, as well as with admixtures and combinations thereof. Other indication agents include, but are not limited to, anti-angiogenic agents, mitotic inhibitors, alkylating agents, anti-metabolites, DNA-intercalating antibiotics, growth factor inhibitors, cell cycle inhibitors, enzyme inhibitors, toposisomerase inhibitors, biological response modifiers, or anti-hormones.

The additional pharmaceutical agent can be aldesleukin, alendronic acid, alfaferone, alitretinoin, allopurinol, aloprim, aloxi, altretamine, aminoglutethimide, amifostine, amrubicin, amsacrine, anastrozole, anzmet, aranesp, arglabin, arsenic trioxide, aromasin, 5-azacytidine, azathioprine, BCG or tice BCG, bestatin, betamethasone acetate, betamethasone sodium phosphate, bexarotene, bleomycin sulfate, broxuridine , bortezomib, busulfan, calcitonin, campath, capecitabine, carboplatin, casodex, cefesone, celmoleukin, cerubidine, chlorambucil, cisplatin, cladribine, cladribine, clodronic acid, cyclophosphamide, cytarabine, dacarbazine, dactinomycin, DaunoXome, decadron, decadron phosphate, delestrogen, denileukin diftitox, depo-medrol, deslorelin, dexrazoxane, diethylstilbestrol, diflucan, docetaxel, doxifluridine, doxorubicin, dronabinol, DW-166HC, eligard, elitek, ellence, emend, epirubicin, epoetin alfa, epogen, eptaplatin, ergamisol, estrace, estradiol, estramustine phosphate sodium, ethinyl estradiol, ethyol, etidronic acid, etopophos, etoposide, fadrozole, farston, filgrastim, finasteride, fligrastim, floxuridine, fluconazole, fludarabine, 5-fluorodeoxyuridine monophosphate, 5-fluorouracil (5-FU), fluoxymesterone, flutamide, formestane, fosteabine, fotemustine, fulvestrant, gammagard, gemcitabine, gemtuzumab, gleevec, gliadel, goserelin, granisetron HCl, histrelin, hycamtin, hydrocortone, eyrthro-hydroxynonyladenine, hydroxyurea, ibritumomab tiuxetan, idarubicin, ifosfamide, interferon alpha, interferon-alpha 2, interferon alfa-2A, interferon alfa-2B, interferon alfa-n1, interferon alfa-n3, interferon beta, interferon gamma-1a, interleukin-2, intron A, iressa, irinotecan, kytril, lentinan sulfate, letrozole, leucovorin, leuprolide, leuprolide acetate, levamisole, levofolinic acid calcium salt, levothroid, levoxyl, lomustine, lonidamine, marinol, mechlorethamine, mecobalamin, medroxyprogesterone acetate, megestrol acetate, melphalan, menest, 6-mercaptopurine, Mesna, methotrexate, metvix, miltefosine, minocycline, mitomycin C, mitotane, mitoxantrone, Modrenal, Myocet, nedaplatin, neulasta, neumega, neupogen, nilutamide, nolvadex, NSC-631570, OCT-43, octreotide, ondansetron HCl, orapred, oxaliplatin, paclitaxel, pediapred, pegaspargase, Pegasys, pentostatin, picibanil, pilocarpine HCl, pirarubicin, plicamycin, porfimer sodium, prednimustine, prednisolone, prednisone, premarin, procarbazine, procrit, raltitrexed, rebif, rhenium-186 etidronate, rituximab, roferon-A, romurtide, salagen, sandostatin, sargramostim, semustine, sizofiran, sobuzoxane, solu-medrol, sparfosic acid, stem-cell therapy, streptozocin, strontium-89 chloride, synthroid, tamoxifen, tamsulosin, tasonermin, tastolactone, taxotere, teceleukin, temozolomide, teniposide, testosterone propionate, testred, thioguanine, thiotepa, thyrotropin, tiludronic acid, topotecan, toremifene, tositumomab, trastuzumab, treosulfan, tretinoin, trexall, trimethylmelamine, trimetrexate, triptorelin acetate, triptorelin pamoate, UFT, uridine, valrubicin, vesnarinone, vinblastine, vincristine, vindesine, vinorelbine, virulizin, zinecard, zinostatin stimalamer, zofran, ABI-007, acolbifene, actimmune, affinitak, aminopterin, arzoxifene, asoprisnil, atamestane, atrasentan, sorafenib, avastin, CCI-779, CDC-501, celebrex, cetuximab, crisnatol, cyproterone acetate, decitabine, DN-101, doxorubicin-MTC, dSLIM, dutasteride, edotecarin, eflornithine, exatecan, fenretinide, histamine dihydrochloride, histrelin hydrogel implant, holmium-166 DOTMP, ibandronic acid, interferon gamma, intron-PEG, ixabepilone, keyhole limpet hemocyanin, L-651582, lanreotide, lasofoxifene, libra, lonafarnib, miproxifene, minodronate, MS-209, liposomal MTP-PE, MX-6, nafarelin, nemorubicin, neovastat, nolatrexed, oblimersen, onco-TCS, osidem, paclitaxel polyglutamate, pamidronate disodium, PN-401, QS-21, quazepam, R-1549, raloxifene, ranpirnase, 13-cis -retinoic acid, satraplatin, seocalcitol, T-138067, tarceva, taxoprexin, thymosin alpha 1, tiazofurine, tipifarnib, tirapazamine, TLK-286, toremifene, TransMID-107R, valspodar, vapreotide, vatalanib, verteporfin, vinflunine, Z-100, zoledronic acid or combinations thereof.

Optional anti-hyper-proliferative agents which can be added to the composition include but are not limited to compounds listed on the cancer chemotherapy drug regimens in the 11th Edition of the Merck Index, (1996), which is hereby incorporated by reference, such as asparaginase, bleomycin, carboplatin, carmustine, chlorambucil, cisplatin, colaspase, cyclophosphamide, cytarabine, dacarbazine, dactinomycin, daunorubicin, doxorubicin (adriamycine), epirubicin, etoposide, 5-fluorouracil, hexamethylmelamine, hydroxyurea, ifosfamide, irinotecan, leucovorin, lomustine, mechlorethamine, 6-mercaptopurine, mesna, methotrexate, mitomycin C, mitoxantrone, prednisolone, prednisone, procarbazine, raloxifen, streptozocin, tamoxifen, thioguanine, topotecan, vinblastine, vincristine, and vindesine.

Other anti-hyper-proliferative agents suitable for use with the composition of the invention include but are not limited to those compounds acknowledged to be used in the treatment of neoplastic diseases in Goodman and Gilman's The Pharmacological Basis of Therapeutics (Ninth Edition), editor Molinoff et al., publ. by McGraw-Hill, pages 1225-1287, (1996), which is hereby incorporated by reference, such as aminoglutethimide, L-asparaginase, azathioprine, 5-azacytidine cladribine, busulfan, diethylstilbestrol, 2',2'-difluorodeoxycytidine, docetaxel, erythrohydroxynonyl adenine, ethinyl estradiol, 5-fluorodeoxyuridine, 5-fluorodeoxyuridine monophosphate, fludarabine phosphate, fluoxymesterone, flutamide, hydroxyprogesterone caproate, idarubicin, interferon, medroxyprogesterone acetate, megestrol acetate, melphalan, mitotane, paclitaxel, pentostatin, N-phosphonoacetyl-L-aspartate (PALA), plicamycin, semustine, teniposide, testosterone propionate, thiotepa, trimethylmelamine, uridine, and vinorelbine.

Other anti-hyper-proliferative agents suitable for use with the composition of the invention include but are not limited to other anti-cancer agents such as epothilone and its derivatives, irinotecan, raloxifen and topotecan.

The compounds of the invention may also be administered in combination with protein therapeutics. Such protein therapeutics suitable for the treatment of cancer or other angiogenic disorders and for use with the compositions of the invention include, but are not limited to, an interferon (e.g., interferon .alpha., .beta., or .gamma.) supraagonistic monoclonal antibodies, Tuebingen, TRP-1 protein vaccine, Colostrinin, anti-FAP antibody, YH-16, gemtuzumab, infliximab, cetuximab, trastuzumab, denileukin diftitox, rituximab, thymosin alpha 1, bevacizumab, mecasermin, mecasermin rinfabate, oprelvekin, natalizumab, rhMBL, MFE-CP1 + ZD-2767-P, ABT-828, ErbB2-specific immunotoxin, SGN-35, MT-103, rinfabate, AS-1402, B43-genistein, L-19 based radioimmunotherapeutics, AC-9301, NY-ESO-1 vaccine, IMC-1C11, CT-322, rhCC10, r(m)CRP, MORAb-009, aviscumine, MDX-1307, Her-2 vaccine, APC-8024, NGR-hTNF, rhH1.3, IGN-311, Endostatin, volociximab, PRO-1762, lexatumumab, SGN-40, pertuzumab, EMD-273063, L19-IL-2 fusion protein, PRX-321, CNTO-328, MDX-214, tigapotide, CAT-3888, labetuzumab, alpha-particle-emitting radioisotope-llinked lintuzumab, EM-1421, HyperAcute vaccine, tucotuzumab celmoleukin, galiximab, HPV-16-E7, Javelin - prostate cancer, Javelin - melanoma, NY-ESO-1 vaccine, EGF vaccine, CYT-004-MelQbG10, WT1 peptide, oregovomab, ofatumumab, zalutumumab, cintredekin besudotox, WX-G250, Albuferon, aflibercept, denosumab, vaccine, CTP-37, efungumab, or 131I-chTNT-1/B. Monoclonal antibodies useful as the protein therapeutic include, but are not limited to, muromonab-CD3, abciximab, edrecolomab, daclizumab, gentuzumab, alemtuzumab, ibritumomab, cetuximab, bevicizumab, efalizumab, adalimumab, omalizumab, muromomab-CD3, rituximab, daclizumab, trastuzumab, palivizumab, basiliximab, and infliximab.

Generally, the use of cytotoxic and/or cytostatic agents in combination with a compound or composition of the present invention will serve to:
(1) yield better efficacy in reducing the growth of a tumor or even eliminate the tumor as compared to administration of either agent alone,
(2) provide for the administration of lesser amounts of the administered chemotherapeutic agents,
(3) provide for a chemotherapeutic treatment that is well tolerated in the patient with fewer deleterious pharmacological complications than observed with single agent chemotherapies and certain other combined therapies,
(4) provide for treating a broader spectrum of different cancer types in mammals, especially humans,
(5) provide for a higher response rate among treated patients,
(6) provide for a longer survival time among treated patients compared to standard chemotherapy treatments,
(7) provide a longer time for tumor progression, and/or
(8) yield efficacy and tolerability results at least as good as those of the agents used alone, compared to known instances where other cancer agent combinations produce antagonistic effects.

### Methods of Sensitizing Cells to Radiation

In a distinct embodiment of the present invention, a compound of the present invention may be used to sensitize a cell to radiation. That is, treatment of a cell with a compound of the present invention prior to radiation treatment of the cell renders the cell more susceptible to DNA damage and cell death than the cell would be in the absence of any treatment with a compound of the invention. In one aspect, the cell is treated with at least one compound of the invention.

Thus, the present invention also provides a method of killing a cell, wherein a cell is administered one or more compounds of the invention in combination with conventional radiation therapy.

The present invention also provides a method of rendering a cell more susceptible to cell death, wherein the cell is treated one or more compounds of the invention prior to the treatment of the cell to cause or induce cell death. In one aspect, after the cell is treated with one or more compounds of the invention, the cell is treated with at least one compound, or at least one method, or a combination thereof, in order to cause DNA damage for the purpose of inhibiting the function of the normal cell or killing the cell.

In one embodiment, a cell is killed by treating the cell with at least one DNA damaging agent. That is, after treating a cell with one or more compounds of the invention to sensitize the cell to cell death, the cell is treated with at least one DNA damaging agent to kill the cell. DNA damaging agents useful in the present invention include, but are not limited to, chemotherapeutic agents (*e.g.,* cisplatinum), ionizing radiation (X-rays, ultraviolet radiation), carcinogenic agents, and mutagenic agents.

In another embodiment, a cell is killed by treating the cell with at least one method to cause or induce DNA damage. Such methods include, but are not limited to, activation of a cell signalling pathway that results in DNA damage when the pathway is activated, inhibiting of a cell signalling pathway that results in DNA damage when the pathway is inhibited, and inducing a biochemical change in a cell, wherein the change results in DNA damage. By way of a non-limiting example, a DNA repair pathway in a cell can be inhibited, thereby preventing the repair of DNA damage and resulting in an abnormal accumulation of DNA damage in a cell.

In one aspect of the invention, a compound of the invention is administered to a cell prior to the radiation or orther induction of DNA damage in the cell. In another aspect of the invention, a compound of the invention is administered to a cell concomitantly with the radiation or orther induction of DNA damage in the cell. In yet another aspect of the invention, a compound of the invention is administered to a cell immediately after radiation or orther induction of DNA damage in the cell has begun.

In another aspect, the cell is *in vitro.* In another embodiment, the cell is *in vivo.*

As mentioned *supra,* the compounds of the present invention have surprisingly been found to effectively inhibit Mps-1 and may therefore be used for the treatment or prophylaxis of diseases of uncontrolled cell growth, proliferation and/or survival, inappropriate cellular immune responses, or inappropriate cellular inflammatory responses, or diseases which are accompanied with uncontrolled cell growth, proliferation and/or survival, inappropriate cellular immune responses, or inappropriate cellular inflammatory responses, particularly in which the uncontrolled cell growth, proliferation and/or survival, inappropriate cellular immune responses, or inappropriate cellular inflammatory responses is mediated by Mps-1, such as, for example, haematological tumours, solid tumours, and/or metastases thereof, e.g. leukaemias and myelodysplastic syndrome, malignant lymphomas, head and neck tumours including brain tumours and brain metastases, tumours of the thorax including non-small cell and small cell lung tumours, gastrointestinal tumours, endocrine tumours, mammary and other gynaecological tumours, urological tumours including renal, bladder and prostate tumours, skin tumours, and sarcomas, and/or metastases thereof.

In accordance with another aspect therefore, the present invention covers a compound of general formula (I), or a stereoisomer, a tautomer, an N-oxide, a hydrate, a solvate, or a salt thereof, particularly a pharmaceutically acceptable salt thereof, or a mixture of same, as described and defined herein, for use in the treatment or prophylaxis of a disease, as mentioned *supra.*

Another particular aspect of the present invention is therefore the use of a compound of general formula (I) described *supra* for manufacturing a pharmaceutical composition for the treatment or prophylaxis of a disease.

The diseases referred to in the two preceding paragraphs are diseases of uncontrolled cell growth, proliferation and/or survival, inappropriate cellular immune responses, or inappropriate cellular inflammatory responses, or diseases which are accompanied with uncontrolled cell growth, proliferation and/or survival, inappropriate cellular immune responses, or inappropriate cellular inflammatory responses, particularly in which the uncontrolled cell growth, proliferation and/or survival, inappropriate cellular immune responses, or inappropriate cellular inflammatory responses is mediated by Mps-1, such as, for example, haematological tumours, solid tumours, and/or metastases thereof, *e.g.* leukaemias and myelodysplastic syndrome, malignant lymphomas, head and neck tumours including brain tumours and brain metastases, tumours of the thorax including non-small cell and small cell lung tumours, gastrointestinal tumours, endocrine tumours, mammary and other gynaecological tumours, urological tumours including renal, bladder and prostate tumours, skin tumours, and sarcomas, and/or metastases thereof.

The term "inappropriate" within the context of the present invention, in particular in the context of "inappropriate cellular immune responses, or inappropriate cellular inflammatory responses", as used herein, is to be understood as preferably meaning a response which is less than, or greater than normal, and which is associated with, responsible for, or results in, the pathology of said diseases.

Preferably, the use is in the treatment or prophylaxis of diseases, wherein the diseases are haemotological tumours, solid tumours and/or metastases thereof.

### Method of treating hyper-proliferative disorders

The present invention relates to a method for using the compounds of the present invention and compositions thereof, to treat mammalian hyper-proliferative disorders. Compounds can be utilized to inhibit, block, reduce, decrease, etc., cell proliferation and/or cell division, and/or produce apoptosis. This method comprises administering to a mammal in need thereof, including a human, an amount of a compound of this invention, or a pharmaceutically acceptable salt, isomer, polymorph, metabolite, hydrate, solvate or ester thereof ; etc. which is effective to treat the disorder. Hyper-proliferative disorders include but are not limited, e.g., psoriasis, keloids, and other hyperplasias affecting the skin, benign prostate hyperplasia (BPH), solid tumors, such as cancers of the breast, respiratory tract, brain, reproductive organs, digestive tract, urinary tract, eye, liver, skin, head and neck, thyroid, parathyroid and their distant metastases. Those disorders also include lymphomas, sarcomas, and leukemias.

Examples of breast cancer include, but are not limited to invasive ductal carcinoma, invasive lobular carcinoma, ductal carcinoma in situ, and lobular carcinoma in situ.

Examples of cancers of the respiratory tract include, but are not limited to small-cell and non-small-cell lung carcinoma, as well as bronchial adenoma and pleuropulmonary blastoma.

Examples of brain cancers include, but are not limited to brain stem and hypophtalmic glioma, cerebellar and cerebral astrocytoma, medulloblastoma, ependymoma, as well as neuroectodermal and pineal tumor.

Tumors of the male reproductive organs include, but are not limited to prostate and testicular cancer. Tumors of the female reproductive organs include, but are not limited to endometrial, cervical, ovarian, vaginal, and vulvar cancer, as well as sarcoma of the uterus.

Tumors of the digestive tract include, but are not limited to anal, colon, colorectal, esophageal, gallbladder, gastric, pancreatic, rectal, small-intestine, and salivary gland cancers.

Tumors of the urinary tract include, but are not limited to bladder, penile, kidney, renal pelvis, ureter, urethral and human papillary renal cancers.

Eye cancers include, but are not limited to intraocular melanoma and retinoblastoma.

Examples of liver cancers include, but are not limited to hepatocellular carcinoma (liver cell carcinomas with or without fibrolamellar variant), cholangiocarcinoma (intrahepatic bile duct carcinoma), and mixed hepatocellular cholangiocarcinoma.

Skin cancers include, but are not limited to squamous cell carcinoma, Kaposi's sarcoma, malignant melanoma, Merkel cell skin cancer, and non-melanoma skin cancer.

Head-and-neck cancers include, but are not limited to laryngeal, hypopharyngeal, nasopharyngeal, oropharyngeal cancer, lip and oral cavity cancer and squamous cell. Lymphomas include, but are not limited to AIDS- related lymphoma, non-Hodgkin's lymphoma, cutaneous T-cell lymphoma, Burkitt lymphoma, Hodgkin's disease, and lymphoma of the central nervous system.

Sarcomas include, but are not limited to sarcoma of the soft tissue, osteosarcoma, malignant fibrous histiocytoma, lymphosarcoma, and rhabdomyosarcoma.

Leukemias include, but are not limited to acute myeloid leukemia, acute lymphoblastic leukemia, chronic lymphocytic leukemia, chronic myelogenous leukemia, and hairy cell leukemia.

These disorders have been well characterized in humans, but also exist with a similar etiology in other mammals, and can be treated by administering pharmaceutical compositions of the present invention.

The term "treating" or "treatment" as stated throughout this document is used conventionally, e.g., the management or care of a subject for the purpose of combating, alleviating, reducing, relieving, improving the condition of, *etc.*, of a disease or disorder, such as a carcinoma.

### Methods of treating kinase disorders

The present invention also provides methods for the treatment of disorders associated with aberrant mitogen extracellular kinase activity, including, but not limited to stroke, heart failure, hepatomegaly, cardiomegaly, diabetes, Alzheimer's disease, cystic fibrosis, symptoms of xenograft rejections, septic shock or asthma.

Effective amounts of compounds of the present invention can be used to treat such disorders, including those diseases (e.g., cancer) mentioned in the Background section above. Nonetheless, such cancers and other diseases can be treated with compounds of the present invention, regardless of the mechanism of action and/or the relationship between the kinase and the disorder.

The phrase "aberrant kinase activity" or "aberrant tyrosine kinase activity," includes any abnormal expression or activity of the gene encoding the kinase or of the polypeptide it encodes. Examples of such aberrant activity, include, but are not limited to, over-expression of the gene or polypeptide ; gene amplification ; mutations which produce constitutively-active or hyperactive kinase activity ; gene mutations, deletions, substitutions, additions, etc.

The present invention also provides for methods of inhibiting a kinase activity, especially of mitogen extracellular kinase, comprising administering an effective amount of a compound of the present invention, including salts, polymorphs, metabolites, hydrates, solvates, prodrugs (e.g.: esters) thereof, and diastereoisomeric forms thereof. Kinase activity can be inhibited in cells (*e.g., in vitro*)*,* or in the cells of a mammalian subject, especially a human patient in need of treatment.

### Methods of treating angiogenic disorders

The present invention also provides methods of treating disorders and diseases associated with excessive and/or abnormal angiogenesis.

Inappropriate and ectopic expression of angiogenesis can be deleterious to an organism. A number of pathological conditions are associated with the growth of extraneous blood vessels. These include, e.g., diabetic retinopathy, ischemic retinal-vein occlusion, and retinopathy of prematurity [Aiello et al. New Engl. J. Med. 1994, 331, 1480 ; Peer et al. Lab. Invest. 1995, 72, 638], age-related macular degeneration [AMD; see, Lopez et al. Invest. Opththalmol. Vis. Sci. 1996, 37, 855], neovascular glaucoma, psoriasis, retrolental fibroplasias, angiofibroma, inflammation, rheumatoid arthritis (RA), restenosis, in-stent restenosis, vascular graft restenosis, etc. In addition, the increased blood supply associated with cancerous and neoplastic tissue, encourages growth, leading to rapid tumor enlargement and metastasis. Moreover, the growth of new blood and lymph vessels in a tumor provides an escape route for renegade cells, encouraging metastasis and the consequence spread of the cancer. Thus, compounds of the present invention can be utilized to treat and/or prevent any of the aforementioned angiogenesis disorders, e.g., by inhibiting and/or reducing blood vessel formation; by inhibiting, blocking, reducing, decreasing, etc. endothelial cell proliferation or other types involved in angiogenesis, as well as causing cell death or apoptosis of such cell types.

### Dose and administration

Based upon standard laboratory techniques known to evaluate compounds useful for the treatment of hyper-proliferative disorders and angiogenic disorders, by standard toxicity tests and by standard pharmacological assays for the determination of treatment of the conditions identified above in mammals, and by comparison of these results with the results of known medicaments that are used to treat these conditions, the effective dosage of the compounds of this invention can readily be determined for treatment of each desired indication. The amount of the active ingredient to be administered in the treatment of one of these conditions can vary widely according to such considerations as the particular compound and dosage unit employed, the mode of administration, the period of treatment, the age and sex of the patient treated, and the nature and extent of the condition treated.

The total amount of the active ingredient to be administered will generally range from about 0.001 mg/kg to about 200 mg/kg body weight per day, and preferably from about 0.01 mg/kg to about 20 mg/kg body weight per day. Clinically useful dosing schedules will range from one to three times a day dosing to once every four weeks dosing. In addition, "drug holidays" in which a patient is not dosed with a drug for a certain period of time, may be beneficial to the overall balance between pharmacological effect and tolerability. A unit dosage may contain from about 0.5 mg to about 1500 mg of active ingredient, and can be administered one or more times per day or less than once a day. The average daily dosage for administration by injection, including intravenous, intramuscular, subcutaneous and parenteral injections, and use of infusion techniques will preferably be from 0.01 to 200 mg/kg of total body weight. The average daily rectal dosage regimen will preferably be from 0.01 to 200 mg/kg of total body weight. The average daily vaginal dosage regimen will preferably be from 0.01 to 200 mg/kg of total body weight. The average daily topical dosage regimen will preferably be from 0.1 to 200 mg administered between one to four times daily. The transdermal concentration will preferably be that required to maintain a daily dose of from 0.01 to 200 mg/kg. The average daily inhalation dosage regimen will preferably be from 0.01 to 100 mg/kg of total body weight.

Of course the specific initial and continuing dosage regimen for each patient will vary according to the nature and severity of the condition as determined by the attending diagnostician, the activity of the specific compound employed, the age and general condition of the patient, time of administration, route of administration, rate of excretion of the drug, drug combinations, and the like. The desired mode of treatment and number of doses of a compound of the present invention or a pharmaceutically acceptable salt or ester or composition thereof can be ascertained by those skilled in the art using conventional treatment tests.

Preferably, the diseases of said method are haematological tumours, solid tumour and/or metastases thereof.

The compounds of the present invention can be used in particular in therapy and prevention, i.e. prophylaxis, of tumour growth and metastases, especially in solid tumours of all indications and stages with or without pre-treatment of the tumour growth.

Methods of testing for a particular pharmacological or pharmaceutical property are well known to persons skilled in the art.

The example testing experiments described herein serve to illustrate the present invention and the invention is not limited to the examples given.

### Biological assay: Proliferation Assay

Cultivated tumor cells (MCF7, hormone dependent human mammary carcinoma cells, ATCC HTB22; NCI-H460, human non-small cell lung carcinoma cells, ATCC HTB-177; DU 145, hormone-independent human prostate carcinoma cells, ATCC HTB-81; HeLa-MaTu, human cervical carcinoma cells, EPO-GmbH, Berlin; HeLa-MaTu-ADR, multidrug-resistant human cervical carcinoma cells, EPO-GmbH, Berlin; HeLa human cervical tumor cells, ATCC CCL-2; B16F10 mouse melanoma cells, ATCC CRL-6475) were plated at a density of 5000 cells/well (MCF7, DU145, HeLa-MaTu-ADR), 3000 cells/well (NCI-H460, HeLa-MaTu, HeLa), or 1000 cells/well (B16F10) in a 96-well multititer plate in 200 µl of their respective growth medium supplemented 10% fetal calf serum. After 24 hours, the cells of one plate (zero-point plate) were stained with crystal violet (see below), while the medium of the other plates was replaced by fresh culture medium (200 µl), to which the test substances were added in various concentrations (0 µM, as well as in the range of 0.01-30 µM; the final concentration of the solvent dimethyl sulfoxide was 0.5%). The cells were incubated for 4 days in the presence of test substances. Cell proliferation was determined by staining the cells with crystal violet: the cells were fixed by adding 20 µl/measuring point of an 11 % glutaric aldehyde solution for 15 minutes at room temperature. After three washing cycles of the fixed cells with water, the plates were dried at room temperature. The cells were stained by adding 100 µ/measuring point of a 0.1% crystal violet solution (pH 3.0). After three washing cycles of the stained cells with water, the plates were dried at room temperature. The dye was dissolved by adding 100 µl/measuring point of a 10% acetic acid solution. The extinction was determined by photometry at a wavelength of 595 nm. The change of cell number, in percent, was calculated by normalization of the measured values to the extinction values of the zero-point plate (=0%) and the extinction of the untreated (0 µm) cells (=100%). The IC50 values were determined by means of a 4 parameter fit using an inhouse software.

### Mps-1 kinase assay with 10 µM ATP

The human kinase Mps-1 phosphorylates a biotinylated substrate peptide. Detection of the phosphorylated product is achieved by time-resolved fluorescence resonance energy transfer (TR-FRET) from Europium-labelled anti- phospho-Serine/Threonine antibody as donor to streptavidin labelled with cross-linked allophycocyanin (SA-XLent) as acceptor. Compounds are tested for their inhibition of the kinase activity.

N-terminally GST-tagged human full length recombinant Mps-1 kinase (purchased from Invitrogen, Karslruhe, Germany, cat. no PV4071) was used. As substrate for the kinase reaction a biotinylated peptide of the amino-acid sequence biotin-Ahx-PWDPDDADITEILG (C-terminus in amide form, purchased from Biosynthan GmbH, Berlin) was used.

For the assay 50 nl of a 100-fold concentrated solution of the test compound in DMSO was pipetted into a black low volume 384 well microtiter plate (Greiner Bio-One, Frickenhausen, Germany), 2 µL of a solution of Mps-1 in assay buffer [0.1 mM sodium-ortho-vanadate, 10 mM MgCl₂, 2 mM DTT, 25 mM Hepes pH 7.7, 0.05% BSA (w/v), 0.001% Pluronic F-127] were added and the mixture was incubated for 15 min at 22°C to allow pre-binding of the test compounds to Mps-1 before the start of the kinase reaction. Then the kinase reaction was started by the addition of 3 µL of a solution of 16.7 µM adenosine-triphosphate (ATP, 16.7 µM => final conc. in the 5 µL assay volume is 10 µM) and peptide substrate (1.67 µM => final conc. in the 5 µL assay volume is 1 µM) in assay buffer and the resulting mixture was incubated for a reaction time of 60 min at 22°C. The concentration of Mps-1 in the assay was adjusted to the activity of the enzyme lot and was chosen appropriate to have the assay in the linear range, typical enzyme concentrations were in the range of about 0.5 nM (final conc. in the 5 µL assay volume). The reaction was stopped by the addition of 5 µL of a solution of TR-FRET detection reagents (100 mM Hepes pH 7.4, 0.1% BSA, 40 mM EDTA, 140 nM Streptavidin-XLent [# 61GSTXLB, Fa. Cis Biointernational, Marcoule, France], 1.5 nM anti-phospho(Ser/Thr)-Europium- antibody [#AD0180, PerkinElmer LAS, Rodgau-Jügesheim, Germany]. Instead of the 1.5 nM anti-phospho(Ser/Thr)-Europium-antibody a mixture of 2 nM unlabeled anti-phospho ser/thr-pro antibody MPM-2 [Millipore cat. # 05-368] and 1 nM LANCE EU-W1024 labeled anti-mouse IgG antibody [Perkin-Elmer, product no. AD0077] can be used).

The resulting mixture was incubated 1 h at 22°C to allow the binding of the phosphorylated peptide to the anti-phospho(Ser/Thr)-Europium-antibody. Subsequently the amount of phosphorylated substrate was evaluated by measurement of the resonance energy transfer from the Europium-labelled anti-phospho(Ser/Thr) antibody to the Streptavidin-XLent. Therefore, the fluorescence emissions at 620 nm and 665 nm after excitation at 350 nm was measured in a Viewlux TR-FRET reader (PerkinElmer LAS, Rodgau-Jugesheim, Germany). The "blank-corrected normalized ratio" (a Viewlux specific readout, similar to the traditional ratio of the emissions at 665 nm and at 622 nm, in which blank and Eu-donor crosstalk are subtracted from the 665 nm signal before the ratio is calculated) was taken as the measure for the amount of phosphorylated substrate. The data were normalised (enzyme reaction without inhibitor = 0 % inhibition, all other assay components but no enzyme = 100 % inhibition). Test compounds were tested on the same microtiter plate at 10 different concentrations in the range of 20 µM to 1 nM (20 µM, 6.7 µM, 2.2 µM, 0.74 µM, 0.25 µM, 82 nM, 27 nM, 9.2 nM, 3.1 nM and 1 nM, dilution series prepared before the assay at the level of the 100fold conc. stock solutions by serial 1:3 dilutions) in duplicate values for each concentration and IC₅₀ values were calculated by a 4 parameter fit using an inhouse software.

### Mps-1 kinase assay with 2 mM ATP

The human kinase Mps-1 phosphorylates a biotinylated substrate peptide. Detection of the phosphorylated product is achieved by time-resolved fluorescence resonance energy transfer (TR-FRET) from Europium-labelled anti-phospho-Serine/Threonine antibody as donor to streptavidin labelled with cross-linked allophycocyanin (SA-XLent) as acceptor. Compounds are tested for their inhibition of the kinase activity.

N-terminally GST-tagged human full length recombinant Mps-1 kinase (purchased from Invitrogen, Karslruhe, Germany, cat. no PV4071) was used. As substrate for the kinase reaction a biotinylated peptide of the amino-acid sequence biotin-Ahx-PWDPDDADITEILG (C-terminus in amide form, purchased from Biosynthan GmbH, Berlin) was used.

For the assay 50 nl of a 100-fold concentrated solution of the test compound in DMSO was pipetted into a black low volume 384well microtiter plate (Greiner Bio-One, Frickenhausen, Germany), 2 µL of a solution of Mps-1 in assay buffer [0.1 mM sodium-ortho-vanadate, 10 mM MgCl₂, 2 mM DTT, 25 mM Hepes pH 7.7, 0.05% BSA (w/v), 0.001% Pluronic F-127] were added and the mixture was incubated for 15 min at 22°C to allow pre-binding of the test compounds to Mps-1 before the start of the kinase reaction. Then the kinase reaction was started by the addition of 3 µL of a solution of 3.33 mM adenosine-triphosphate (ATP, 3.3 mM => final conc. in the 5 µL assay volume is 2 mM) and peptide substrate (1.67 µM => final conc. in the 5 µL assay volume is 1 µM) in assay buffer and the resulting mixture was incubated for a reaction time of 60 min at 22°C. The concentration of Mps-1 in the assay was adjusted to the activity of the enzyme lot and was chosen appropriate to have the assay in the linear range, typical enzyme concentrations were in the range of about 0.5 nM (final conc. in the 5 µL assay volume). The reaction was stopped by the addition of 5 µL of a solution of TR-FRET detection reagents (100 mM Hepes pH 7.4, 0.1% BSA, 40 mM EDTA, 140 nM Streptavidin-XLent [# 61 GSTXLB, Fa. Cis Biointernational, Marcoule, France], 1.5 nM anti-phospho(Ser/Thr)-Europium- antibody [#AD0180, PerkinElmer LAS, Rodgau-Jügesheim, Germany]. Instead of the 1.5 nM anti-phospho(Ser/Thr)-Europium-antibody a mixture of 2 nM unlabeled anti-phospho ser/thr-pro antibody MPM-2 [Millipore cat. # 05-368] and 1 nM LANCE EU-W1024 labeled anti-mouse IgG antibody [Perkin-Elmer, product no. AD0077] can be used).

The resulting mixture was incubated 1 h at 22°C to allow the binding of the phosphorylated peptide to the anti-phospho(Ser/Thr)-Europium-antibody. Subsequently the amount of phosphorylated substrate was evaluated by measurement of the resonance energy transfer from the Europium-labelled anti-phospho(Ser/Thr) antibody to the Streptavidin-XLent. Therefore, the fluorescence emissions at 620 nm and 665 nm after excitation at 350 nm was measured in a Viewlux TR-FRET reader (PerkinElmer LAS, Rodgau-Jügesheim, Germany). The "blank-corrected normalized ratio" ( a Viewlux specific readout, similar to the traditional ratio of the emissions at 665 nm and at 622 nm, in which blank and Eu-donor crosstalk are subtracted from the 665 nm signal before the ratio is calculated) was taken as the measure for the amount of phosphorylated substrate. The data were normalised (enzyme reaction without inhibitor = 0 % inhibition, all other assay components but no enzyme = 100 % inhibition). Test compounds were tested on the same microtiter plate at 10 different concentrations in the range of 20 µM to 1 nM (20 µM, 6.7 µM, 2.2 µM, 0.74 µM, 0.25 µM, 82 nM, 27 nM, 9.2 nM, 3.1 nM and 1 nM, dilution series prepared before the assay at the level of the 100fold conc. stock solutions by serial 1:3 dilutions) in duplicate values for each concentration and IC₅₀ values were calculated by a 4 parameter fit using an inhouse software.

| Example Number | Mps-1 Inhibition; Assay with 10 µM ATP; IC₅₀ in nM | Mps-1 Inhibition; Assay with 2 mM ATP; IC₅₀ in nM |
|---|---|---|
| Example01.1 | ≤ 1.0 | 1.2 |
| Example01.2 | ≤ 1.0 | 4.1 |
| Example01.3 | ≤ 1.0 | 1.1 |
| Example01.4 | ≤ 1.0 | ≤ 1.0 |
| Example01.5 | ≤ 1.0 | ≤ 1.0 |
| Example01.6 | ≤ 1.0 | ≤ 1.0 |
| Example01.7 | ≤ 1.0 | ≤ 1.0 |
| Example01.8 | ≤ 1.0 | ≤ 1.0 |
| Example01.9 | ≤ 1.0 | ≤ 1.0 |
| Example01.10 | ≤ 1.0 | ≤ 1.0 |
| Example01.11 | ≤ 1.0 | ≤ 1.0 |
| Example01.12 | ≤ 1.0 | ≤ 1.0 |
| Example01.13 | ≤ 1.0 | ≤ 1.0 |
| Example01.14 | ≤ 1.0 | ≤ 1.0 |
| Example01.15 | ≤ 1.0 | ≤ 1.0 |
| Example01.16 | ≤ 1.0 | ≤ 1.0 |
| Example01.17 | ≤ 1.0 | ≤ 1.0 |
| Example01.18 | ≤ 1.0 | ≤ 1.0 |
| Example01.19 | ≤ 1.0 | ≤ 1.0 |
| Example01.20 | ≤ 1.0 | ≤ 1.0 |
| Example01.21 | ≤ 1.0 | ≤ 1.0 |
| Example01.22 | ≤ 1.0 | ≤ 1.0 |
| Example01.23 | ≤ 1.0 | 1.1 |
| Example01.24 | ≤ 1.0 | 1.2 |
| Example01.25 | ≤ 1.0 | 1.3 |
| Example01.26 | ≤ 1.0 | 21 |
| Example01.27 | ≤ 1.0 | ≤ 1.0 |
| Example01.28 | ≤ 1.0 | ≤ 1.0 |
| Example01.29 | ≤ 1.0 | ≤ 1.0 |
| Example01.30 | ≤ 1.0 | ≤ 1.0 |
| Example01.31 | ≤ 1.0 | 5.0 |
| Example01.32 | ≤ 1.0 | ≤ 1.0 |
| Example01.33 | ≤ 1.0 | 1.3 |
| Example01.34 | ≤ 1.0 | ≤ 1.0 |
| Example01.35 | ≤ 1.0 | 1.2 |
| Example01.36 | ≤ 1.0 | ≤ 1.0 |
| Example01.37 | ≤ 1.0 | 1.6 |
| Example02.1 | ≤ 1.0 | 1.1 |
| Example02.2 | ≤ 1.0 | ≤ 1.0 |
| Example02.3 | ≤ 1.0 | ≤ 1.0 |
| Example02.4 | ≤ 1.0 | ≤ 1.0 |
| Example02.5 | ≤ 1.0 | 1.8 |
| Example02.6 | ≤ 1.0 | 4.3 |
| Example02.7 | ≤ 1.0 | ≤ 1.0 |
| Example02.8 | ≤ 1.0 | 1.5 |
| Example02.9 | ≤ 1.0 | 2.2 |
| Example02.10 | ≤ 1.0 | 2.3 |
| Example02.11 | ≤ 1.0 | ≤ 1.0 |
| Example02.12 | ≤ 1.0 | ≤ 1.0 |
| Example02.13 | ≤ 1.0 | ≤ 1.0 |
| Example02.14 | ≤ 1.0 | 2.4 |
| Example02.15 | ≤ 1.0 | ≤ 1.0 |
| Example02.16 | ≤ 1.0 | 3.8 |
| Example02.17 | ≤ 1.0 | 4.6 |
| Example02.18 | ≤ 1.0 | ≤ 1.0 |
| Example02.19 | ≤ 1.0 | ≤ 1.0 |
| Example02.20 | ≤ 1.0 | 1.7 |
| Example02.21 | ≤ 1.0 | 2.4 |
| Example02.22 | ≤ 1.0 | ≤ 1.0 |
| Example02.23 | ≤ 1.0 | ≤ 1.0 |
| Example02.24 | ≤ 1.0 | 5.9 |
| Example02.25 | ≤ 1.0 | 3.3 |
| Example02.26 | ≤ 1.0 | 6.1 |
| Example02.27 | ≤ 1.0 | ≤ 1.0 |
| Example02.28 | ≤ 1.0 | 3.5 |
| Example02.29 | ≤ 1.0 | 7.5 |
| Example03.1 | ≤ 1.0 | 6.9 |
| Example04.1 | ≤ 1.0 | 16 |
| Example04.2 | ≤ 1.0 | 15 |
| Example05.1 | ≤ 1.0 | 29 |
| Example06.1 | ≤ 1.0 | 29 |
| Example06.2 | 1.4 | 26 |
| Example06.3 | ≤ 1.0 | 21 |
| Example06.4 | ≤ 1.0 | 11 |
| Example06.5 | ≤ 1.0 | 19 |
| Example07.1 | ≤ 1.0 | 14 |
| Example08.1 | ≤ 1.0 | 4.3 |
| Example09.1 | ≤ 1.0 | 19 |
| Example10.01 | ≤ 1.0 | 17 |
| Example11.01 | ≤ 1.0 | 11 |
| Example12.01 | ≤ 1.0 | ≤ 1.0 |
| Example12.02 | ≤ 1.0 | ≤ 1.0 |
| Example12.03 | ≤ 1.0 | ≤ 1.0 |
| Example12.04 | ≤ 1.0 | ≤ 1.0 |
| Example12.05 | ≤ 1.0 | ≤ 1.0 |
| Example12.06 | ≤ 1.0 | ≤ 1.0 |
| Example12.07 | ≤ 1.0 | ≤ 1.0 |
| Example12.08 | ≤ 1.0 | ≤ 1.0 |
| Example12.09 | ≤ 1.0 | 2.5 |
| Example12.10 | ≤ 1.0 | ≤ 1.0 |
| Example12.11 | ≤ 1.0 | 1.2 |
| Example12.12 | ≤ 1.0 | 13 |
| Example12.13 | ≤ 1.0 | ≤ 1.0 |
| Example12.14 | ≤ 1.0 | 8.5 |
| Example12.15 | ≤ 1.0 | 1.6 |
| Example12.16 | ≤ 1.0 | ≤ 1.0 |
| Example12.17 | ≤ 1.0 | ≤ 1.0 |
| Example12.18 | ≤ 1.0 | 1.5 |
| Example12.19 | ≤ 1.0 | ≤ 1.0 |
| Example12.20 | ≤ 1.0 | ≤ 1.0 |
| Example12.21 | ≤ 1.0 | ≤ 1.0 |
| Example12.22 | ≤ 1.0 | ≤ 1.0 |
| Example12.23 | ≤ 1.0 | ≤ 1.0 |
| Example12.24 | ≤ 1.0 | ≤ 1.0 |
| Example12.25 | ≤ 1.0 | 2.7 |
| Example12.26 | ≤ 1.0 | 1.2 |
| Example12.27 | ≤ 1.0 | 9.0 |
| Example12.28 | ≤ 1.0 | ≤ 1.0 |
| Example12.29 | ≤ 1.0 | 2.3 |
| Example12.30 | ≤ 1.0 | 4.3 |
| Example12.31 | ≤ 1.0 | 3.8 |
| Example12.32 | ≤ 1.0 | 2.2 |
| Example12.33 | ≤ 1.0 | 1.4 |
| Example12.34 | ≤ 1.0 | 6.6 |
| Example12.35 | ≤ 1.0 | 2.1 |
| Example12.36 | ≤ 1.0 | 3.5 |
| Example12.37 | ≤ 1.0 | 13 |
| Example12.38 | ≤ 1.0 | 29 |
| Example12.39 | ≤ 1.0 | 16 |
| Example12.40 | ≤ 1.0 | 9.9 |
| Example12.41 | ≤ 1.0 | 7.2 |
| Example12.42 | ≤ 1.0 | 12 |
| Example12.43 | ≤ 1.0 | ≤ 1.0 |
| Example12.44 | ≤ 1.0 | 1.6 |
| Example12.45 | ≤ 1.0 | 1.4 |
| Example12.46 | ≤ 1.0 | 3.5 |
| Example12.47 | ≤ 1.0 | 2.4 |
| Example12.48 | ≤ 1.0 | 1.3 |
| Example12.49 | ≤ 1.0 | 3.4 |
| Example12.50 | ≤ 1.0 | 3.1 |
| Example12.51 | ≤ 1.0 | 2.6 |
| Example12.52 | ≤ 1.0 | 14 |
| Example12.53 | ≤ 1.0 | 6.9 |
| Example12.54 | ≤ 1.0 | 1.1 |
| Example12.55 | ≤ 1.0 | ≤ 1.0 |
| Example12.56 | ≤ 1.0 | ≤ 1.0 |
| Example12.57 | ≤ 1.0 | ≤ 1.0 |
| Example12.58 | ≤ 1.0 | ≤ 1.0 |
| Example12.59 | ≤ 1.0 | ≤ 1.0 |
| Example12.60 | ≤ 1.0 | 5.0 |
| Example12.61 | ≤ 1.0 | 6.1 |
| Example12.62 | ≤ 1.0 | 3.5 |
| Example12.63 | ≤ 1.0 | 26 |
| Example12.64 | ≤ 1.0 | 1.6 |
| Example12.65 | ≤ 1.0 | ≤ 1.0 |
| Example12.66 | ≤ 1.0 | 2.0 |
| Example12.67 | ≤ 1.0 | 5.0 |
| Example12.68 | ≤ 1.0 | 1.9 |
| Example12.69 | ≤ 1.0 | 4.5 |
| Example12.70 | ≤ 1.0 | 9.5 |
| Example12.71 | ≤ 1.0 | 8.8 |
| Example12.72 | ≤ 1.0 | 16 |
| Example12.73 | ≤ 1.0 | 16 |
| Example12.74 | ≤ 1.0 | 16 |
| Example12.75 | ≤ 1.0 | 5.1 |
| Example12.76 | ≤ 1.0 | 7.4 |
| Example12.77 | ≤ 1.0 | 11 |
| Example12.78 | ≤ 1.0 | 7.6 |
| Example12.79 | ≤ 1.0 | 23 |
| Example12.80 | ≤ 1.0 | 18 |
| Example12.81 | ≤ 1.0 | 7.3 |
| Example12.82 | ≤ 1.0 | ≤ 1.0 |
| Example12.83 | ≤ 1.0 | 1.8 |
| Example12.84 | ≤ 1.0 | ≤ 1.0 |
| Example12.85 | ≤ 1.0 | 2.2 |

### Spindle Assembly Checkpoint Assay

The spindle assembly checkpoint assures the proper segregation of chromosomes during mitosis. Upon entry into mitosis, chromosomes begin to condensate which is accompanied by the phosphorylation of histone H3 on serine 10. Dephosphorylation of histone H3 on serine 10 begins in anaphase and ends at early telophase. Accordingly, phosphorylation of histone H3 on serine 10 can be utilized as a marker of cells in mitosis. Nocodazole is a microtubule destabilizing substance. Thus, nocodazole interferes with microtubule dynamics and mobilises the spindle assembly checkpoint. The cells arrest in mitosis at G2/M transition and exhibit phosphorylated histone H3 on serine 10. An inhibition of the spindle assembly checkpoint by Mps-1 inhibitors overrides the mitotic blockage in the presence of nocodazole, and the cells complete mitosis prematurely. This alteration is detected by the decrease of cells with phosphorylation of histone H3 on serine 10. This decline is used as a marker to determine the capability of compounds of the present invention to induce a mitotic breakthrough.

Cultivated cells of the human cervical tumor cell line HeLa (ATCC CCL-2) were plated at a density of 2500 cells/well in a 384-well microtiter plate in 20 µl Dulbeco's Medium (w/o phenol red, w/o sodium pyruvate, w 1000 mg/mL glucose, w pyridoxine) supplemented with 1% (v/v) glutamine, 1% (v/v) penicillin, 1% (v/v) streptomycin and 10% (v/v) fetal calf serum. After incubation overnight at 37°C, 10 µL/well nocodazole at a final concentration of 0.1 µg/mL were added to cells. After 24 h incubation, cells were arrested at G2/M phase of the cell cycle progression. Test compounds solubilised in dimethyl sulfoxide (DMSO) were added at various concentrations (0 µM, as well as in the range of 0.005 µM - 10 µM; the final concentration of the solvent DMSO was 0.5% (v/v)). Cells were incubated for 4 h at 37°C in the presence of test compounds. Thereafter, cells were fixed in 4% (v/v) paraformaldehyde in phosphate buffered saline (PBS) at 4°C overnight then permeabilised in 0.1% (v/v) Triton X™ 100 in PBS at room temperature for 20 min and blocked in 0.5% (v/v) bovine serum albumin (BSA) in PBS at room temperature for 15 min. After washing with PBS, 20 µL/well antibody solution (anti-phospho-histone H3 clone 3H10, FITC; Upstate, Cat# 16-222; 1:200 dilution) was added to cells, which were incubated for 2 h at room temperature. Afterwards, cells were washed with PBS and 20 µL/well HOECHST 33342 dye solution (5 µg/mL) was added to cells and cells were incubated 12 min at room temperature in the dark. Cells were washed twice with PBS then covered with PBS and stored at 4°C until analysis. Images were acquired with a Perkin Elmer OPERA™ High-Content Analysis reader. Images were analyzed with image analysis software MetaXpress™ from Molecular devices utilizing the Cell Cycle application module. In this assay both labels HOECHST 33342 and phosphorylated Histone H3 on serine 10 were measured. HOECHST 33342 labels DNA and is used to count cell number. The staining of phosphorylated Histone H3 on serine 10 determines the number of mitotic cells. Inhibition of Mps-1 decreases the number of mitotic cells in the presence of nocodazole indicating an inappropriate mitotic progression. The raw assay data were further analysed by four parameter logistic regression analysis to determine the IC₅₀ value for each tested compound.

It will be apparent to persons skilled in the art that assays for other Mps kinases may be performed in analogy using the appropriate reagents.

Thus the compounds of the present invention effectively inhibit one or more Mps-1 kinases and are therefore suitable for the treatment or prophylaxis of diseases of uncontrolled cell growth, proliferation and/or survival, inappropriate cellular immune responses, or inappropriate cellular inflammatory responses, particularly in which the uncontrolled cell growth, proliferation and/or survival, inappropriate cellular immune responses, or inappropriate cellular inflammatory responses is mediated by Mps-1, more particularly in which the diseases of uncontrolled cell growth, proliferation and/or survival, inappropriate cellular immune responses, or inappropriate cellular inflammatory responses are haemotological tumours, solid tumours and/or metastases thereof, e.g. leukaemias and myelodysplastic syndrome, malignant lymphomas, head and neck tumours including brain tumours and brain metastases, tumours of the thorax including non-small cell and small cell lung tumours, gastrointestinal tumours, endocrine tumours, mammary and other gynaecological tumours, urological tumours including renal, bladder and prostate tumours, skin tumours, and sarcomas, and/or metastases thereof.

### Hydrolytic stability Assay

The hydrolytic stability assay investigates the stability of a compound in an aqueous buffer system. Standard solution stability assay is run in 0.05 M Phosphate buffer at pH 7.4 (pH of blood plasma) at 37°C. As compounds in the GI tract are exposed to a wide variety of pHs any relevant pH (as pH 2 to simulate the acidic condition of the GI tract in the following experiment) can be chosen. Compounds are incubated in relevant solution at 37°C and analyzed by HPLC immediately after incubation and after 1, 2 and 24 hrs. Degredation rate (decay in %) is calculated by relating peak areas after 1, 2 and 24 hrs to the time zero injection.

Compound is available as 10 mM in DMSO (solution 1). 2.5 µL of solution 1 is dissolved in 1 mL acetonitrile leading to solution 2. Poorly soluble compounds may demand another dilution step of solution 2 (1:5 and 1:10 respectively) in Acetonitrile). Solution 2 is incubated at 37°C in a tempered HPLC autosampler. 1 mL buffer pH 2 is transferred into an HPLC vial. 100 µL of solution 2 is added to the buffer pH 2 solution and mixed thoroughly. Immediately after mixing the combined solution is injected into the HPLC to give the time zero injection. Injections are repeated after 1, 2 and 24 hrs.

The degredation rate (decay in %) is calculated using HPLC software Millennium and Excel respectively.

| Example Number | Hydrolysis at pH 2; Decay in % after 24 h |
|---|---|
| Example01.1 | < 10 % |
| Example01.2 | < 10 % |
| Example01.3 | < 10 % |
| Example01.4 | < 10 % |
| Example01.5 | < 10 % |
| Example01.6 | < 10 % |
| Example01.7 | < 10 % |
| Example01.8 | < 10 % |
| Example01.9 | < 10 % |
| Example01.10 | < 10 % |
| Example01.11 | < 10 % |
| Example01.12 | < 10 % |
| Example01.13 | < 10 % |
| Example01.14 | < 10 % |
| Example01.15 | < 10 % |
| Example01.16 | < 10 % |
| Example01.17 | < 10 % |
| Example01.18 | < 10 % |
| Example01.19 | < 10 % |
| Example01.20 | < 10 % |
| Example01.21 | < 10 % |
| Example01.22 | < 10 % |
| Example01.23 | < 10 % |
| Example01.24 | < 10 % |
| Example01.25 | < 10 % |
| Example01.26 | < 10 % |
| Example01.27 | < 10 % |
| Example01.28 | < 10 % |
| Example01.29 | < 10 % |
| Example02.1 | < 10 % |
| Example02.2 | < 10 % |
| Example02.3 | < 10 % |
| Example02.4 | < 10 % |
| Example02.5 | < 10 % |
| Example02.6 | < 10 % |
| Example02.7 | < 10 % |
| Example02.8 | < 10 % |
| Example02.9 | < 10 % |
| Example02.10 | < 10 % |
| Example02.11 | < 10 % |
| Example02.12 | < 10 % |
| Example02.13 | < 10 % |
| Example02.14 | < 10 % |
| Example02.15 | < 10 % |
| Example02.16 | < 10 % |
| Example02.17 | < 10 % |
| Example02.18 | < 10 % |
| Example02.19 | < 10 % |
| Example02.20 | < 10 % |
| Example02.21 | < 10 % |
| Example02.22 | < 10 % |
| Example02.23 | < 10 % |
| Example02.24 | < 10 % |
| Example03.1 | < 10 % |
| Example04.1 | < 10 % |
| Example05.1 | < 10 % |
| Example06.1 | < 10 % |
| Example06.2 | < 10 % |
| Example06.3 | < 10 % |
| Example06.4 | < 10 % |
| Example06.5 | < 10 % |
| Example07.1 | < 10 % |
| Example08.1 | < 10 % |

**Comparison with compounds specified in** WO2011/063908 **Mps-1 Inhibition in Inhibition Assay with 10 µM ATP:**

| Structure | No. | Mps-1 Inhibition; Assay with 10 µM ATP; IC₅₀ in nM |
|---|---|---|
| 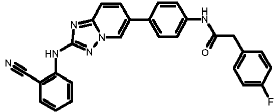 | 1 | ≤ 1.0 |
| 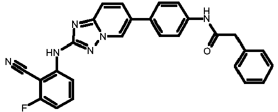 | 2 | ≤ 1.0 |
| 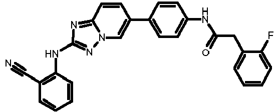 | 3 | ≤ 1.0 |
| 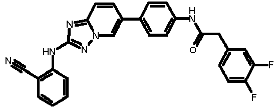 | 4 | ≤ 1.0 |
| 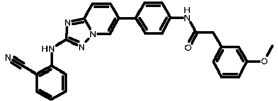 | 5 | ≤ 1.0 |
| 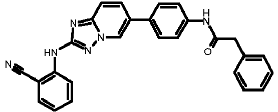 | 6 | ≤ 1.0 |
| 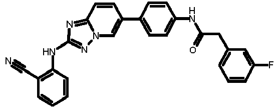 | 7 | 1.1 |
| 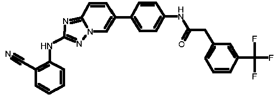 | 8 | 1.4 |
| 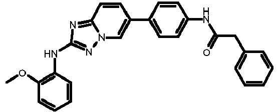 | 9 | 1.6 |
| 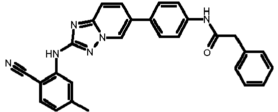 | 10 | 2.1 |

Comparison with compounds specified in WO2011/063908 Mps-1 Inhibition in Inhibition Assay with 2 mM ATP:

| Structure | No. | Mps-1 Inhibition; Assay with 2 mM ATP; IC₅₀ in nM |
|---|---|---|
| 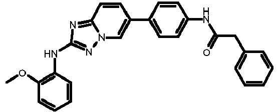 | 9 | 55 |

**Comparison with compounds specified in** WO2011/063908 **Hydrolytic stability:**

| Structure | No. | Hydrolysis at pH 2; Decay in % after 24 h |
|---|---|---|
| 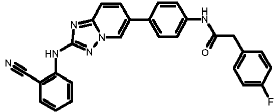 | 1 | ≥ 90% |
| 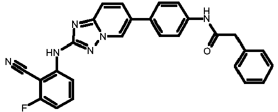 | 2 | ≥ 20% |
| 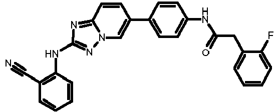 | 3 | ≥ 70% |
| 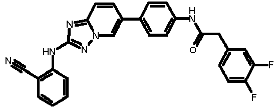 | 4 | ≥ 70% |
| 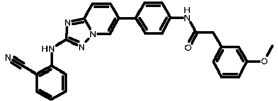 | 5 | ≥ 70% |
| 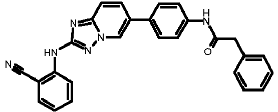 | 6 | ≥ 90% |
| 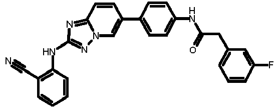 | 7 | ≥ 70% |
| 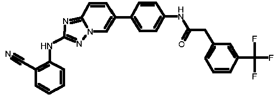 | 8 | ≥ 70% |
| 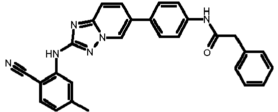 | 10 | ≥ 70 % |

The half maximal inhibitory concentration (IC₅₀) of the most potent compounds specified in WO2011/063908, determined in an Mps-1 kinase assay with a concentration of 10 µM ATP, was lower than 2 nM (more potent than 2 nM). However, all these compounds show either an IC₅₀ higher than 30 nM (less potent than 30 nM) in an Mps-1 kinase assay with a concentration of 2 mM ATP, or they show a low hydrolytic stability at pH 2 with more than 15 % decay after 24 h.

The compounds of the present invention are characterized by
- an IC₅₀ lower than 2 nM (more potent than 2 nM) in an Mps-1 kinase assay with a concentration of 10 µM ATP, and
- an IC₅₀ lower than 30 nM (more potent than 30 nM) in an Mps-1 kinase assay with a concentration of 2 mM ATP, and
- a high hydrolytic stability, with less than 10 % decay after 24 h at pH 2.

## Claims

1. A compound of general formula (I) : 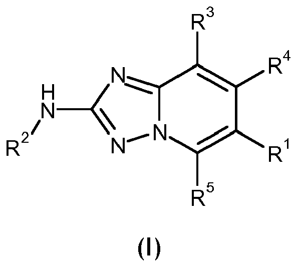 in which:
R¹ represents a phenyl or a pyridyl group
- which is substituted, one or more times, identically or differently, with a substituent selected from:
R⁶-(C₁-C₆-alkoxy)-, R⁶-O-, -C(=O)R⁶, -C(=O)O-R⁶, -N(H)C(=O)R⁶, -N(H)C(=O)NR⁶R⁷, -NR⁶R⁷, -C(=O)N(H)R⁶, -C(=O)NR⁶R⁷, R⁶-S-, R⁶-S(=O)₂-, -N(H)S(=O)₂R⁶, -S(=O)₂N(H)R⁶; and
- which is optionally substituted, one or more times, identically or differently, with a substituent selected from:
halo-, hydroxy-, nitro-, C₁-C₆-alkyl-, C₁-C₆-alkoxy-, hydroxy-C₁-C₆-alkyl-, -N(H)C(=O)R⁸, -N(H)C(=O)NR⁸R⁷, -C(=O)N(H)R⁸, -N(H)S(=O)₂R⁸;
R² represents a 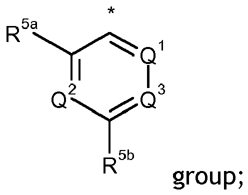 wherein * indicates the point of attachment of said group with the rest of the molecule;
Q¹ represents a group selected from: N, CH, C-(C₁-C₆-alkyl),
C-(C₁-C₆-alkoxy), C-halo;
Q² represents a group selected from: N, CH, CR^{5b};
Q³ represents a group selected from: N, CH, CR^{5b};
R^{5a} represents a group selected from:
halo-, nitro-, C₁-C₆-alkyl-, halo-C₁-C₆-alkyl-, C₁-C₆-alkoxy-, halo-C₁-C₆-alkoxy-, hydroxy-C₁-C₆-alkyl-, C₁-C₆-alkoxy-C₁-C₆-alkyl-, halo-C₁-C₆-alkoxy-C₁-C₆-alkyl-, R⁸-(C₁-C₆-alkoxy)-, R⁸-O-, -NR⁸R⁷, R⁸-S-, R⁸-S(=O)-, R⁸-S(=O)₂-, (C₃-C₆-cycloalkyl)-(CH₂)ₙ-O-;
R^{5b} represents a group selected from:
halo-, hydroxy-, cyano-, nitro-, C₁-C₆-alkyl-, halo-C₁-C₆-alkyl-, C₁-C₆-alkoxy-, halo-C₁-C₆-alkoxy-, hydroxy-C₁-C₆-alkyl-, C₁-C₆-alkoxy-C₁-C₆-alkyl-, halo-C₁-C₆-alkoxy-C₁-C₆-alkyl-, R⁸-(C₁-C₆-alkyl)-, R⁸-(CH₂)ₙ(CHOH)(CH₂)ₘ-, R⁸-(C₁-C₆-alkoxy)-, R⁸-(CH₂)ₙ(CHOH)(CH₂)ₚ-O-, R⁸-(C₁-C₆-alkoxy-C₁-C₆-alkyl)-, R⁸-(C₁-C₆-alkoxy-C₁-C₆-alkyl)-O-, -O-(CH₂)ₙ-C(=O)NR⁸R⁷, R⁸-O-, -C(=O)R⁸, -C(=O)O-R⁸, -OC(=O)-R⁸, -N(H)C(=O)R⁸, -N(R⁷)C(=O)R⁸, -N(H)C(=O)NR⁸R⁷, -N(R⁷)C(=O)NR⁸R⁷, -NR⁸R⁷, -NR⁷R⁷, -C(=O)N(H)R⁸, -C(0=ONR⁸R⁷, R⁸-S-, R⁸-S(=O)-, R⁸-S(=O)²-, -N(H)S(=O)R⁸, -N(R⁷)S(=O)R⁸, -S(=O)N(H)R⁸, -S(=O)NR⁸R⁷, -N(H)S(=O)₂R⁸, -N(R⁷)S(=O)²R⁸, -S(=O)₂N(H)R⁸, -S(=O)₂NR⁸R⁷, -S(=O)(=NR⁸)R⁷, -S(=O)(=NR⁷)R⁸, -N=S(=O)(R⁸)R⁷;
R³ represents a hydrogen atom, a halogen atom, a hydroxy-, amino-, cyano-, nitro-, C₁-C₄-alkyl-, halo-C₁-C₄-alkyl-, C₁-C₄-alkoxy-, halo-C₁-C₄-alkoxy-, hydroxy-C₁-C₄-alkyl-, C₁-C₄-alkoxy-C₁-C₄-alkyl-, halo-C₁-C₄-alkoxy-C₁-C₄-alkyl-, C₂-C₆-alkenyl-, C₂-C₆-alkynyl-, halo-C₂-C₆-alkenyl-, halo-C₂-C₆-alkynyl-, C₃-C₆-cycloalkyl-, or halo-C₃-C₆-cycloalkyl- group;
R⁴ represents a hydrogen atom, a halogen atom, a hydroxy-, amino-, cyano-, nitro-, C₁-C₄-alkyl-, halo-C₁-C₄-alkyl-, C₁-C₄-alkoxy-, halo-C₁-C₄-alkoxy-, hydroxy-C₁-C₄-alkyl-, C₁-C₄-alkoxy-C₁-C₄-alkyl-, halo-C₁-C₄-alkoxy-C₁-C₄-alkyl-, C₂-C₆-alkenyl-, C₂-C₆-alkynyl-, halo-C₂-C₆-alkenyl-, halo-C₂-C₆-alkynyl-, C₃-C₆-cycloalkyl-, or halo-C₃-C₆-cycloalkyl- group;
R⁵ represents a hydrogen atom;
R⁶ represents a group selected from C₃-C₆-cycloalkyl-, 3- to 10-membered heterocyclyl-, aryl-, heteroaryl-, -(CH₂)_{q}-(C₃-C₆-cycloalkyl), -(CH₂)_{q}-(3- to 10-membered heterocyclyl), -(CH₂)_{q}-aryl, or -(CH₂)_{q}-heteroaryl,
wherein said group being optionally substituted, one or more times, identically or differently, with a substituent selected from: halo-, hydroxy-, cyano-, nitro-, C₁-C₆-alkyl-, halo-C₁-C₆-alkyl-, C₁-C₆-alkoxy-, halo-C₁-C₆-alkoxy-, hydroxy-C₁-C₆-alkyl-, C₁-C₆-alkoxy-C₁-C₆-alkyl-, halo-C₁-C₆-alkoxy-C₁-C₆-alkyl-, R⁸-(C₁-C₆-alkyl)-, R⁸-(CH₂)ₙ(CHOH)(CH₂)ₘ-, R⁸-(C₁-C₆-alkoxy)-, R⁸-(CH₂)ₙ(CHOH)(CH₂)ₚ-O-, R⁸-(C₁-C₆-alkoxy-C₁-C₆-alkyl)-, R⁸-(C₁-C₆-alkoxy-C₁-C₆-alkyl)-O-, aryl-, R⁸-O-, -C(=O)R⁸, -C(=O)O-R⁸, -OC(=O)-R⁸, -N(H)C(=O)R⁸, -N(R⁷)C(=O)R⁸, -N(H)C(=O)NR⁸R⁷, -N(R7)C(=O)NR⁸R⁷, -NR⁸R⁷, -C(=O)N(H)R⁸, -C(=O)NR⁸R⁷, R⁸-S-, R⁸-S(=O)-, R⁸-S(=O)₂-, -N(H)S(=O)R⁸, -N(R⁷)S(=O)R⁸, -S(=O)N(H)R⁸, -S(=O)NR⁸R⁷, -N(H)S(=O)₂R⁸, -N(R⁷)S(=O)²R⁸, -S(=O)₂N(H)R⁸, -S(=O)₂NR⁸R⁷, -S(=O)(=NR⁸)R⁷,- S(=O)(=NR⁷)R⁸, -N=S(=O)(R⁸)R⁷;
R⁷ represents a hydrogen atom, a C₁-C₆-alkyl- or a C₃-C₆-cycloalkyl- group;
R⁸ represents a hydrogen atom or a C₁-C₆-alkyl- or C₃-C₆-cycloalkyl- group, wherein said C₁-C₆-alkyl- or C₃-C₆-cycloalkyl- group is optionally substituted, one or more times, identically or differently, with a substituent selected from:
halo-, hydroxy-, -NHR⁷, -NR⁷R⁷, -N(C₁-C₃-alkyl)-C(=O)R⁷,
-N(C₁-C₃-alkyl)-C(=O)OR⁷, C₁-C₃-alkyl-, R⁷-S(=O)₂-, C₁-C₃-alkoxy-, halo-C₁-C₃-alkoxy- ;
n, m, p, represent, independently from each other, an integer of 0, 1, 2 or 3;
and
q represents an integer of 0, 1, 2 or 3;
or a stereoisomer, a tautomer, an N-oxide, a hydrate, a solvate, or a salt thereof, or a mixture of same.

2. The compound according to claim 1, wherein:
R¹ represents a phenyl group
- which is substituted, one or more times, identically or differently, with a substituent selected from:
R⁶-(C₁-C₆-alkoxy)-, R⁶-O-, -N(H)C(=O)R⁶, -N(H)C(=O)NR⁷, -C(=O)N(H)R⁶, -C(=O)NR⁶R⁷; and
- which is optionally substituted, one or more times, identically or differently, with a substituent selected from:
halo-, C₁-C₆-alkyl-, C₁-C₆-alkoxy-, -N(H)C(=O)R⁸, -C(=O)N(H)R⁸;
R³ represents a hydrogen atom, halo-, hydroxy-, C₁-C₄-alkyl-, halo-C₁-C₄-alkyl-, or C₁-C₄-alkoxy- group;
R⁴ represents a hydrogen atom, halo-, a C₁-C₆-alkyl-, halo-C₁-C₆-alkyl- or C₁-C₆-alkoxy- group;
R^{5a} represents a group selected from:
halo-, C₁-C₆-alkyl-,C₁-C₆-alkoxy-, halo-C₁-C₆-alkoxy-, hydroxy-C₁-C₆-alkyl-, C₁-C₆-alkoxy-C₁-C₆-alkyl-,
halo-C₁-C₆-alkoxy-C₁-C₆-alkyl-, R⁸-(C₁-C₆-alkoxy)-, R⁸-O- , R⁸-S-, R⁸-S(=O)₂-, (C₃-C₆-cycloalkyl)-(CH₂)ₙ-O-;
R^{5b} represents a group selected from:
halo-, cyano-, nitro-, C₁-C₆-alkyl-, halo-C₁-C₆-alkyl-, C₁-C₆-alkoxy-, halo-C₁-C₆-alkoxy-, R⁸-O-, -C(=O)R⁸, -C(=O)O-R⁸, -N(H)C(=O)R⁸, -N(R⁷)C(=O)R⁸, -NR⁸R⁷, -NR⁷R⁷, -C(=O)N(H)R⁸, -C(=O)NR⁸R⁷, R⁸-S(=O)-, R⁸-S(=O)₂-, -S(=O)(=NR⁷)R⁸;
Q¹ represents a group selected from: CH, C-(C₁-C₆-alkyl), C-(C₁-C₆-alkoxy), C-halo;
Q² represents a group selected from: N, CH, C-R^{5c};
wherein R^{5c} is selected from the groups consisting of:
halo-, cyano-, C₁-C₆-alkyl-, C₁-C₆-alkoxy-, -N(H)C(=O)R⁷, -N(R⁷)C(=O)R⁷, -C(=O)N(H)R⁸, -C(=O)NR⁸R⁷, R⁸-S(=O)-, R⁸-S(=O)₂-, -S(=O)(=NR⁷)R⁸;
Q³ represents a group selected from: N, CH, C-R^{5c}, wherein R^{5c} is selected from the groups consisting of: halo-, cyano-, C₁-C₆-alkyl-, C₁-C₆-alkoxy-, -N(H)C(=O)R⁷, -N(R⁷)C(=O)R⁷, -C(=O)N(H)R⁸, -C(=O)NR⁸R⁷, R⁸-S(=O)-, R⁸-S(=O)₂-, -S(=O)(=NR⁷)R⁸;
R⁶ represents a group selected from: C₃-C₆-cycloalkyl-, 3- to 10-membered heterocyclyl-, aryl-, heteroaryl-, -(CH₂)_{q}-(C₃-C₆-cycloalkyl), -(CH₂)_{q}-(3- to 10-membered heterocyclyl), -(CH₂)_{q}-aryl, or -(CH₂)_{q}-heteroaryl;
wherein said group is optionally substituted, one or more times, identically or differently, with a substituent selected from:
halo-, hydroxy-, cyano-, nitro-, C₁-C₆-alkyl-, halo-C₁-C₆-alkyl-, C₁-C₆-alkoxy-, halo-C₁-C₆-alkoxy-, hydroxy-C₁-C₆-alkyl-, C₁-C₆-alkoxy-C₁-C₆-alkyl-, halo-C₁-C₆-alkoxy-C₁-C₆-alkyl-;
n represents an integer of 0, 1 or 2;
q represents an integer of 0, 1 or 2;
or a stereoisomer, a tautomer, an N-oxide, a hydrate, a solvate, or a salt thereof, or a mixture of same.

3. The compound according to claim 1 or 2, wherein:
R¹ represents a phenyl group
- which is substituted, one or more times, identically or differently, with a substituent selected from:
-N(H)C(=O)R⁶, -C(=O)N(H)R⁶; and
- which is optionally substituted, one or more times, identically or differently, with a substituent selected from:
halo-, C₁-C₆-alkyl-, C₁-C₆-alkoxy-;
R³ and R⁴ represent a hydrogen atom;
Q¹ represents CH;
Q² represents CH;
Q³ represents CH or N;
R^{5a} represents a group selected from:
halo-, C₁-C₆-alkyl-,C₁-C₆-alkoxy-, halo-C₁-C₆-alkoxy-, (C₃-C₆-cycloalkyl)-(CH₂)ₙ-O-;
R^{5b} represents a group selected from:
halo-, cyano-, C₁-C₆-alkoxy-, -N(H)C(=O)R⁸, -N(R⁷)C(=O)R⁸, -C(=O)N(H)R⁸, -C(=O)NR⁸R⁷, R⁸-S(=O)-, R⁸-S(=O)₂-, -S(=O)(=NR⁷)R⁸;
R⁶ represents a group selected from:
-(CH₂)_{q}-(C₃-C₆-cycloalkyl) or -(CH₂)_{q}-aryl;
wherein said group is optionally substituted, one or more times, identically or differently, with a substituent selected from:
halo-, hydroxy-, cyano-, nitro-, C₁-C₆-alkyl-, halo-C₁-C₆-alkyl-, C₁-C₆-alkoxy-, halo-C₁-C₆-alkoxy-, hydroxy-C₁-C₆-alkyl-, C₁-C₆-alkoxy-C₁-C₆-alkyl-, halo-C₁-C₆-alkoxy-C₁-C₆-alkyl-;
n represents an integer of 0, 1 or 2;
q represents an integer of 1 or 2;
or a stereoisomer, a tautomer, an N-oxide, a hydrate, a solvate, or a salt thereof, or a mixture of same.

4. The compound according to any one of claims 1, 2 or 3, wherein:
R⁶ represents a group selected from:
-CH₂-(C₃-C₆-cycloalkyl) or -CH₂-aryl;
wherein said group is optionally substituted, one or more times, identically or differently, with a substituent selected from:
halo-, C₁-C₆-alkyl-, halo-C₁-C₆-alkyl-, halo-C₁-C₆-alkoxy-;
n represents an integer of 0 or 1;
q represents an integer of 1;
or a stereoisomer, a tautomer, an N-oxide, a hydrate, a solvate, or a salt thereof, or a mixture of same.

5. The compound according to claim 1, which is selected from the group consisting of:
*N*,*N*-diethyl-4-{[6-(4-{[(4-fluorophenyl)acetyl]amino}phenyl)[1,2,4]triazolo[1,5-*a*]-pyridin-2-yl]amino}-3-methoxybenzamide,
*N*-(4-{2-[(4-cyano-2-methoxyphenyl)amino][1,2,4]triazolo[1,5-*a*]pyridin-6-yl}-phenyl)-2-(4-fluorophenyl)acetamide,
*N*-(4-{2-[(2-ethoxy-4-fluorophenyl)amino][1,2,4]triazolo[1,5-*a*]pyridin-6-yl}phenyl)-2-(4-fluorophenyl)acetamide,
*N*-ethyl-4-{[6-(4-{[(4-fluorophenyl)acetyl]amino}phenyl)[1,2,4]triazolo[1,5-*a*]pyridin-2-yl]amino}-3-methoxybenzamide,
*N*-*tert*-butyl-4-{[6-(4-{[(4-fluorophenyl)acetyl]amino}phenyl)[1,2,4]triazolo[1,5-*a*]pyridin-2-yl]amino}-3-methoxybenzamide,
4-{[6-(4-{[(4-fluorophenyl)acetyl]amino}phenyl)[1,2,4]triazolo[1,5-*a*]pyridin-2-yl]amino}-*N*-(2-hydroxyethyl)-3-methoxybenzamide,
*N*-(2-ethoxyethyl)-4-{[6-(4-{[(4-fluorophenyl)acetyl]amino}phenyl)[1,2,4]triazolo-[1,5-*a*]pyridin-2-yl]amino}-3-methoxybenzamide,
3-ethoxy-4-{[6-(4-{[(4-fluorophenyl)acetyl]amino}phenyl)[1,2,4]triazolo[1,5-*a*]pyridin-2-yl]amino}-N-(2-hydroxyethyl)benzamide,
3-ethoxy-4-{[6-(4-{[(4-fluorophenyl)acetyl]amino}phenyl)[1,2,4]triazolo[1,5-*a*]pyridin-2-yl]amino}-*N*-(1-hydroxy-2-methylpropan-2-yl)benzamide,
3-ethoxy-*N*,*N*-diethyl-4-{[6-(4-{[(4-fluorophenyl)acetyl]amino}phenyl)[1,2,4]-triazolo[1,5-*a*]pyridin-2-yl]amino}benzamide,
4-{[6-(4-{[(4-fluorophenyl)acetyl]amino}phenyl)[1,2,4]triazolo[1,5-*a*]pyridin-2-yl]-amino}-*N*-(2-hydroxyethyl)-3-(2,2,2-trifluoroethoxy)benzamide,
4-{[6-(4-{[(4-fluorophenyl)acetyl]amino}phenyl)[1,2,4]triazolo[1,5-*a*]pyridin-2-yl]amino}-*N*-(2-hydroxy-2-methylpropyl)-3-methoxybenzamide,
4-{[6-(4-{[(4-fluorophenyl)acetyl]amino}phenyl)[1,2,4]triazolo[1,5-*a*]pyridin-2-yl]amino}-*N*-(1-hydroxy-2-methylpropan-2-yl)-3-methoxybenzamide,
*N*-{2-[acetyl(methyl)amino]ethyl}-4-{[6-(4-{[(4-fluorophenyl)acetyl]amino}phenyl)-[1,2,4]triazolo[1,5-*a*]pyridin-2-yl]amino}-3-methoxy-N-methylbenzamide,
2-(4-fluorophenyl)-*N*-[4-(2-{[2-methoxy-4-(methylsulfonyl)phenyl]amino}[1,2,4]-triazolo[1,5-*a*]pyridin-6-yl)phenyl]acetamide,
3-ethoxy-*N*-ethyl-4-{[6-(4-{[(4-fluorophenyl)acetyl]amino}phenyl)[1,2,4]triazolo-[1,5-*a*]pyridin-2-yl]amino}benzamide,
3-ethoxy-4-{[6-(4-{[(4-fluorophenyl)acetyl]amino}phenyl)[1,2,4]triazolo[1,5-*a*]pyridin-2-yl]amino}-*N*-(2-hydroxy-2-methylpropyl)benzamide,
3-ethoxy-*N*-ethyl-4-{[6-(4-{[(4-fluorophenyl)acetyl]amino}phenyl)[1,2,4]-triazolo[1,5-*a*]pyridin-2-yl]amino}-*N*-(2-methoxyethyl)benzamide,
3-ethoxy-4-{[6-(4-{[(4-fluorophenyl)acetyl]amino}phenyl)[1,2,4]triazolo[1,5-*a*]pyridin-2-yl]amino}-*N*-(2-hydroxyethyl)-*N*-methylbenzamide,
4-{[6-(4-{[(4-fluorophenyl)acetyl]amino}phenyl)[1,2,4]triazolo[1,5-*a*]pyridin-2-yl]-amino}-3-methoxy-*N*-methyl-*N*-[2-(methylamino)ethyl]benzamide,
*N*-*tert*-butyl-4-{[6-(4-{[(4-fluorophenyl)acetyl]amino}phenyl)[1,2,4]triazolo[1,5-*a*]pyridin-2-yl]amino}-3-(2,2,2-trifluoroethoxy)benzamide,
*N*,*N*-diethyl-4-{[6-(4-{[(4-fluorophenyl)acetyl]amino}phenyl)[1,2,4]triazolo[1,5-*a*]pyridin-2-yl]amino}-3-(2,2,2-trifluoroethoxy)benzamide,
*N*,*N*-diethyl-4-{[6-(4-{[(4-fluorophenyl)acetyl]amino}phenyl)[1,2,4]triazolo[1,5-*a*]pyridin-2-yl]amino}-3-propoxybenzamide,
3-(cyclopropylmethoxy)*-N*,*N*-diethyl-4-{[6-(4-{[(4-fluorophenyl)acetyl]amino}-phenyl)[1,2,4]triazolo[1,5-*a*]pyridin-2-yl]amino}benzamide,
*N*,*N*-diethyl-4-{[6-(4-{[(4-fluorophenyl)acetyl]amino}phenyl)[1,2,4]triazolo[1,5-*a*]pyridin-2-yl]amino}-3-isopropoxybenzamide,
*N*,*N*-diethyl-4-{[6-(4-{[(4-fluorophenyl)acetyl]amino}phenyl)[1,2,4]triazolo[1,5-*a*]pyridin-2-yl]amino}-3-(2-methoxyethoxy)benzamide,
4-{[6-(4-{[(4-fluorophenyl)acetyl]amino}phenyl)[1,2,4]triazolo[1,5-*a*]pyridin-2-yl]-amino}-3-(2,2,2-trifluoroethoxy)-*N*-(2,2,2-trifluoroethyl)benzamide,
3-ethoxy-4-{[6-(4-{[(4-fluorophenyl)acetyl]amino}phenyl)[1,2,4]triazolo[1,5-*a*]pyridin-2-yl]amino}-*N*-[2-(methylsulfonyl)ethyl]benzamide,
4-{[6-(4-{[(4-fluorophenyl)acetyl]amino}phenyl)[1,2,4]triazolo[1,5-*a*]pyridin-2-yl]amino}-*N*-(2-hydroxy-2-methylpropyl)-3-(2,2,2-trifluoroethoxy)benzamide,
4-{[6-(4-{[(4-fluorophenyl)acetyl]amino}phenyl)[1,2,4]triazolo[1,5-*a*]pyridin-2-yl]amino}-3-methoxy-*N*-methylbenzamide,
4-[(6-{4-[(4-fluorobenzyl)carbamoyl]phenyl}[1,2,4]triazolo[1,5-*a*]pyridin-2-yl)-amino]-*N*-(2-hydroxyethyl)-3-methoxybenzamide,
3-ethoxy-4-[(6-{4-[(4-fluorobenzyl)carbamoyl]phenyl}[1,2,4]triazolo[1,5-*a*]pyridin-2-yl)amino]-*N*-(2-hydroxyethyl)benzamide,
3-ethoxy-4-[(6-{4-[(4-fluorobenzyl)carbamoyl]phenyl}[1,2,4]triazolo[1,5-*a*]pyridin-2-yl)amino]-*N*-(1-hydroxy-2-methylpropan-2-yl)benzamide,
4-[(6-{4-[(4-fluorobenzyl)carbamoyl]phenyl}[1,2,4]triazolo[1,5-*a*]pyridin-2-yl)-amino]-*N*-(2-hydroxyethyl)-3-(2,2,2-trifluoroethoxy)benzamide, 4-[(6-{4-[(4-fluorobenzyl)carbamoyl]phenyl}[1,2,4]triazolo[1,5-*a*]pyridin-2-yl)-amino]-*N*-(2-hydroxy-2-methylpropyl)-3-methoxybenzamide, *N*-(4-fluorobenzyl)-4-(2-{[2-methoxy-4-(methylsulfonyl)phenyl]amino}[1,2,4]-triazolo[1,5-*a*]pyridin-6-yl)benzamide,
3-ethoxy-4-[(6-{4-[(4-fluorobenzyl)carbamoyl]phenyl}[1,2,4]triazolo[1,5-*a*]pyridin-2-yl)amino]-*N*-(2-hydroxy-2-methylpropyl)benzamide,
4-[(6-{4-[(4-fluorobenzyl)carbamoyl]phenyl}[1,2,4]triazolo[1,5-*a*]pyridin-2-yl)-amino]-*N*-(1-hydroxy-2-methylpropan-2-yl)-3-methoxybenzamide,
*N*-{2-[acetyl(methyl)amino]ethyl}-4-[(6-{4-[(4-fluorobenzyl)carbamoyl]phenyl}-[1,2,4]triazolo[1,5-*a*]pyridin-2-yl)amino]-3-methoxy-*N*-methylbenzamide, *N*-(2-ethoxyethyl)-4-[(6-{4-[(4-fluorobenzyl)carbamoyl]phenyl}[1,2,4]triazolo[1,5-*a*]pyridin-2-yl)amino]-3-methoxybenzamide,
4-[(6-{4-[(4-fluorobenzyl)carbamoyl]phenyl}[1,2,4]triazolo[1,5-*a*]pyridin-2-yl)-amino]-3-methoxy-*N*-methyl-*N*-[2-(methylamino)ethyl]benzamide, 3-ethoxy-4-[(6-{4-[(4-fluorobenzyl)carbamoyl]phenyl}[1,2,4]triazolo[1,5-*a*]pyridin-2-yl)amino]-*N*-(2-hydroxyethyl)-*N*-methylbenzamide,
4-[(6-{4-[(4-fluorobenzyl)carbamoyl]phenyl}[1,2,4]triazolo[1,5-*a*]pyridin-2-yl)-amino]-3-methoxy-*N*-methylbenzamide,
*N*-*tert*-butyl-4-[(6-{4-[(4-fluorobenzyl)carbamoyl]phenyl}[1,2,4]triazolo[1,5-*a*]-pyridin-2-yl)amino]-3-methoxybenzamide,
4-[(6-{4-[(4-fluorobenzyl)carbamoyl]phenyl}[1,2,4]triazolo[1,5-*a*]pyridin-2-yl)-amino]-3-methoxy-*N*-[2-(methylsulfonyl)ethyl]benzamide,
4-{2-[(2,4-dimethoxyphenyl)amino][1,2,4]triazolo[1,5-*a*]pyridin-6-yl}-*N*-(4-fluoro-benzyl)benzamide,
4-[(6-{4-[(4-fluorobenzyl)carbamoyl]phenyl}[1,2,4]triazolo[1,5-*a*]pyridin-2-yl)-amino]-3-methoxy-*N*-(2,2,2-trifluoroethyl)benzamide,
4-[(6-{4-[(4-fluorobenzyl)carbamoyl]phenyl}[1,2,4]triazolo[1,5-*a*]pyridin-2-yl)-amino]-*N*-(1-hydroxy-2-methylpropan-2-yl)-3-(2,2,2-trifluoroethoxy)benzamide,
3-ethoxy-4-[(6-{4-[(4-fluorobenzyl)carbamoyl]phenyl}[1,2,4]triazolo[1,5-*a*]pyridin-2-yl)amino]-*N*-[2-(methylsulfonyl)ethyl]benzamide,
*N*-ethyl-4-[(6-{4-[(4-fluorobenzyl)carbamoyl]phenyl}[1,2,4]triazolo[1,5-*a*]pyridin-2-yl)amino]-3-methoxybenzamide,
*N*-*tert*-butyl-3-ethoxy-4-[(6-{4-[(4-fluorobenzyl)carbamoyl]phenyl}[1,2,4]-triazolo[1,5-*a*]pyridin-2-yl)amino]benzamide,
4-[(6-{4-[(4-fluorobenzyl)carbamoyl]phenyl}[1,2,4]triazolo[1,5-*a*]pyridin-2-yl)-amino]-*N*-(2-hydroxy-2-methylpropyl)-3-(2,2,2-trifluoroethoxy)benzamide,
4-[(6-{4-[(4-fluorobenzyl)carbamoyl]phenyl}[1,2,4]triazolo[1,5-*a*]pyridin-2-yl)-amino]-3-(2,2,2-trifluoroethoxy)-*N*-(2,2,2-trifluoroethyl)benzamide,
4-(2-{[4-(dimethylamino)-2-methylphenyl]amino}[1,2,4]triazolo[1,5-*a*]pyridin-6-yl)-*N*-(4-fluorobenzyl)benzamide,
2-fluoro-*N*-(4-fluorobenzyl)-4-[2-({4-[(1-hydroxy-2-methylpropan-2-yl)carbamoyl]-2-methoxyphenyl}amino)[1,2,4]triazolo[1,5-*a*]pyridin-6-yl]benzamide,
*N*-(4-fluorobenzyl)-4-[2-({4-[(1-hydroxy-2-methylpropan-2-yl)carbamoyl]-2-methoxyphenyl}amino)[1,2,4]triazolo[1,5-*a*]pyridin-6-yl]-2-methylbenzamide,
2-chloro-*N*-(4-fluorobenzyl)-4-[2-({4-[(1-hydroxy-2-methylpropan-2-yl)carbamoyl]-2-methoxyphenyl}amino)[1,2,4]triazolo[1,5-*a*]pyridin-6-yl]benzamide,
4-[(6-{4-[(cyclopropylmethyl)carbamoyl]phenyl}[1,2,4]triazolo[1,5-*a*]pyridin-2-yl)-amino]-3-ethoxy-*N*-ethylbenzamide,
4-[(6-{4-[(cyclopropylmethyl)carbamoyl]phenyl}[1,2,4]triazolo[1,5-*a*]pyridin-2-yl)-amino]-3-ethoxy-*N*-ethyl-*N*-(2-methoxyethyl)benzamide,
4-[(6-{4-[(cyclopropylmethyl)carbamoyl]phenyl}[1,2,4]triazolo[1,5-*a*]pyridin-2-yl)-amino]-3-ethoxy-*N*-(2-hydroxyethyl)benzamide,
4-[(6-{4-[(cyclopropylmethyl)carbamoyl]phenyl}[1,2,4]triazolo[1,5-*a*]pyridin-2-yl)amino]-*N*-(2-hydroxyethyl)-3-(2,2,2-trifluoroethoxy)benzamide,
4-[(6-{4-[(cyclopropylmethyl)carbamoyl]phenyl}[1,2,4]triazolo[1,5-*a*]pyridin-2-yl)-amino]-*N*-ethyl-3-(2,2,2-trifluoroethoxy)benzamide,
*N*-ethyl-4-[(6-{4-[(3-fluorobenzyl)carbamoyl]phenyl}[1,2,4]triazolo[1,5-*a*]pyridin-2-yl)amino]-3-methoxybenzamide,
*N*-(4-fluorobenzyl)-4-[2-({4-[(1-hydroxy-2-methylpropan-2-yl)carbamoyl]-2-methoxyphenyl}amino)[1,2,4]triazolo[1,5-*a*]pyridin-6-yl]-2-methoxybenzamide,
4-{[6-(4-{[(4-fluorophenyl)acetyl]amino}phenyl)[1,2,4]triazolo[1,5-*a*]pyridin-2-yl]-amino}-3-methoxy-*N*-(2,2,2-trifluoroethyl)benzamide,
N-[4-(2-{[2-ethoxy-4-(methylsulfonyl)phenyl]amino}[1,2,4]triazolo[1,5-*a*]pyridin-6-yl)phenyl]-2-(4-fluorophenyl)acetamide,
N-[4-(2-{[2-ethoxy-4-(ethylsulfonyl)phenyl]amino}[1,2,4]triazolo[1,5-*a*]pyridin-6-yl)phenyl]-2-(4-fluorophenyl)acetamide,
2-(4-fluorophenyl)-*N*-[4-(2-{[4-(methylsulfonyl)-2-(2,2,2-trifluoroethoxy)phenyl]-amino}[1,2,4]triazolo[1,5-*a*]pyridin-6-yl)phenyl]acetamide,
*N*-[4-(2-{[2-(difluoromethoxy)-4-(methylsulfonyl)phenyl]amino}[1,2,4]triazolo[1,5-*a*]pyridin-6-yl)phenyl]-2-(4-fluorophenyl)acetamide,
*N*-[4-(2-{[2-(difluoromethoxy)-4-(ethylsulfonyl)phenyl]amino}[1,2,4]triazolo[1,5-*a*]pyridin-6-yl)phenyl]-2-(4-fluorophenyl)acetamide,
*N*-[4-(2-{[2-(cyclopropyloxy)-4-(methylsulfonyl)phenyl]amino}[1,2,4]triazolo[1,5-*a*]-pyridin-6-yl)phenyl]-2-(4-fluorophenyl)acetamide,
4-(2-{[2-ethoxy-4-(methylsulfonyl)phenyl]amino}[1,2,4]triazolo[1,5-*a*]pyridin-6-yl)-*N*-(4-fluorobenzyl)benzamide,
4-(2-{[2-ethoxy-4-(ethylsulfonyl)phenyl]amino}[1,2,4]triazolo[1,5-*a*]pyridin-6-yl)-*N-*(4-fluorobenzyl)benzamide,
*N*-(4-fluorobenzyl)-4-(2-{[4-(methylsulfonyl)-2-(2,2,2-trifluoroethoxy)phenyl]-amino}[1,2,4]triazolo[1,5-*a*]pyridin-6-yl)benzamide,
4-(2-{[2-(difluoromethoxy)-4-(methylsulfonyl)phenyl]amino}[1,2,4]triazolo[1,5-*a*]-pyridin-6-yl)-N-(4-fluorobenzyl)benzamide,
4-(2-{[2-(cyclopropyloxy)-4-(methylsulfonyl)phenyl]amino}[1,2,4]triazolo[1,5-*a*]-pyridin-6-yl)-*N*-(4-fluorobenzyl)benzamide,
*N*-(4-fluorobenzyl)-2-methyl-4-(2-{[4-(methylsulfonyl)-2-(2,2,2-trifluoroethoxy)-phenyl]amino}[1,2,4]triazolo[1,5-*a*]pyridin-6-yl)benzamide,
*N*-(4-fluorobenzyl)-2-methoxy-4-(2-{[2-methoxy-4-(methylsulfonyl)phenyl]amino}-[1,2,4]triazolo[1,5-*a*]pyridin-6-yl)benzamide,
2-(2,4-difluorophenyl)-*N*-[4-(2-{[2-methoxy-4-(methylsulfonyl)phenyl]amino}-[1,2,4]triazolo[1,5-*a*]pyridin-6-yl)phenyl]acetamide,
*N*-(2,4-difluorobenzyl)-4-(2-{[2-methoxy-4-(methylsulfonyl)phenyl]amino}-[1,2,4]triazolo[1,5-*a*]pyridin-6-yl)benzamide,
4-{[6-(4-{[(4-fluorophenyl)acetyl]amino}phenyl)[1,2,4]triazolo[1,5-*a*]pyridin-2-yl]amino}-3-methoxy-*N*-(2-methoxyethyl)benzamide,
4-{[6-(4-{[(4-fluorophenyl)acetyl]amino}phenyl)[1,2,4]triazolo[1,5-*a*]pyridin-2-yl]amino}-3-methoxy-*N*,*N*-dimethylbenzamide,
4-{[6-(4-{[(4-fluorophenyl)acetyl]amino}phenyl)[1,2,4]triazolo[1,5-*a*]pyridin-2-yl]amino}-3-methoxy-*N*-[2-(methylsulfonyl)ethyl]benzamide,
4-{[6-(4-{[(4-fluorophenyl)acetyl]amino}phenyl)[1,2,4]triazolo[1,5-*a*]pyridin-2-yl]amino}-3-methoxybenzamide,
*N*-(2-fluoroethyl)-4-{[6-(4-{[(4-fluorophenyl)acetyl]amino}phenyl)[1,2,4]-triazolo[1,5-*a*]pyridin-2-yl]amino}-3-methoxybenzamide,
*N*-ethyl-4-{[6-(4-{[(4-fluorophenyl)acetyl]amino}phenyl)[1,2,4]triazolo[1,5-*a*]-pyridin-2-yl]amino}-3-methoxy-*N*-methylbenzamide,
*N*-(2,2-difluoroethyl)-4-{[6-(4-{[(4-fluorophenyl)acetyl]amino}phenyl)[1,2,4]-triazolo[1,5-*a*]pyridin-2-yl]amino}-3-methoxybenzamide,
*N*-[2-(dimethylamino)ethyl]-4-{[6-(4-{[(4-fluorophenyl)acetyl]amino}phenyl)[1,2,4]-triazolo[1,5-*a*]pyridin-2-yl]amino}-3-methoxy-*N*-methylbenzamide,
4-{[6-(4-{[(4-fluorophenyl)acetyl]amino}phenyl)[1,2,4]triazolo[1,5-*a*]pyridin-2-yl]-amino}-3-methoxy-*N*-methyl-*N*-(2,2,2-trifluoroethyl)benzamide,
*N*-[2-(dimethylamino)ethyl]-4-{[6-(4-{[(4-fluorophenyl)acetyl]amino}phenyl)-[1,2,4]triazolo[1,5-*a*]pyridin-2-yl]amino}-3-methoxybenzamide,
4-{[6-(4-{[(4-fluorophenyl)acetyl]amino}phenyl)[1,2,4]triazolo[1,5-*a*]pyridin-2-yl]amino}-*N*-(3-fluoropropyl)-3-methoxybenzamide,
*N*-[4-(2-{[5-fluoro-2-methoxy-4-(methylsulfonyl)phenyl]amino}[1,2,4]triazolo[1,5-*a*]pyridin-6-yl)phenyl]-2-(4-fluorophenyl)acetamide,
2-(4-fluorophenyl)-*N*-[4-(2-{[2-methoxy-4-(methylsulfinyl)phenyl]amino}[1,2,4]-triazolo[1,5-a]pyridin-6-yl)phenyl]acetamide,
*N*-[4-(2-{[5-fluoro-2-methoxy-4-(methylsulfinyl)phenyl]amino}[1,2,4]triazolo[1,5-*a*]pyridin-6-yl)phenyl]-2-(4-fluorophenyl)acetamide,
*N*-[4-(2-{[4-(tert-butylsulfamoyl)-2-methoxyphenyl]amino}[1,2,4]triazolo[1,5-*a*]-pyridin-6-yl)phenyl]-2-(4-fluorophenyl)acetamide,
2-(4-fluorophenyl)-*N*-[4-(2-{[4-(2-hydroxypropan-2-yl)-2-methoxyphenyl]amino}-[1,2,4]triazolo[1,5-*a*]pyridin-6-yl)phenyl]acetamide,
*N*-(4-{2-[(2,4-dimethoxyphenyl)amino][1,2,4]triazolo[1,5-*a*]pyridin-6-yl}phenyl)-2-(4-fluorophenyl)acetamide,
3-ethoxy-*N*-ethyl-4-{[6-(4-{[(4-fluorophenyl)acetyl]amino}phenyl)[1,2,4]triazolo-[1,5-*a*]pyridin-2-yl]amino}-*N*-methylbenzamide,
*N*-[2-(dimethylamino)ethyl]-3-ethoxy-4-{[6-(4-{[(4-fluorophenyl)acetyl]amino}-phenyl)[1,2,4]triazolo[1,5-*a*]pyridin-2-yl]amino}benzamide,
4-{[6-(4-{[(4-fluorophenyl)acetyl]amino}phenyl)[1,2,4]triazolo[1,5-*a*]pyridin-2-yl]-amino}-*N*-[2-(methylsulfonyl)ethyl]-3-(2,2,2-trifluoroethoxy)benzamide,
4-{[6-(4-{[(4-fluorophenyl)acetyl]amino}phenyl)[1,2,4]triazolo[1,5-*a*]pyridin-2-yl]amino}-*N*-(1-hydroxy-2-methylpropan-2-yl)-3-(2,2,2-trifluoroethoxy)benzamide,
*N*-[2-(dimethylamino)ethyl]-4-{[6-(4-{[(4-fluorophenyl)acetyl]amino}phenyl)-[1,2,4]triazolo[1,5-*a*]pyridin-2-yl]amino}-3-(2,2,2-trifluoroethoxy)benzamide,
2-(4-fluorophenyl)-*N*-[4-(2-{[4-(methylsulfinyl)-2-(2,2,2-trifluoroethoxy)phenyl]-amino}[1,2,4]triazolo[1,5-*a*]pyridin-6-yl)phenyl]acetamide,
4-{[6-(4-{[(4-fluorophenyl)acetyl]amino}phenyl)[1,2,4]triazolo[1,5-*a*]pyridin-2-yl]amino}-3-(trifluoromethoxy)benzamide,
*N*-[4-(2-{[2-(difluoromethoxy)-4-(propan-2-ylsulfonyl)phenyl]amino}[1,2,4]triazolo[1,5-*a*]pyridin-6-yl)phenyl]-2-(4-fluorophenyl)acetamide,
*N*-[4-(2-{[2-(difluoromethoxy)-4-fluorophenyl]amino}[1,2,4]triazolo[1,5-*a*]pyridin-6-yl)phenyl]-2-(4-fluorophenyl)acetamide,
4-{[6-(4-{[(4-fluorophenyl)acetyl]amino}phenyl)[1,2,4]triazolo[1,5-*a*]pyridin-2-yl]amino}-3-methylbenzamide,
4-{[6-(4-{[(4-fluorophenyl)acetyl]amino}phenyl)[1,2,4]triazolo[1,5-*a*]pyridin-2-yl]amino}-3-methyl-*N*-(2,2,2-trifluoroethyl)benzamide,
*N*-(2-fluoroethyl)-4-{[6-(4-{[(4-fluorophenyl)acetyl]amino}phenyl)[1,2,4]-triazolo[1,5-*a*]pyridin-2-yl]amino}-3-methylbenzamide,
4-{[6-(4-{[(4-fluorophenyl)acetyl]amino}phenyl)[1,2,4]triazolo[1,5-*a*]pyridin-2-yl]amino}-*N*,*N*,3-trimethylbenzamide,
2-(4-fluorophenyl)-*N*-[4-(2-{[2-methyl-4-(methylsulfonyl)phenyl]amino}[1,2,4]-triazolo[1,5-*a*]pyridin-6-yl)phenyl]acetamide,
2-(4-fluorophenyl)-*N*-[4-(2-{[2-methyl-4-(methylsulfinyl)phenyl]amino}[1,2,4]-triazolo[1,5-*a*]pyridin-6-yl)phenyl]acetamide,
2-(4-fluorophenyl)-*N*-{4-[2-({2-methyl-4-[(methylsulfonyl)amino]phenyl}amino)-[1,2,4]triazolo[1,5-*a*]pyridin-6-yl]phenyl}acetamide,
2-(4-fluorophenyl)-*N*-(4-{2-[(4-methoxy-2-methylphenyl)amino][1,2,4]triazolo[1,5-*a*]pyridin-6-yl}phenyl)acetamide,
*N*-[4-(2-{[4-(dimethylamino)-2-methylphenyl]amino}[1,2,4]triazolo[1,5-*a*]pyridin-6-yl)phenyl]-2-(4-fluorophenyl)acetamide,
*N*-ethyl-5-{[6-(4-{[(4-fluorophenyl)acetyl]amino}phenyl)[1,2,4]triazolo[1,5-*a*]pyridin-2-yl]amino}-4-methylpyridine-2-carboxamide,
5-{[6-(4-{[(4-fluorophenyl)acetyl]amino}phenyl)[1,2,4]triazolo[1,5-*a*]pyridin-2-yl]amino}-4-methyl-*N*-(2,2,2-trifluoroethyl)pyridine-2-carboxamide,
*N*-[4-(2-{[2-fluoro-4-(methylsulfonyl)phenyl]amino}[1,2,4]triazolo[1,5-*a*]pyridin-6-yl)phenyl]-2-(4-fluorophenyl)acetamide,
2-(4-fluoro-3-methylphenyl)-*N*-[4-(2-{[2-methoxy-4-(methylsulfonyl)phenyl]-amino}[1,2,4]triazolo[1,5-*a*]pyridin-6-yl)phenyl]acetamide,
2-(4-chlorophenyl)*-N*-[4-(2-{[2-methoxy-4-(methylsulfonyl)phenyl]amino}-[1,2,4]triazolo[1,5-*a*]pyridin-6-yl)phenyl]acetamide,
*N*-[4-(2-{[2-methoxy-4-(methylsulfonyl)phenyl]amino}[1,2,4]triazolo[1,5-*a*]pyridin-6-yl)phenyl]-2-phenylacetamide,
4-[(6-{4-[(4-fluorobenzyl)carbamoyl]phenyl}[1,2,4]triazolo[1,5-*a*]pyridin-2-yl)-amino]-3-methoxybenzamide,
4-[(6-{4-[(4-fluorobenzyl)carbamoyl]phenyl}[1,2,4]triazolo[1,5-*a*]pyridin-2-yl)-amino]-3-methoxy-*N*-(2-methoxyethyl)benzamide,
4-[(6-{4-[(4-fluorobenzyl)carbamoyl]phenyl}[1,2,4]triazolo[1,5-*a*]pyridin-2-yl)-amino]-*N*-(2-fluoroethyl)-3-methoxybenzamide,
4-[(6-{4-[(4-fluorobenzyl)carbamoyl]phenyl}[1,2,4]triazolo[1,5-*a*]pyridin-2-yl)-amino]-3-methoxy-*N*,*N*-dimethylbenzamide,
*N*-(2,2-difluoroethyl)-4-[(6-{4-[(4-fluorobenzyl)carbamoyl]phenyl}[1,2,4]-triazolo[1,5-*a*]pyridin-2-yl)amino]-3-methoxybenzamide,
*N*-[2-(dimethylamino)ethyl]-4-[(6-{4-[(4-fluorobenzyl)carbamoyl]phenyl}-[1,2,4]triazolo[1,5-*a*]pyridin-2-yl)amino]-3-methoxybenzamide,
*N*-[2-(dimethylamino)ethyl]-4-[(6-{4-[(4-fluorobenzyl)carbamoyl]phenyl}-[1,2,4]triazolo[1,5-*a*]pyridin-2-yl)amino]-3-methoxy-N-methylbenzamide,
4-[(6-{4-[(4-fluorobenzyl)carbamoyl]phenyl}[1,2,4]triazolo[1,5-*a*]pyridin-2-yl)-amino]-*N*-(3-fluoropropyl)-3-methoxybenzamide,
*N*-(4-fluorobenzyl)-4-(2-{[2-methoxy-4-(methylsulfinyl)phenyl]amino}[1,2,4]-triazolo[1,5-*a*]pyridin-6-yl)benzamide,
4-(2-{[4-(*tert*-butylsulfamoyl)-2-methoxyphenyl]amino}[1,2,4]triazolo[1,5-*a*]-pyridin-6-yl)-*N*-(4-fluorobenzyl)benzamide,
*N*-(4-fluorobenzyl)-4-(2-{[4-(2-hydroxypropan-2-yl)-2-methoxyphenyl]amino}-[1,2,4]triazolo[1,5-*a*]pyridin-6-yl)benzamide,
*N*-[2-(dimethylamino)ethyl]-3-ethoxy-4-[(6-{4-[(4-fluorobenzyl)carbamoyl]-phenyl}[1,2,4]triazolo[1,5-*a*]pyridin-2-yl)amino]benzamide,
3-ethoxy-*N*-ethyl-4-[(6-{4-[(4-fluorobenzyl)carbamoyl]phenyl}[1,2,4]triazolo[1,5-*a*]pyridin-2-yl)amino]-N-methylbenzamide,
3-ethoxy-4-[(6-{4-[(4-fluorobenzyl)carbamoyl]phenyl}[1,2,4]triazolo[1,5-*a*]pyridin-2-yl)amino]-*N*-(2-fluoroethyl)benzamide,
*N*-[2-(dimethylamino)ethyl]-4-[(6-{4-[(4-fluorobenzyl)carbamoyl]phenyl}-[1,2,4]triazolo[1,5-*a*]pyridin-2-yl)amino]-3-(2,2,2-trifluoroethoxy)benzamide,
4-[(6-{4-[(4-fluorobenzyl)carbamoyl]phenyl}[1,2,4]triazolo[1,5-*a*]pyridin-2-yl)-amino]-*N*-(2-fluoroethyl)-3-(2,2,2-trifluoroethoxy)benzamide,
4-[(6-{4-[(4-fluorobenzyl)carbamoyl]phenyl}[1,2,4]triazolo[1,5-*a*]pyridin-2-yl)-amino]-3-(trifluoromethoxy)benzamide,
4-(2-{[2-(difluoromethoxy)-4-(ethylsulfonyl)phenyl]amino}[1,2,4]triazolo[1,5-*a*]-pyridin-6-yl)-*N*-(4-fluorobenzyl)benzamide,
4-(2-{[2-(difluoromethoxy)-4-(propan-2-ylsulfonyl)phenyl]amino}[1,2,4]-triazolo[1,5-*a*]pyridin-6-yl)-N-(4-fluorobenzyl)benzamide,
4-(2-{[2-(difluoromethoxy)-4-fluorophenyl]amino}[1,2,4]triazolo[1,5-*a*]pyridin-6-yl)-*N*-(4-fluorobenzyl)benzamide,
*N*-ethyl-4-[(6-{4-[(4-fluorobenzyl)carbamoyl]phenyl}[1,2,4]triazolo[1,5-*a*]pyridin-2-yl)amino]-3-methylbenzamide,
4-[(6-{4-[(4-fluorobenzyl)carbamoyl]phenyl}[1,2,4]triazolo[1,5-*a*]pyridin-2-yl)-amino]-3-methylbenzamide,
4-[(6-{4-[(4-fluorobenzyl)carbamoyl]phenyl}[1,2,4]triazolo[1,5-*a*]pyridin-2-yl)-amino]-3-methyl-*N*-(2,2,2-trifluoroethyl)benzamide,
4-[(6-{4-[(4-fluorobenzyl)carbamoyl]phenyl}[1,2,4]triazolo[1,5-*a*]pyridin-2-yl)-amino]-*N*-(2-fluoroethyl)-3-methylbenzamide,
*N*-(4-fluorobenzyl)-4-(2-{[2-methyl-4-(methylsulfonyl)phenyl]amino}[1,2,4]-triazolo[1,5-*a*]pyridin-6-yl)benzamide,
*N*-(4-fluorobenzyl)-4-(2-{[2-methyl-4-(methylsulfinyl)phenyl]amino}[1,2,4]-triazolo[1,5-*a*]pyridin-6-yl)benzamide,
*N*-(4-fluorobenzyl)-4-[2-({2-methyl-4-[(methylsulfonyl)amino]phenyl}amino)[1,2,4]-triazolo[1,5-*a*]pyridin-6-yl]benzamide,
*N*-(4-fluorobenzyl)-4-{2-[(4-methoxy-2-methylphenyl)amino][1,2,4]triazolo[1,5-*a*]pyridin-6-yl}enzamide,
4-[(6-{4-[(4-chlorobenzyl)carbamoyl]phenyl}[1,2,4]triazolo[1,5-*a*]pyridin-2-yl)-amino]-3-methoxy-*N*-(2,2,2-trifluoroethyl)benzamide,
*N*-(4-chlorobenzyl)-4-(2-{[2-methoxy-4-(methylsulfonyl)phenyl]amino}[1,2,4]-triazolo[1, 5-*a*] pyridin-6-yl)benzamide,
*N*-(4-chlorobenzyl)-4-(2-{[2-methyl-4-(methylsulfonyl)phenyl]amino}[1,2,4]triazolo[1,5-*a*]pyridin-6-yl)benzamide,
*N*-(4-chlorobenzyl)-4-(2-{[2-methyl-4-(methylsulfinyl)phenyl]amino}[1,2,4]-triazolo[1,5-*a*]pyridin-6-yl)benzamide,
4-(2-{[2-methoxy-4-(methylsulfonyl)phenyl]amino}[1,2,4]triazolo[1,5-*a*]pyridin-6-yl)-*N*-(4-methylbenzyl)benzamide,
*N*-(4-methylbenzyl)-4-(2-{[2-methyl-4-(methylsulfonyl)phenyl]amino}[1,2,4]-triazolo[1,5-a]pyridin-6-yl)benzamide,
*N*-(4-methylbenzyl)-4-(2-{[2-methyl-4-(methylsulfinyl)phenyl]amino}[1,2,4]-triazolo[1,5-*a*]pyridin-6-yl)benzamide,
4-[(6-{4-[(2,4-difluorobenzyl)carbamoyl]phenyl}[1,2,4]triazolo[1,5-*a*]pyridin-2-yl)-amino]-3-methoxy-*N*-(2,2,2-trifluoroethyl)benzamide,
*N*-(2,4-difluorobenzyl)-2-methyl-4-(2-{[4-(methylsulfonyl)-2-(2,2,2-trifluoro-ethoxy)phenyl]amino}[1,2,4]triazolo[1,5-*a*]pyridin-6-yl)benzamide,
*N*-(4-fluorobenzyl)-4-(2-{[4-(2-hydroxypropan-2-yl)-2-methoxyphenyl]amino}-[1,2,4]triazolo[1,5-*a*]pyridin-6-yl)-2-methoxybenzamide,
2-(4-fluorophenyl)-*N*-[4-(2-{[2-methoxy-4-(*S*-methylsulfonimidoyl)phenyl]amino}-[1,2,4]triazolo[1,5-*a*]pyridin-6-yl)phenyl]acetamide,
*N*-(4-fluorobenzyl)-4-(2-{[2-methoxy-4-(*S*-methylsulfonimidoyl)phenyl]amino}-[1,2,4]triazolo[1,5-*a*]pyridin-6-yl)benzamide,
2-(4-fluorophenyl)-*N*-[4-(2-{[4-(hydroxymethyl)-2-methoxyphenyl]amino}[1,2,4]-triazolo[1,5-*a*]pyridin-6-yl)phenyl]acetamide,
and
*N*-(4-fluorobenzyl)-4-(2-{[4-(hydroxymethyl)-2-methoxyphenyl]amino}[1,2,4]-triazolo[1,5-*a*]pyridin-6-yl)benzamide,
or a stereoisomer, a tautomer, an N-oxide, a hydrate, a solvate, or a salt thereof, or a mixture of same.

6. A method of preparing a compound of general formula (I) according to any one of claims 1 to 5, in which method an intermediate compound of general formula (5) : 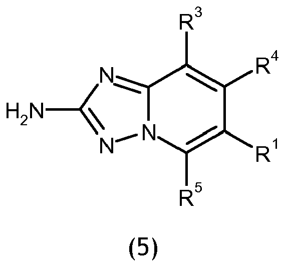 in which R¹, R³, R⁴, and R⁵ are as defined for the compounds of general formula (I) in any one of claims 1 to 5,
is allowed to react with an aryl halide of general formula (5a) :
R²-Y (5a)
in which R² is as defined for the compounds of general formula (I) in any one of claims 1 to 5, and Y represents a leaving group, such as a halogen atom or a trifluoromethylsulphonyloxy or nonafluorobutylsulphonyloxy group for example, thus providing a compound of general formula (I) : 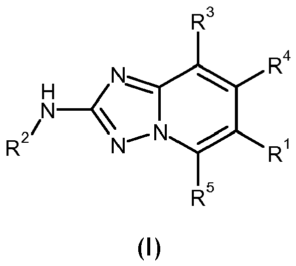 in which R¹, R², R³, R⁴, and R⁵ are as defined for the compounds of general formula (I) in any one of claims 1 to 5.

7. A method of preparing a compound of general formula (I) according to any one of claims 1 to 5, in which method an intermediate compound of general formula (7) : 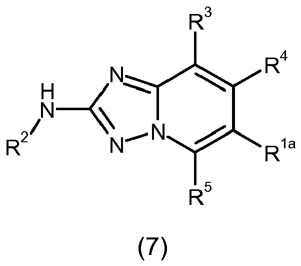 in which R², R³, R⁴, and R⁵ are as defined for the compounds of general formula (I) in any one of claims 1 to 5, and R^{1a} is an aryl group to which an -NH₂ substituent is bound,
is allowed to react with a compound of general formula :
R^{1b}-X (7a)
in which R^{1b} is -C(=O)R⁶, -C(=O)NR⁶R⁷, -S(=O)R⁶, -S(=O₂R⁶, and X is a suitable functional group via which the R^{1b} of the R^{1b}-X compound (7a) can be coupled, via a coupling reaction, such as an amide coupling reaction for example, onto the -NH₂ substituent bound to the aryl group R^{1a} of compound (7), thereby replacing said X with said R^{1a},
thus providing a compound of general formula (I) : 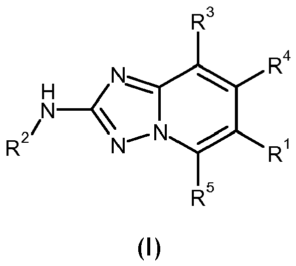 in which R¹, R², R³, R⁴, and R⁵ are as defined for the compounds of general formula (I) in any one of claims 1 to 5.

8. A compound according to any one of claims 1 to 5, or a stereoisomer, a tautomer, an N-oxide, a hydrate, a solvate, or a salt thereof, particularly a pharmaceutically acceptable salt thereof, or a mixture of same, for use in the treatment or prophylaxis of a disease.

9. A compound for use according to claim 8, wherein said disease is a disease of uncontrolled cell growth, proliferation and/or survival, an inappropriate cellular immune response, or an inappropriate cellular inflammatory response, particularly in which the uncontrolled cell growth, proliferation and/or survival, inappropriate cellular immune response, or inappropriate cellular inflammatory response is mediated by Mps-1, more particularly in which the disease of uncontrolled cell growth, proliferation and/or survival, inappropriate cellular immune response, or inappropriate cellular inflammatory response is a haemotological tumour, a solid tumour and/or metastases thereof, e.g. leukaemias and myelodysplastic syndrome, malignant lymphomas, head and neck tumours including brain tumours and brain metastases, tumours of the thorax including non-small cell and small cell lung tumours, gastrointestinal tumours, endocrine tumours, mammary and other gynaecological tumours, urological tumours including renal, bladder and prostate tumours, skin tumours, and sarcomas, and/or metastases thereof.

10. A pharmaceutical composition comprising a compound according to any one of claims 1 to 5, or a stereoisomer, a tautomer, an N-oxide, a hydrate, a solvate, or a salt thereof, particularly a pharmaceutically acceptable salt thereof, or a mixture of same, and a pharmaceutically acceptable diluent or carrier.

11. A pharmaceutical combination comprising :
- one or more compounds according to any one of claims 1 to 5, or a stereoisomer, a tautomer, an N-oxide, a hydrate, a solvate, or a salt thereof, particularly a pharmaceutically acceptable salt thereof, or a mixture of same ;
and
- one or more agents selected from : a taxane, such as Docetaxel, Paclitaxel, or Taxol; an epothilone, such as Ixabepilone, Patupilone, or Sagopilone; Mitoxantrone; Predinisolone; Dexamethasone; Estramustin; Vinblastin; Vincristin; Doxorubicin; Adriamycin; Idarubicin; Daunorubicin; Bleomycin; Etoposide; Cyclophosphamide; Ifosfamide; Procarbazine; Melphalan; 5-Fluorouracil; Capecitabine; Fludarabine; Cytarabine; Ara-C; 2-Chloro-2'-deoxyadenosine; Thioguanine; an anti-androgen, such as Flutamide, Cyproterone acetate, or Bicalutamide; Bortezomib; a platinum derivative, such as Cisplatin, or Carboplatin; Chlorambucil; Methotrexate; and Rituximab.

12. Use of a compound according to any one of claims 1 to 5, or a stereoisomer, a tautomer, an N-oxide, a hydrate, a solvate, or a salt thereof, particularly a pharmaceutically acceptable salt thereof, or a mixture of same, for the preparation of a medicament for the prophylaxis or treatment of a disease.

13. Use according to claim 12, wherein said disease is a disease of uncontrolled cell growth, proliferation and/or survival, an inappropriate cellular immune response, or an inappropriate cellular inflammatory response, particularly in which the uncontrolled cell growth, proliferation and/or survival, inappropriate cellular immune response, or inappropriate cellular inflammatory response is mediated by Mps-1, more particularly in which the disease of uncontrolled cell growth, proliferation and/or survival, inappropriate cellular immune response, or inappropriate cellular inflammatory response is a haemotological tumour, a solid tumour and/or metastases thereof, *e.g.* leukaemias and myelodysplastic syndrome, malignant lymphomas, head and neck tumours including brain tumours and brain metastases, tumours of the thorax including non-small cell and small cell lung tumours, gastrointestinal tumours, endocrine tumours, mammary and other gynaecological tumours, urological tumours including renal, bladder and prostate tumours, skin tumours, and sarcomas, and/or metastases thereof.

14. A compound of general formula (7) : 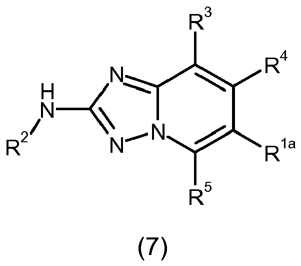 in which R², R³, R⁴, and R⁵ are as defined for the compounds of general formula (I) in any one of claims 1 to 5, and R^{1a} is an aryl group to which an -NH₂ substituent is bound.

15. Use of a compound of general formula (5) 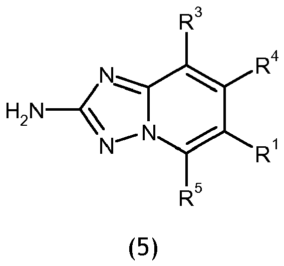 in which R¹, R³, R⁴, and R⁵ are as defined for the compounds of general formula (I) in any one of claims 1 to 5;
for the preparation of a compound of general formula (I) according to any one of claims 1 to 5.

16. Use of a compound of general formula (7) according to claim 14 for the preparation of a compound of general formula (I) according to any one of claims 1 to 5.

## Patentansprüche

1. Verbindung der allgemeinen Formel (I): 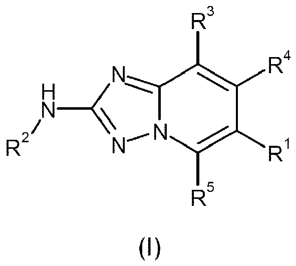 in welcher:
R¹ für eine Phenyl- oder eine Pyridylgruppe steht,
- welche ein- oder mehrfach gleich oder verschieden substituiert ist durch einen Substituenten ausgewählt aus:
R⁶-(C₁-C₆-Alkoxy)-, R⁶-O-, -C(=OR⁶, -C(=O)O-R⁶, -N(H)C(=O)R⁶, -N(H)C(=O)NR⁶R⁷, -NR⁶R⁷, -C(=O)N(H)R⁶, -C(=O)NR⁶R⁷, R⁶-S-, R⁶-S(=O)₂-, -N(H)S(=O)₂R⁶, -S(=O)₂N(H)R⁶; und
- welche gegebenenfalls ein- oder mehrfach gleich oder verschieden substituiert ist durch einen Substituenten ausgewählt aus:
Halogen-, Hydroxy-, Nitro-, C₁-C₆-Alkyl-, C₁-C₆-Alkoxy-, Hydroxy-C₁-C₆-alkyl-, -N(H)C(=O)R⁸, -N(H)C(=O) NR⁸R⁷, -C(=O)N(H) R⁸, -N(H)S(=O)₂R⁸;
R² für eine 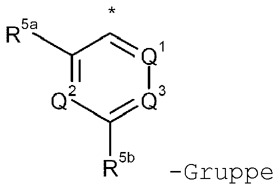 steht;
in welcher * den Verknüpfungspunkte der Gruppe mit dem Rest des Moleküls anzeigt;
Q¹ für eine Gruppe steht, die ausgewählt ist aus: N, CH, C-(C₁-C₆-Alkyl), C-(C₁-C₆-Alkoxy), C-Halogen;
Q² für eine Gruppe steht, die ausgewählt ist aus: N, CH, CR^{5b};
Q³ für eine Gruppe steht, die ausgewählt ist aus: N, CH, CR^{5b};
R^{5a} für eine Gruppe steht, die ausgewählt ist aus:
Halogen-, Nitro-, C₁-C₆-Alkyl-, Halogen-C₁-C₆-alkyl-, C₁-C₆-Alkoxy-, Halogen-C₁-C₆-alkoxy-, Hydroxy-C₁-C₆-alkyl-, C₁-C₆-Alkoxy-C₁-C₆-alkyl-, Halogen-C₁-C₆-alkoxy-C₁-C₆-alkyl-, R⁸-(C₁-C₆-Alkoxy) -, R⁸-O-, -NR⁸R⁷, R⁸-S-, R⁸-S(=O)-, R⁸-S(=O)₂-, (C₃-C₆-Cycloalkyl) - (CH₂)ₙ-O-;
R^{5b} für eine Gruppe steht, die ausgewählt ist aus:
Halogen-, Hydroxy-, Cyano-, Nitro-, C₁-C₆-Alkyl-, Halogen-C₁-C₆-alkyl-, C₁-C₆-Alkoxy-, Halogen-C₁-C₆-alkoxy-, Hydroxy-C₁-C₆-alkyl-, C₁-C₆-Alkoxy-C₁-C₆-alkyl-, Halogen-C₁-C₆-alkoxy-C₁-C₆-alkyl-, R⁸-(C₁-C₆-Alkyl)-, R⁸-(CH₂)ₙ(CHOH) (CH₂)ₘ-, R⁸-(C₁-C₆-Alkoxy) -, R⁸-(CH₂)ₙ(CHOH)(CH₂)ₚ-O-, R⁸-(C₁-C₆-Alkoxy-C₁-C₆-alkyl)-, R⁸-(C₁-C₆-Alkoxy-C₁-C₆-alkyl)-O-, -O-(CH₂)ₙ-C(=O)NR⁸R⁷, R⁸-O-, -C(=O)R⁸, -C(=O)O-R⁸, -OC(=O)-R⁸, -N(H)C(=O) R⁸, -N(R⁷)C(=O)R⁸, -N(H)C(=O)NR⁸R⁷, -N(R⁷)C(=O)NR⁸R⁷, -NR⁸R⁷, -NR⁷R⁷, -C(=O)N(H)R⁸, -C(=O)NR⁸R⁷, R⁸-S-, R⁸-S(=0)-, R⁸-S(=O)₂-, -N(H)S(=O)R⁸, -N(R⁷)S(=O)R⁸, -S(=O)N(H)R⁸, -S(=O)NR⁸R⁷, -N(H)S(=O)₂R⁸, -N(R⁷)S(=O) ₂R⁸, -S(=O)₂N(H)R⁸, -S(=O)₂NR⁸R⁷, -S(=O)(=NR⁸)R⁷, -S(=O)(=NR⁷)R⁸, -N=S(=O)(R⁸)R⁷;
R³ für ein Wasserstoffatom, ein Halogenatom, eine Hydroxy-, Amino-, Cyano-, Nitro-, C₁-C₄-Alkyl-, Halogen-C₁-C₄-alkyl-, C₁-C₄-Alkoxy-, Halogen-C₁-C₄-alkoxy-, Hydroxy-C₁-C₄-alkyl-, C₁-C₄-Alkoxy-C₁-C₄-alkyl-, Halogen-C₁-C₄-alkoxy-C₁-C₄-alkyl-, C₂-C₆-Alkenyl-, C₂-C₆-Alkinyl-, Halogen-C₂-C₆-alkenyl-, Halogen-C₂-C₆-alkinyl-, C₃-C₆-Cycloalkyl- oder Halogen-C₃-C₆-cycloalkylgruppe steht;
R⁴ für ein Wasserstoffatom, ein Halogenatom, eine Hydroxy-, Amino-, Cyano-, Nitro-, C₁-C₄-Alkyl-, Halogen-C₁-C₄-alkyl-, C₁-C₄-Alkoxy-, Halogen-C₁-C₄-alkoxy-, Hydroxy-C₁-C₄-alkyl-, C₁-C₄-Alkoxy-C₁-C₄-alkyl-, Halogen-C₁-C₄-alkoxy-C₁-C₄-alkyl-, C₂-C₆-Alkenyl-, C₂-C₆-Alkinyl-, Halogen-C₂-C₆-alkenyl-, Halogen-C₂-C₆-alkinyl-, C₃-C₆-Cycloalkyl- oder Halogen-C₃-C₆-cycloalkylgruppe steht;
R⁵ für ein Wasserstoffatom steht;
R⁶ für eine aus C₃-C₆-Cycloalkyl-, 3- bis 10-gliedrigem Heterocyclyl-, Aryl-, Heteroaryl-, -(CH₂)_{q}-(C₃-C₆-Cycloalkyl), -(CH₂)_{q}-(₃- bis 10-gliedriges Heterocyclyl), -(CH₂)_{q}-Aryl und -(CH₂)_{q}-Heteroaryl ausgewählte Gruppe steht, wobei diese Gruppe gegebenenfalls ein- oder mehrfach gleich oder verschieden substituiert ist durch einen Substituenten ausgewählt aus: Halogen-, Hydroxy-, Cyano-, Nitro-, C₁-C₆-Alkyl-, Halogen-C₁-C₆-alkyl-, C₁-C₆-Alkoxy-, Halogen-C₁-C₆-alkoxy-, Hydroxy-C₁-C₆-alkyl-, C₁-C₆-Alkoxy-C₁-C₆-alkyl-, Halogen-C₁-C₆-alkoxy-C₁-C₆-alkyl-, R⁸-(C₁-C₆-Alkyl)-, R⁸-(CH₂)ₙ(CHOH)(CH₂)ₘ-, R⁸-(C₁-C₆-Alkoxy)R⁸-(CH₂)ₙ(CHOH)(CH₂)ₚ-O-, R⁸-(C₁-C₆-Alkoxy-C₁-C₆-alkyl)-, R⁸-(C₁-C₆-Alkoxy-C₁-C₆-alkyl)-O-, Aryl-, R⁸-O-, -C(=O)R⁸, -C(=O)O-R⁸, -OC(=O)-R⁸, -N(H)C(=O)R⁸, -N(R⁷)C(=O)R⁸, -N(H)C(=O)NR⁸R⁷, -N(R⁷)C(=O)NR⁸R⁷, -NR⁸R⁷, -C(=O)N(H)R⁸, -C(=O)NR⁸R⁷, R⁸-S-, R⁸-S(=O)-, R⁸-S(=O)₂-, -N(H)S(=O)R⁸, -N(R⁷)S(=O)R⁸, -S(=O)N(H) R⁸, -S(=O)NR⁸R⁷, -N(H)S(=O)₂R⁸, -N(R⁷)S(=O)₂R⁸, -S(=O)₂N(H)R⁸, -S(=O)₂NR⁸R⁷, -S(=O)(=NR⁸)R⁷, -S(=O)(=NR⁷)R⁸, -N=S(=O)(R⁸) R⁷;
R⁷ für ein Wasserstoffatom, eine C₁-C₆-Alkyl- oder eine C₃-C₆-Cycloalkylgruppe steht;
R⁸ für ein Wasserstoffatom, eine C₁-C₆-Alkyl- oder eine C₃-C₆-Cycloalkylgruppe steht,
wobei die C₁-C₆-Alkyl- oder C₃-C₆-Cycloalkylgruppe gegebenenfalls ein- oder mehrfach gleich oder verschieden substituiert ist durch einen Substituenten ausgewählt aus:
Halogen-, Hydroxy-, -NHR⁷, -NR⁷R⁷, -N(C₁-C₃-Alkyl)-C(=O)R⁷, -N(C₁-C₃-Alkyl) -C(=O)OR⁷, C₁-C₃-Alkyl-, R⁷-S(=O)₂-, C₁-C₃-Alkoxy-, Halogen-C₁-C₃-alkoxy-;
n, m, p unabhängig voneinander für eine ganze Zahl 0, 1, 2 oder 3 stehen;
und
q für eine ganze Zahl 0, 1, 2 oder 3 steht;
oder ein Stereoisomer, ein Tautomer, ein N-Oxid, ein Hydrat, ein Solvat oder ein Salz davon oder eine Mischung dieser.

2. Verbindung nach Anspruch 1, wobei:
R¹ für eine Phenylgruppe steht,
- welche ein- oder mehrfach gleich oder verschieden substituiert ist durch einen Substituenten ausgewählt aus:
R⁶-(C₁-C₆-Alkoxy) -, R⁶-O-, -N(H)C(=O)R⁶, -N(H)C(=O)NR⁶R⁷, -C(=O)N(H)R⁶, -C(=O)NR⁶R⁷; und
- welche gegebenenfalls ein- oder mehrfach gleich oder verschieden substituiert ist durch einen Substituenten ausgewählt aus:
Halogen-, C₁-C₆-Alkyl-, C₁-C₆-Alkoxy-, -N(H)C(=O)R⁸, -C(=O)N(H)R⁸;
R³ für ein Wasserstoffatom, eine Halogen-, Hydroxy-, C₁-C₄-Alkyl-, Halogen-C₁-C₄-alkyl- oder C₁-C₄-Alkoxygruppe steht;
R⁴ für ein Wasserstoffatom, Halogen-, eine C₁-C₆-Alkyl-, Halogen-C₁-C₆-alkyl- oder C₁-C₆-Alkoxygruppe steht;
für eine Gruppe steht, die ausgewählt ist aus:
R^{5a} Halogen-, C₁-C₆-Alkyl-, C₁-C₆-Alkoxy-, Halogen-C₁-C₆-alkoxy-, Hydroxy-C₁-C₆-alkyl-, C₁-C₆-Alkoxy-C₁-C₆-alkyl-, Halogen-C₁-C₆-alkoxy-C₁-C₆-alkyl-, R⁸-(C₁-C₆-Alkoxy)-, R⁸-O-, R⁸-S-, R⁸-S(=O)₂-, (C₃-C₆-Cycloalkyl)-(CH2)ₙ-O-;
für eine Gruppe steht, die ausgewählt ist aus:
R^{5b} Halogen-, Cyano-, Nitro-, C₁-C₆-Alkyl-, Halogen-C₁-C₆-alkyl-, C₁-C₆-Alkoxy-, Halogen-C₁-C₆-alkoxy-, R⁸-O-, -C(=O)R⁸, -C(=O)O-R⁸, -N(H)C(=O)R⁸, -N(R⁷)C(=O)R⁸, -NR⁸R⁷, -NR⁷R⁷, -C(=O)N(H)R⁸, -C(=O)NR⁸R⁷, R⁸-S(=O)-, R⁸-S(=O)₂-, -S(=O)(=NR⁷)R⁸;
Q¹ für eine Gruppe steht, die ausgewählt ist aus: CH, C- (C₁-C₆-Alkyl), C-(C₁-C₆-Alkoxy), C-Halogen;
Q² für eine Gruppe steht, die ausgewählt ist aus: N, CH, CR^{5c};
wobei R^{5c} ausgewählt ist aus den Gruppen bestehend aus:
Halogen-, Cyano-, C₁-C₆-Alkyl-, C₁-C₆-Alkoxy-, -N (H) C (=O) R⁷, -N (R⁷) C (=O) R⁷, -C (=O) N (H) R⁸, -C (=O) NR⁸R⁷, R⁸-S (=O) -, R⁸-S(=O)₂-, -S (=O) (=NR⁷) R⁸;
Q³ für eine Gruppe steht, die ausgewählt ist aus: N, CH, CR^{5c};
wobei R^{5c} ausgewählt ist aus den Gruppen bestehend aus:
Halogen-, Cyano-, C₁-C₆-Alkyl-, C₁-C₆-Alkoxy-, -N (H) C (=O) R⁷, -N (R⁷) C (=O) R⁷, -C (=O) N (H) R⁸, -C(=O)NR⁸R⁷, R⁸-S (=O) -, R⁸-S(=O)₂-, -S (=O) (=NR⁷) R⁸;
R⁶ für eine Gruppe steht, die ausgewählt ist aus: C₃-C₆-Cycloalkyl-, 3- bis 10-gliedrigem Heterocyclyl-, Aryl-, Heteroaryl-, - (CH₂)_{q}-(C₃-C₆-Cycloalkyl), -(CH₂)_{q}-(3- bis 10-gliedriges Heterocyclyl), -(CH₂)_{q}-Aryl und -(CH₂)_{q}-Heteroaryl;
wobei diese Gruppe gegebenenfalls ein- oder mehrfach gleich oder verschieden substituiert ist durch einen Substituenten ausgewählt aus:
Halogen-, Hydroxy-, Cyano-, Nitro-, C₁-C₆-Alkyl-, Halogen-C₁-C₆-alkyl-, C₁-C₆-Alkoxy-, Halogen-C₁-C₆-alkoxy-, Hydroxy-C₁-C₆-alkyl-, C₁-C₆-Alkoxy-C₁-C₆-alkyl-, Halogen-C₁-C₆-alkoxy-C₁-C₆-alkyl- ;
n für eine ganze Zahl 0, 1 oder 2 steht;
q für eine ganze Zahl 0, 1 oder 2 steht;
oder ein Stereoisomer, ein Tautomer, ein N-Oxid, ein Hydrat, ein Solvat oder ein Salz davon oder eine Mischung dieser.

3. Verbindung nach Anspruch 1 oder 2, wobei:
R¹ für eine Phenylgruppe steht,
- welche ein- oder mehrfach gleich oder verschieden substituiert ist durch einen Substituenten ausgewählt aus:
-N(H)C(=O)R⁶, -C(=O)N(H)R⁶; und
- welche gegebenenfalls ein- oder mehrfach gleich oder verschieden substituiert ist durch einen Substituenten ausgewählt aus: Halogen-, C₁-C₆-Alkyl-, C₁-C₆-Alkoxy-;
R³ und R⁴ für ein Wasserstoffatom stehen;
Q¹ für CH steht;
Q² für CH steht;
Q³ für CH oder N steht;
R^{5a} für eine Gruppe steht, die ausgewählt ist aus: Halogen-, C₁-C₆-Alkyl-, C₁-C₆-Alkoxy-, Halogen-C₁-C₆-alkoxy-, (C₃-C₆-Cycloalkyl)-(CH₂)ₙ-O-;
R^{5b} für eine Gruppe steht, die ausgewählt ist aus: Halogen-, Cyano-, C₁-C₆-Alkoxy-, -N(H)C(=O)R⁸, -N(R⁷)C (=O)R⁸, -C(=O) N (H) R⁸, -C(=O)NR⁸R⁷, R⁸-S(=O)-, R⁸-S(=O)₂-, -S(=O)(=NR⁷)R⁸;
R⁶ für eine Gruppe steht, die ausgewählt ist aus: -(CH₂)_{q}-(C₃-C₆-Cycloalkyl) und -(CH₂)_{q}-Aryl;
wobei diese Gruppe gegebenenfalls ein- oder mehrfach gleich oder verschieden substituiert ist durch einen Substituenten ausgewählt aus:
Halogen-, Hydroxy-, Cyano-, Nitro-, C₁-C₆-Alkyl-, Halogen-C₁-C₆-alkyl-, C₁-C₆-Alkoxy-, Halogen-C₁-C₆-alkoxy-, Hydroxy-C₁-C₆-alkyl-, C₁-C₆-Alkoxy-C₁-C₆-alkyl-, Halogen-C₁-C₆-alkoxy-C₁-C₆-alkyl- ;
n für eine ganze Zahl 0, 1 oder 2 steht;
q für eine ganze Zahl 0, 1 oder 2 steht;
oder ein Stereoisomer, ein Tautomer, ein N-Oxid, ein Hydrat, ein Solvat oder ein Salz davon oder eine Mischung dieser.

4. Verbindung nach Anspruch 1, 2 oder 3, wobei:
R⁶ für eine Gruppe steht, die ausgewählt ist aus: -CH₂-(C₃-C₆-Cycloalkyl) und -(CH₂)_{q}-Aryl;
wobei diese Gruppe gegebenenfalls ein- oder mehrfach gleich oder verschieden substituiert ist durch einen Substituenten ausgewählt aus:
Halogen-, C₁-C₆-Alkyl-, Halogen-C₁-C₆-alkyl-, Halogen-C₁-C₆-alkoxy-;
n für eine ganze Zahl 0 oder 1 steht;
q für eine ganze Zahl 1 steht;
oder ein Stereoisomer, ein Tautomer, ein N-Oxid, ein Hydrat, ein Solvat oder ein Salz davon oder eine Mischung dieser.

5. Verbindung nach Anspruch 1, ausgewählt aus der Gruppe bestehend aus:
*N,N*-Diethyl-4-{[6-(4-{[(4-fluorphenyl)acetyl]-amino}phenyl)[1,2,4]triazolo[1,5-*a*]pyridin-2-yl]amino}-3-methoxybenzamid,
*N*-(4-{2-[(4-Cyano-2-methoxyphenyl)amino][1,2,4]-triazolo[1,5-*a*]pyridin-6-yl}phenyl)-2-(4-fluor-phenyl)acetamid,
*N*-(4-{2-[(2-Ethoxy-4-fluorphenyl)amino][1,2,4]-triazolo[1,5-*a*]pyridin-6-yl}phenyl)-2-(4-fluor-phenyl)acetamid,
*N*-Ethyl-4-{[6-(4-{[(4-fluorphenyl)acetyl]amino}-phenyl)[1,2,4]triazolo[1,5-*a*]pyridin-2-yl]amino}-3-methoxybenzamid,
*N*-*tert*-Butyl-4-{[6-(4-{[(4-fluorphenyl)acetyl]-amino}phenyl)[1,2,4]triazolo[1,5-*a*]pyridin-2-yl]-amino}-3-methoxybenzamid,
4-{[6-(4-{[(4-Fluorphenyl)acetyl]amino}phenyl)-[1,2,4]triazolo[1,5-*a*]pyridin-2-yl]amino}-*N*-(2-hydroxyethyl)-3-methoxybenzamid,
*N*-(2-Ethoxyethyl)-4-{[6-(4-{[(4-fluorphenyl)-acetyl]amino}phenyl)[1,2,4]triazolo[1,5-*a*]pyridin-2-yl]amino}-3-methoxybenzamid,
3-Eethoxy-4-{[6-(4-{[(4-fluorphenyl)acetyl]amino}-phenyl)[1,2,4]triazolo[1,5-*a*]pyridin-2-yl]amino}-N-(2-hydroxyethyl)benzamid,
3-Ethoxy-4-{[6-(4-{[(4-fluorphenyl)acetyl]amino}-phenyl)[1,2,4]triazolo[1,5-*a*]pyridin-2-yl]amino}-*N*-(1-hydroxy-2-methylpropan-2-yl)benzamid,
3-Ethoxy-*N,N*-diethyl-4-{[6-(4-{[(4-fluorphenyl)-acetyl]amino}phenyl)[1,2,4]triazolo[1,5-*a*]pyridin-2-yl]amino}benzamid,
4-{[6-(4-{[(4-Fluorphenyl)acetyl]amino}phenyl)-[1,2,4]triazolo[1,5-*a*]pyridin-2-yl]amino}-*N*-(2-hydroxyethyl)-3-(2,2,2-trifluorethoxy)benzamid,
4-{[6-(4-{[(4-Fluorphenyl)acetyl]amino}phenyl)-[1,2,4]triazolo[1,5-*a*]pyridin-2-yl]amino}-*N*-(2-hydroxy-2-methylpropyl)-3-methoxybenzamid,
4-{[6-(4-{[(4-Fluorphenyl)acetyl]amino}phenyl)-[1,2,4]triazolo[1,5-*a*]pyridin-2-yl]amino}-*N*-(1-hydroxy-2-methylpropan-2-yl)-3-methoxybenzamid,
*N*-{2-[Acetyl(methyl)amino]ethyl}-4-{[6-(4-{[(4-fluorphenyl)acetyl]amino}phenyl)[1,2,4]triazolo-[1,5-*a*]pyridin-2-yl]amino}-3-methoxy-*N*-methyl-benzamid,
2-(4-Fluorphenyl)-*N*-[4-(2-{[2-methoxy-4-(methyl-sulfonyl)phenyl]amino}[1,2,4]triazolo[1,5-*a*]-pyridin-6-yl)phenyl]acetamid,
3-Ethoxy-*N*-ethyl-4-{[6-(4-{[(4-fluorphenyl)-acetyl]amino}phenyl)[1,2,4]triazolo[1,5-*a*]pyridin-2-yl]amino}benzamid,
3-Ethoxy-4-{[6-(4-{[(4-fluorphenyl)acetyl]amino}-phenyl)[1,2,4]triazolo[1,5-*a*]pyridin-2-yl]amino}-*N*-(2-hydroxy-2-methylpropyl)benzamid,
3-Ethoxy-*N*-ethyl-4-{[6-(4-{[(4-fluorphenyl)-acetyl]amino}phenyl)[1,2,4]triazolo[1,5-*a*]pyridin-2-yl]amino}-*N*-(2-methoxyethyl)benzamid,
3-Ethoxy-4-{[6-(4-{[(4-fluorphenyl)acetyl]amino}-phenyl)[1,2,4]triazolo[1,5-*a*]pyridin-2-yl]amino}-*N*-(2-hydroxyethyl)-*N*-methylbenzamid,
4-{[6-(4-{[(4-Fluorphenyl)acetyl]amino}phenyl)-[1,2,4]triazolo[1,5-*a*]pyridin-2-yl]amino}-3-methoxy-*N*-methyl-*N*-[2-(methylamino)ethyl]benzamid,
*N*-*tert*-Butyl-4-{[6-(4-{[(4-fluorphenyl)acetyl]-amino}phenyl)[1,2,4]triazolo[1,5-*a*]pyridin-2-yl]-amino}-3-(2,2,2-trifluorethoxy)benzamid,
*N,N*-Diethyl-4-{[6-(4-{[(4-fluorphenyl)acetyl]-amino}phenyl)[1,2,4]triazolo[1,5-*a*]pyridin-2-yl]-amino}-3-(2,2,2-trifluorethoxy)benzamid,
*N,N*-Diethyl-4-{[6-(4-{[(4-fluorphenyl)acetyl]-amino}phenyl)[1,2,4]triazolo[1,5-*a*]pyridin-2-yl]-amino}-3-propoxybenzamid,
3-(Cyclopropylmethoxy)-*N,N*-diethyl-4-{[6-(4-{[(4-fluorphenyl)acetyl]amino}phenyl)[1,2,4]triazolo-[1,5-*a*]pyridin-2-yl]amino}benzamid,
*N,N*-Diethyl-4-{[6-(4-{[(4-fluorphenyl)acetyl]-amino}phenyl)[1,2,4]triazolo[1,5-*a*]pyridin-2-yl]-amino}-3-isopropoxybenzamid,
*N,N*-Diethyl-4-{[6-(4-{[(4-fluorphenyl)acetyl]-amino}phenyl)[1,2,4]triazolo[1,5-*a*]pyridin-2-yl]-amino}-3-(2-methoxyethoxy)benzamid,
4-{[6-(4-{[(4-Fluorphenyl)acetyl]amino}phenyl)-[1,2,4]triazolo[1,5-*a*]pyridin-2-yl]amino}-3-(2,2,2-trifluorethoxy)-*N*-(2,2,2-trifluorethyl)-benzamid,
3-Ethoxy-4-{[6-(4-{[(4-fluorphenyl)acetyl]amino}-phenyl)[1,2,4]triazolo[1,5-*a*]pyridin-2-yl]amino}-*N*-[2-(methylsulfonyl)ethyl]benzamid,
4-{[6-(4-{[(4-Fluorphenyl)acetyl]amino}phenyl)-[1,2,4]triazolo[1,5-*a*]pyridin-2-yl]aminol-*N*-(2-hydroxy-2-methylpropyl)-3-(2,2,2-trifluorethoxy)-benzamid,
4-{[6-(4-{[(4-Fluorphenyl)acetyl]amino}phenyl)-[1,2,4]triazolo[1,5-*a*]pyridin-2-yl]amino}-3-methoxy-*N*-methylbenzamid,
4-[(6-{4-[(4-Fluorbenzyl)carbamoyl]phenyl}[1,2,4]-triazolo[1,5-*a*]pyridin-2-yl)amino]-*N*-(2-hydroxy-ethyl)-3-methoxybenzamid,
3-Ethoxy-4-[(6-{4-[(4-fluorbenzyl)carbamoyl]-phenyl}[1,2,4]triazolo[1,5-*a*]pyridin-2-yl)amino]-*N*-(2-hydroxyethyl)benzamid,
3-Ethoxy-4-[(6-{4-[(4-fluorbenzyl)carbamoyl]-phenyl}[1,2,4]triazolo[1,5-*a*]pyridin-2-yl)amino]-*N*-(1-hydroxy-2-methylpropan-2-yl)benzamid,
4-[(6-{4-[(4-Fluorbenzyl)carbamoyl]phenyl}[1,2,4]-triazolo[1,5-*a*]pyridin-2-yl)amino]-*N*-(2-hydroxy-ethyl)-3-(2,2,2-trifluorethoxy)benzamid,
4-[(6-{4-[(4-Fluorbenzyl)carbamoyl]phenyl}[1,2,4]-triazolo[1,5-*a*]pyridin-2-yl)amino]-*N*-(2-hydroxy-2-methylpropyl)-3-methoxybenzamid,
*N*-(4-Fluorbenzyl)-4-(2-{[2-methoxy-4-(methylsulfonyl)phenyl]amino}[1,2,4]triazolo[1,5-*a*]-pyridin-6-yl)benzamid,
3-Ethoxy-4-[(6-{4-[(4-fluorbenzyl)carbamoyl]-phenyl}[1,2,4]triazolo[1,5-*a*]pyridin-2-yl)amino]-*N*-(2-hydroxy-2-methylpropyl)benzamid,
4-[(6-{4-[(4-Fluorbenzyl)carbamoyl]phenyl}[1,2,4]-triazolo[1,5-*a*]pyridin-2-yl)amino]-*N*-(1-hydroxy-2-methylprop-2-yl)-3-methoxybenzamid,
*N*-{2-[Acetyl(methyl)amino]ethyl}-4-[(6-{4-[(4-fluorbenzyl)carbamoyl]phenyl}[1,2,4]triazolo[1,5-*a*]pyridin-2-yl)amino]-3-methoxy-*N*-methylbenzamid,
*N*-(2-Ethoxyethyl)-4-[(6-{4-[(4-fluorbenzyl)-carbamoyl]phenyl}[1,2,4]triazolo[1,5-*a*]pyridin-2-yl)amino]-3-methoxybenzamid,
4-[(6-{4-[(4-Fluorbenzyl)carbamoyl]phenyl}[1,2,4]-triazolo[1,5-*a*]pyridin-2-yl)amino]-3-methoxy-*N-*methyl-*N*-[2-(methylamino)ethyl]benzamid,
3-Ethoxy-4-[(6-{4-[(4-fluorbenzyl)carbamoyl]-phenyl}[1,2,4]triazolo[1,5-*a*]pyridin-2-yl)amino]-*N*-(2-hydroxyethyl)-*N*-methylbenzamid,
4-[(6-{4-[(4-Fluorbenzyl)carbamoyl]phenyl}[1,2,4]-triazolo[1,5-*a*]pyridin-2-yl)amino]-3-methoxy-*N-*methylbenzamid,
*N*-tert-Butyl-4-[(6-{4-[(4-fluorbenzyl)carbamoyl]-phenyl}[1,2,4]triazolo[1,5-*a*]pyridin-2-yl)amino]-3-methoxybenzamid,
4-[(6-{4-[(4-Fluorbenzyl)carbamoyl]phenyl}[1,2,4]-triazolo[1,5-*a*]pyridin-2-yl)amino]-3-methoxy-*N*-[2-(methylsulfonyl)ethyl]benzamid,
4-{2-[(2,4-Dimethoxyphenyl)amino][1,2,4]triazolo-[1,5-*a*]pyridin-6-yl}-*N*-(4-fluorbenzyl)benzamid,
4-[(6-{4-[(4-Fluorbenzyl)carbamoyl]phenyl}[1,2,4]-triazolo[1,5-*a*]pyridin-2-yl)amino]-3-methoxy-*N-*(2,2,2-trifluorethyl)benzamid,
4-[(6-{4-[(4-Fluorbenzyl)carbamoyl]phenyl}[1,2,4]-triazolo[1,5-*a*]pyridin-2-yl)aminol-*N*-(1-hydroxy-2-methylpropan-2-yl)-3-(2,2,2-trifluorethoxy)benzamid,
3-Ethoxy-4-[(6-{4-[(4-fluorbenzyl)carbamoyl]-phenyl}[1,2,4]triazolo[1,5-*a*]pyridin-2-yl)amino]-*N*-[2-(methylsulfonyl)ethyl]benzamid,
*N*-Ethyl-4-[(6-{4-[(4-fluorbenzyl)carbamoyl]-phenyl}[1,2,4]triazolo[1,5-*a*]pyridin-2-yl)amino]-3-methoxybenzamid,
*N*-*tert*-Butyl-3-ethoxy-4-[(6-{4-[(4-fluorbenzyl)-carbamoyl]phenyl}[1,2,4]triazolo[1,5-*a*]pyridin-2-yl)amino]benzamid,
4-[(6-{4-[(4-Fluorbenzyl)carbamoyl]phenyl}[1,2,4]-triazolo[1,5-*a*]pyridin-2-yl)amino]-*N*-(2-hydroxy-2-methylpropyl)-3-(2,2,2-trifluorethoxy)benzamid,
4-[(6-{4-[(4-Fluorbenzyl)carbamoyl]phenyl}[1,2,4]-triazolo[1,5-*a*]pyridin-2-yl)amino]-3-(2,2,2-trifluorethoxy)-*N*-(2,2,2-trifluorethyl)benzamid,
4-(2-{[4-(Dimethylamino)-2-methylphenyl]amino}-[1,2,4]triazolo[1,5-*a*]pyridin-6-yl)-*N*-(4-fluor-benzyl)benzamid,
2-Fluor-*N*-(4-fluorbenzyl)-4-[2-({4-[(1-hydroxy-2-methylpropan-2-yl)carbamoyl]-2-methoxyphenyl}amino)[1,2,4]triazolo[1,5-*a*]pyridin-6-yl]benzamid,
*N*-(4-Fluorbenzyl)-4-[2-({4-[(1-hydroxy-2-methyl-propan-2-yl)carbamoyl]-2-methoxyphenyl}amino)-[1,2,4]triazolo[1,5-*a*]pyridin-6-yl]-2-methylbenz-amid,
2-Chlor-*N*-(4-fluorbenzyl)-4-[2-({4-[(1-hydroxy-2-methylpropan-2-yl)carbamoyl]-2-methoxyphenyl}amino)[1,2,4]triazolo[1,5-*a*]pyridin-6-yl]benzamid,
4-[(6-{4-[(Cyclopropylmethyl)carbamoyl]phenyl}-[1,2,4]triazolo[1,5-*a*]pyridin-2-yl)amino]-3-ethoxy-*N*-ethylbenzamid,
4-[(6-{4-[(Cyclopropylmethyl)carbamoyl]phenyl}-[1,2,4]triazolo[1,5-*a*]pyridin-2-yl)amino]-3-ethoxy-*N*-ethyl-N-(2-methoxyethyl)benzamid,
4-[(6-{4-[(Cyclopropylmethyl)carbamoyl]phenyl}-[1,2,4]triazolo[1,5-*a*]pyridin-2-yl)amino]-3-ethoxy-*N*-(2-hydroxyethyl)benzamid,
4-[(6-{4-[(Cyclopropylmethyl)carbamoyl]phenyl}-[1,2,4]triazolo[1,5-*a*]pyridin-2-yl)amino]-*N*-(2-hydroxyethyl)-3-(2,2,2-trifluorethoxy)benzamid,
4-[(6-{4-[(Cyclopropylmethyl)carbamoyl]phenyl}-[1,2,4]triazolo[1,5-*a*]pyridin-2-yl)amino]-*N*-ethyl-3-(2,2,2-trifluorethoxy)benzamid,
N-Ethyl-4-[(6-{4-[(3-fluorbenzyl)carbamoyl]-phenyl}[1,2,4]triazolo[1,5-*a*]pyridin-2-yl)amino]-3-methoxybenzamid,
*N*-(4-Fluorbenzyl)-4-[2-({4-[(1-hydroxy-2-methyl-propan-2-yl)carbamoyl]-2-methoxyphenyl}amino)-[1,2,4]triazolo[1,5-*a*]pyridin-6-yl]-2-methoxybenzamid,
4-{[6-(4-{[(4-Fluorphenyl)acetyl]amino}phenyl)-[1,2,4]triazolo[1,5-*a*]pyridin-2-yl]amino}-3-methoxy-*N*-(2,2,2-trifluorethyl)benzamid,
*N*-[4-(2-{[2-Ethoxy-4-(methylsulfonyl)phenyl]amino}-[1,2,4]triazolo[1,5-*a*]pyridin-6-yl)phenyl]-2-(4-fluorphenyl)acetamid,
*N*-[4-(2-{[2-Ethoxy-4-(ethylsulfonyl)phenyl]amino}-[1,2,4]triazolo[1,5-*a*]pyridin-6-yl)phenyl]-2-(4-fluorphenyl)acetamid,
2-(4-Fluorphenyl)-*N*-[4-(2-{[4-(methylsulfonyl)-2-(2,2,2-trifluorethoxy)phenyl]amino}[1,2,4]-triazolo[1,5-*a*]pyridin-6-yl)phenyl]acetamid,
*N*-[4-(2-{[2-(Difluormethoxy)-4-(methylsulfonyl)-phenyl]amino}[1,2,4]triazolo[1,5-*a*]pyridin-6-yl)-phenyl]-2-(4-fluorphenyl)acetamid,
*N*-[4-(2-{[2-(Difluormethoxy)-4-(ethylsulfonyl)-phenyl]amino}[1,2,4]triazolo[1,5-*a*]pyridin-6-yl)phenyl]-2-(4-fluorphenyl)acetamid,
*N*-[4-(2-{[2-(Cyclopropyloxy)-4-(methylsulfonyl)-phenyl]amino}[1,2,4]triazolo[1,5-*a*]pyridin-6-yl)-phenyl]-2-(4-fluorphenyl)acetamid,
4-(2-{[2-Ethoxy-4-(methylsulfonyl)phenyl]amino}-[1,2,4]triazolo[1,5-*a*]pyridin-6-yl)-*N*-(4-fluorbenzyl)benzamid,
4-(2-{[2-Ethoxy-4-(ethylsulfonyl)phenyl]amino}-[1,2,4]triazolo[1,5-*a*]pyridin-6-yl)-*N*-(4-fluorbenzyl)benzamid,
*N*-(4-Fluorbenzyl)-4-(2-{[4-(methylsulfonyl)-2-(2,2,2-trifluorethoxy)phenyl]amino}[1,2,4]-triazolo[1,5-*a*]pyridin-6-yl)benzamid,
4-(2-{[2-(Difluormethoxy)-4-(methylsulfonyl)-phenyl]amino}[1,2,4]triazolo[1,5-*a*]pyridin-6-yl)-*N*-(4-fluorbenzyl)benzamid,
4-(2-{[2-(Cyclopropyloxy)-4-(methylsulfonyl)-phenyl]amino}[1,2,4]triazolo[1,5-*a*]pyridin-6-yl)-*N*-(4-fluorbenzyl)benzamid,
*N*-(4-Fluorbenzyl)-2-methyl-4-(2-{[4-(methylsulfonyl)-2-(2,2,2-trifluorethoxy)phenyl]amino}-[1,2,4]triazolo[1,5-*a*]pyridin-6-yl)benzamid,
N-(4-Fluorbenzyl)-2-methoxy-4-(2-{[2-methoxy-4-(methylsulfonyl)phenyl]amino}[1,2,4]triazolo[1,5-*a*]pyridin-6-yl)benzamid,
2-(2,4-Difluorphenyl)-*N*-[4-(2-{[2-methoxy-4-(methylsulfonyl)phenyl]amino}[1,2,4]triazolo[1,5-*a*]pyridin-6-yl)phenyl]acetamid,
*N*-(2,4-Difluorbenzyl)-4-(2-{[2-methoxy-4-(methyl-sulfonyl)phenyl]amino}[1,2,4]triazolo[1,5-*a*]-pyridin-6-yl)benzamid,
4-{[6-(4-{[(4-Fluorphenyl)acetyl]amino}phenyl)-[1,2,4]triazolo[1,5-*a*]pyridin-2-yl]amino}-3-methoxy-*N*-(2-methoxyethyl)benzamid,
4-{[6-(4-{[(4-Fluorphenyl)acetyl]amino}phenyl)-[1,2,4]triazolo[1,5-*a*]pyridin-2-yl]amino}-3-methoxy-*N,N*-dimethylbenzamid,
4-{[6-(4-{[(4-Fluorphenyl)acetyl]amino}phenyl)-[1,2,4]triazolo[1,5-*a*]pyridin-2-yl]amino}-3-methoxy-*N*-[2-(methylsulfonyl)ethyl]benzamid,
4-{[6-(4-{[(4-Fluorphenyl)acetyl]amino}phenyl)-[1,2,4]triazolo[1,5-*a*]pyridin-2-yl]amino}-3-methoxybenzamid,
*N*-(2-Fluorethyl)-4-{[6-(4-{[(4-fluorphenyl)-acetyl]amino}phenyl)[1,2,4]triazolo[1,5-*a*]pyridin-2-yl]amino}-3-methoxybenzamid,
*N*-Ethyl-4-{[6-(4-{[(4-fluorphenyl)acetyl]amino}-phenyl)[1,2,4]triazolo[1,5-*a*]pyridin-2-yl]amino}-3-methoxy-*N*-methylbenzamid,
*N*-(2,2-Difluorethyl)-4-{[6-(4-{[(4-fluorphenyl)-acetyl]amino}phenyl)[1,2,4]triazolo[1,5-*a*]pyridin-2-yl]amino}-3-methoxybenzamid,
*N*-[2-(Dimethylamino)ethyl]-4-{[6-(4-{[(4-fluorphenyl)acetyl]amino}phenyl)[1,2,4]triazolo[1,5-*a*]-pyridin-2-yl]amino}-3-methoxy-*N*-methylbenzamid,
4-{[6-(4-{[(4-Fluorphenyl)acetyl]amino}phenyl)-[1,2,4]triazolo[1,5-*a*]pyridin-2-yl]amino}-3-methoxy-*N*-methyl-*N*-(2,2,2-trifluorethyl)benzamid,
*N*-[2-(Dimethylamino)ethyl]-4-{[6-(4-{[(4-fluorphenyl)acetyl]amino}phenyl)[1,2,4]triazolo[1,5-*a*]-pyridin-2-yl]amino}-3-methoxybenzamid,
4-{[6-(4-{[(4-Fluorphenyl)acetyl]amino}phenyl)-[1,2,4]triazolo[1,5-*a*]pyridin-2-yl]amino}-*N*-(3-fluorpropyl)-3-methoxybenzamid,
*N*-[4-(2-{[5-Fluor-2-methoxy-4-(methylsulfonyl)-phenyl]amino}[1,2,4]triazolo[1,5-*a*]pyridin-6-yl)phenyl]-2-(4-fluorphenyl)acetamid,
2-(4-Fluorphenyl)-*N*-[4-(2-{[2-methoxy-4-(methyl-sulfinyl)phenyl]amino}[1,2,4]triazolo[1,5-*a*]-pyridin-6-yl)phenyllacetamid,
*N*-[4-(2-{[5-Fluor-2-methoxy-4-(methylsulfinyl)-phenyl]amino}[1,2,4]triazolo[1,5-*a*]pyridin-6-yl)-phenyl]-2-(4-fluorphenyl)acetamid,
*N*-[4-(2-{[4-(*tert*-Butylsulfamoyl)-2-methoxyphenyl]-amino}[1,2,4]triazolo[1,5-*a*]pyridin-6-yl)phenyl]-2-(4-fluorphenyl)acetamid,
2-(4-Fluorphenyl)-*N*-[4-(2-{[4-(2-hydroxypropan-2-yl)-2-methoxyphenyl]amino}[1,2,4]triazolo[1,5-*a*]-pyridin-6-yl)phenyl]acetamid,
*N*-(4-{2-[(2,4-Dimethoxyphenyl)amino][1,2,4]-triazolo[1,5-*a*]pyridin-6-yl}phenyl)-2-(4-fluorphenyl)acetamid,
3-Ethoxy-*N*-ethyl-4-{[6-(4-{[(4-fluorphenyl)-acetyl]amino}phenyl)[1,2,4]triazolo[1,5-*a*]pyridin-2-yl]amino}-*N*-methylbenzamid,
*N*-[2-(Dimethylamino)ethyl]-3-ethoxy-4-{[6-(4-{[(4-fluorphenyl)acetyl]amino}phenyl)[1,2,4]triazolo-[1,5-*a*]pyridin-2-yl]amino}benzamid,
4-{[6-(4-{[(4-Fluorphenyl)acetyl]amino}phenyl)-[1,2,4]triazolo[1,5-*a*]pyridin-2-yl]amino}-*N*-[2-(methylsulfonyl)ethyl]-3-(2,2,2-trifluorethoxy)-benzamid,
4-{[6-(4-{[(4-Fluorphenyl)acetyl]amino}phenyl)-[1,2,4]triazolo[1,5-*a*]pyridin-2-yl]amino}-*N*-(1-hydroxy-2-methylpropan-2-yl)-3-(2,2,2-trifluor-ethoxy)benzamid,
*N*-[2-(Dimethylamino)ethyl]-4-{[6-(4-{[(4-fluorphenyl)acetyl]amino}phenyl)[1,2,4]triazolo[1,5-*a*]-pyridin-2-yl]amino}-3-(2,2,2-trifluorethoxy)-benzamid,
2-(4-Fluorphenyl)-*N*-[4-(2-{[4-(methylsulfinyl)-2-(2,2,2-trifluorethoxy)phenyl]amino}[1,2,4]-triazolo[1,5-*a*]pyridin-6-yl)phenyl]acetamid,
4-{[6-(4-{[(4-Fluorphenyl)acetyl]amino}phenyl)-[1,2,4]triazolo[1,5-*a*]pyridin-2-yl]amino}-3-(trifluormethoxy)benzamid,
*N*-[4-(2-{[2-(Difluormethoxy)-4-(propan-2-yl-sulfonyl)phenyl]amino}[1,2,4]triazolo[1,5-*a*]-pyridin-6-yl)phenyl]-2-(4-fluorphenyl)acetamid,
*N*-[4-(2-{[2-(Difluormethoxy)-4-fluorphenyl]amino}-[1,2,4]triazolo[1,5-*a*]pyridin-6-yl)phenyl]-2-(4-fluorphenyl)acetamid,
4-{[6-(4-{[(4-Fluorphenyl)acetyl]amino}phenyl)-[1,2,4]triazolo[1,5-*a*]pyridin-2-yl]amino}-3-methylbenzamid,
4-{[6-(4-{[(4-Fluorphenyl)acetyl]amino}phenyl)-[1,2,4]triazolo[1,5-*a*]pyridin-2-yl]amino}-3-methyl-*N*-(2,2,2-trifluorethyl)benzamid,
*N*-(2-Fluorethyl)-4-{[6-(4-{[(4-fluorphenyl)-acetyl]amino}phenyl)[1,2,4]triazolo[1,5-*a*]pyridin-2-yl]amino}-3-methylbenzamid,
4-{[6-(4-{[(4-Fluorphenyl)acetyl]amino}phenyl)-[1,2,4]triazolo[1,5-*a*]pyridin-2-yl]amino}-*N,N*,3-trimethylbenzamid,
2-(4-Fluorphenyl)-*N*-[4-(2-{[2-methyl-4-(methyl-sulfonyl)phenyl]amino}[1,2,4]triazolo[1,5-*a*]-pyridin-6-yl)phenyl]acetamid,
2-(4-Fluorphenyl)-*N*-[4-(2-{[2-methyl-4-(methyl-sulfinyl)phenyl]amino}[1,2,4]triazolo[1,5-*a*]-pyridin-6-yl)phenyl]acetamid,
2-(4-Fluorphenyl)-*N*-{4-[2-({2-methyl-4-[(methyl-sulfonyl)amino]phenyl}amino)[1,2,4]triazolo[1,5-*a*]pyridin-6-yl]phenyl}acetamid,
2-(4-Fluorphenyl)-*N*-(4-{2-[(4-methoxy-2-methyl-phenyl)amino][1,2,4]triazolo[1,5-*a*]pyridin-6-yl}-phenyl)acetamid,
*N*-[4-(2-{[4-(Dimethylamino)-2-methylphenyl]amino}-[1,2,4]triazolo[1,5-*a*]pyridin-6-yl)phenyl]-2-(4-fluorphenyl)acetamid,
*N*-Ethyl-5-{[6-(4-{[(4-fluorphenyl)acetyl]amino}-phenyl)[1,2,4]triazolo[1,5-*a*]pyridin-2-yl]amino}-4-methylpyridin-2-carboxamid,
5-{[6-(4-{[(4-Fluorphenyl)acetyl]amino}phenyl)-[1,2,4]triazolo[1,5-*a*]pyridin-2-yl]amino}-4-methyl-*N*-(2,2,2-trifluorethyl)pyridin-2-carboxamid,
*N*-[4-(2-{[2-Fluor-4-(methylsulfonyl)phenyl]amino}-[1,2,4]triazolo[1,5-*a*]pyridin-6-yl)phenyl]-2-(4-fluorphenyl)acetamid,
2-(4-Fluor-3-methylphenyl)-*N*-[4-(2-{[2-methoxy-4-(methylsulfonyl)phenyl]amino}[1,2,4]triazolo[1,5-*a*]pyridin-6-yl)phenyl]acetamid,
2-(4-Chlorphenyl)-*N*-[4-(2-{[2-methoxy-4-(methyl-sulfonyl)phenyl]amino}[1,2,4]triazolo[1,5-*a*]-pyridin-6-yl)phenyl]acetamid,
*N*-[4-(2-{[2-Methoxy-4-(methylsulfonyl)phenyl]-amino}[1,2,4]triazolo[1,5-*a*]pyridin-6-yl)phenyl]-2-phenylacetamid,
4-[(6-{4-[(4-Fluorbenzyl)carbamoyl]phenyl}[1,2,4]-triazolo[1,5-*a*]pyridin-2-yl)amino]-3-methoxybenzamid,
4-[(6-{4-[(4-Fluorbenzyl)carbamoyl]phenyl}[1,2,4]-triazolo[1,5-*a*]pyridin-2-yl)amino]-3-methoxy-*N*-(2-methoxyethyl)benzamid,
4-[(6-{4-[(4-Fluorbenzyl)carbamoyl]phenyl}[1,2,4]-triazolo[1,5-*a*]pyridin-2-yl)amino]-*N*-(2-fluorethyl)-3-methoxybenzamid,
4-[(6-{4-[(4-Fluorbenzyl)carbamoyl]phenyl}[1,2,4]-triazolo[1,5-*a*]pyridin-2-yl)amino]-3-methoxy-*N,N-*dimethylbenzamid,
*N*-(2,2-Difluorethyl)-4-[(6-{4-[(4-fluorbenzyl)-carbamoyl]phenyl}[1,2,4]triazolo[1,5-*a*]pyridin-2-yl)amino]-3-methoxybenzamid,
*N*-[2-(Dimethylamino)ethyl]-4-[(6-{4-[(4-fluorbenzyl)carbamoyl]phenyl}[1,2,4]triazolo[1,5-*a*]-pyridin-2-yl)amino]-3-methoxybenzamid,
*N*-[2-(Dimethylamino)ethyl]-4-[(6-{4-[(4-fluorbenzyl)carbamoyl]phenyl}[1,2,4]triazolo[1,5-*a*]-pyridin-2-yl)amino]-3-methoxy-*N*-methylbenzamid,
4-[(6-{4-[(4-Fluorbenzyl)carbamoyl]phenyl}[1,2,4]-triazolo[1,5-*a*]pyridin-2-yl)amino]-*N*-(3-fluorpropyl)-3-methoxybenzamid,
*N*-(4-Fluorbenzyl)-4-(2-{[2-methoxy-4-(methylsulfinyl)phenyl]amino}[1,2,4]triazolo[1,5-*a*]-pyridin-6-yl)benzamid,
4-(2-{[4-(*tert*-Butylsulfamoyl)-2-methoxyphenyl]-amino}[1,2,4]triazolo[1,5-*a*]pyridin-6-yl)-*N*-(4-fluorbenzyl)benzamid,
*N*-(4-Fluorbenzyl)-4-(2-{[4-(2-hydroxypropan-2-yl)-2-methoxyphenyl]amino}[1,2,4]triazolo[1,5-a]pyridin-6-yl)benzamid,
*N*-[2-(Dimethylamino)ethyl]-3-ethoxy-4-[(6-{4-[(4-fluorbenzyl)carbamoyl]phenyl}[1,2,4]triazolo[1,5-*a*]pyridin-2-yl)amino]benzamid,
3-Ethoxy-*N*-ethyl-4-[(6-{4-[(4-fluorbenzyl)-carbamoyl]phenyl}[1,2,4]triazolo[1,5-*a*]pyridin-2-yl)amino]-*N*-methylbenzamid,
3-Ethoxy-4-[(6-{4-[(4-fluorbenzyl)carbamoyl]-phenyl}[1,2,4]triazolo[1,5-*a*]pyridin-2-yl)amino]-*N*-(2-fluorethyl)benzamid,
*N*-[2-(Dimethylamino)ethyl]-4-[(6-{4-[(4-fluorbenzyl)carbamoyl]phenyl}[1,2,4]triazolo[1,5-*a*]-pyridin-2-yl)amino]-3-(2,2,2-trifluorethoxy)-benzamid,
4-[(6-{4-[(4-Fluorbenzyl)carbamoyl]phenyl}[1,2,4]-triazolo[1,5-*a*]pyridin-2-yl)amino]-*N*-(2-fluorethyl)-3-(2,2,2-trifluorethoxy)benzamid,
4-[(6-{4-[(4-Fluorbenzyl)carbamoyl]phenyl}[1,2,4]-triazolo[1,5-*a*]pyridin-2-yl)amino]-3-(trifluormethoxy)benzamid,
4-(2-{[2-(Difluormethoxy)-4-(ethylsulfonyl)-phenyl]amino}[1,2,4]triazolo[1,5-*a*]pyridin-6-yl)-*N*-(4-fluorbenzyl)benzamid,
4-(2-{[2-(Difluormethoxy)-4-(propan-2-ylsulfonyl)-phenyl]amino}[1,2,4]triazolo[1,5-*a*]pyridin-6-yl)-*N*-(4-fluorbenzyl)benzamid,
4-(2-{[2-(Difluormethoxy)-4-fluorphenyl]amino}-[1,2,4]triazolo[1,5-*a*]pyridin-6-yl)-*N*-(4-fluor-benzyl)benzamid,
*N*-Ethyl-4-[(6-{4-[(4-fluorbenzyl)carbamoyl]-phenyl}[1,2,4]triazolo[1,5-*a*]pyridin-2-yl)amino]-3-methylbenzamid,
4-[(6-{4-[(4-Fluorbenzyl)carbamoyl]phenyl}[1,2,4]-triazolo[1,5-*a*]pyridin-2-yl)amino]-3-methyl-benzamid,
4-[(6-{4-[(4-Fluorbenzyl)carbamoyl]phenyl}[1,2,4]-triazolo[1,5-*a*]pyridin-2-yl)amino]-3-methyl-*N-*(2,2,2-trifluorethyl)benzamid,
4-[(6-{4-[(4-Fluorbenzyl)carbamoyl]phenyl}[1,2,4]-triazolo[1,5-*a*]pyridin-2-yl)amino]-*N*-(2-fluor-ethyl)-3-methylbenzamid,
*N*-(4-Fluorbenzyl)-4-(2-{[2-methyl-4-(methyl-sulfonyl)phenyl]amino}[1,2,4]triazolo[1,5-*a*]-pyridin-6-yl)benzamid,
*N*-(4-Fluorbenzyl)-4-(2-{[2-methyl-4-(methyl-sulfinyl)phenyl]amino}[1,2,4]triazolo[1,5-*a*]-pyridin-6-yl)benzamid,
*N*-(4-Fluorbenzyl)-4-[2-({2-methyl-4-[(methylsulfonyl)amino]phenyl}amino)[1,2,4]triazolo[1,5-*a*]pyridin-6-yl]benzamid,
*N*-(4-Fluorbenzyl)-4-{2-[(4-methoxy-2-methyl-phenyl)amino][1,2,4]triazolo[1,5-*a*]pyridin-6-yl}-benzamid,
4-[(6-{4-[(4-Chlorbenzyl)carbamoyl]phenyl}[1,2,4]-triazolo[1,5-*a*]pyridin-2-yl)amino]-3-methoxy-*N-*(2,2,2-trifluorethyl)benzamid,
*N*-(4-Chlorbenzyl)-4-(2-{[2-methoxy-4-(methyl-sulfonyl)phenyl]amino}[1,2,4]triazolo[1,5-*a*]-pyridin-6-yl)benzamid,
*N*-(4-Chlorbenzyl)-4-(2-{[2-methyl-4-(methyl-sulfonyl)phenyl]amino}[1,2,4]triazolo[1,5-*a*]-pyridin-6-yl)benzamid,
*N*-(4-Chlorbenzyl)-4-(2-{[2-methyl-4-(methyl-sulfinyl)phenyl]amino}[1,2,4]triazolo[1,5-*a*]-pyridin-6-yl)benzamid,
4-(2-{[2-Methoxy-4-(methylsulfonyl)phenyl]amino}-[1,2,4]triazolo[1,5-*a*]pyridin-6-yl)-*N*-(4-methyl-benzyl)benzamid,
*N*-(4-Methylbenzyl)-4-(2-{[2-methyl-4-(methyl-sulfonyl)phenyl]amino}[1,2,4]triazolo[1,5-*a*]-pyridin-6-yl)benzamid,
*N*-(4-Methylbenzyl)-4-(2-{[2-methyl-4-(methyl-sulfinyl)phenyl]amino}[1,2,4]triazolo[1,5-*a*]-pyridin-6-yl)benzamid,
4-[(6-{4-[(2,4-Difluorbenzyl)carbamoyl]phenyl}-[1,2,4]triazolo[1,5-*a*]pyridin-2-yl)amino]-3-methoxy-*N*-(2,2,2-trifluorethyl)benzamid,
*N*-(2,4-Difluorbenzyl)-2-methyl-4-(2-{[4-(methylsulfonyl)-2-(2,2,2-trifluorethoxy)phenyl]amino}-[1,2,4]triazolo[1,5-*a*]pyridin-6-yl)benzamid,
*N*-(4-Fluorbenzyl)-4-(2-{[4-(2-hydroxypropan-2-yl)-2-methoxyphenyl]amino}[1,2,4]triazolo[1,5-*a*]pyridin-6-yl)-2-methoxybenzamid,
2-(4-Fluorphenyl)-*N*-[4-(2-{[2-methoxy-4-(*S*-methyl-sulfonimidoyl)phenyl]amino}[1,2,4]triazolo[1,5-*a*]-pyridin-6-yl)phenyl]acetamid,
*N*-(4-Fluorbenzyl)-4-(2-{[2-methoxy-4-(*S*-methyl-sulfonimidoyl)phenyl]amino}[1,2,4]triazolo[1,5-*a*]-pyridin-6-yl)benzamid,
2-(4-Fluorphenyl)-*N*-[4-(2-{[4-(hydroxymethyl)-2-methoxyphenyl]amino}[1,2,4]triazolo[1,5-*a*]pyridin-6-yl)phenyl]acetamid und
*N*-(4-Fluorbenzyl)-4-(2-{[4-(hydroxymethyl)-2-methoxyphenyl]amino}[1,2,4]triazolo[1,5-*a*]pyridin-6-yl)benzamid,
oder ein Stereoisomer, ein Tautomer, ein N-Oxid, ein Hydrat, ein Solvat oder ein Salz davon oder eine Mischung dieser.

6. Verfahren zur Herstellung einer Verbindung der allgemeinen Formel (I) nach einem der Ansprüche 1 bis 5, bei dem man ein Zwischenprodukt der allgemeinen Formel (5): 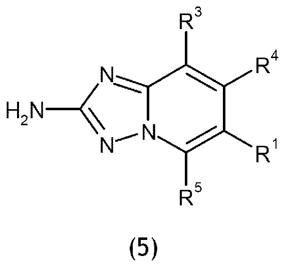 in welcher R¹, R³, R⁴ und R⁵ wie in einem der Ansprüche 1 bis 5 für die Verbindungen der allgemeinen Formel (I) definiert sind,
mit einem Arylhalogenid der allgemeinen Formel (5a):
R²-Y (5a)
in welcher R² wie in einem der Ansprüche 1 bis 5 für die Verbindungen der allgemeinen Formel (I) definiert ist und Y für eine Abgangsgruppe, wie zum Beispiel ein Halogenatom oder eine Trifluor-methylsulfonyloxy- oder Nonafluorbutylsulfonyloxygruppe, steht,
reagieren lässt und so eine Verbindung der allgemeinen Formel (I) : 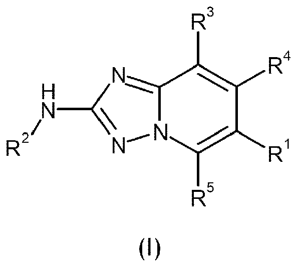 in welcher R¹, R², R³, R⁴ und R⁵ wie in einem der Ansprüche 1 bis 5 für die Verbindungen der allgemeinen Formel (I) definiert sind, bereitstellt.

7. Verfahren zur Herstellung einer Verbindung der allgemeinen Formel (I) nach einem der Ansprüche 1 bis 5, bei dem man ein Zwischenprodukt der allgemeinen Formel (7): 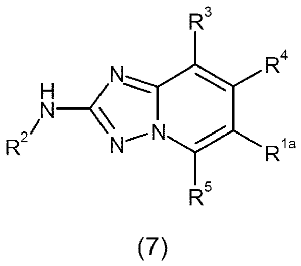 in welcher R², R³, R⁴ und R⁵ wie in einem der Ansprüche 1 bis 5 für die Verbindungen der allgemeinen Formel (I) definiert sind und R^{1a} für eine Arylgruppe, an die ein -NH₂-Substituent gebunden ist, steht,
mit einer Verbindung der allgemeinen Formel:
R^{1b}-X (7a)
in welcher R^{1b} für -C(=O)R⁶, -C(=O)NR⁶R⁷, -S(=O)R⁶, -S(=O)₂R⁶ steht und X für eine geeignete funktionelle Gruppe steht, über die das R^{1b} der R^{1b}-X-Verbindung (7a) mittels einer Kupplungsreaktion, wie zum Beispiel einer Amidkupplungsreaktion, an den an die Arylgruppe R^{1a} der Verbindung (7) gebundenen -NH₂-Substituenten gekuppelt werden kann, wodurch das X durch das R^{1a} ersetzt wird, reagieren lässt und so eine Verbindung der allgemeinen Formel (I) : 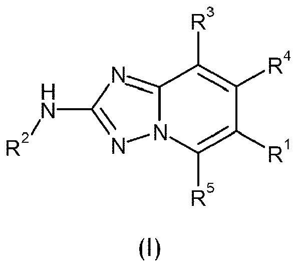 in welcher R¹, R², R³, R⁴ und R⁵ wie in einem der Ansprüche 1 bis 5 für die Verbindungen der allgemeinen Formel (I) definiert sind, bereitstellt.

8. Verbindung nach einem der Ansprüche 1 bis 5 oder ein Stereoisomer, ein Tautomer, ein N-Oxid, ein Hydrat, ein Solvat oder ein Salz davon, insbesondere ein pharmazeutisch unbedenkliches Salz davon, oder eine Mischung dieser, zur Verwendung bei der Behandlung oder Prophylaxe einer Krankheit.

9. Verbindung zur Verwendung nach Anspruch 8, wobei es sich bei der Krankheit um eine Krankheit mit unkontrolliertem Zellwachstum, unkontrollierter Zellproliferation und/oder unkontrolliertem Zellüberleben, eine unangemessene zelluläre Immunreaktion oder eine unangemessene zelluläre Entzündungsreaktion handelt, wobei das unkontrollierte Zellwachstum, die unkontrollierte Zellproliferation und/oder das unkontrollierte Zellüberleben, die unangemessene zelluläre Immunreaktion bzw. die unangemessene zelluläre Entzündungsreaktion insbesondere durch Mps-1 vermittelt wird, wobei es sich bei der Krankheit mit unkontrolliertem Zellwachstum, unkontrollierter Zellproliferation und/oder unkontrolliertem Zellüberleben, der unangemessenen zellulären Immunreaktion oder der unangemessenen zellulären Entzündungsreaktion spezieller um einen hämatologischen Tumor, einen soliden Tumor und/oder Metastasen davon, z.B. Leukämien und myelodysplastisches Syndrom, maligne Lymphome, Kopf- und Halstumore einschließlich Hirntumoren und Hirnmetastasen, Tumore des Thorax einschließlich nichtkleinzelliger und kleinzelliger Lungentumore, Tumore des Magen-Darm-Trakts, endokrine Tumore, Brust- und andere gynäkologische Tumore, urologische Tumore einschließlich Nieren-, Blasen- und Prostatatumoren, Hauttumore und Sarkome und/oder Metastasen davon handelt.

10. Pharmazeutische Zusammensetzung, umfassend eine Verbindung nach einem der Ansprüche 1 bis 5 oder ein Stereoisomer, ein Tautomer, ein N-Oxid, ein Hydrat, ein Solvat oder ein Salz davon, insbesondere ein pharmazeutisch unbedenkliches Salz davon, oder eine Mischung dieser und ein pharmazeutisch unbedenkliches Verdünnungsmittel oder einen pharmazeutisch unbedenklichen Träger.

11. Pharmazeutische Kombination, umfassend:
- eine oder mehrere Verbindungen nach einem der Ansprüche 1 bis 5 oder ein Stereoisomer, ein Tautomer, ein N-Oxid, ein Hydrat, ein Solvat oder ein Salz davon, insbesondere ein pharmazeutisch unbedenkliches Salz davon, oder eine Mischung dieser;
und
- ein oder mehrere Mittel ausgewählt aus: einem Taxan, wie Docetaxel, Paclitaxel oder Taxol; einem Epothilon, wie Ixabepilon, Patupilon oder Sagopilon; Mitoxantron; Prednisolon; Dexamethason; Estramustin; Vinblastin; Vincristin; Doxorubicin; Adriamycin; Idarubicin; Daunorubicin; Bleomycin; Etoposid; Cyclophosphamid; Ifosfamid; Procarbazin; Melphalan; 5-Fluoruracil; Capecitabin; Fludarabin; Cytarabin; Ara-C; 2-Chlor-2'-desoxyadenosin; Thioguanin; einem Antiandrogen, wie Flutamid, Cyproteronacetat oder Bicalutamid; Bortezomib; einem Platinderivat, wie Cisplatin oder Carboplatin; Chlorambucil; Methotrexat und Rituximab.

12. Verwendung einer Verbindung nach einem der Ansprüche 1 bis 5 oder eines Stereoisomers, eines Tautomers, eines N-Oxids, eines Hydrats, eines Solvats oder eines Salzes davon, insbesondere eines pharmazeutisch unbedenklichen Salzes davon, oder einer Mischung dieser zur Herstellung eines Medikaments zur Prophylaxe oder Behandlung einer Krankheit.

13. Verwendung nach Anspruch 12, wobei es sich bei der Krankheit um eine Krankheit mit unkontrolliertem Zellwachstum, unkontrollierter Zellproliferation und/oder unkontrolliertem Zellüberleben, eine unangemessene zelluläre Immunreaktion oder eine unangemessene zelluläre Entzündungsreaktion handelt, wobei das unkontrollierte Zellwachstum, die unkontrollierte Zellproliferation und/oder das unkontrollierte Zellüberleben, die unangemessene zelluläre Immunreaktion bzw. die unangemessene zelluläre Entzündungsreaktion insbesondere durch Mps-1 vermittelt wird, wobei es sich bei der Krankheit mit unkontrolliertem Zellwachstum, unkontrollierter Zellproliferation und/oder unkontrolliertem Zellüberleben, der unangemessenen zellulären Immunreaktion oder der unangemessenen zellulären Entzündungsreaktion spezieller um einen hämatologischen Tumor, einen soliden Tumor und/oder Metastasen davon, z.B. Leukämien und myelodysplastisches Syndrom, maligne Lymphome, Kopf- und Halstumore einschließlich Hirntumoren und Hirnmetastasen, Tumore des Thorax einschließlich nichtkleinzelliger und kleinzelliger Lungentumore, Tumore des Magen-Darm-Trakts, endokrine Tumore, Brust- und andere gynäkologische Tumore, urologische Tumore einschließlich Nieren-, Blasen- und Prostatatumoren, Hauttumore und Sarkome und/oder Metastasen davon handelt.

14. Verbindung der allgemeinen Formel (7): 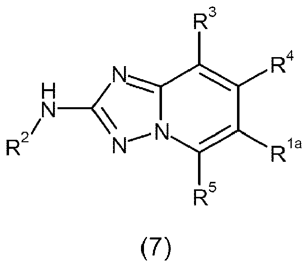 in welcher R², R³, R⁴ und R⁵ wie in einem der Ansprüche 1 bis 5 für die Verbindungen der allgemeinen Formel (I) definiert sind und R^{1a} für eine Arylgruppe, an die ein -NH₂-Substituent gebunden ist, steht.

15. Verwendung einer Verbindung der allgemeinen Formel (5) 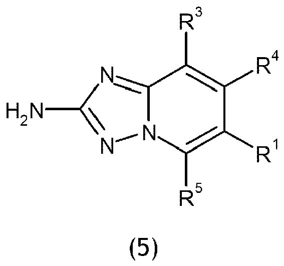 in welcher R¹, R³, R⁴ und R⁵ wie in einem der Ansprüche 1 bis 5 für die Verbindungen der allgemeinen Formel (I) definiert sind,
zur Herstellung einer Verbindung der allgemeinen Formel (I) nach einem der Ansprüche 1 bis 5.

16. Verwendung einer Verbindung der allgemeinen Formel (7) nach Anspruch 14 zur Herstellung einer Verbindung der allgemeinen Formel (I) nach einem der Ansprüche 1 bis 5.

## Revendications

1. Composé de formule générale (I) 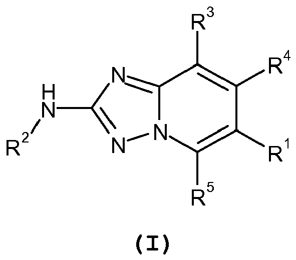 dans laquelle :
R¹ représente un groupe phényle ou pyridyle
- qui est substitué, une ou plusieurs fois, de façon identique ou de façon différente, par un substituant choisi parmi les substituants :
R⁶-(alcoxy en C₁-C₆)-, R⁶-O-, -C(=O)R⁶, -C(=O)OR⁶, -N (H) C (=O) R⁶, -N (H) C (=O) NR⁶R⁷, -NR⁶R⁷, -C (=O) N (H) R⁶, -C(=O)NR⁶R⁷, R⁶-S-, R⁶-S(=O)₂-, -N(H) S (=O)₂R⁶, -S (=O)₂N(H)R⁶ ; et
- qui est facultativement substitué, une ou plusieurs fois, de façon identique ou de façon différente, par un substituant choisi parmi les substituants :
halogéno-, hydroxy-, nitro-, (alkyle en C₁-C₆)-, (alcoxy en C₁-C₆)-, hydroxy-(alkyle en C₁-C₆)-, -N (H) C (=O) R⁸, -N (H) C (=O) NR⁸R⁷, -C (=O) N (H) R⁸, -N(H)S(=O)₂R⁸ ;
R² représente un groupe 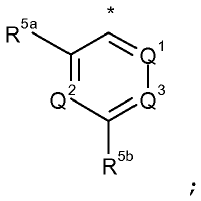 dans lequel
* indique le point d'attache dudit groupe au reste de la molécule ;
Q¹ représente un groupe choisi parmi les groupes : N, CH, C-(alkyle en C₁-C₆), C-(alcoxy en C₁-C₆), C-halogéno ;
Q² représente un groupe choisi parmi les groupes : N, CH, CR^{5b} ;
Q³ représente un groupe choisi parmi les groupes : N, CH, CR^{5b} ;
R^{5a} représente un groupe choisi parmi les groupes . halogéno-, nitro-, (alkyle en C₁-C₆)-, halogéno-(alkyle en C₁-C₆)-, (alcoxy en C₁-C₆)-, halogéno-(alcoxy en C₁-C₆)-, hydroxy-(alkyle en C₁-C₆)-, (alcoxy en C₁-C₆)-(alkyle en C₁-C₆-, halogéno-(alcoxy en C₁-C₆)-(alkyle en C₁-C₆-, R⁸-(alcoxy en C₁-C₆-, R⁸-O-, -NR⁸R⁷, R⁸-S-, R⁸-S (=O) -, R⁸-S(=O)₂-, (cycloalkyl en C₃-C₆)-(CH₂)ₙ-O- ;
R^{5b} représente un groupe choisi parmi les groupes : halogéno-, hydroxy-, cyano-, nitro-, (alkyle en C₁-C₆)-, halogéno-(alkyle en C₁-C₆)-, (alcoxy en C₁-C₆)-, halogéno-(alcoxy en C₁-C₆-, hydroxy- (alkyle en C₁-C₆)-, (alcoxy en C₁-C₆)-(alkyle en C₁-C₆)-, halogéno-(alcoxy en C₁-C₆)-(alkyle en C₁-C₆)-, R⁸-(alkyle en C₁-C₆)-, R⁸-(CH₂)ₙ(CHOH) (CH₂)ₘ-, R⁸-(alcoxy en C₁-C₆-, R⁸-(CH₂)ₙ (CHOH) (CH₂)ₚ-O-, R⁸-((alcoxy en C₁-C₆)- (alkyle en C₁-C₆))-, R⁸-((alcoxy en C₁-C₆-(alkyle en C₁-C6))-O-, -O-(CH₂)ₙ-C(=O)NR⁸R⁷, R⁸-O-, -C(=O)R⁸ -C(=O)O-R⁸ -OC(=O)-R⁸, -N (H) C (=O) R⁸, -N (R⁷) C (=O) R⁸, -N (H) C (=O) NR⁸R⁷, -N (R⁷) C (=O) NR⁸R⁷, -NR⁸R⁷, -NR⁷R⁷, -C (=O) N (H) R⁸, -C(=O)NR⁸R⁷, R⁸-S-, R⁸-S (=O) -, R⁸-S(=O)₂-, -N (H) S (=O) R⁸, -N (R⁷) S (=O) R⁸, -S (=O) N (H) R⁸, -S (=O) NR⁸R⁷, -N (H) S (=O) ₂R⁸, -N (R⁷) S (=O) ₂R⁸, -S (=O) ₂N (H) R⁸, -S (=O) ₂NR⁸R⁷, -S (=O) (=NR⁸) R⁷, -S (=O) (=NR⁷) R⁸, -N=S (=O) (R⁸) R⁷ ;
R³ représente un atome d'hydrogène, un atome d'halogène ou un groupe hydroxy-, amino-, cyano-, nitro-, (alkyle en C₁-C₄)-, halogéno-(alkyle en C₁-C₄)-, (alcoxy en C₁-C₄)-, halogéno-(alcoxy en C₁-C₄)-, hydroxy-(alkyle en C₁-C₄)-, (alcoxy en C₁-C₄)-(alkyle en C₁-C₄)-, halogéno-(alcoxy en C₁-C₄)-(alkyle en C₁-C₄)-, (alcényle en C₂-C₆)-, (alcynyle en C₂-C₆)-, halogéno-(alcényle en C₂-C₆)-, halogéno-(alcynyle en C₂-C₆)-, (cycloalkyle en C₃-C₆)- ou halogéno-(cycloalkyle en C₃-C₆)- ;
R⁴ représente un atome d'hydrogène, un atome d'halogène ou un groupe hydroxy-, amino-, cyano-, nitro-, (alkyle en C₁-C₄)-, halogéno-(alkyle en C₁-C₄)-, (alcoxy en C₁-C₄)-, halogéno-(alcoxy en C₁-C₄)-, hydroxy-(alkyle en C₁-C₄)-, (alcoxy en C₁-C₄)-(alkyle en C₁-C₄)-, halogéno-(alcoxy en C₁-C₄)-(alkyle en C₁-C₄)-, (alcényle en C₂-C₆)-, (alcynyle en C₂-C₆)-, halogéno-(alcényle en C₂-C₆)-, halogéno-(alcynyle en C₂-C₆)-, (cycloalkyle en C₃-C₆)- ou halogéno-(cycloalkyle en C₃-C₆)- ;
R⁵ représente un atome d'hydrogène ;
R⁶ représente un groupe choisi parmi les groupes (cycloalkyle en C₃-C₆)-, (hétérocyclyle à 3 à 10 chaînons)-, aryle-, hétéroaryle-, -(CH₂)_{q}-(cycloalkyle en C₃-C₆), -(CH₂)_{q}-(hétérocyclyle à 3 à 10 chaînons), -(CH₂)_{q}-aryle ou -(CH₂)_{q}-hétéroaryle, ledit groupe étant facultativement substitué, une ou plusieurs fois, de façon identique ou de façon différente, par un substituant choisi parmi les substituants :
halogéno-, hydroxy-, cyano-, nitro-, (alkyle en C₁-C₆)-, halogéno-(alkyle en C₁-C₆-, (alcoxy en C₁-C₆)-, halogéno-(alcoxy en C₁-C₆)-, hydroxy-(alkyle en C₁-C₆)-, (alcoxy en C₁-C₆)-(alkyle en C₁-C₆)-, halogéno-(alcoxy en C₁-C₆)- (alkyle en C₁-C₆)-, R⁸-(alkyle en C₁-C₆)-, R⁸-(CH₂)ₙ-(CHOH) (CH₂)ₘ-, R⁸-(alcoxy en C₁-C₆)-, R⁸-(CH₂), (CHOH) (CH₂)ₚ-O-, R⁸-((alcoxy en C₁-C₆)-(alkyle en C₁-C₆)-, R⁸-((alcoxy en C₁-C₆)-(alkyle en C₁-C₆))-O-, aryle-, R⁸-O-, -C(=O)R⁸, -C(=O)O-R⁸, -OC(=O)-R⁸, -N(H)C(=O)R⁸, -N(R⁷) C (=O) R⁸, -N (H) C (=O) NR⁸R⁷, -N (R⁷) C (=O) NR⁸R⁷, -NR⁸R⁷, -C(=O)N(H)R⁸, -C(=O)NR⁸R⁷, R⁸-S-, R⁸-S(=O)-, R⁸-S(=O)₂-, -N (H) S (=O) R⁸, -N (R⁷) S (=O) R⁸, -S (=O) N (H) R⁸, -S (=O) NR⁸R⁷, -N (H) S (=O) ₂R⁸, -N(R⁷) S (=O) ₂R⁸, -S (=O) ₂N (H) R⁸, -S (=O) ₂NR⁸R⁷, -S (=O) (=NR⁸) R⁷, -S (=O) (=NR⁷) R⁸, -N=S (=O) (R⁸) R⁷ ;
R⁷ représente un atome d'hydrogène ou un groupe (alkyle en C₁-C₆)- ou (cycloalkyle en C₃-C₆)- ;
R⁸ représente un atome d'hydrogène ou un groupe (alkyle en C₁-C₆)- ou (cycloalkyle en C₃-C₆)-, ledit groupe (alkyle en C₁-C₆)- ou (cycloalkyle en C₃-C₆)-étant facultativement substitué, une ou plusieurs fois, de façon identique ou de façon différente, par un substituant choisi parmi les substituants : halogéno-, hydroxy-, -NHR⁷, -NR⁷R⁷, -N(alkyl en C₁-C₃)-C(=O)R⁷, -N(alkyl en C₁-C₃)-C(=O)OR⁷, (alkyle en C₁-C₃)-, R⁷-S(=O)₂-, (alcoxy en C₁-C₃)-, halogéno-(alcoxy en C₁-C₃)- ;
n, m et p représentent, chacun indépendamment des autres, un nombre entier valant 0, 1, 2 ou 3 ;
et
q représente un nombre entier valant 0, 1, 2 ou 3 ;
ou stéréoisomère, tautomère, N-oxyde, hydrate, solvate ou sel de celui-ci ou mélange de ceux-ci.

2. Composé selon la revendication 1, dans lequel :
R¹ représente un groupe phényle
- qui est substitué, une ou plusieurs fois, de façon identique ou de façon différente, par un substituant choisi parmi les substituants : R⁶-(alcoxy en C₁-C₆)-, R⁶-O-, -N(H)C(=O)R⁶, -N(H)C(=O)NR⁶R⁷, -C(=O)N(H)R⁶, -C(=O)NR⁶R⁷ ; et
- qui est facultativement substitué, une ou plusieurs fois, de façon identique ou de façon différente, par un substituant choisi parmi les substituants :
halogéno-, (alkyle en C₁-C₆) -, (alcoxy en C₁-C₆) -, -N(H)C(=O)R⁸, -C(=O)N(H)R⁸ ;
R³ représente un atome d'hydrogène ou un groupe halogéno-, hydroxy-, (alkyle en C₁-C₄)-, halogéno-(alkyle en C₁-C₄) - ou (alcoxy en C₁-C₄) - ;
R⁴ représente un atome d'hydrogène ou un groupe halogéno-, (alkyle en C₁-C₆)-, halogéno-(alkyle en C₁-C₆) - ou (alcoxy en C₁-C₆) - ;
R^{5a} représente un groupe choisi parmi les groupes : halogéno-, (alkyle en C₁-C₆)-, (alcoxy en C₁-C₆)-, halogéno-(alcoxy en C₁-C₆)-, hydroxy-(alkyle en C₁-C₆)-, (alcoxy en C₁-C₆)-(alkyle en C₁-C₆)-, halogéno-(alcoxy en C₁-C₆)- (alkyle en C₁-C₆)-, R⁸-(alcoxy en C₁-C₆)-, R⁸-O-, R⁸-S-, R⁸-S(=O)₂-, (cycloalkyl en C₃-C₆)-(CH₂)ₙ-O-;
R^{5b} représente un groupe choisi parmi les groupes : halogéno-, cyano-, nitro-, (alkyle en C₁-C₆)-, halogéno-(alkyle en C₁-C₆)-, (alcoxy en C₁-C₆) -, halogéno-(alcoxy en C₁-C₆)-, R⁸-O-, -C(=O)R⁸, -C(=O)O-R⁸, -N (H) C (=O)R⁸, -N (R⁷) C (=O) R⁸, -NR⁸R⁷, -NR⁷R⁷, -C(=O)N (H) R⁸, -C(=O)NR⁸R⁷, R⁸-S(=O)-, R⁸-S(=O)₂-, -S(=O)(=NR⁷)R⁸ ;
Q¹ représente un groupe choisi parmi les groupes : CH, C-(alkyle en C₁-C₆), C-(alcoxy en C₁-C₆), C-halogéno ;
Q² représente un groupe choisi parmi les groupes : N, CH, C-R^{5c};
R^{5c} étant choisi dans le groupe constitué par les groupes :
halogéno-, cyano-, (alkyle en C₁-C₆)-, (alcoxy en C₁-C₆)-, -N(H)C(=O)R⁷, -N(R⁷)C(=O)R⁷, -C(=O)N(H)R⁸, -C(=O)NR⁸R⁷, R⁸-S(=O)-, R⁸-S(=O)₂-, -S(=O)(=NR⁷)R⁸ ;
Q³ représente un groupe choisi parmi les groupes : N, CH, C-R^{5c},
R^{5c} étant choisi dans le groupe constitué par les groupes :
halogéno-, cyano-, (alkyle en C₁-C₆)-, (alcoxy en C₁-C₆) -, -N(H)C(=O)R⁷, -N(R⁷)C(=O)R⁷, -C(=O)N(H)R⁸, -C(=O)NR⁸R⁷, R⁸-S(=O)-, R⁸-S(=O)₂-, -S(=O)(=NR⁷)R⁸ ;
R⁶ représente un groupe choisi parmi les groupes : (cycloalkyle en C₃-C₆)-, (hétérocyclyle à 3 à 10 chaînons) -, aryle-, hétéroaryle-, -(CH₂)_{q}-(cycloalkyle en C₃-C₆), -(CH₂)_{q}-(hétérocyclyle à 3 à 10 chaînons), - (CH₂)_{q}-aryle ou -(CH₂)_{q}-hétéroaryle, ledit groupe étant facultativement substitué, une ou plusieurs fois, de façon identique ou de façon différente, par un substituant choisi parmi les substituants :
halogéno-, hydroxy-, cyano-, nitro-, (alkyle en C₁-C₆)-, halogéno-(alkyle en C₁-C₆)-, (alcoxy en C₁-C₆)-, halogéno-(alcoxy en C₁-C₆)-, hydroxy-(alkyle en C₁-C₆)-, (alcoxy en C₁-C₆)-(alkyle en C₁-C₆)-, halogéno-(alcoxy en C₁-C₆)-(alkyle en C₁-C₆)- ;
n représente un nombre entier valant 0, 1 ou 2 ; et
q représente un nombre entier valant 0, 1 ou 2 ;
ou stéréoisomère, tautomère, N-oxyde, hydrate, solvate ou sel de celui-ci ou mélange de ceux-ci.

3. Composé selon la revendication 1 ou 2, dans lequel :
R¹ représente un groupe phényle
- qui est substitué, une ou plusieurs fois, de façon identique ou de façon différente, par un substituant choisi parmi les substituants :
-N(H)C(=O)R⁶, -C(=O)N(H)R⁶ ; et
- qui est facultativement substitué, une ou plusieurs fois, de façon identique ou de façon différente, par un substituant choisi parmi les substituants :
halogéno-, (alkyle en C₁-C₆)-, (alcoxy en C₁-C₆) - ;
R³ et R⁴ représentent chacun un atome d'hydrogène ;
Q¹ représente CH ;
Q² représente CH ;
Q³ représente CH ou N ;
R^{5a} représente un groupe choisi parmi les groupes :
halogéno-, (alkyle en C₁-C₆) -, (alcoxy en C₁-C₆)-, halogéno-(alcoxy en C₁-C₆)-, (cycloalkyl en C₃-C₆)-(CH₂)ₙ-O- ;
R^{5b} représente un groupe choisi parmi les groupes :
halogéno-, cyano-, (alcoxy en C₁-C₆)-, -N(H)C(=O)R⁸, -N(R⁷)C(=O)R⁸, -C(=O)N(H)R⁸, -C(=O)NR⁸R⁷, R⁸-S(=O)-, R⁸-S(=O)₂-, -S(=O)(=NR⁷) R⁸ ;
R⁶ représente un groupe choisi parmi les groupes :
-(CH₂)_{q}-(cycloalkyle en C₃-C₆) ou - (CH₂)_{q}-aryle ; ledit groupe étant facultativement substitué, une ou plusieurs fois, de façon identique ou de façon différente, par un substituant choisi parmi les substituants :
halogéno-, hydroxy-, cyano-, nitro-, (alkyle en C₁-C₆)-, halogéno- (alkyle en C₁-C₆)-, (alcoxy en C₁-C₆)-, halogéno-(alcoxy en C₁-C₆)-, hydroxy- (alkyle en C₁-C₆)-, (alcoxy en C₁-C₆)-(alkyle en C₁-C₆)-, halogéno- (alcoxy en C₁-C₆)-(alkyle en C₁-C₆)- ;
n représente un nombre entier valant 0, 1 ou 2 ; et
q représente un nombre entier valant 1 ou 2 ;
ou stéréoisomère, tautomère, N-oxyde, hydrate, solvate ou sel de celui-ci ou mélange de ceux-ci.

4. Composé selon l'une quelconque des revendications 1, 2 ou 3, dans lequel :
R⁶ représente un groupe choisi parmi les groupes :
-CH₂-(cycloalkyle en C₃-C₆) ou -CH₂-aryle ;
ledit groupe étant facultativement substitué, une ou plusieurs fois, de façon identique ou de façon différente, par un substituant choisi parmi les substituants :
halogéno-, (alkyle en C₁-C₆)-, halogéno-(alkyle en C₁-C₆)-, halogéno-(alcoxy en C₁-C₆)- ;
n représente un nombre entier valant 0 ou 1 ; et
q représente un nombre entier valant 1 ;
ou stéréoisomère, tautomère, *N*-oxyde, hydrate, solvate ou sel de celui-ci ou mélange de ceux-ci.

5. Composé selon la revendication 1, qui est choisi dans le groupe constitué par :
le *N*,*N*-diéthyl-4-{[6-(4-{[(4-fluorophényl)acétyl]amino}phényl)[1,2,4]triazolo[1,5-a]pyridin-2-yl]amino}-3-méthoxybenzamide,
le *N*-(4-{2-[(4-cyano-2-méthoxyphényl)amino][1,2,4]triazolo[1,5-a]pyridin-6-yl}phényl)-2-(4-fluorophényl)acétamide,
le *N*-(4-{2-[(2-éthoxy-4-fluorophényl)amino][1,2,4]triazolo[1,5-a]pyridin-6-yl}phényl)-2-(4-fluorophényl)acétamide,
le *N*-éthyl-4-{[6-(4-{[(4-fluorophényl)acétyl]amino}phényl)[1,2,4]triazolo[1,5-a]pyridin-2-yl]amino}-3-méthoxybenzamide,
le *N*-*tert*-butyl-4-{[6-(4-{[(4-fluorophényl)acétyl]amino}phényl)[1,2,4]triazolo[1,5-*a*]pyridin-2-yl]amino}-3-méthoxybenzamide,
le 4-{[6-(4-{[(4-fluorophényl)acétyl]amino}phényl)[1,2,4]triazolo[1,5-*a*]pyridin-2-yl]amino}-*N*-(2-hydroxyéthyl)-3-méthoxybenzamide,
le *N*-(2-éthoxyéthyl)-4-{[6-(4-{[(4-fluorophényl)acétyl]amino}phényl)[1,2,4]triazolo[1,5-a]pyridin-2-yl]amino}-3-méthoxybenzamide,
le 3-éthoxy-4-{[6-(4-{[(4-fluorophényl)acétyl]amino}phényl)[1,2,4]triazolo[1,5-a]pyridin-2-yl]amino}-*N*-(2-hydroxyéthyl)benzamide,
le 3-éthoxy-4-{[6-(4-{[(4-fluorophényl)acétyl]amino}phényl)[1,2,4]triazolo[1,5-*a*]pyridin-2-yl]amino}-*N*-(1-hydroxy-2-méthylprop-2-yl)benzamide,
le 3-éthoxy-*N*,*N*-diéthyl-4-{[6-(4-{[(4-fluorophényl)acétyl]amino}phényl)[1,2,4]triazolo[1,5-*a*]pyridin-2-yl]amino}benzamide,
le 4-{[6-(4-{[(4-fluorophényl)acétyl]amino}phényl)[1,2,4]triazolo[1,5-*a*]pyridin-2-yl]amino}-*N*-(2-hydroxyéthyl)-3-(2,2,2-trifluoroéthoxy)benzamide,
le 4-{[6-(4-{[(4-fluorophényl)acétyl]amino}phényl)[1,2,4]triazolo[1,5-*a*]pyridin-2-yl]amino}-*N*-(2-hydroxy-2-méthylpropyl)-3-méthoxybenzamide,
le 4-{[6-(4-{[(4-fluorophényl)acétyl]amino}phényl)[1,2,4]triazolo[1,5-*a*]pyridin-2-yl]amino}-*N*-(1-hydroxy-2-méthylprop-2-yl)-3-méthoxybenzamide,
le *N*-{2-[acétyl(méthyl)amino]éthyl}-4-{[6-(4-{[(4-fluorophényl)acétyl]amino}phényl)[1,2,4]triazolo[1,5-*a*]pyridin-2-yl]amino}-3-méthoxy-*N*-méthylbenzamide,
le 2-(4-fluorophényl)-*N*-[4-(2-{[2-méthoxy-4-(méthylsulfonyl)phényl]amino}[1,2,4]triazolo[1,5-*a*]pyridin-6-yl)phényl]acétamide,
le 3-éthoxy-*N*-éthyl-4-{[6-(4-{[(4-fluorophényl)acétyl]amino}phényl)[1,2,4]triazolo[1,5-*a*]pyridin-2-yl]amino}benzamide,
le 3-éthoxy-4-{[6-(4-{[(4-fluorophényl)acétyl]amino}phényl)[1,2,4]triazolo[1,5-*a*]pyridin-2-yl]amino}-*N*-(2-hydroxy-2-méthylpropyl)benzamide,
le 3-éthoxy-*N*-éthyl-4-{[6-(4-{[(4-fluorophényl)acétyl]amino}phényl)[1,2,4]triazolo[1,5-*a*]pyridin-2-yl]amino}-*N*-(2-méthoxyéthyl)benzamide,
le 3-éthoxy-4-{[6-(4-{[(4-fluorophényl)acétyl]amino}phényl)[1,2,4]triazolo[1,5-*a*]pyridin-2-yl]amino}-*N*-(2-hydroxyéthyl)-*N-*méthylbenzamide,
le 4-{[6-(4-{[(4-fluorophényl)acétyl]amino}phényl)[1,2,4]triazolo[1,5-*a*]pyridin-2-yl]amino}-3-méthoxy-*N*-méthyl-*N*-[2-(méthylamino)éthyl]benzamide,
le *N*-*tert*-butyl-4-{[6-(4-{[(4-fluorophényl)acétyl]amino}phényl)[1,2,4]triazolo[1,5-*a*]pyridin-2-yl]amino}-3-(2,2,2-trifluoroéthoxy)benzamide,
le *N*,*N*-diéthyl-4-{[6-(4-{[(4-fluorophényl)acétyl]amino}phényl)[1,2,4]triazolo[1,5-a]pyridin-2-yl]amino}-3-(2,2,2-trifluoroéthoxy)benzamide,
le *N*,*N*-diéthyl-4-{[6-(4-{[(4-fluorophényl)acétyl]amino}phényl)[1,2,4]triazolo[1,5-a]pyridin-2-yl]amino}-3-propoxybenzamide,
le 3-(cyclopropylméthoxy)-*N*,*N*-diéthyl-4-{[6-(4-{[(4-fluorophényl)acétyl]amino}phényl)[1,2,4]triazolo[1,5-a]pyridin-2-yl]amino}benzamide,
le *N*,*N*-diéthyl-4-{[6-(4-{[(4-fluorophényl)acétyl]amino}phényl)[1,2,4]triazolo[1,5-*a*]pyridin-2-yl]amino}-3-isopropoxybenzamide,
le *N*,*N*-diéthyl-4-{[6-(4-{[(4-fluorophényl)acétyl]amino}phényl)[1,2,4]triazolo[1,5-*a*]pyridin-2-yl]amino}-3-(2-méthoxyéthoxy)benzamide,
le 4-{[6-(4-{[(4-fluorophényl)acétyl]amino}phényl)[1,2,4]triazolo[1,5-*a*]pyridin-2-yl]amino}-3-(2,2,2-trifluoroéthoxy)-*N-*(2,2,2-trifluoroéthyl)benzamide,
le 3-éthoxy-4-{[6-(4-{[(4-fluorophényl)acétyl]amino}phényl)[1,2,4]triazolo[1,5-*a*]pyridin-2-yl]amino}-*N*-[2-(méthylsulfonyl)éthyl]benzamide,
le 4-{[6-(4-{[(4-fluorophényl)acétyl]amino}phényl)[1,2,4]triazolo[1,5-*a*]pyridin-2-yl]amino}-*N*-(2-hydroxy-2-méthylpropyl)-3-(2,2,2-trifluoroéthoxy)benzamide,
le 4-{[6-(4-{[(4-fluorophényl)acétyl]amino}phényl)[1,2,4]triazolo[1,5-*a*]pyridin-2-yl]amino}-3-méthoxy-*N*-méthylbenzamide,
le 4-[(6-{4-[(4-fluorobenzyl)carbamoyl]phényl}[1,2,4]triazolo[1,5-a]pyridin-2-yl)amino]-*N*-(2-hydroxyéthyl)-3-méthoxybenzamide,
le 3-éthoxy-4-[(6-{4-[(4-fluorobenzyl)carbamoyl]phényl}[1,2,4]triazolo[1,5-*a*]pyridin-2-yl)amino]-*N*-(2-hydroxyéthyl)benzamide,
le 3-éthoxy-4-[(6-{4-[(4-fluorobenzyl)carbamoyl]phényl}[1,2,4]triazolo[1,5-*a*]pyridin-2-yl)amino]-*N*-(1-hydroxy-2-méthylprop-2-yl)benzamide,
le 4-[(6-{4-[(4-fluorobenzyl)carbamoyl]phényl}[1,2,4]triazolo[1,5-*a*]pyridin-2-yl)amino]-*N*-(2-hydroxyéthyl)-3-(2,2,2-trifluoroéthoxy)benzamide,
le 4-[(6-{4-[(4-fluorobenzyl)carbamoyl]phényl}[1,2,4]triazolo[1,5-*a*]pyridin-2-yl)amino]-*N*-(2-hydroxy-2-méthylpropyl)-3-méthoxybenzamide,
le *N*-(4-fluorobenzyl)-4-(2-{[2-méthoxy-4-(méthylsulfonyl)phényl]amino}[1,2,4]triazolo[1,5-*a*]pyridin-6-yl)benzamide,
le 3-éthoxy-4-[(6-{4-[(4-fluorobenzyl)carbamoyl]phényl}[1,2,4]triazolo[1,5-*a*]pyridin-2-yl)amino]-*N*-(2-hydroxy-2-méthylpropyl)benzamide,
le 4-[(6-{4-[(4-fluorobenzyl)carbamoyl]phényl}[1,2,4]triazolo[1,5-*a*]pyridin-2-yl)amino]-*N*-(1-hydroxy-2-méthylprop-2-yl)-3-méthoxybenzamide,
le *N*-{2-[acétyl(méthyl)amino]éthyl}-4-[(6-{4-[(4-fluorobenzyl)carbamoyl]phényl}[1,2,4]triazolo[1,5-*a*]pyridin-2-yl)amino]-3-méthoxy-*N*-méthylbenzamide,
le *N*-(2-éthoxyéthyl)-4-[(6-{4-[(4-fluorobenzyl)carbamoyl]phényl}[1,2,4]triazolo[1,5-*a*]pyridin-2-yl)amino]-3-méthoxybenzamide,
le 4-[(6-{4-[(4-fluorobenzyl)carbamoyl]phényl}[1,2,4]triazolo[1,5-*a*]pyridin-2-yl)amino]-3-méthoxy-*N*-méthyl-*N*-[2-(méthylamino)éthyl]benzamide,
le 3-éthoxy-4-[(6-{4-[(4-fluorobenzyl)carbamoyl]phényl}[1,2,4]triazolo[1,5-*a*]pyridin-2-yl)amino]-N-(2-hydroxyéthyl)-*N-*méthylbenzamide,
le 4-[(6-{4-[(4-fluorobenzyl)carbamoyl]phényl}[1,2,4]triazolo[1,5-a]pyridin-2-yl)amino]-3-méthoxy-*N*-méthylbenzamide,
le *N*-*tert*-butyl-4-[(6-{4-[(4-fluorobenzyl)carbamoyl]phényl}[1,2,4]triazolo[1,5-*a*]pyridin-2-yl)amino]-3-méthoxybenzamide,
le 4-[(6-{4-[(4-fluorobenzyl)carbamoyl]phényl}[1,2,4]triazolo[1,5-*a*]pyridin-2-yl)amino]-3-méthoxy-*N*-[2-(méthylsulfonyl)éthyl]benzamide,
le 4-{2-[(2,4-diméthoxyphényl)amino][1,2,4]triazolo[1,5-*a*]pyridin-6-yl}-*N*-(4-fluorobenzyl)benzamide,
le 4-[(6-{4-[(4-fluorobenzyl)carbamoyl]phényl}[1,2,4]triazolo[1,5-*a*]pyridin-2-yl)amino]-3-méthoxy-*N*-(2,2,2-trifluoroéthyl)benzamide,
le 4-[(6-{4-[(4-fluorobenzyl)carbamoyl]phényl}[1,2,4]triazolo[1,5-*a*]pyridin-2-yl)amino]-*N*-(1-hydroxy-2-méthylprop-2-yl)-3-(2,2,2-trifluoroéthoxy)benzamide,
le 3-éthoxy-4-[(6-{4-[(4-fluorobenzyl)carbamoyl]phényl}[1,2,4]triazolo[1,5-*a*]pyridin-2-yl)amino]-*N*-[2-(méthylsulfonyl)éthyl]benzamide,
le *N*-éthyl-4-[(6-{4-[(4-fluorobenzyl)carbamoyl]phényl}[1,2,4]triazolo[1,5-*a*]pyridin-2-yl)amino]-3-méthoxybenzamide,
le *N*-*tert*-butyl-3-éthoxy-4-[(6-{4-[(4-fluorobenzyl)carbamoyl]phényl}[1,2,4]triazolo[1,5-*a*]pyridin-2-yl)amino]benzamide,
le 4-[(6-{4-[(4-fluorobenzyl)carbamoyl]phényl}[1,2,4]triazolo[1,5-*a*]pyridin-2-yl)amino]-*N*-(2-hydroxy-2-méthylpropyl)-3-(2,2,2-trifluoroéthoxy)benzamide,
le 4-[(6-{4-[(4-fluorobenzyl)carbamoyl]phényl}[1,2,4]triazolo[1,5-*a*]pyridin-2-yl)amino]-3-(2,2,2-trifluoroéthoxy)-*N-*(2,2,2-trifluoroéthyl)benzamide,
le 4-(2-{[4-(diméthylamino)-2-méthylphényl]amino}[1,2,4]triazolo[1,5-*a*]pyridin-6-yl)-*N*-(4-fluorobenzyl)benzamide,
le 2-fluoro-*N*-(4-fluorobenzyl)-4-[2-({4-[(1-hydroxy-2-méthylprop-2-yl)carbamoyl]-2-méthoxyphényl}amino)[1,2,4]triazolo[1,5-*a*]pyridin-6-yl]benzamide,
le *N*-(4-fluorobenzyl)-4-[2-({4-[(1-hydroxy-2-méthylprop-2-yl)carbamoyl]-2-méthoxyphényl}amino)[1,2,4]triazolo[1,5-*a*]pyridin-6-yl]-2-méthylbenzamide,
le 2-chloro-*N*-(4-fluorobenzyl)-4-[2-({4-[(1-hydroxy-2-méthylprop-2-yl)carbamoyl]-2-méthoxyphényl}amino)[1,2,4]triazolo[1,5-*a*]pyridin-6-yl]benzamide,
le 4-[(6-{4-[(cyclopropylméthyl)carbamoyl]phényl}[1,2,4]triazolo[1, 5-*a*]pyridin-2-yl)amino]-3-éthoxy-*N*-éthylbenzamide,
le 4-[(6-{4-[(cyclopropylméthyl)carbamoyl]phényl}[1,2,4]triazolo[1, 5-a]pyridin-2-yl)amino]-3-éthoxy-*N*-éthyl-*N*-(2-méthoxyéthyl)benzamide,
le 4-[(6-{4-[(cyclopropylméthyl)carbamoyl]phényl}[1,2,4]triazolo[1, 5-*a*]pyridin-2-yl)amino]-3-éthoxy-*N*-(2-hydroxyéthyl)benzamide,
le 4-[(6-{4-[(cyclopropylméthyl)carbamoyl]phényl}[1,2,4]triazolo[1, 5-*a*]pyridin-2-yl)amino]-*N*-(2-hydroxyéthyl)-3-(2,2,2-trifluoroéthoxy)benzamide,
le 4-[(6-{4-[(cyclopropylméthyl)carbamoyl]phényl}[1,2,4]triazolo[1, 5-a]pyridin-2-yl)amino]-*N*-éthyl-3-(2,2,2-trifluoroéthoxy)benzamide,
le *N*-éthyl-4-[(6-{4-[(3-fluorobenzyl)carbamoyl]phényl}[1,2,4]triazolo[1,5-*a*]pyridin-2-yl)amino]-3-méthoxybenzamide,
le *N*-(4-fluorobenzyl)-4-[2-({4-[(1-hydroxy-2-méthylprop-2-yl)carbamoyl]-2-méthoxyphényl}amino)[1,2,4]triazolo[1,5-*a*]pyridin-6-yl]-2-méthoxybenzamide,
le 4-{[6-(4-{[(4-fluorophényl)acétyl]amino}phényl)[1,2,4]triazolo[1,5-*a*]pyridin-2-yl]amino}-3-méthoxy-*N*-(2,2,2-trifluoroéthyl)benzamide,
le *N*-[4-(2-{[2-éthoxy-4-(méthylsulfonyl)phényl]amino}[1,2,4]triazolo[1,5-*a*]pyridin-6-yl)phényl]-2-(4-fluorophényl)acétamide,
le *N*-[4-(2-{[2-éthoxy-4-(éthylsulfonyl)phényl]amino}[1,2,4]triazolo[1,5-*a*]pyridin-6-yl)phényl]-2-(4-fluorophényl)acétamide,
le 2-(4-fluorophényl)-*N*-[4-(2-{[4-(méthylsulfonyl)-2-(2,2,2-trifluoroéthoxy)phényl]amino}[1,2,4]triazolo[1,5-*a*]pyridin-6-yl)phényl]acétamide,
le *N*-[4-(2-{[2-(difluorométhoxy)-4-(méthylsulfonyl)phényl]amino}[1,2,4]triazolo[1,5-*a*]pyridin-6-yl)phényl]-2-(4-fluorophényl)acétamide,
le *N*-[4-(2-{[2-(difluorométhoxy)-4-(éthylsulfonyl)phényl]amino}[1,2,4]triazolo[1,5-*a*]pyridin-6-yl)phényl]-2-(4-fluorophényl)acétamide,
le *N*-[4-(2-{[2-(cyclopropyloxy)-4-(méthylsulfonyl)phényl]amino}[1,2,4]triazolo[1,5-*a*]pyridin-6-yl)phényl]-2-(4-fluorophényl)acétamide,
le 4-(2-{[2-éthoxy-4-(méthylsulfonyl)phényl]amino}[1,2,4]triazolo[1,5-*a*]pyridin-6-yl)-N-(4-fluorobenzyl)benzamide,
le 4-(2-{[2-éthoxy-4-(éthylsulfonyl)phényl]amino}[1,2,4]triazolo[1,5-*a*]pyridin-6-yl)-*N*-(4-fluorobenzyl)benzamide,
le *N*-(4-fluorobenzyl)-4-(2-{[4-(méthylsulfonyl)-2-(2,2,2-trifluoroéthoxy)phényl]amino}[1,2,4]triazolo[1,5-*a*]pyridin-6-yl)benzamide,
le 4-(2-{[2-(difluorométhoxy)-4-(méthylsulfonyl)phényl]amino}[1,2,4]triazolo[1,5-*a*]pyridin-6-yl)-*N*-(4-fluorobenzyl)benzamide,
le 4-(2-{[2-(cyclopropyloxy)-4-(méthylsulfonyl)phényl]amino}[1,2,4]triazolo[1,5-*a*]pyridin-6-yl)-*N*-(4-fluorobenzyl)benzamide,
le *N*-(4-fluorobenzyl)-2-méthyl-4-(2-{[4-(méthylsulfonyl)-2-(2,2,2-trifluoroéthoxy)phényl]amino}[1,2,4]triazolo[1,5-*a*]pyridin-6-yl)benzamide,
le *N*-(4-fluorobenzyl)-2-méthoxy-4-(2-{[2-méthoxy-4-(méthylsulfonyl)phényl]amino}[1,2,4]triazolo[1,5-*a*]pyridin-6-yl)benzamide,
le 2-(2,4-difluorophényl)-*N*-[4-(2-{[2-méthoxy-4-(méthylsulfonyl)phényl]amino}[1,2,4]triazolo[1,5-*a*]pyridin-6-yl)phényl]acétamide,
le *N*-(2,4-difluorobenzyl)-4-(2-{[2-méthoxy-4-(méthylsulfonyl)phényl]amino}[1,2,4]triazolo[1,5-*a*]pyridin-6-yl)benzamide,
le 4-{[6-(4-{[(4-fluorophényl)acétyl]amino}phényl)[1,2,4]triazolo[1,5-*a*]pyridin-2-yl]amino}-3-méthoxy-*N*-(2-méthoxyéthyl)benzamide,
le 4-{[6-(4-{[(4-fluorophényl)acétyl]amino}phényl)[1,2,4]triazolo[1,5-*a*]pyridin-2-yl]amino}-3-méthoxy-*N*,*N*-diméthylbenzamide,
le 4-{[6-(4-{[(4-fluorophényl)acétyl]amino}phényl)[1,2,4]triazolo[1,5-*a*]pyridin-2-yl]amino}-3-méthoxy-*N*-[2-(méthylsulfonyl)éthyl]benzamide,
le 4-{[6-(4-{[(4-fluorophényl)acétyl]amino}phényl)[1,2,4]triazolo[1,5-*a*]pyridin-2-yl]amino}-3-méthoxybenzamide,
le *N*-(2-fluoroéthyl)-4-{[6-(4-{[(4-fluorophényl)acétyl]amino}phényl)[1,2,4]triazolo[1,5-*a*]pyridin-2-yl]amino}-3-méthoxybenzamide,
le *N*-éthyl-4-{[6-(4-{[(4-fluorophényl)acétyl]amino}phényl)[1,2,4]triazolo[1,5-*a*]pyridin-2-yl]amino}-3-méthoxy-*N*-méthylbenzamide,
le *N*-(2,2-difluoroéthyl)-4-{[6-(4-{[(4-fluorophényl)acétyl]amino}phényl)[1,2,4]triazolo[1,5-*a*]pyridin-2-yl]amino}-3-méthoxybenzamide,
le *N*-[2-(diméthylamino)éthyl]-4-{[6-(4-{[(4-fluorophényl)acétyl]amino}phényl)[1,2,4]triazolo[1,5-*a*]pyridin-2-yl]amino}-3-méthoxy-*N*-méthylbenzamide,
le 4-{[6-(4-{[(4-fluorophényl)acétyl]amino}phényl)[1,2,4]triazolo[1,5-*a*]pyridin-2-yl]amino}-3-méthoxy-*N*-méthyl-*N*-(2,2,2-trifluoroéthyl)benzamide,
le *N*-[2-(diméthylamino)éthyl]-4-{[6-(4-{[(4-fluorophényl)acétyl]amino}phényl)[1,2,4]triazolo[1,5-*a*]pyridin-2-yl]amino}-3-méthoxybenzamide,
le 4-{[6-(4-{[(4-fluorophényl)acétyl]amino}phényl)[1,2,4]triazolo[1,5-*a*]pyridin-2-yl]amino}-*N*-(3-fluoropropyl)-3-méthoxybenzamide,
le *N*-[4-(2-{[5-fluoro-2-méthoxy-4-(méthylsulfonyl)phényl]amino}[1,2,4]triazolo[1,5-*a*]pyridin-6-yl)phényl]-2-(4-fluorophényl)acétamide,
le 2-(4-fluorophényl)-*N*-[4-(2-{[2-méthoxy-4-(méthylsulfinyl)phényl]amino}[1,2,4]triazolo[1,5-*a*]pyridin-6-yl)phényl]acétamide,
le *N*-[4-(2-{[5-fluoro-2-méthoxy-4-(méthylsulfinyl)phényl]amino}[1,2,4]triazolo[1,5-*a*]pyridin-6-yl)phényl]-2-(4-fluorophényl)acétamide,
le *N*-[4-(2-{[4-(*tert*-butylsulfamoyl)-2-méthoxyphényl]amino}[1,2,4]triazolo[1,5-*a*]pyridin-6-yl)phényl]-2-(4-fluorophényl)acétamide,
le 2-(4-fluorophényl)-*N*-[4-(2-{[4-(2-hydroxyprop-2-yl)-2-méthoxyphényl]amino}[1,2,4]triazolo[1,5-*a*]pyridin-6-yl)phényl]acétamide,
le *N*-(4-{2-[(2,4-diméthoxyphényl)amino][1,2,4]triazolo[1,5-*a*]pyridin-6-yl}phényl)-2-(4-fluorophényl)acétamide,
le 3-éthoxy-*N*-éthyl-4-{[6-(4-{[(4-fluorophényl)acétyl]amino}phényl)[1,2,4]triazolo[1,5-*a*]pyridin-2-yl]amino}-*N*-méthylbenzamide,
le *N*-[2-(diméthylamino)éthyl]-3-éthoxy-4-{[6-(4-{[(4-fluorophényl)acétyl]amino}phényl)[1,2,4]triazolo[1,5-*a*]pyridin-2-yl]amino}benzamide,
le 4-{[6-(4-{[(4-fluorophényl)acétyl]amino}phényl)[1,2,4]triazolo[1,5-*a*]pyridin-2-yl]amino}-*N*-[2-(méthylsulfonyl)éthyl]-3-(2,2,2-trifluoroéthoxy)benzamide,
le 4-{[6-(4-{[(4-fluorophényl)acétyl]amino}phényl)[1,2,4]triazolo[1,5-*a*]pyridin-2-yl]amino}-*N*-(1-hydroxy-2-méthylprop-2-yl)-3-(2,2,2-trifluoroéthoxy)benzamide,
le *N*-[2-(diméthylamino)éthyl]-4-{[6-(4-{[(4-fluorophényl)acétyl]amino}phényl)[1,2,4]triazolo[1,5-*a*]pyridin-2-yl]amino}-3-(2,2,2-trifluoroéthoxy)benzamide,
le 2-(4-fluorophényl)-*N*-[4-(2-{[4-(méthylsulfinyl)-2-(2,2,2-trifluoroéthoxy)phényl]amino}[1,2,4]triazolo[1,5-*a*]pyridin-6-yl)phényl]acétamide,
le 4-{[6-(4-{[(4-fluorophényl)acétyl]amino}phényl)[1,2,4]triazolo[1,5-*a*]pyridin-2-yl]amino}-3-(trifluorométhoxy)benzamide,
le *N*-[4-(2-{[2-(difluorométhoxy)-4-(prop-2-ylsulfonyl)phényl]amino}[1,2,4]triazolo[1,5-*a*]pyridin-6-yl)phényl]-2-(4-fluorophényl)acétamide,
le *N-*[4-(2-{[2-(difluorométhoxy)-4-fluorophényl]amino}[1,2,4]triazolo[1,5-*a*]pyridin-6-yl)phényl]-2-(4-fluorophényl)acétamide,
le 4-{[6-(4-{[(4-fluorophényl)acétyl]amino}phényl)[1,2,4]triazolo[1,5-*a*]pyridin-2-yl]amino}-3-méthylbenzamide,
le 4-{[6-(4-{[(4-fluorophényl)acétyl]amino}phényl)[1,2,4]triazolo[1,5-*a*]pyridin-2-yl]amino}-3-méthyl-*N*-(2,2,2-trifluoroéthyl)benzamide,
le *N*-(2-fluoroéthyl)-4-{[6-(4-{[(4-fluorophényl)acétyl]amino}phényl)[1,2,4]triazolo[1,5-*a*]pyridin-2-yl]amino}-3-méthylbenzamide,
le 4-{[6-(4-{[(4-fluorophényl)acétyl]amino}phényl)[1,2,4]triazolo[1,5-*a*]pyridin-2-yl]amino}-*N*,*N*,3-triméthylbenzamide,
le 2-(4-fluorophényl)-*N*-[4-(2-{[2-méthyl-4-(méthylsulfonyl)phényl]amino}[1,2,4]triazolo[1,5-*a*]pyridin-6-yl)phényl]acétamide,
le 2-(4-fluorophényl)-*N*-[4-(2-{[2-méthyl-4-(méthylsulfinyl)phényl]amino}[1,2,4]triazolo[1,5-*a*]pyridin-6-yl)phényl]acétamide,
le 2-(4-fluorophényl)-*N*-{4-[2-({2-méthyl-4-[(méthylsulfonyl)amino]phényl}amino)[1,2,4]triazolo[1,5 -*a*]pyridin-6-yl]phényl}acétamide,
le 2-(4-fluorophényl)-*N*-(4-{2-[(4-méthoxy-2-méthylphényl)amino][1,2,4]triazolo[1,5-*a*]pyridin-6-yl}phényl)acétamide,
le *N*-[4-(2-{[4-(diméthylamino)-2-méthylphényl]amino}[1,2,4]triazolo[1,5-*a*]pyridin-6-yl)phényl]-2-(4-fluorophényl)acétamide,
le *N*-éthyl-5-{[6-(4-{[(4-fluorophényl)acétyl]amino}phényl)[1,2,4]triazolo[1,5-*a*]pyridin-2-yl]amino}-4-méthylpyridine-2-carboxamide,
le 5-{[6-(4-{[(4-fluorophényl)acétyl]amino}phényl)[1,2,4]triazolo[1,5-*a*]pyridin-2-yl]amino}-4-méthyl-*N*-(2,2,2-trifluoroéthyl)pyridine-2-carboxamide,
le *N*-[4-(2-{[2-fluoro-4-(méthylsulfonyl)phényl]amino}[1,2,4]triazolo[1,5-*a*]pyridin-6-yl)phényl]-2-(4-fluorophényl)acétamide,
le 2-(4-fluoro-3-méthylphényl)-*N*-[4-(2-{[2-méthoxy-4-(méthylsulfonyl)phényl]amino}[1,2,4]triazolo[1,5-*a*]pyridin-6-yl)phényl]acétamide,
le 2-(4-chlorophényl)-*N*-[4-(2-{[2-méthoxy-4-(méthylsulfonyl)phényl]amino}[1,2,4]triazolo[1,5-*a*]pyridin-6-yl)phényl]acétamide,
le *N*-[4-(2-{[2-méthoxy-4-(méthylsulfonyl)phényl]amino}[1,2,4]triazolo[1,5-*a*]pyridin-6-yl)phényl]-2-phénylacétamide,
le 4-[(6-{4-[(4-fluorobenzyl)carbamoyl]phényl}[1,2,4]triazolo[1,5-*a*]pyridin-2-yl)amino]-3-méthoxybenzamide,
le 4-[(6-{4-[(4-fluorobenzyl)carbamoyl]phényl}[1,2,4]triazolo[1,5-*a*]pyridin-2-yl)amino]-3-méthoxy-*N*-(2-méthoxyéthyl)benzamide,
le 4-[(6-{4-[(4-fluorobenzyl)carbamoyl]phényl}[1,2,4]triazolo[1,5-*a*]pyridin-2-yl)amino]-*N*-(2-fluoroéthyl)-3-méthoxybenzamide,
le 4-[(6-{4-[(4-fluorobenzyl)carbamoyl]phényl}[1,2,4]triazolo[1,5-*a*]pyridin-2-yl)amino]-3-méthoxy-*N*,*N*-diméthylbenzamide,
le *N*-(2,2-difluoroéthyl)-4-[(6-{4-[(4-fluorobenzyl)carbamoyl]phényl}[1,2,4]triazolo[1,5-*a*]pyridin-2-yl)amino]-3-méthoxybenzamide,
le *N*-[2-(diméthylamino)éthyl]-4-[(6-{4-[(4-fluorobenzyl)carbamoyl]phényl}[1,2,4]triazolo[1,5-*a*]pyridin-2-yl)amino]-3-méthoxybenzamide,
le *N*-[2-(diméthylamino)éthyl]-4-[(6-{4-[(4-fluorobenzyl)carbamoyl]phényl}[1,2,4]triazolo[1,5-*a*]pyridin-2-yl)amino]-3-méthoxy-*N*-méthylbenzamide,
le 4-[(6-{4-[(4-fluorobenzyl)carbamoyl]phényl}[1,2,4]triazolo[1,5-*a*]pyridin-2-yl)amino]-*N*-(3-fluoropropyl)-3-méthoxybenzamide,
le *N*-(4-fluorobenzyl)-4-(2-{[2-méthoxy-4-(méthylsulfinyl)phényl]amino}[1,2,4]triazolo[1,5-*a*]pyridin-6-yl)benzamide,
le 4-(2-{[4-(*tert*-butylsulfamoyl)-2-méthoxyphényl]amino}[1,2,4]triazolo[1,5-*a*]pyridin-6-yl)-*N*-(4-fluorobenzyl)benzamide,
le *N*-(4-fluorobenzyl)-4-(2-{[4-(2-hydroxyprop-2-yl)-2-méthoxyphényl]amino}[1,2,4]triazolo[1,5-*a*]pyridin-6-yl)benzamide,
le *N*-[2-(diméthylamino)éthyl]-3-éthoxy-4-[(6-{4-[(4-fluorobenzyl)carbamoyl]phényl}[1,2,4]triazolo[1,5-*a*]pyridin-2-yl)amino]benzamide,
le 3-éthoxy-*N*-éthyl-4-[(6-{4-[(4-fluorobenzyl)carbamoyl]phényl}[1,2,4]triazolo[1,5-*a*]pyridin-2-yl)amino]-*N*-méthylbenzamide,
le 3-éthoxy-4-[(6-{4-[(4-fluorobenzyl)carbamoyl]phényl}[1,2,4]triazolo[1,5-*a*]pyridin-2-yl)amino]-*N*-(2-fluoroéthyl)benzamide,
le *N*-[2-(diméthylamino)éthyl]-4-[(6-{4-[(4-fluorobenzyl)carbamoyl]phényl}[1,2,4]triazolo[1,5-*a*]pyridin-2-yl)amino]-3-(2,2,2-trifluoroéthoxy)benzamide,
le 4-[(6-{4-[(4-fluorobenzyl)carbamoyl]phényl}[1,2,4]triazolo[1,5-*a*]pyridin-2-yl)amino]-*N*-(2-fluoroéthyl)-3-(2,2,2-trifluoroéthoxy)benzamide,
le 4-[(6-{4-[(4-fluorobenzyl)carbamoyl]phényl}[1,2,4]triazolo[1,5-*a*]pyridin-2-yl)amino]-3-(trifluorométhoxy)benzamide,
le 4-(2-{[2-(difluorométhoxy)-4-(éthylsulfonyl)phényl]amino}[1,2,4]triazolo[1,5-*a*]pyridin-6-yl)-*N*-(4-fluorobenzyl)benzamide,
le 4-(2-{[2-(difluorométhoxy)-4-(prop-2-ylsulfonyl)phényl]amino}[1,2,4]triazolo[1,5-*a*]pyridin-6-yl)-*N*-(4-fluorobenzyl)benzamide,
le 4-(2-{[2-(difluorométhoxy)-4-fluorophényl]amino}[1,2,4]triazolo[1,5-*a*]pyridin-6-yl)-*N*-(4-fluorobenzyl)benzamide,
le *N*-éthyl-4-[(6-{4-[(4-fluorobenzyl)carbamoyl]phényl}[1,2,4]triazolo[1,5-*a*]pyridin-2-yl)amino]-3-méthylbenzamide,
le 4-[(6-{4-[(4-fluorobenzyl)carbamoyl]phényl}[1,2,4]triazolo[1,5-*a*]pyridin-2-yl)amino]-3-méthylbenzamide,
le 4-[(6-{4-[(4-fluorobenzyl)carbamoyl]phényl}[1,2,4]triazolo[1,5-*a*]pyridin-2-yl)amino]-3-méthyl-*N*-(2,2,2-trifluoroéthyl)benzamide,
le 4-[(6-{4-[(4-fluorobenzyl)carbamoyl]phényl}[1,2,4]triazolo[1,5-*a*]pyridin-2-yl)amino]-*N*-(2-fluoroéthyl)-3-méthylbenzamide,
le *N*-(4-fluorobenzyl)-4-(2-{[2-méthyl-4-(méthylsulfonyl)phényl]amino}[1,2,4]triazolo[1,5-*a*]pyridin-6-yl)benzamide,
le *N*-(4-fluorobenzyl)-4-(2-{[2-méthyl-4-(méthylsulfinyl)phényl]amino}[1,2,4]triazolo[1,5-*a*]pyridin-6-yl)benzamide,
le *N*-(4-fluorobenzyl)-4-[2-({2-méthyl-4-[(méthylsulfonyl)amino]phényl}amino)[1,2,4]triazolo[1,5 -*a*]pyridin-6-yl]benzamide,
le *N*-(4-fluorobenzyl)-4-{2-[(4-méthoxy-2-méthylphényl)amino][1,2,4]triazolo[1,5-*a*]pyridin-6-yl}benzamide,
le 4-[(6-{4-[(4-chlorobenzyl)carbamoyl]phényl}[1,2,4]triazolo[1,5-*a*]pyridin-2-yl)amino]-3-méthoxy-*N*-(2,2,2-trifluoroéthyl)benzamide,
le *N*-(4-chlorobenzyl)-4-(2-{[2-méthoxy-4-(méthylsulfonyl)phényl]amino}[1,2,4]triazolo[1,5-*a*]pyridin-6-yl)benzamide,
le *N*-(4-chlorobenzyl)-4-(2-{[2-méthyl-4-(méthylsulfonyl)phényl]amino}[1,2,4]triazolo[1,5-*a*]pyridin-6-yl)benzamide,
le *N*-(4-chlorobenzyl)-4-(2-{[2-méthyl-4-(méthylsulfinyl)phényl]amino}[1,2,4]triazolo[1,5-*a*]pyridin-6-yl)benzamide,
le 4-(2-{[2-méthoxy-4-(méthylsulfonyl)phényl]amino}[1,2,4]triazolo[1,5-*a*]pyridin 6-yl)-*N*-(4-méthylbenzyl)benzamide,
le *N*-(4-méthylbenzyl)-4-(2-{[2-méthyl-4-(méthylsulfonyl)phényl]amino}[1,2,4]triazolo[1,5-*a*]pyridin-6-yl)benzamide,
le *N*-(4-méthylbenzyl)-4-(2-{[2-méthyl-4-(méthylsulfinyl)phényl]amino}[1,2,4]triazolo[1,5-*a*]pyridin-6-yl)benzamide,
le 4-[(6-{4-[(2,4-difluorobenzyl)carbamoyl]phényl}[1,2,4]triazolo[1,5-*a*]pyridin-2-yl)amino]-3-méthoxy-*N*-(2,2,2-trifluoroéthyl)benzamide,
le *N*-(2,4-difluorobenzyl)-2-méthyl-4-(2-{[4-(méthylsulfonyl)-2-(2,2,2-trifluoroéthoxy)phényl]amino}[1,2,4]triazolo[1,5-*a*]pyridin-6-yl)benzamide,
le *N*-(4-fluorobenzyl)-4-(2-{[4-(2-hydroxyprop-2-yl)-2-méthoxyphényl]amino}[1,2,4]triazolo[1,5-*a*]pyridin-6-yl)-2-méthoxybenzamide,
le 2-(4-fluorophényl)-*N*-[4-(2-{[2-méthoxy-4-(*S-*méthylsulfonimidoyl)phényl]amino}[1,2,4]triazolo[1,5-*a*]pyridin-6-yl)phényl]acétamide,
le *N*-(4-fluorobenzyl)-4-(2-{[2-méthoxy-4-(*S-*méthylsulfonimidoyl)phényl]amino[1,2,4]triazolo[1,5-*a*]pyridin-6-yl)benzamide,
le 2-(4-fluorophényl)-*N*-[4-(2-{[4-(hydroxyméthyl)-2-méthoxyphényl]amino}[1,2,4]triazolo[1,5-a]pyridin-6-yl)phényl]acétamide et
le *N*-(4-fluorobenzyl)-4-(2-{[4-(hydroxyméthyl)-2-méthoxyphényl]amino}[1,2,4]triazolo[1,5-a]pyridin-6-yl)benzamide
ou stéréoisomère, tautomère, *N*-oxyde, hydrate, solvate ou sel de celui-ci ou mélange de ceux-ci.

6. Procédé de préparation d'un composé de formule générale (I) selon l'une quelconque des revendications 1 à 5, dans lequel procédé un composé intermédiaire de formule générale (5) : 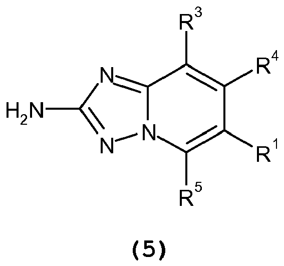 dans laquelle R¹, R³, R⁴ et R⁵ sont tels que définis pour les composés de formule générale (I) dans l'une quelconque des revendications 1 à 5,
est amené à réagir avec un halogénure d'aryle de formule générale (5a) :
R²-Y (5a)
dans laquelle R² est tel que défini pour les composés de formule générale (I) dans l'une quelconque des revendications 1 à 5 et Y représente un groupe partant, tel qu'un atome d'halogène ou un groupe trifluorométhylsulfonyloxy ou nonafluorobutylsulfonyloxy par exemple,
ce qui fournit ainsi un composé de formule générale (I) : 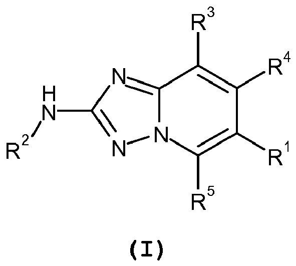 dans laquelle R¹, R², R³, R⁴ et R⁵ sont tels que définis pour les composés de formule générale (I) dans l'une quelconque des revendications 1 à 5.

7. Procédé de préparation d'un composé de formule générale (I) selon l'une quelconque des revendications 1 à 5, dans lequel procédé un composé intermédiaire de formule générale (7) : 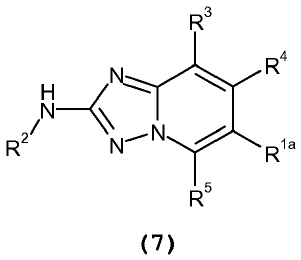 dans laquelle R², R³, R⁴ et R⁵ sont tels que définis pour les composés de formule générale (I) dans l'une quelconque des revendications 1 à 5 et R^{1a} est un groupe aryle auquel un substituant -NH₂ est lié,
est amené à réagir avec un composé de formule générale :
**R^{1b}-X** (7a)
dans laquelle R^{1b} est -C(=O)R⁶, -C(=O)NR⁶R⁷, -S(=O)R⁶, -S(=O)₂R⁶ et X est un groupe fonctionnel approprié par l'intermédiaire duquel le groupe R^{1b} du composé (7a) R^{1b}-X peut être couplé, par l'intermédiaire d'une réaction de couplage, telle qu'une réaction de couplage par formation d'amide par exemple, au substituant -NH₂ lié au groupe aryle R^{1a} du composé (7), ce qui remplace de cette manière ledit X par ledit R^{1a},
ce qui fournit ainsi un composé de formule générale (I) : 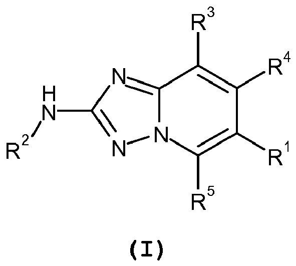 dans laquelle R¹, R², R³, R⁴ et R⁵ sont tels que définis pour les composés de formule générale (I) dans l'une quelconque des revendications 1 à 5.

8. Composé selon l'une quelconque des revendications 1 à 5, ou stéréoisomère, tautomère, N-oxyde, hydrate, solvate ou sel de celui-ci, en particulier un sel pharmaceutiquement acceptable de celui-ci, ou mélange de ceux-ci, destiné à être utilisé dans le traitement ou la prophylaxie d'une maladie.

9. Composé destiné à être utilisé selon la revendication 8, ladite maladie étant une maladie de croissance, prolifération et/ou survie non maîtrisées des cellules, une réponse immunitaire cellulaire inappropriée ou une réponse inflammatoire cellulaire inappropriée, en particulier la croissance, la prolifération et/ou la survie non maîtrisées des cellules, la réponse immunitaire cellulaire inappropriée ou la réponse inflammatoire cellulaire inappropriée étant à médiation par Mps-1, plus particulièrement la maladie de croissance, prolifération et/ou survie non maîtrisées des cellules, la réponse immunitaire cellulaire inappropriée ou la réponse inflammatoire cellulaire inappropriée étant une tumeur hématologique, une tumeur solide et/ou des métastases de celles-ci, par exemple des leucémies et un syndrome myélodysplasique, des lymphomes malins, des tumeurs de la tête et du cou notamment des tumeurs cérébrales et des métastases cérébrales, des tumeurs du thorax notamment des tumeurs du poumon non à petites cellules et à petites cellules, des tumeurs gastro-intestinales, des tumeurs endocrines, des tumeurs mammaires et autres tumeurs gynécologiques, des tumeurs urologiques notamment des tumeurs rénales, de la vessie et de la prostate, des tumeurs de la peau et des sarcomes et/ou des métastases de ceux-ci.

10. Composition pharmaceutique comprenant un composé selon l'une quelconque des revendications 1 à 5, ou un stéréoisomère, un tautomère, un N-oxyde, un hydrate, un solvate ou un sel de celui-ci, en particulier un sel pharmaceutiquement acceptable de celui-ci, ou un mélange de ceux-ci, et un diluant ou véhicule pharmaceutiquement acceptable.

11. Association pharmaceutique comprenant :
- un ou plusieurs composés selon l'une quelconque des revendications 1 à 5, ou un stéréoisomère, un tautomère, un N-oxyde, un hydrate, un solvate ou un sel de ceux-ci, en particulier un sel pharmaceutiquement acceptable de ceux-ci, ou un mélange de ceux-ci ;
et
- un ou plusieurs agents choisis parmi : un taxane, tel que le docétaxel, le paclitaxel ou le taxol ; un épothilone, tel que l'ixabépilone, le patupilone ou le sagopilone ; la mitoxantrone ; la prednisolone ; la dexaméthasone ; l'estramustine ; la vinblastine ; la vincristine ; la doxorubicine ; l'adriamycine ; l'idarubicine ; la daunorubicine ; la bléomycine ; l'étoposide ; le cyclophosphamide ; l'ifosfamide ; la procarbazine ; le melphalan ; le 5-fluorouracile ; la capécitabine ; la fludarabine ; la cytarabine ; l'Ara-C ; la 2-chloro-2'-désoxyadénosine ; la thioguanine ; un anti-androgène, tel que le flutamide, l'acétate de cyprotérone ou le bicalutamide ; le bortézomib ; un dérivé du platine, tel que le cisplatine ou le carboplatine ; le chlorambucil ; le méthotrexate ; et le rituximab.

12. Utilisation d'un composé selon l'une quelconque des revendications 1 à 5, ou d'un stéréoisomère, d'un tautomère, d'un *N*-oxyde, d'un hydrate, d'un solvate ou d'un sel de celui-ci, en particulier d'un sel pharmaceutiquement acceptable de celui-ci, ou d'un mélange de ceux-ci, pour la préparation d'un médicament pour la prophylaxie ou le traitement d'une maladie.

13. Utilisation selon la revendication 12, ladite maladie étant une maladie de croissance, prolifération et/ou survie non maîtrisées des cellules, une réponse immunitaire cellulaire inappropriée ou une réponse inflammatoire cellulaire inappropriée, en particulier la croissance, la prolifération et/ou la survie non maîtrisées des cellules, la réponse immunitaire cellulaire inappropriée ou la réponse inflammatoire cellulaire inappropriée étant à médiation par Mps-1, plus particulièrement la maladie de croissance, prolifération et/ou survie non maîtrisées des cellules, la réponse immunitaire cellulaire inappropriée ou la réponse inflammatoire cellulaire inappropriée étant une tumeur hématologique, une tumeur solide et/ou des métastases de celles-ci, par exemple des leucémies et un syndrome myélodysplasique, des lymphomes malins, des tumeurs de la tête et du cou notamment des tumeurs cérébrales et des métastases cérébrales, des tumeurs du thorax notamment des tumeurs du poumon non à petites cellules et à petites cellules, des tumeurs gastro-intestinales, des tumeurs endocrines, des tumeurs mammaires et autres tumeurs gynécologiques, des tumeurs urologiques notamment des tumeurs rénales, de la vessie et de la prostate, des tumeurs de la peau et des sarcomes et/ou des métastases de ceux-ci.

14. Composé de formule générale (7) : 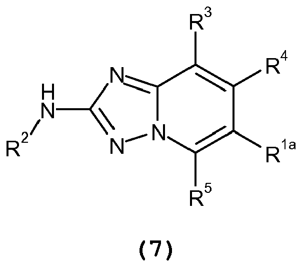 dans laquelle R², R³, R⁴ et R⁵ sont tels que définis pour les composés de formule générale (I) dans l'une quelconque des revendications 1 à 5 et R^{1a} est un groupe aryle auquel un substituant -NH₂ est lié.

15. Utilisation d'un composé de formule générale (5) 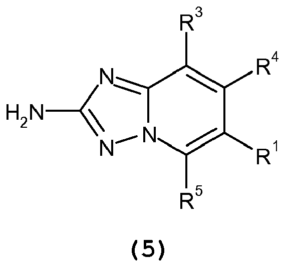 dans laquelle R¹, R³, R⁴ et R⁵ sont tels que définis pour les composés de formule générale (I) dans l'une quelconque des revendications 1 à 5 ;
pour la préparation d'un composé de formule générale (I) selon l'une quelconque des revendications 1 à 5.

16. Utilisation d'un composé de formule générale (7) selon la revendication 14 pour la préparation d'un composé de formule générale (I) selon l'une quelconque des revendications 1 à 5.
